# EUROPEAN PATENT APPLICATION

(11) **EP 4 755 427 A2**
(43) Date of publication of application: **10.06.2026**
(21) Application number: 26174035.1
(22) Date of filing: 25.03.2021
(51) Int. Cl.: A61M 15/00

(54) **MULTI-CARRIER MEDICAMENT DISPENSERS**

(30) Priority: 25.03.2020 US 202062994307 P
(62) Divisional of application: 21719437.2
(71) Applicant: Lupin Inc., Baltimore, Maryland 21202 (US)
(72) Inventor: SHAIKH, Imran, BALTIMORE, 21202 (US); DALVI, Mukul, BALTIMORE, 21202 (US); ZENG, Xian Ming, BALTIMORE, 21202 (US); ALBERG, Cameron, CHICAGO, 60640 (US); MATUSAITIS, Tomas, CHICAGO, 60640 (US)
(74) Representative: Ipsilon

(57) **Abstract**

The invention of the present disclosure relates to a medicament dispenser for dispensing medicament from a plurality of elongate form medicament carriers, the dispenser comprising a dispensing mechanism for dispensing the medicament held within the dispenser, wherein the dispensing mechanism is capable of independently advancing each medicament carrier held within the dispenser, the dispensing mechanism comprising a driver, a carrier engagement for each medicament carrier for individually advancing the medicament dose portions thereof, an advancer for each carrier engagement, a flow director capable of dispensing a medicament dose from a plurality of medicament carriers, and a cover for the medicament access body operating as a component of the dispenser actuation mechanism.

## Description

### FIELD

The present disclosure relates to medicament dispensers for dispensing medicaments.

### BACKGROUND

Medicaments are administered to a patient by inhalation using dry powder dispenser devices. Such devices are often referred to as dry powder inhalers (DPIs) and are used for the treatment and prophylaxis of respiratory diseases, including, but not limited to asthma, chronic obstructive pulmonary disease (COPD), bronchitis, lung infections, interstitial pulmonary fibrosis (IPF), pulmonary arterial hypertension (PAH), cystic fibrosis, Non-CF bronchiectasis, and lung transplant.

Generally described, dry powder inhalers carry one or more medicaments in dose storage areas, e.g., reservoirs such as capsules or blisters packages, and other similar mechanisms capable of holding or maintaining medicament until the time required for dispensation. The medicament carrier can include a blister strip containing several discrete doses of powdered medicament. Such dry powder dispensing (e.g., inhaler) devices can contain a mechanism of accessing these doses, either through the incorporation of a piercing means or a means of peeling a lid or cover sheet away from a base sheet. The powdered medicament can then be accessed by the device and inhaled via the device by the user. Such a mechanism may also be used for dispensing medicament in tablets, capsules, pellets or pucks form wherein peeling away the lid sheet from the base sheet reveals a particular medicament form such as a tablet for removal and subsequent consumption, particularly for oral administration.

U.S. Pat. No. 5,873,360 discloses an inhalation device, wherein a cover is capable of being pivoted between an open position, in which the mouthpiece is exposed, and a closed position in which it is not. In operation, the user moves the cover to its open position and then presses on a finger tab associated with a lever to cause the finger tab to move ("slide") as the lever pivots. The lever associated with the finger tab actuates a gear mechanism as the finger tab is slid from end to end. Accordingly, a blister strip contained within the inhalation device is advanced; one blister is opened, and one dose of the dry powder medicament contained in the properly aligned blister cavity becomes available for inhalation by the user. The cover is held stationary as the body is rotated counter-clockwise. As the cover of the device is not directly connected to the lever mechanism, the user is required first to move the cover and then move the finger tab and associated lever, and thus multiple steps are required to operate the device.

U.S. Pat. No. 8,161,968 discloses a medicament dispenser capable of containing multiple elongate form medicament carriers, wherein the medicament dispenser comprises a movable cover for the outlet of blister strips which is movable from an at-rest position, in which the cover covers the outlet, to a primed position, and further to an actuated position to uncover the outlet. The outlet cover cooperates with the dispensing mechanism such that movement of the outlet cover from the primed position to the actuated position actuates one or more components of the dispensing mechanism, and movement of the cover from the at-rest position to the primed position does not actuate any one or more components of the dispensing mechanism.

U.S. Pat. No. 8,746,242 discloses a medicament dispenser for use with at least one medicament carrier carrying multiple distinct medicament portions. This art discloses a medicament dispenser comprising (a) a dispensing mechanism actuatable for dispensing the distinct medicament portions carried by at least one of the medicament carriers; (b) a mouthpiece; and (c) a cover for the mouthpiece. The mouthpiece cover is movably mounted to the dispenser for sequential movement from a first position, in which the mouthpiece is covered, to a second position, in which the mouthpiece is partially covered and partially uncovered, to a third position in which the mouthpiece is uncovered; wherein further the cover is adapted to couple with the dispensing mechanism such that movement of the cover from the first position to the second position does not result in actuation of the dispensing mechanism, but any further movement of the cover from the second position to the third position results in actuation of the dispensing mechanism.

U.S. Pat. No. 8,511,304 discloses an inhalation device comprising two elongate form medicament carriers, the medicament dose portions of one blister strip containing a medicament active, or a mixture of medicament actives, which is different from that in the medicament dose portions of the other blister strip, an actuating member for actuating the inhalation device, a dispensing mechanism which is adapted to act upon each blister strip together to dispense a single distinct medicament dose portion carried by each medicament carrier, the dispensing mechanism comprising a) at least one receiving station for receiving the blister strips; b) a release for releasing in combination a distinct medicament dose portion from each of the blister strips upon receipt of an actuation by a receiving mechanism; c) an indexer for each medicament carrier for individually indexing the distinct medicament dose portions thereof, the indexers being mutually coupled; and d) an outlet, positioned to be in communication with the combination of distinct medicament dose portions releasable by the release and which a user is able to inhale for simultaneous inhalation of the distinct medicament dose portions releasable by said release.

### SUMMARY

The inventors of the medicament dispensers described herein have recognized a need in the art for a medicament dispenser, which, in operation, comprises a minimum number of steps to operate. A combination inhaled medicament product wherein medicament from one or more medicament carriers within the dispenser can be inhaled in response to a single operation of the dispenser is simultaneously desirable.

The invention described herein provides simple and cost-effective medicament dispensers capable of being operated with a single hand by a user and having fewer moving components than the devices of the prior art and that further allows the advancement of each medicament carrier within the dispenser independently of each other, releasing the medicament portions successively. The dispensing mechanism of such devices typically includes, essentially includes, or only includes three steps to operate the medicament dispenser: open, inhale and close.

According to one aspect, which can be combined with any other aspect, embodiment, or combination(s) of aspects and/or embodiments of the present disclosure, is the invention provides a medicament dispenser for dispensing medicament from at least one single elongate form medicament carrier, such an elongate form medicament carrier commonly existing in the art as blister pack packaging or strip packaging (but which could incorporate any multiple dose medicament carrier wherein the carrier houses multiple, distinctly separate medicament doses in a form suited for being housed within the dispenser), said dispenser comprising: a) a dispensing mechanism comprising: i) a driver, ii) a carrier engagement, iii) an advancer, iv) a flow director, v) a first waste collector for collecting a first waste stream, vi) a second waste collector for collecting a second waste stream; b) a medicament access body; and c) a cover for the medicament access body.

Another aspect of the present invention, which can be combined with any other aspect, embodiment, or combination(s) of aspects and/or embodiments of the present disclosure, is a medicament dispenser for dispensing medicament from plural (e.g., two, three, or four) elongate form medicament carriers, each carrier having multiple, distinct dose portions, said dispenser comprising: a) a dispensing mechanism comprising: i) a driver, ii) a carrier engagement for each medicament carrier for individually advancing the distinct medicament dose portions thereof, iii) an advancer for each carrier engagement for rotating thereof, iv) a flow director for dispensing the medicament, v) a first waste collector for collecting a first waste stream vi) a second waste collector for collecting a second waste stream; b) a medicament access body; and c) a cover for the medicament access body.

Another aspect of the disclosure, which can be combined with any other aspect, embodiment, or combination(s) of aspects and/or embodiments of the present disclosure, relates to a medicament dispenser for dispensing medicament from plural elongate form medicament carriers, each carrier having multiple, distinct dose portions, said dispenser comprising: a) a dispensing mechanism comprising: i) a driver, ii) a carrier engagement for each medicament carrier for individually advancing the distinct medicament dose portions thereof, iii) an advancer for each carrier engagement for rotating thereof, iv) a flow director for each medicament carrier for dispensing the medicament, the medicament dispenser having a plurality of medicament carriers held therein, v) a first waste collector capable of collecting a first waste stream, and vi) a second waste collector capable of collecting a second waste stream; and b) a medicament access body; and c) a cover for the medicament access body.

According to one aspect, which can be combined with any other aspect, embodiment, or combination(s) of aspects and/or embodiments of the present disclosure, the invention relates to a waste collector, such a waste collector capable of being utilized within a medicament dispenser described herein and capable of collecting a waste stream, the waste collector comprising: a) a tensioning mechanism comprising: i) a base and, ii) ratchet teeth; and b) a waste capture component comprising: i) waste collection surface for receipt of a sheet of said medicament carrier, ii) inner teeth; and iii) outer teeth.

According to one aspect, which can be combined with any other aspect, embodiment, or combination(s) of aspects and/or embodiments of the disclosure provided herein, the invention relates to a flow director for use in a medicament dispenser. According to some embodiments, which can be combined with any other aspect, embodiment, or combination(s) of aspects and/or embodiments, the flow director can be an element of the dispensing mechanism of a medicament dispenser and can provide for the delivery of medicament in powder form from an open or otherwise available dose storage area, e.g., a blister pocket, of a medicament carrier having a series of dose storage areas, or from a similar or equivalent open individual compartment or area holding medicament in a similar or equivalent medicament carrier which, unless being made available for administration, is otherwise closed. In one aspect, which can be combined with any other aspect, embodiment, or combination(s) of aspects and/or embodiments the flow director can comprise an inlet compartment, an outlet compartment, and a number of grids or similar structures, such as a pair of grids (e.g., a pair of non-contacting grids), for directing airflow from the inlet compartment to the outlet compartment. In some aspects, which can be combined with any other aspect, embodiment, or combination(s) of aspects and/or embodiments, when in use, the grids are associated with an open or otherwise available dose storage area of the medicament carrier, such that when the open or otherwise available dose storage area of the medicament carrier is positioned adjacent to the grids, the airflow is directed from the inlet compartment to the outlet compartment via the open dose storage area to entrain the medicament powder within the airflow, enabling transport of the medicament in the airflow from the inlet compartment to the outlet compartment.

Another aspect of the disclosure, which can be combined with any other aspect, embodiment, or combination of aspects and embodiments described herein, relates to a flow director for use in a medicament dispenser for the delivery of medicament powder from an open or otherwise available dose storage area of each of the medicament carrier, the flow director comprising a pair of inlet compartments, a pair of outlet compartments, and a plurality of (e.g., at least two sets of two) grids for directing airflow from the inlet compartments to the outlet compartments. In some aspects, which can be combined with any other aspect, embodiment, or combination(s) of aspects and/or embodiments, a plurality of grids or a plurality of grid pairs, when in use, are associated with an open or otherwise available dose storage area of the each of the medicament carriers acted upon by the device; such that when the open or otherwise available dose storage area of each of the medicament carriers is positioned adjacent to each of the multiple grids, the airflow is directed from the inlet compartments to the outlet compartments via the open dose storage area of the medicament carrier to entrain the medicament powders and enable transport of the medicament powders in the airflow from the inlet compartments to the outlet compartments.

One aspect of the disclosure, which can be combined with any other aspect or embodiment of the disclosure, is a medicament dispenser for dispensing medicament from a plurality of elongate form medicament carriers held therein, each carrier comprising multiple dose portions of one or more medicaments, said dispenser comprising:
a. A dispensing mechanism for dispensing the medicament held within the dispenser comprising:
   i. a driver,
   ii. a carrier engagement for each medicament carrier for individually advancing the medicament dose portions thereof,
   iii. an advancer for each carrier engagement located coaxially with the carrier engagement for rotating thereof,
   iv. a flow director; and
b. a medicament access body; and
c. a cover for the medicament access body,
wherein the medicament access body cover is engaged with the driver and wherein sufficient movement of the medicament access body cover results in movement of the driver, the driver being movingly engaged, either directly or indirectly, with the advancer(s) such that sufficient movement of the driver results in movement of the advancers, each advancer being engaged, either directly or indirectly, with a carrier engagement such that sufficient movement of the advancers results in movement of the carrier engagements, and further wherein movement of each carrier engagement results in advancement of a medicament carrier to a position wherein a medicament dose portion of the medicament carrier, when present, is in sufficient alignment with the flow director such that airflows directed by the flow director entrain at least one medicament dose portion extracted from a dose storage area, and further wherein the airflows containing the entrained medicament(s) are separated and separately maintained until the medicament exits the dispenser.

In one aspect of the disclosure, the invention is a medicament dispenser for dispensing medicament from a plurality of elongate form medicament carriers held therein, each carrier comprising multiple dose portions of one or more medicaments, said dispenser comprising:
a. A dispensing mechanism for dispensing the medicament held within the dispenser comprising:
   i. a driver,
   ii. a carrier engagement for each medicament carrier for individually advancing the medicament dose portions thereof,
   iii. an advancer for each carrier engagement located coaxially or non-coaxially with the carrier engagement for rotating thereof,
   iv. a flow director for directing airflow within the dispenser,
   v. at least one first waste collector, and
   vi. at least one second waste collector; and
b. a medicament access body; and
c. a cover for the medicament access body,
wherein the medicament access body cover is engaged with the driver and wherein sufficient movement of the medicament access body cover results in movement of the driver, the driver being movingly engaged, either directly or indirectly, with the advancers such that sufficient movement of the driver results in movement of the advancers, at least one advancer being movingly engaged, either directly or indirectly, with a first waste collector and each advancer being engaged, either directly or indirectly, with a carrier engagement such that sufficient movement of the advancers results in movement of at least one first waste collector and in movement of the carrier engagements, movement of at least one carrier engagement resulting in movement of at least one second waste collector, the movement of each carrier engagement resulting in advancement of a medicament carrier to a position wherein a medicament dose portion of the medicament carrier, when present, is in sufficient alignment with the flow director such that separate airflows directed by the flow director entrain at least one medicament dose portion extracted from a present dose storage area, and further wherein the separate airflows containing the entrained medicament are maintained until the medicament exits the dispenser. In certain embodiments, the airflows each entraining their respective medicament exit the dispenser as a single airflow.

In one aspect of the disclosure herein, the invention relates to a medicament dispenser for dispensing medicament from a plurality of elongate form medicament carriers held therein, each carrier comprising multiple dose portions of one or more medicaments, said dispenser comprising:
a. a dispensing mechanism for dispensing the medicament held within the dispenser comprising:
   i. a driver that movingly engages, either directly or indirectly, with a plurality of advancers,
   ii. an advancer for each carrier engagement for advancing the carrier engagement wherein at least one advancer is directly movingly engaged with the driver and wherein at least a second advancer is acted upon indirectly by movement of the driver via an interim component; or, alternatively, an advancer for each carrier engagement for advancing the carrier engagement wherein each of a plurality of advancers is directly movingly engaged with the driver;
   iii. a carrier engagement for each medicament carrier for individually advancing the medicament dose portions thereof, wherein at least one carrier engagement is movingly engaged with an advancer; or, alternatively, wherein each of a plurality of carrier engagements is movingly engaged with an advancer; and
   iv. each advancer is positioned coaxially with the carrier engagement for rotating thereof, or, alternatively, at least one advancer is positioned coaxially with a carrier engagement for rotating thereof and at least one advancer is positioned non-coaxially with a carrier engagement for rotating thereof; and
      wherein sufficient movement of the driver results in direct movement of at least a first advancer, movement of each advancer resulting in movement of each respective carrier engagement, the movement of each carrier engagement resulting in the advancement of a medicament carrier;
   v. a flow director for receiving and directing airflow,
   wherein, upon dispenser use, a separate airflow entrains each of the at least one medicament dose portions available within a dose storage area when present of at least one medicament carrier brought into position by a carrier engagement, and further wherein the separate airflows containing the entrained medicament(s) are maintained until the medicament exits the dispenser;
   i. at least one waste collector for collecting medicament carrier material after at least one dose of medicament contained therein has been removed; and
b. a medicament access body for generating an airflow via user inhalation and for receipt of the medicament by the user as it exits the dispenser; and
c. a cover for the medicament access body engaged with the driver such that sufficient movement of the medicament access body cover results in movement of the driver.

In some aspects of the above described embodiment, the separate airflows containing the entrained medicament(s) exit the dispenser as a single airflow.

In one aspect of the present invention, the disclosure relates to a medicament dispenser for dispensing one or more medicaments from plural elongate form medicament carriers, each carrier comprising multiple dose portions, said dispenser comprising:
a. a dispensing mechanism comprising:
   i. a driver,
   ii. a carrier engagement for each medicament carrier for individually advancing the medicament dose portions thereof,
   iii. an advancer for each carrier engagement, at least one such advancer located coaxially with the carrier engagement for rotating thereof,
   iv. a flow director wherein separate airflows entrain medicament dose portions extracted from a dose storage area when present for administration, of a medicament carrier and further wherein the separate airflows containing the entrained medicament are maintained until the medicament exits the dispenser,
   v. a first waste collector; and
   vi. a second waste collector, and
b. a medicament access body; and
c. a cover for medicament access body.

In one aspect of the present disclosure, the invention relates to a medicament dispenser for dispensing medicament from a plurality of elongate form medicament carriers held therein, each carrier comprising multiple dose portions of one or more medicaments, said dispenser comprising a dispensing mechanism for dispensing the medicament held within the dispenser, further an advancer for each medicament carrier held therein, each advancer:
a. being engaged, either directly or indirectly with an actuator, for actuating the dispensation of medicament from the dispenser;
   being engaged with a carrier engagement for each medicament carrier for individually advancing the medicament dose portions thereof, such an engagement being either direct or indirect, wherein within the same dispenser one engagement may be direct, one may be indirect, or also or alternatively both may be direct; and
wherein at least one advancer is positioned coaxially with a carrier engagement.

In one aspect of the present disclosure, the invention relates to a medicament dispenser for dispensing medicament from a plurality of elongate form medicament carriers held therein, each carrier comprising multiple dose portions of one or more medicaments, said dispenser comprising a dispensing mechanism for dispensing the medicament held within the dispenser, further comprising at least one waste collector for collecting a portion of a medicament carrier having had a dose of medicament held therein dispensed, each waste collector:
a. comprising a component capable of creating tension on the waste element being collected by the waste collector;
b. comprising a component capable of maintaining tension on the waste element being collected by the waste collector;
c. comprising a component capable of collecting the waste element being collected by the waste collector;
wherein the component (a) and component (b) are positioned within the component (c) and wherein the component (a), component (b), and component (c) share a common axis of rotation.

In one aspect of the present disclosure, the invention relates to a medicament dispenser for dispensing medicament from a plurality of elongate form medicament carriers held therein, each carrier comprising multiple dose portions of one or more medicaments, said dispenser comprising a dispensing mechanism for dispensing the medicament held within the dispenser, further comprising a flow director, said flow director:
a. receiving and separating an airflow into two or more separate airflows each within a separate inlet compartment;
b. directing each of the separated airflows of (a) within the separated inlet compartments such that each separate airflow entrains the medicament held within the dose storage areas, when present, of separate medicament carriers; and
c. maintaining the separation of each airflow comprising entrained medicament from separate dose storage areas until each airflow exits the dispenser.

In one aspect of the present disclosure, the invention relates to a medicament dispenser for dispensing medicament from a plurality of elongate form medicament carriers held therein, each carrier comprising multiple dose portions of one or more medicaments, said dispenser comprising a dispensing mechanism for dispensing the medicament held within the dispenser, further comprising a flow director, said flow director:
a. receiving and separating an airflow into two or more separate airflows each within a separate inlet compartment;
b. directing each of the separated airflows of (a) within the separated inlet compartments such that each separate airflow entrains the medicament held within the dose storage areas, when present, of separate medicament carriers; and
c. providing a single exit for the combined airflows to exit the dispenser.

In one aspect of the present disclosure, the invention relates to a medicament dispenser capable of independently advancing a plurality of medicament carriers at varying rates and/or at varying times comprising i) a driver for actuating the dispensing mechanism, ii) a carrier engagement for each medicament carrier for individually advancing the distinct medicament dose portions thereof, iii) an advancer for each carrier engagement, iv) a flow director wherein separate airflows entrain medicament extracted from separate medicament, v) at least one first waste collector for collecting a first waste stream, vi) at least one second waste collector for collecting at least one second waste stream; b) a medicament access body for user access to the medicaments held within the dispenser; and c) a cover for the medicament access body to protect the medicament access body and which is engaged with the driver such that sufficient movement of the medicament access body results in movement of the driver.

According to one aspect of the disclosure, the invention relates to the method of treating a condition treatable by a medicament administered via inhalation, the method comprising administering one or more medicaments via a medicament dispenser, the administration comprising:
a. loading a medicament dispenser configured to receive a plurality of elongate form medicament carriers and to dispense medicament therefrom with a plurality of elongate form medicament carriers comprising a plurality of discrete medicament doses in powder form;
b. actuating the medicament dispenser in a manner such that prior to any advancement of any medicament carrier upon actuation, tension is applied to at least a portion of each elongate form, typically a separable portion, typically corresponding to one part of the elongate form that corresponds to one of the waste streams formed by the elongate form;
c. advancing the plurality of medicament carriers within the dispenser independently from one another to a common opening location in the dispenser such that a medicament dose within a dose storage location, if present, of each medicament carrier becomes available for administration; and
d. the user inhaling the one or more medicament doses available for administration via a medicament access body, the airstream generated by the inhalation separately entraining the medicament of each available medicament dose and either being maintained separately from one another until the airstream exits the dispenser and reaches the user via the medicament access body, or, alternatively, being combined such that a single airstream entraining all medicament being dispensed exits the dispenser and reaches the user via the medicament access body.

In one further aspect of the disclosure, the invention relates to a method of administering a plurality of medicaments by inhalation to an individual from a medicament dispenser containing the medicaments and comprising an orifice for delivering the medicaments to a user, the plurality of medicaments being maintained in separate airstreams prior to reaching the orifice and the user, the method comprising administering the plurality of medicaments to the individual via the dispenser having any combination of the aspects or embodiments described above and herein.

According to one independent embodiment of the present invention, the invention described herein provides a collection system for capturing and storing an elongate media, which system comprises (a) a tensioning engagement component for applying an initial, engaging tension on an end of the media (e.g., a portion of the media that has been subjected to processing), (b) a tension maintenance component or means for maintaining tension on the media, and (c) a collection component for collecting the media. In some aspects, the component capable of creating tension on the elongate media and the component capable of maintaining tension on the elongate media are positioned within the component capable of collecting the elongate media. In some aspects, all three components share a common axis of rotation. In one aspect, the tensioning engagement component comprises a mechanical attachment component such as a slot, a clip, a clamp, a protrusion to engage the media or to engage a component of the media such as a hole, loop, or indentation in the media or alternatively a hole or indentation to engage a protrusion in the media. In some aspects, the tensioning engagement component is a post. In some aspects, the post is capable of capturing an end of the media via a loop in the media which can be positioned about the post so as to secure the end of the media to the collection system. In some embodiments the collection system also or alternatively comprises a tensioning maintenance component. In some aspects, the tensioning maintenance component can be a guide, a clamp, or a surface over which the media is stretched or against which the media is positioned during capture and storage of an elongate media. In some aspects, the component comprises a curved wall or a curved panel. In some aspects, the curved wall has a surface which increases the ability of the curved wall to engage with the media. In some aspects, such a surface is a sticky surface, such as a surface having an adhesive additive. In some aspects, such a surface is a rubber surface. In some aspects, the surface is a serrated surface. In some aspects, the collection system also or alternatively comprises a collection component for collecting the media. In some aspects, the collection component comprises a surface about which an elongate media is wound or wrapped as the collection component moves, e.g., rotates. In some aspects, the collection component can comprise a media access point, e.g., an opening through which the elongate media can pass from one side of the collection component to the other (e.g., a slit, slot, or hole). In certain embodiments, the collection component is a curved wall surface, optionally wherein the tensioning engagement component and the tension maintenance component reside within the area defined, in part, by the curved wall of the collection component. In some aspects, the media access point is a slit or slot or hole in the curved surface. In some embodiments, the collection component rotates and media is collected along a surface, e.g., the outer surface, of the collection component as the collection component moves (e.g., rotates), while the tensioning maintenance component maintains tension on the elongate media. According to certain aspects of the present invention, the collection system is capable of collecting a waste stream, such as a waste stream generated within a medicament dispenser as an elongate medicament media is processed by the dispenser.

Any of the preceding embodiments and aspects can be combined with any other suitable aspect or embodiment described above and may incorporate any of the corresponding features and characteristics described below in the Detailed Description portion of this disclosure.

### DETAILED DESCRIPTION

According to certain embodiments which can be combined with any other aspect, embodiment, or combination(s) of aspects and/or embodiments described herein, the invention described herein provides a simple and cost-effective medicament dispenser having fewer moving components than the devices of the prior art and which is capable of being operated using a single hand of a user. In one aspect, the invention provides a medicament dispenser that allows for the advancement of each medicament carrier held within the dispenser independently of any other medicament carrier held within the dispenser. In one aspect, any two or more carrier engagement components (or carrier engagements) that engage the medicament carrier are capable of advancing their individually associated medicament carrier at the same time, e.g., two or more carrier engagements are in motion at the same time. In another aspect, the carrier engagements are also or alternatively capable of advancing their individually associated medicament carriers sequentially, e.g., successively, such that one advances first followed by a second (or more) in sequence, and in some embodiments the manner of such engagement is such that there is no substantial overlap or no detectable overlap in movement of at least some, most, or all of the carrier engagements. In another aspect, the carrier engagements are also capable of advancing their individually associated carrier in such a manner that at least one carrier advances first followed by advancement of second after completing all discrete doses of first carrier. In certain aspects, the medicament dispenser is capable of releasing the medicament portions of medicament held within each medicament carrier substantially simultaneously with that from any other one or more medicament carriers (if a plurality of medicament carriers are present). In certain other aspects, the medicament dispenser is capable of releasing the medicament portions of the medicament held within each medicament carrier in sequence, or successively, such that one is released prior to that from any other one or more medicament carriers (if a plurality of medicament carriers are present). In certain aspects, regardless of whether any two or more carrier engagements are in motion at the same time or whether any two or more carrier engagements are in motion in sequence, e.g., one finishing its motion prior to the next engagement starting its motion, and further regardless of whether any two or more medicament portions are released or made available for administration at substantially the same time or in sequence, e.g., one medicament carrier is opened or a dose is made available from that carrier prior to a second or more medicament carriers being opened or a dose from such a carrier is made available from that carrier, the doses are available for administration to the user in a single administration, that is, in a single inhalation by the user. In certain aspects, the advancement of medicament carriers allows dosing to be administered successively to accomplish a sequence of administrations. In certain aspects, the advancement of medicament carriers takes place in such a manner that only one carrier engagements is in motion at a time. In certain aspects, the advancement of medicament carriers takes place in such a manner that only one carrier engagements is in motion at a time and wherein the second medicament carrier is not in motion or substantially at stationary position.

As used herein, the term substantially simultaneously is intended to mean at essentially the same time, such as no significant or intended difference in time (e.g., within about 5 seconds or less, about 3 seconds or less, about 2 seconds or less, or about 1 second or less, such as within about 0.1-5 seconds, about 0.1-3 seconds, or about 0.1-2 seconds).

As used herein, a sequence of administrations is intended to describe the ability to use the dispenser repeatedly to administer a series of doses of medicament held therein, to be able to deliver an, e.g., once-per-day, twice-per-day, three-times-per-day, or more frequent dose administration of one or more medicaments held therein for about three or more days, about four or more days, about five or more days, about six or more days, at least about one week, at least about two weeks, at least about three weeks, at least about four weeks, at least about six weeks, at least about eight weeks or more, at least about two months, at least about three months, at least about four months, at least about six months or longer.

The devices provided herein typically will require relatively few components as compared to those systems previously known in the art (e.g., they may require 20 separate components or less, 15 components or less, 12 components, or even less than ten components that are involved in the functioning of the dispensing mechanism of the dispenser). Each such component can have features and elements which aid in its functionality and allow certain components to interface, engage, or otherwise interact with other components of the dispenser.

In some aspects, some or all components of the dispensing mechanism are common for each of the medicament carriers present within the dispenser; that is, medicament carriers share the functionality of some or all components of the dispensing mechanism within the medicament dispenser. In some aspects, the use of two or more identical or substantially similar components to provide similar functions (e.g., gears, tensioning mechanisms, and springs, such as are described herein) can help to reduce the number of different individual components required for successful dispenser operation.

In some aspects, a common or shared component can be engaged, coupled, or otherwise interfacing with, in common embodiments in a movably engageable fashion, with another one or more common components of the dispenser such that movement of the common component results in movement of multiple components. Such engagement, coupling, or otherwise interfacing between at least two components with a common component can be achieved by any suitable fashion including mechanical interfaces (e.g., the interaction of gears, e.g., toothed-wheels present on each component) or via the use of coupling arms, rods, pins, or the like). Alternatively, multiple components can interface electronically, such as by the use of electromechanical coupling mechanisms. One advantage of such coupled engagement can be that the indexing/advancement of each medicament carrier may be achieved in a coupled fashion.

In some aspects, some or all components of the dispensing mechanism are present individually for each medicament carrier present within the dispenser. That is, the number of each such component is determined by the number of medicament carriers present such that each medicament carrier is capable of being advanced independently and separately from any other medicament carrier present within the dispenser. The use of such separate assemblies for handling different medicament carriers can be preferable in scenarios wherein the medicament within one carrier comprises an administration schedule that is different from that of the medicament within another one or more carriers within the dispenser.

According to common embodiments, one aspect, which can be combined with any other aspect, embodiment, or combination(s) of aspects and/or embodiments of the present invention is embodied in a medicament dispenser for dispensing medicament from a plurality of (e.g., two, three, or four) elongate form medicament carriers, each such carrier having multiple, distinct dose portions, the medicament dispenser (sometimes referred to as "the dispenser") comprising:
a) a dispensing mechanism comprising:
   i) a driver;
   ii) a carrier engagement for each medicament carrier for individually advancing the distinct medicament dose portions thereof;
   iii) an advancer for each carrier engagement for rotating thereof;
   iv) a flow director for dispensing the medicament;
   v) a waste collector for collecting a first waste stream; and
   vi) a waste collector for collecting a second waste stream; and further comprising
b) a medicament access body; and
c) a cover for the medicament access body.

According to certain aspects, which can be combined with any other aspect, embodiment, or combination(s) of aspects and/or embodiments, the medicament dispenser can be hand-held. In some embodiments which can be combined with any other aspect, embodiment, or combination(s) of aspects and/or embodiments which can be combined with any other aspect, embodiment, or combination(s) of aspects and/or embodiments, the medicament dispenser can be operated by a single either right or left hand of the user. In some aspects, which can be combined with any other aspect, embodiment, or combination(s) of aspects and/or embodiments, the medicament dispenser can be held and operated using two hands.

According to certain aspects, which can be combined with any other aspect, embodiment, or combination(s) of aspects and/or embodiments, the medicament dispenser can be capable of dispensing medicament in powder form. In some aspects, which can be combined with any other aspect, embodiment, or combination(s) of aspects and/or embodiments, the medicament dispenser can be capable of providing a medicament in powder form via inhalation. Such inhalation can be, in some aspects, which can be combined with any other aspect, embodiment, or combination(s) of aspects and/or embodiments, via oral inhalation. In certain embodiments that can be combined with any other aspect, embodiment, or combination(s) of aspects and/or embodiments, such inhalation can be via nasal inhalation. In some aspects, which can be combined with any other aspect, embodiment, or combination(s) of aspects and/or embodiments, the medicament dispenser is an inhalation device. According to particular embodiments which can be combined with any other aspect, embodiment, or combination(s) of aspects and/or embodiments, the medicament dispenser is a dry powder inhaler (DPI).

In some aspects, which can be combined with any other aspect, embodiment, or combination(s) of aspects and/or embodiments, the medicament dispenser described herein can comprise a shape providing for optimal functionality, the convenience of use, comfort of use, or any combination thereof, by the user. According to certain embodiments which can be combined with any other aspect, embodiment, or combination(s) of aspects and/or embodiments, the medicament dispenser can comprise any shape capable of supporting optimal performance characteristics, ease of use, user convenience, or any combination thereof, such as but not limited to comprising a circular form, a non-circular form, a squircular form, an elongate form, an elliptical form, an ovular form, an oblong form, a triangular form, a quadrilateral form, a pentagonal form, or a hexagonal form, including, but not limited to a square form, a rectangular form, a trapezium form, an hourglass form, or other shapes and forms supportive of and/or not interfering with operation of the dispenser and such that the shape fits within a single hand of an average user. As used here, an "average user" would be a human user of at least about ten years old, such as at least about 12 years old, at least about 15 years old, at least about 18 years old, or at least about 21 years old.

In certain embodiments of the present invention, which can be combined with any other aspect, embodiment, or combination(s) of aspects and/or embodiments, the medicament dispenser can comprise one or more flat surfaces. In some aspects, which can be combined with any other aspect, embodiment, or combination(s) of aspects and/or embodiments, one or more of any flat surfaces present can be a resting surface. In some aspects, which can be combined with any other aspect, embodiment, or combination(s) of aspects and/or embodiments, the resting surface can be a surface which is capable of maintaining the device in a preferred stored position (e.g., in an upright or stable position) while at rest on a flat surface such as a table.

According to some aspects of the present invention, which can be combined with any other aspect, embodiment, or combination(s) of aspects and/or embodiments, the medicament dispenser can comprise at least a portion of its outer form which is shaped for ease of grip by the user. In certain embodiments, which can be combined with any other aspect, embodiment, or combination(s) of aspects and/or embodiments, one or more shape elements or components designed to facilitate a stable grip by the user such that the user can easily hold and simultaneously operate the dispenser are incorporated. In certain embodiments that can be combined with any other aspect, embodiment, or combination(s) of aspects and/or embodiments, such elements can provide a comfortable fit for the user when holding the device. In some aspects, which can be combined with any other aspect, embodiment, or combination(s) of aspects and/or embodiments such an element can include but may not be limited to one or more curved elements such as or to fit within the curve of a human palm or to partially nest one or more human fingers. Such elements can be bulges, indentations, or curves, variations in thickness and/or thinness, variations in width and or height, or any other similar aesthetic and operationally advantageous design elements which aid in the comfort and stability of the device when held in the hand of a user.

In general, the medicament dispensers of the invention can be of any suitable size and include components of any suitable size therein. In some aspects, which can be combined with any other aspect, embodiment, or combination(s) of aspects and/or embodiments, the medicament dispenser described herein is of a size providing for optimal functionality and ease and/or the convenience of use by the user. Such factors which may contribute to the optimal size of a dispenser in any particular context can include, but may not be limited to the size of the components housed within the medicament dispenser, the operational space required by such components, or characteristics dictated by user preference, such as the ability and convenience to carry the device in a clothing pocket or within a personal carrying device such as a purse, backpack, or the like. In some aspects, which can be combined with any other aspect, embodiment, or combination(s) of aspects and/or embodiments, the device has no dimension in any one direction having a length of more than 90 mm, such as for example no more no more than about 89 mm, no more than about 88 mm, or no more than about 87 mm. In some aspects, the device has a height of between about 80 - about 90 mm, such as for example between about 82 - about 89 mm, between about 84 - about 88 mm, or between about 86 - about 88 mm, such as for example about 87 mm. In some aspects, the device also or alternatively has a width of no more than about 80 mm, such as no more than about 80 mm, no more than about 78 mm, no more than about 76 mm, no more than about 74 mm, or no more than about 72 mm, such as for example having a width of about 70 mm, about 68 mm, about 66 mm, about 64 mm, or about 62 mm, such as about 60 mm. In some aspects the device has a width of between about 65 - 75 mm, such as between about 65 - about 75 mm, between about 66 - about 74 mm, between about 67 - about 73 mm, between about 68 - about 72 mm, between about 69 - about 71 mm, or about 70 mm. In some aspects, the device also or alternatively has a depth of no more than about 40 mm, such as no more than about 40 mm, no more than about 38 mm, no more than about 36 mm, no more than about 34 mm, no more than about 32 mm, such as having a width of about 30 mm, about 28 mm, about 26 mm, about 24 mm, about 22 mm, or about 20 mm, In some aspects, the device has a depth of between about 26 - about 38 mm, such as between about 28 - about 36 mm, between about 30 - about 34 mm, or such as for example a width of about 32 mm. In some aspects, the device has a height of between about 85 - 90 mm, a width of between about 66 - 74 mm, and a depth of between about 28 - about 36 mm. In some aspects, the device has a height of about 87 mm, a width of about 70 mm, and a depth of about 32 mm. Thus, in one exemplary aspect, the device has a height of about 80-90 mm (e.g., about 84-88 mm), a width of about 65-75 mm (e.g., about 68-72 mm), and a depth of about 26-38 mm (e.g., about 30-34 mm).

The medicament dispenser of the present invention is designed to dispense one or more medicaments contained therein. Such a medicament or number of medicaments can be any one or more medicament(s) housed within one or more medicament carrier(s) capable of being received by and housed within the medicament dispenser.

According to certain aspects, which can be combined with any other aspect, embodiment, or combination(s) of aspects and/or embodiments, the medicament dispenser is designed to receive an elongate form medicament carrier carrying multiple distinct medicament dose portions. In one aspect, which can be combined with any other aspect, embodiment, or combination(s) of aspects and/or embodiments, the medicament dispenser can receive a single elongate form medicament carrier. In another aspect, the dispenser can receive plural elongate form medicament carriers. In yet another aspect, the dispenser can receive one to four elongate form medicament carriers, such as one, two, three, or four elongate form medicament carriers. In one aspect, which can be combined with any other aspect, embodiment, or combination(s) of aspects and/or embodiments, the dispenser can receive two elongate form medicament carriers. In certain embodiments, which can be combined with any other aspect, embodiment, or combination(s) of aspects and/or embodiments, the medicament dispenser can receive a single elongate form medicament carrier which houses one, two, three, four, or more different medicaments, each housed in distinct dose housing or dose storage areas (e.g., blister pockets), one or more such pockets being positioned such that they are accessible when the elongate medicament carrier is appropriately positioned within the dispenser. According to alternative aspects, which can be combined with any other aspect, embodiment, or combination(s) of aspects and/or embodiments, the medicament dispenser can receive a plurality (e.g., two, three, or four) elongate form medicament carriers which can optionally house one, two, three, four, or more different medicaments, each medicament dose being housed in distinct dose housing or dose storage areas (e.g., blister pockets) however one or more being positioned such that they are accessible substantially simultaneously, that is at about the same time, along with one or more dose storage areas, of one or more other medicament carriers held within the dispenser. In certain aspects, the medicament dispenser can advance a plurality of medicament carriers substantially simultaneously. In certain other aspects, the medicament dispenser can advance a plurality of medicament carriers sequentially, e.g., such that there is no substantial overlap or no detectable overlap in movement of two or more medicament carrier engagement mechanisms in operation. In another aspect, the carrier engagements are also capable of advancing their individually associated carrier in such a manner that at least one carrier advances first followed by advancement of second after completing all discrete doses of first carrier.

According to certain embodiments, which can be combined with any other aspect, embodiment, or combination(s) of aspects and/or embodiments, the elongate form medicament carrier can be any elongate form medicament carrier that houses a plurality of discrete doses of medicament in a sequence, such that when positioned in an appropriate position for access by the medicament dispenser a single dose of medicament within the carrier is available for administration and additional discrete doses of medicament are subsequently made available for administration upon further actuation and positioning. Such successive availability of different medicament dosages provides the dispenser with the ability to provide successive dosings of one or more medicaments, such as multiple dose-per-day administration of one or more medicaments, or sequential daily administration of one or more medicaments, and the like.

In some embodiments, medicament housed within one or more medicament carrier comprises one or more active pharmaceutical ingredients (APIs) and optionally, one or more pharmaceutically acceptable carriers. The active pharmaceutical ingredients and pharmaceutically acceptable carriers may be present in micronized form, non-micronized form, or mixtures thereof.

According to some embodiments, the medicament within each of the medicament storage areas of any single medicament carrier are the same. In alternative embodiments, the medicament within at least one of the medicament storage areas is different from the medicament within any at least one other medicament storage area within the same carrier. In certain aspects, the medicament within at least one of the medicament storage areas of a first carrier varies from the medicament held within at least one of the medicament storage areas of a second carrier.

According to certain aspects of the present invention, the dose portions of medicament within any single medicament carrier are all the same. In alternative aspects, at least one of the individual doses of medicament within any single medicament carrier is different from at least one other separated individual dose of medicament within the same single medicament carrier. According to yet further aspects, at least one of the individual doses of medicament within any one medicament carrier is different from at least one of the individual doses of medicament within any second or more medicament carriers.

According to one aspect of the present invention, one or more active pharmaceutical ingredients (APIs) that can be used in the inventions selected from, analgesics, e.g., codeine, dihydromorphine, ergotamine, fentanyl or morphine; anginal preparations, e.g., diltiazem; antiallergics, e.g., cromoglycate (e.g., as the sodium salt), ketotifen or nedocromil (e.g., as the sodium salt); anti-infectives, e.g., cephalosporins, penicillins, streptomycin, sulphonamides, tetracyclines and pentamidine, particularly tobramycin, aztreonam, colistin, ceftazidime, gentamycin, levofloxacin, ciprofloxacin, amikacin, vancomycin, rifampin and isoniazid; mucolytics e.g., N-acetylcysteine, carbocysteine, erdosteine, and dornase alfa.

According to embodiments, the medicaments used in the device can include agents for treating idiopathic pulmonary fibrosis e.g., nintedanib and pirfenidone; antihistamines, e.g., methapyrilene; anti-fungals, e.g., Pulmazole and voriconazole; anti-inflammatories, e.g., beclomethasone (e.g., as the dipropionate ester), fluticasone (e.g., as the propionate or furoate ester), flunisolide, budesonide, rofleponide, mometasone e.g., as the furoate ester), ciclesonide, triamcinolone (e.g., as the acetonide) or 6α,9α-difluoro-11β-hydroxy-16α-methyl-3-oxo-17α.-propionyloxy-androsta-1,4-diene-17β-carbothioic acid S-(2-oxo-tetrahydro-furan-3-yl) ester; antitussives, e.g., noscapine; bronchodilators, e.g., albuterol (e.g., as free base or sulphate), levoalbuterol, procaterol, salmeterol (e.g., as xinafoate), ephedrine, adrenaline, fenoterol (e.g., as hydrobromide), formoterol (e.g., as fumarate), arformoterol, clenbuterol, olodaterol, indacaterol, vilanterol, isoprenaline, metaproterenol, phenylephrine, phenylpropanolamine, pirbuterol (e.g., as acetate), reproterol (e.g., as hydrochloride), rimiterol, terbutaline (e.g., as sulphate), isoetharine, tulobuterol or 4-hydroxy-7-[2-[[2-[[3-(2-phenylethoxy)propyl]sulfonyl]ethyl]amino]ethyl-2(3H)-benzothiazolone; adenosine 2a agonists, e.g., 2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol (e.g., as maleate);. α4 integrin inhibitors, e.g., (2S)-3-[4-({[4-(aminocarbonyl)-1-piperidinyl]carbonyl}oxy)phenyl]-2-[((2S-)-4-methyl-2-{[2-(2-methylphenoxy)acetyl]amino }pentanoyl)amino]propanoic acid (e.g., as free acid or potassium salt), diuretics, e.g., amiloride; anticholinergics, e.g., ipratropium (e.g., as bromide), tiotropium, umeclidinium, aclidinium, glycopyrronium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon; vaccines, diagnostics, and gene therapies. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimize the activity and/or stability of the medicament.

In some embodiments, the medicament can be a mono-therapy (for example, single active medicament containing) product, or it may be a combination therapy (for example, plural active medicaments containing) product. In certain aspects, the medicament carrier can contain mono-active medicament dose portions or multi-active medicament dose portions (i.e., two or more than two APIs). Suitable medicaments or medicament components of a combination therapy product are typically selected from the group consisting of anti-inflammatory agents (for example a corticosteroid particularly inhaled corticosteroid (ICS) or an NSAID), anticholinergic agents (for example, an M₁, M₂, M₁/M₂ or M₃ receptor antagonist particularly long-acting muscarinic antagonist (LAMA), other β₂-adrenoreceptor agonists particularly long-acting β₂- agonist (LABA), anti-infective agents (e.g., an antibiotic or an antiviral), and antihistamines. All suitable combinations are envisaged. The present invention, in some embodiments, also provides combination therapy products, such as double or triple LAMA, LABA, and/or ICS combinations.

According to certain aspects, suitable anti-inflammatory agents include corticosteroids and NSAIDs. Suitable corticosteroids are those oral and inhaled corticosteroids and their pro-drugs which have anti-inflammatory activity. Examples of such agents include but are not limited to include methyl prednisolone, prednisolone, dexamethasone, fluticasone propionate, fluticasone furoate, 6α,9α-difluoro-17α-[(2-furanylcarbonyl)oxy]-11β-hydroxy-16α-methyl-3-oxo-androsta-1,4-diene-17β-carbothioic acid S-fluoromethyl ester, 6α,9α-difluoro-11β-hydroxy-16α-methyl-3-oxo-17α-propionyloxy-androsta-1,4-diene-17β-carbothioic acid S-(2-oxo-tetrahydro-furan-3S-yl) ester, beclomethasone esters (e.g., the 17-propionate ester or the 17,21-dipropionate ester), budesonide, flunisolide, mometasone esters (e.g., the furoate ester), triamcinolone acetonide, rofleponide, ciclesonide, butixocort propionate, RPR-106541, and ST-126. In some aspects, preferred corticosteroids include fluticasone propionate, fluticasone furoate 6α,9α-difluoro-11β-hydroxy-16α-methyl-17α-[(-4-methyl-1,3-thiazole-5-carbonyl)oxy]-3-oxo-androsta-1,4-diene-17β-carbothioic acid S-fluoromethyl ester and 6α,9α-difluoro-17α-[(2-furanylcarbonyl)oxy]-11β-hydroxy-16α-methyl-3-oxo-androsta-1,4-diene-17β-carbothioic acid S-fluoromethyl ester, more preferably 6α,9α-difluoro-17α-[(2-furanylcarbonyl)oxy]-11β-hydroxy-16α-methyl-3-oxo-androsta-1,4-diene-17β-carbothioic acid S-fluoromethyl ester.

According to some embodiments, suitable NSAIDs include cromolyns (e.g., sodium cromoglycate, nedocromil sodium), phosphodiesterase (PDE) inhibitors (e.g., theophylline, PDE4 inhibitors or mixed PDE3/PDE4 inhibitors), leukotriene antagonists, inhibitors of leukotriene synthesis, iNOS inhibitors, tryptase and elastase inhibitors, β₂integrin antagonists and adenosine receptor agonists or antagonists (e.g., adenosine 2a agonists), cytokine antagonists (e.g., chemokine antagonists) or inhibitors of cytokine synthesis. Suitable other β₂-adrenoreceptor agonists include salmeterol (e.g., as the xinafoate), salbutamol (e.g., as the sulphate or the free base), formoterol (e.g., as the fumarate), vilanterol (e.g., as the trifenatate), indacaterol (e.g., as the maleate), fenoterol or terbutaline and salts thereof.

In some aspects, suitable phosphodiesterase 4 (PDE4) inhibitors include compounds that are known to inhibit the PDE4 enzyme or which are discovered to act as a PDE4 inhibitor, and which are only PDE4 inhibitors, not compounds which inhibit other members of the PDE family as well as PDE4. According to certain aspects, it may be preferred to use a PDE4 inhibitor which has an IC₅₀ ratio of about 0.1 or greater as regards the IC₅₀ for the PDE4 catalytic form which binds rolipram with a high affinity divided by the IC₅₀ for the form which binds rolipram with a low affinity. For the purposes of this disclosure, the cAMP catalytic site which binds R and S rolipram with a low affinity is denominated the "low affinity" binding site (LPDE 4), and the other form of this catalytic site which binds rolipram with a high affinity is denominated the "high affinity" binding site (HPDE 4). This term, "HPDE4," should not be confused with the term "hPDE4," which is used to denote human PDE4.

A method for determining IC₅₀s ratios is set out in U.S. Pat. No. 5,998,428, which is incorporated herein in full by reference as though set out herein. See also PCT application WO 00/51599 for another description of the said assay.

In some embodiments, suitable PDE4 inhibitors include those compounds which have a salutary therapeutic ratio, for example, compounds which preferentially inhibit cAMP catalytic activity where the enzyme is in the form that binds rolipram with a low affinity, thereby reducing the side effects which apparently are linked to inhibiting the form which binds rolipram with a high affinity. Another way to state this is that in some aspects, the preferred compounds will have an IC₅₀ ratio of about 0.1 or greater as regards the IC₅₀ for the PDE4 catalytic form which binds rolipram with a high affinity divided by the IC₅₀ for the form which binds rolipram with a low affinity.

A further refinement of this standard is that of one wherein the PDE4 inhibitor has an IC50 ratio of about 0.1 or greater; said ratio is the ratio of the IC₅₀ value for competing with the binding of 1nM of [³H]R-rolipram to a form of PDE4 which binds rolipram with a high affinity over the IC₅₀ value for inhibiting the PDE4 catalytic activity of a form which binds rolipram with a low affinity using 1 muM[³H]-cAMP as the substrate.

In some embodiments, suitable agents are those PDE4 inhibitors that have an IC₅₀ ratio of greater than 0.5, and particularly those compounds having a ratio of greater than 1.0. Preferred compounds are cis 4-cyano-4-(3-cyclopentyloxy-4-methoxyphenyl)cyclohexan-1-carboxylic acid, 2-carbomethoxy-4-cyano-4-(3-cyclopropylmethoxy-4-difluoromethoxyphenyl)cyclohexan-1-one and cis-[4-cyano-4-(3-cyclopropylmethoxy-4-difluoromethoxyphenyl)cyclohexan-1-ol]; these are examples of compounds which bind preferentially to the low-affinity binding site and which have an IC₅₀ ratio of 0.1 or greater.

Other suitable medicament compounds include, according to certain embodiments: cis-4-cyano-4-[3-(cyclopentyloxy)-4-methoxyphenyl]cyclohexane-1-carboxylic acid (also known as cilomalast) disclosed in U.S. Pat. No. 5,552,438 and its salts, esters, pro-drugs or physical forms; AWD-12-281 from elbion (Hofgen, N. et al. 15th EFMC Int Symp Med Chem (Sep. 6-10, Edinburgh) 1998, Abst P.98; CAS reference No. 247584020-9); a 9-benzyladenine derivative nominated NCS-613 (INSERM); D-4418 from Chiroscience and Schering-Plough; a benzodiazepine PDE4 inhibitor identified as CI-1018 (PD-168787) and attributed to Pfizer; a benzodioxole derivative disclosed by Kyowa Hakko in WO99/16766; K-34 from Kyowa Hakko; V-11294A from Napp (Landells, L. J. et al. Eur Resp J [Annu Cong Eur Resp Soc (Sep. 19-23, Geneva) 1998] 1998, 12 (Suppl. 28): Abst P2393); roflumilast (CAS reference No 162401-32-3) and a pthalazinone (WO99/47505, the disclosure of which is hereby incorporated by reference) from Byk-Gulden; Pumafentrine, (-)-p-[(4aR*,10bS*)-9-ethoxy-1,2,3,4,4a,10b-hexahydro-methoxy-8-methoxy-2-methylbenzo[c][1,6]naphthyridin-6-yl]-N,N-diisopropylbenzamide which is a mixed PDE3/PDE4 inhibitor which has been prepared and published on by Byk-Gulden, now Altana; arofylline under development by Almirall-Prodesfarma; VM554/UM565 from Vernalis; or T-440 (Tanabe Seiyaku; Fuji, K. et al. J Pharmacol Exp Ther, 1998, 284(1): 162), and T2585.

According to certain embodiments, suitable anticholinergic agents are those compounds that act as antagonists at the muscarinic receptor, in particular, those compounds, which are antagonists of the M1 and M2 receptors. Exemplary compounds include but may not be limited to the alkaloids of the belladonna plants as illustrated by the likes of atropine, scopolamine, homatropine, hyoscyamine; these compounds are normally administered as a salt, being tertiary amines.

In some aspects, particularly suitable anticholinergics can include ipratropium (e.g., as the bromide), sold under the name Atrovent, umeclidinium (e.g., as the bromide), aclidinium (e.g., as the bromide), glycopyrronium (e.g., as the bromide), oxitropium (e.g., as the bromide) and tiotropium (e.g., as the bromide) (CAS-139404-48-1). Also of interest in some embodiments are methantheline (CAS-53-46-3), propantheline bromide (CAS-50-34-9), anisotropine methyl bromide or Valpin 50 (CAS-80-50-2), clidinium bromide (Quarzan, CAS-3485-62-9), copyrrolate (Robinul), isopropamide iodide (CAS-71-81-8), mepenzolate bromide (U.S. Pat. No. 2,918,408), tridihexethyl chloride (Pathilone, CAS-4310-35-4), and hexocyclium methylsulfate (Tral, CAS-115-63-9). See also cyclopentolate hydrochloride (CAS-5870-29-1), tropicamide (CAS-1508-75-4), trihexyphenidyl hydrochloride (CAS-144-11-6), pirenzepine (CAS-29868-97-1), telenzepine (CAS-80880-90-9), AF-DX 116, or methoctramine, and the compounds disclosed in WO01/04118.

According to some aspects, suitable antihistamines (also referred to as H₁-receptor antagonists) include any one or more of the numerous antagonists known which inhibit Hi-receptors and are safe for human use. All such agents can be reversible, competitive inhibitors of the interaction of histamine with H1-receptors. Examples of such agents include ethanolamines, ethylenediamines, and alkylamines. In addition, other first-generation antihistamines include those which can be characterized as based on piperizine and phenothiazines. Second generation antagonists, which are non-sedating, have a similar structure-activity relationship in that they retain the core ethylene group (the alkylamines) or mimic the tertiary amine group with piperizine or piperidine. Exemplary antagonists are as follows but may not be limited to: Ethanolamines: carbinoxamine maleate, clemastine fumarate, diphenylhydramine hydrochloride, and dimenhydrinate. Ethylenediamines: pyrilamine amleate, tripelennamine HCI, and tripelennamine citrate. Alkylamines: chlropheniramine and its salts such as the maleate salt, and acrivastine. Piperazines: hydroxyzine HCI, hydroxyzine pamoate, cyclizine HCI, cyclizine lactate, meclizine HCI, and cetirizine HCI. Piperidines: Astemizole, levocabastine HCI, loratadine or its descarboethoxy analogue, and terfenadine and fexofenadine hydrochloride or another pharmaceutically acceptable salt.

Azelastine hydrochloride is yet another Hi receptor antagonist that may be used in combination with a PDE4 inhibitor. Particularly suitable anti-histamines in some aspects include methapyrilene and loratadine. In respect of combination products, co-formulation compatibility is generally determined on an experimental basis by known methods and may depend on the chosen type of medicament dispenser action.

According to certain embodiments, the medicament components of a combination product administered by a dispenser described herein are suitably selected from the group consisting of anti-inflammatory agents (for example a corticosteroid or an NSAID), anticholinergic agents (for example, an M₁, M₂, M₁/M₂ or M₃ receptor antagonist), other β₂-adrenoreceptor agonists, anti-infective agents (e.g., an antibiotic or an antiviral), and antihistamines. All suitable combinations are envisaged.

In certain aspects, the co-formulation compatible components comprise a β₂-adrenoreceptor agonist and a corticosteroid; and the co-formulation incompatible component comprises a PDE-4 inhibitor, an anti-cholinergic or a mixture thereof. The β₂-adrenoreceptor agonists may, for example, be salbutamol (e.g., as the free base or the sulphate salt) or salmeterol (e.g., as the xinafoate salt) or formoterol (e.g., as the fumarate salt). The corticosteroid may for example, be a beclomethasone ester (e.g., the dipropionate) or a fluticasone ester (e.g., the propionate) or budesonide.

In one example, the co-formulation compatible components comprise fluticasone propionate and salmeterol, or a salt thereof (particularly the xinafoate salt) and the co-formulation incompatible component comprises a PDE-4 inhibitor, an anti-cholinergic (e.g., ipratropium bromide or tiotropium bromide) or a mixture thereof.

In another example, the co-formulation compatible components comprise budesonide and formoterol (e.g., as the fumarate salt), and the co-formulation incompatible component comprises a PDE-4 inhibitor, an anti-cholinergic (e.g., ipratropium bromide or tiotropium bromide) or a mixture thereof.

Medicament dispensers, as disclosed herein, have a variety of structural configurations and can be used for dispensing powders, tablets, capsules, pellets or pucks, or mixtures thereof for nasal, pulmonary or oral administration. In particular, the medicament dispensers can be used to dispense a powder comprising one or more active pharmaceutical ingredient(s)(API) and optionally, one or more micronized or non-micronized pharmaceutically acceptable carrier(s) and/or excipient(s), wherein the dispensing mechanism comprises of three steps to operate the medicament dispenser: open, dispense and close. This mechanism is described in further detail elsewhere herein.

Generally, powdered medicament particles suitable for delivery to the bronchial or alveolar region of the lung have an aerodynamic diameter of less than about 10 micrometers, preferably less than about 6 micrometers (e.g., about 0.5-10 micrometers, about 0.5-6 micrometers, about 0.5-5 micrometers, about 0.5-3 micrometers, about 1-10 micrometers, about 1-6 micrometers, or about 1-5 micrometers). Other sized particles may be used if delivery to other portions of the respiratory tract is desired, such as the nasal cavity, mouth, or throat. In some aspects, the medicament administered via a dispenser of the present invention can be delivered as a pure drug, but in some aspects, it can be more appropriate or preferable that medicaments are delivered together with one or more excipients or carriers which are suitable for inhalation. Suitable excipients or carriers include organic excipients such as polysaccharides (for example starch, cellulose and the like), lactose, trehalose, leucine, trileucine, pentaleucine and other polyleucines, glucose, mannitol, amino acids, magnesium stearate and maltodextrins, surfactants such as 1,2-Distearoyl-sn-glycero-3-phosphocholine (DSPC), 1,2-Distearoyl-sn-glycero-3-phosphoethanolamine (DSPE), perfluorocarbon such as perflubron (perfluorooctyl bromide) and inorganic excipients such as calcium carbonate, calcium chloride, magnesium chloride or sodium chloride. In a particular embodiment, carrier or excipient is lactose.

Particles of powdered medicament and/or excipient may be produced by conventional techniques, for example by micronization, milling or sieving. Additionally, medicament and/or excipient powders may be engineered with particular densities, size ranges, or characteristics. Particles may comprise active agents, surfactants, wall forming materials, or other components considered desirable by those of ordinary skill.

In some aspects, which can be combined with any other aspect, embodiment, or combination(s) of aspects and/or embodiments, which can be combined with any other aspect, embodiment, or combination(s) of aspects and/or embodiments, any one or more of the medicament carrier(s) to be used with the medicament dispenser (e.g., one or more elongate medicament carrier(s)) can be in the form of a blister strip (blister pack) or a laminate form (e.g., a laminated strip). In some aspects, the carrier can be an elongated carrier onto which medicament has been applied or inserted via any suitable process for providing discrete doses of medicament, such as printing, painting, or vacuum occlusion. In some aspects, which can be combined with any other aspect, embodiment, or combination(s) of aspects and/or embodiments, if a plurality of medicament carriers are housed within the medicament dispenser, all medicament carriers have substantially the same design. According to alternative embodiments, when a plurality of medicament carriers are present within the medicament dispenser, any one medicament carriers may comprise a design that is different from any one or more other medicament carriers present within the dispenser. In certain aspects, which can be combined with any other aspect, embodiment, or combination(s) of aspects and/or embodiments, any single medicament carrier can include a plurality of distinct dose storage areas, such as spots, bubble pockets, or depressed pockets, or cavities, e.g., blister pockets, within an elongated form, such as a strip, wherein each such distinct dose storage areas, for example, each of a plurality of blister pockets comprises one dose of a medicament. In certain aspects, when a plurality of medicament carriers are present, e.g., if such a plurality of medicament carriers vary in design, the medicament dispenser can be capable of advancing each medicament dispenser, regardless of design, at the same time, e.g., a plurality of medicament carrier engagement mechanisms can advance a plurality of medicament carriers such that they are in motion simultaneously or at least substantially simultaneously. In certain other aspects, when a plurality of medicament carriers are present, if such a plurality of medicament carriers vary in design, the medicament dispenser is capable of advancing each, regardless of design, in sequence, e.g., a plurality of medicament carrier engagement mechanisms can advance a plurality of medicament carriers one at a time, such that there is no substantial overlap or no detectable overlap in motion of any two or more medicament carriers in operation. In certain aspects, when a plurality of medicament carriers that vary in design are processed by the medicament dispenser, the medicament dispenser is capable of making a dose of medicament from each such medicament carrier present available at substantially the same time. In certain aspects, when a plurality of medicament carriers are processed that vary in design, the medicament dispenser is capable of making a dose of medicament from each medicament carrier present available in sequence, one dose from one medicament carrier being made available before another dose from the another medicament carrier. In certain embodiments, whether doses of medicament from a plurality of carriers are made available substantially simultaneously or in sequence, the doses made are available for providing to the patient in a single administration, that is, via a single inhalation by the user, in operation of the medicament dispenser.

In certain embodiments, an elongate medicament carrier can be a blister pack having any suitable length and can comprise any suitable number of medicament blister pockets. In certain aspects, which can be combined with any other aspect, embodiment, or combination of aspects and embodiments described herein, can be a blister strip comprising at least about seven blister pockets, such as at least about ten blister pockets, or at least about 14 blister pockets, each containing a distinct dose of medicament ("distinct" in this context means separate, but not necessarily different, as each pocket may contain the same dose of the same medicament or may contain different dosages and/or different medicaments from the contents of other blister pockets). For example, in certain embodiments, each medicament carrier can comprise at least about ten pockets, at least about 20 pockets, at least about 30 pockets, at least about 40 pockets, at least about 50 pockets, at least about 60 pockets, at least about 70 pockets at least about 80 pockets, at least about 90 pockets, or at least about 100 pockets, such as for examples at least approximately 110 pockets, at least approximately 120 pockets, at least approximately 130 pockets, at least approximately 140 pockets, at least approximately 150 pockets, at least approximately 160 pockets, at least approximately 170 pockets, at least approximately 180 pockets, at least approximately 190 pockets, at least about 200 pockets, at least about 220 pockets, or at least about 240 pockets (e.g., about 10-200 pockets, about 10-120 pockets, about 20-120 pockets, about 30-120 pockets, about 40-120 pockets, about 50-150 pockets, or about 40-160 pockets), such that, for example, the medicament dispenser could dispense a daily administration of one or more medicaments over a period comprising approximately one week, approximately one month, approximately two months, approximately three months, approximately four months, approximately five months, approximately six months, or even longer.

In some aspects, which can be combined with any other aspect, embodiment, or combination(s) of aspects and/or embodiments, each pocket, cavity, or other dose storage area (e.g., each blister pocket) comprising a single dose of medicament(s) can be made accessible to the medicament dispenser by appropriate positioning within the dispenser. According to certain aspects, a dose of medicament within the dispenser can be unavailable for dispensation unless it is appropriately positioned within the dispenser device ("device"). In some aspects, which can be combined with any other aspect, embodiment, or combination(s) of aspects and/or embodiments, access to the medicament(s) comprised within, e.g., the blister pockets of a medicament carrier, is accomplished by any suitable access means including, for example, piercing or puncturing, tearing, or peeling apart of blister pockets by a component of the medicament dispenser. In one aspect, which can be combined with any other aspect, embodiment, or combination(s) of aspects and/or embodiments, the medicament carrier can be in the form of a peelable blister strip. In such an aspect, the peelable blister strip can comprise a) a base sheet in which blisters are formed to define pockets therein for containing distinct medicament dose portions and b) a lid sheet or lamination which is hermetically sealed to the base sheet except in the region of the blisters in such a manner that the lid sheet and the base sheet can be separated, e.g., by being peeled apart.

In certain embodiments which can be combined with any other aspect, embodiment, or combination(s) of aspects and/or embodiments, one end of the lid sheet can be in a form which facilitates connecting the end of the lid sheet to a medicament dispenser component which would allow collection, for example by winding into a roll, the lid sheet as it is peeled away from the base sheet upon sequential dose administrations. In some aspects, which can be combined with any other aspect, embodiment, or combination(s) of aspects and/or embodiments, the end of the lid sheet can be in the form of a section of the lid sheet unattached to a base sheet and can comprise a notch, a catch element (e.g., a cut out "keyhole"-type section into which a second element might be inserted to form a connection to the strip), or for example a sealed loop at its end that is capable of being placed in connection or contact with a component of the medicament dispenser, such as a lid connection element, e.g., a post, of a waste collector. In some aspects, which can be combined with any other aspect, embodiment, or combination(s) of aspects and/or embodiments the lid sheet comprise a sealed loop which can connect to a component within a waste collector, such as winding assembly which will be described elsewhere herein, to facilitate application of tension to the lid sheet, winding of the lids sheet after removal from the base sheet, or both application of tension and winding.

In some aspects, which can be combined with any other aspect, embodiment, or combination of aspects and embodiments described here, a lid sheet can be peeled via a constant tension torque applied by a component of a waste collector, such as a winding assembly as is described elsewhere herein, within the medicament dispenser. In such aspects, a lid sheet can wind around a component of the waste collector, e.g., a component of a winding assembly. In some aspects, which can be combined with any other aspect, embodiment, or combination(s) of aspects and/or embodiments, one end of a base sheet can be configured to facilitate connecting or contacting the end of the base sheet to a medicament dispenser component which allows or facilitates advancement of the base sheet upon sequential dose administration. In some aspects, which can be combined with any other aspect, embodiment, or combination(s) of aspects and/or embodiments, the base sheet can be placed in contact with a wrapping wheel of a medicament dispenser. In further aspects, the wrapping wheel can comprise a slit into which the base sheet can be placed such that the base sheet advances via the wrapping wheel.

Each dose/medicament compartment on or within an elongate medicament carrier, e.g., blister pocket of a medicament carrier, can contain or form one or a plurality of separate compartments. In certain embodiments which can be combined with any other aspect, embodiment, or combination(s) of aspects and/or embodiments, a single compartment, e.g., blister pocket, of a medicament carrier can be divided into two, three, four, or more compartments, such as five compartments, six compartments, seven compartments, or eight compartments. In such aspects, the compartments can be formed by a partition wall dividing the blister pocket. A blister pocket comprising one or more compartments can be covered by any suitable lid sheet. Such a design may be helpful in scenarios wherein multiple medicaments are administered together, such medicaments not being formulated within a single composition. A single composition, for example, may not be readily available or for example, one or more medicaments may present issues with respect to compatibility with one or more other medicaments and therefore maintenance of separation of two or more medicaments from one another might be preferable until the time of medicament administration.

In some aspects, the blister pockets of an elongate form can include medicament comprising one or more active pharmaceutical ingredient(s). For example, the blister pocket can include medicament comprising a single active pharmaceutical ingredient. In other examples, the blister pocket can include medicament comprising two, three, four, or more active pharmaceutical ingredients. For example, the blister pocket can include medicament comprising two or three active pharmaceutical ingredients. In some aspects, which can be combined with any other aspect, embodiment, or combination of aspects and embodiments described herein, each blister pocket can contain the same composition of actives as every other blister pocket within the same strip and medicament carrier (elongate form). In alternative aspects, the composition of actives can vary between blister pockets of the same strip or across medicament carriers.

The plural elongate form medicament carriers can be provided in any suitable configuration. One suitable configuration for the dispensation device and dispensation methods described herein is a 'side-by-side' configuration, in which, for example, two carriers (e.g., two coiled blister strips) are arranged to lie in sideways alignment with each other. Another suitable configuration is a 'double-decker' or "staked" configuration, in which for example, two carriers (e.g., two coiled blister strips sharing the same coiling axis) are arranged to lay one on top of the other in the dispenser. A plurality of medicament carriers can be a first medicament carrier and a second medicament carrier and, in some aspects, a third, fourth, or more medicament carrier(s). In common embodiments, a plurality of medicament carriers can be a first medicament carrier and a second medicament carrier. The plural medicament carriers can, in certain embodiments, which can be combined with any other aspect, embodiment, or combination of aspects and embodiments described herein, be separated by a deck separator or deck separators. A deck separator can be used to allow the independent movement of each of the plurality of medicament carriers with no contact between medicament carriers and no friction between medicament carriers due to mutual contact. The pockets of each carrier can contain the same or different medicament powder therein, the same or different amount (dose portion) in each pocket. In certain aspects, each pocket of a single carrier contains the same medicament powder therein, the same amount (dose portion) of medicament therein as the other pockets within the same carrier. In certain embodiments, which can be combined with any other aspect, embodiment, or combination(s) of aspects or embodiments described herein, one carrier of a plurality of carriers will have at least one different active ingredient in its medicament powder than another carrier. Thus, in use, the patient can simultaneously inhale one dose portion from each carrier, each carrier delivering (a) different active ingredient(s). In some embodiments, the medicament dose portions releasable from each of the plural medicament carriers are not combined or mixed within the dispenser. In certain aspects, the medicament dose portions releasable from each of the plural medicament carriers are combined or mixed at the time of inhalation by the user and not before. According to some embodiments, the medicament dose portions from each of the plural medicament carriers are inhaled as a combined product. In some aspects, the medicament dose portions from each of the plural medicament carriers are mixed within the dispenser and exit the dispenser as a single airflow.

The multiple distinct medicament dose portions can be provided to each carrier in uniform series. According to certain aspects, the spacing between each dose storage area of any single medicament carrier is essentially the same. In alternative aspects, the spacing between at least two dose storage areas is different than the spacing, or pitch, between at least two other dose storage areas within a single medicament carrier. In certain aspects, the spacing between any at least two dose storage areas of a first medicament carrier is different from the spacing between any at least two dose storage areas of a second medicament carrier. In particular, the spacing (i.e., pitch) between each dose portion can be uniform throughout the series. In other aspects however, the spacing (i.e., pitch) may vary throughout the series (i.e., be non-uniform). In one aspect, the spacing (i.e., pitch) between each dose portion is equivalent for each carrier of the dispenser. That is to say, each medicament carrier in such an embodiment is equivalently pitched. In other aspects, the spacing between each dose portion is non-equivalent for each carrier of the dispenser. Such variation of the spacing (i.e., pitch) from carrier to carrier can be used to enable flexibility in (combination) dosage patterns. In one particular example, the spacing (i.e., pitch) of a first carrier is arranged to be half that of a second carrier. This arrangement is beneficially employed where the dosing interval (i.e., the time between doses) of the medicament carried by the first carrier is twice that of the medicament carrier by the second carrier (e.g., in a twice-daily / once daily type dosage regime).

The medicament dispenser can include a housing, a medicament access body, and a cover for the medicament access body.

The housing can include a base cover and a top cover for enclosing the medicament carrier(s), an inner housing assembly, a base plate, and components of the dispensing mechanism. The top cover can permanently lock with the base cover through permanent bonding methods, including, but not limited to ultrasonic welding and ultraviolet cured adhesives and the like. Alternatively, the base cover and a top cover can be capable of being separated, for example, simply by a snap-fit or by a locking mechanism such that could be opened/separated by a trained physician or medical practitioner to facilitate, for example, refilling of the medicament dispenser. According to certain embodiments, the housing can provide a mechanism for the user or a trained profession, e.g., a healthcare provider, to gain access to the interior of the dispenser such that one or more expended medicament carriers can be removed and one or more full medicament carriers can be replaced therein, making the dispenser reusable. Such interior access via a mechanism incorporated into the housing can be any suitable access, such as via a hinged hatch or a sliding compartment cover, each having a clasp, clip or other closure element to maintain closure when not in use, a pressure-sensitive (push) clip/clasping door, an access cover attached using screws, or any such similar or equivalent access mechanism common for providing access to the interior of devices comprising a housing, such as access mechanisms to battery storage devices in electronic instruments.

The housing can define the shape of the medicament dispenser. The housing can serve to enclose the components of the dispenser. In certain aspects, the housing can enclose the components of the dispenser and can define the shape of the dispenser as a circular form, non-circular form, squircular form, an elongate form, an elliptical form, an ovular form, an oblong form, a triangular form, a quadrilateral form, a pentagonal form, a hexagonal form, including, but not limited to square form, rectangular form and various trapezium forms, an hourglass form, or other shapes. The housing can include a flat surface or a resting surface. At least a portion of the housing can be shaped for ease of grip by the user, such as comprising curves and elements designed to conform to the human hand, such as depressions for at least partially nesting one or more fingers of a user, an area which can fit comfortably within the palm, which can aid in ease and comfort of securely holding the device and ability to easily and comfortably dispense or activate the device while holding it with for example one hand.

The medicament dispenser of the present invention can have a component that allows the user to receive one or more medicament doses housed therein. Such an element can be referred to herein as a "medicament access body." The term "medicament access body" is used to describe an element containing an opening through which the patient may inhale the medicament dose portions. In some aspects, the medicament access body is shaped such that it promotes efficient cooperation, e.g., docking, fitting, insertion, or interfacing, with either the nose or mouth of a human user. According to certain embodiments, the medicament access body can facilitate inhalation via the nose, such as by incorporation of one or more extended nozzles or the like. In one aspect, the medicament access body can facilitate inhalation by oral means (e.g., via the mouth). According to one embodiment, which can be combined with any other aspect, embodiment, or combination(s) of aspects or embodiments described herein, the medicament access body is a mouthpiece. In such an embodiment, a user can place an opening of the medicament access body (i.e., the mouthpiece) in their mouth or upon their lips. The opening of the mouthpiece can be aligned and communicate with an opening of a flow director. When the patient inhales through the opening of the mouthpiece, air can enter the flow director of the dispenser through inlet vent openings and exit the flow director opening via an outlet compartment or outlet channels, into the mouth of the user, along with the medicament dose portion(s). Design features of the flow director, inlet vent openings, and airflow (or air flow) paths are described in more detail elsewhere herein. Use of the term mouthpiece is used commonly throughout this disclosure but it should be made clear that the medicament access body is not limited to a mouthpiece.

The medicament access body, e.g., the mouthpiece, can be designed to snap, clip, or otherwise fasten onto the housing and/or flow director opening of the medicament dispenser. The medicament access body can be in the form of an extended mouthpiece to assist with deagglomeration of the medicament and ease of delivery in the oral cavity. For example, in certain aspects a medicament access body can comprise an extended form or can incorporate additional components, such as screens, air vents, or the like, to provide for a desirable airflow such that during inhalation, powdered medicament being inhaled from the dose storage area(s), e.g., blister pack(s), of the medicament carrier(s) within the dispenser, is distributed effectively within the airflow and powder that may have aggregated within the dose storage areas, e.g., blister pack(s) can be broken apart for inhalation. In certain aspects, no additional air vents are present within the medicament access body and the only entrance point into the dispenser for a significant source of external air is through the inlet vents of the dispenser upon inhalation by the user, as will be described further elsewhere herein.

The housing and/or the medicament access body, and further many or all of the components of the dispenser housed within the housing, can be made of any suitable material, and many such materials are known in the art. In some aspects, common materials used to form the housing and/or medicament access body can be a plastic material, formed e.g., by common molding techniques such as but not limited to injection molding. In some aspects, it may be appropriate or preferable to have a component within the housing, e.g., another component of the dispensing mechanism, to be comprised of a metal. For example, a spring component in one embodiment of the invention serving as an element of an advancer may be advantageously comprised of a metal, such as but not limited to stainless steel. In certain aspects, a common plastic used in the manufacturing of parts of the dispenser. In some aspects materials that can be used for the housing or any component housed therein can include but may not be limited to acetyl (polyoxymethylene (POM). In some aspects, the housing, mouthpiece, and/or one or more other components of the device comprise, are primarily composed of, are essentially entirely composed of, or are entirely composed of, one or more of acrylonitrile-butadiene-styrene (ABS), high-density polyethylene (HDPE), polycarbonate (PC), a polycarbonate/acrylonitrile butadiene styrene terpolymer blend (PC/ABS), polypropylene (PP), Polyoxymethylene (POM), nylon, metal, and silicone rubber. Such materials while advantageous in some contexts, are exemplary, and those skilled in the art will appreciate that many such alternative materials can be appropriately incorporated into components of the device.

According to embodiments, the housing can include an inner housing assembly. The inner housing assembly can be formed from the same material as the housing, the medicament access body, both the housing and the medicament access body or it can be formed from a material different from both the housing and the medicament access body.

The inner housing assembly can, in some aspects, incorporate features that serve to locate or aid in the placement of the one or more medicament carrier(s) and one or more components of the dispensing mechanism. The inner housing assembly can be placed in the base cover through permanent bonding methods, such as in certain aspects including, but not limited to, ultrasonic welding and ultraviolet cured adhesives and the like. The inner housing assembly can, in some aspects, which can be combined with any other aspect, embodiment, or combination of aspects and embodiments described herein, contain compartments to receive the medicament carrier(s) and the unused portion of the base sheet(s). In some embodiments, one or more of a plurality of such compartments can be separable by a wall. The inner housing assembly can, according to some embodiments, include openings for locating components of the dispensing mechanism to secure their positioning. Such openings can be of any appropriate size, shape, or orientation capable of facilitating necessary interactions of the components of the dispensing mechanism required for the successful operation of the dispenser, such as round, square or other shaped holes or cut-outs, slits, slots, or the like. The inner housing assembly can, in some aspects, which can be combined with any other aspect, embodiment, or combination of aspects and embodiments described herein, also contain a space for locating a flow director. In certain embodiments, the space locating the flow director is in the shape of a "V" or a notch. Such a shaped flow director, as will be described elsewhere, can provide for beneficial airflow patterns for the administration of an inhaled powder medicament from the dispensing device.

In some aspects, which can be combined with any other aspect or embodiment or combination(s) of aspects and embodiments described herein, the underside part of the inner housing assembly can locate a driver, an interim driver, an interim gear, a secondary interim gear and a tertiary interim gear on positioning elements such as, for example, posts or studs. These elements are described elsewhere herein. In certain aspects, the studs of the inner housing assembly help to maintain the optimal positioning of the various components of the dispenser, such as various components having a gearing mechanism (e.g., a mechanism by which one component is movably engaged with a second component or two or more additional components). The inner housing assembly can, in some embodiments, comprise a first compartment in which a medicament carrier containing dose portions is initially located and from which it is dispensed, and a second compartment to receive the used portion of the base sheet after it has been advanced and separated from the lid sheet. In certain aspects, the inner housing assembly can comprise another compartment in which a second of a plurality of medicament carriers containing dose portions is initially located and from which medicament is dispensed. In certain aspects, the inner housing assembly can comprise another compartment in which each of a plurality of medicament carriers containing dose portions is initially located and from which it is dispensed. In some embodiments, the inner housing assembly can comprise a compartment for receiving the used portion of the base sheet from a second medicament carrier when present within the dispenser. In some embodiments, the inner housing assembly can comprise a separate compartment for receiving the used portions of the base sheet from each of a plurality of medicament carriers when present within the dispenser. According to alternative embodiments, the inner housing assembly can comprise a single, common compartment for receiving the used portion of the base sheet from all medicament carriers present within the dispenser.

In certain embodiments, the housing can also include a base plate for housing a dose indicator and covering the medicament carriers over the inner housing assembly. The base plate can be made of any suitable material. The base plate can, in certain aspects, which can be combined with any other aspect or embodiment or any combination(s) of aspects or embodiments described herein, include a pin for locating the medicament access body cover, e.g., mouthpiece cover and openings for locating the wrapping wheel and the advancer. Such a pin can function as, e.g., a point of connection and rotation of the medicament access body cover, e.g., a mouthpiece cover, as is described further in a detailed description of the figures provided herein. The functionality of the wrapping wheel and advancer are also described further herein.

The housing can also comprise, according to certain embodiments which can be combined with any other aspect or embodiment or combination(s) of aspects or embodiments described herein, a dispensing mechanism for dispensing medicament(s) from the dispenser. In one aspect, some or all components are shared commonly between a plurality of medicament carriers. In a common aspect, some or all components of the dispensing mechanism are distinct for each of a plurality of medicament carriers maintained within the device.

The dispensing mechanism can include a driver, a carrier engagement, an advancer, a first waste collector for collecting a first waste stream, a second waste collector for collecting a second waste stream, and a flow director. The dispensing mechanism can also include an interim driver, an interim gear, a secondary interim gear, and a tertiary interim gear.

A driver can be provided for driving the actuation of the dispensing mechanism. According to certain embodiments, the driver can actuate the dispensing mechanism in cooperation with movement of the cover of the medicament access body, the driver being movably engaged with the medicament access body cover in such a way that movement of the medicament access cover can result in movement of the driver. In turn, the driver can be movably engaged with the advancer.

In certain embodiments, the driver can actuate the dispensing mechanism in cooperation with movement of lever, the driver being movably engaged with the lever in such a way that movement of the lever can result in movement of the driver. In turn, the driver can be movably engaged with the advancer.

Hence according to certain embodiments, the driver can be or can be characterized as an actuator. The driver can be any mechanism capable of receiving an actuation energy and passing that activation energy on to a second or more mechanism(s) of the dispenser. According to certain aspects, the driver may directly drive an advancer. According to certain aspects, the driver can indirectly drive an advancer by way of an interim driver.

According to certain embodiments that can be combined with any other aspect or embodiment, or a combination of aspects or embodiments described herein, the driver can be in the form of a repositionable engagement unit. Such a repositionable engagement unit can be any unit which is capable of movably engaging with one or more other components of the dispenser. In some aspects, the driver can be in the form of a conveyor, lever, switch, wheel or the like. In certain aspects, the driver can be in the form of a wheel comprising engagement units capable of engaging with corresponding engagement units of one or more other components. Such engagement units, in some aspects, can be protrusions or indentations of any shape or size. In some aspects, the engagement units are teeth, and the driver is a gear.

It should be noted here that a plurality of components of the dispenser mechanism as described herein can be preferably designed for engagement with one another using a gear-like mechanism, the gear-like mechanism comprising what is described herein as teeth, such as teeth commonly exemplified in the art. Such teeth of any such component described herein can comprise any suitable have any size, shape or design capable of cooperating with, communicating with, movably engaging with, or otherwise interacting with suitable complementary size or shape. Such teeth can have a square-, rectangular-, triangular-, trapezoidal-, or polygonal-shape, or any other such exemplary shape and can be arranged circumferentially about the element, e.g., about the gear. Further, in some aspects such a gear-like mechanism comprising teeth can have any number of teeth unless stated otherwise, to facilitate its function, such as about two teeth, about five teeth, about ten teeth, about 15 teeth, about 20 teeth, about 40 teeth, about 60 teeth, about 80 teeth, or about 100 teeth, such as between about two - about 100 teeth, between about five - about 80 teeth, between about 10 - about 60 teeth, or for example between about ten and about 40 teeth, such as between approximately ten and about 30 teeth. In some aspects, the teeth can be evenly distributed about the circumference of the engaging element, e.g., the gear-like element. In some aspects, the teeth may not be evenly distributed. In some aspects, there may be gaps or variations in the distance between any one tooth and the next tooth. In some aspects, this creates a mutilated gear. Similarly, in some aspects, a gear can comprise teeth that are intentionally unable to engage with the corresponding engagement element of the device otherwise in movably engageable communication (e.g., via other engageable teeth of the same mechanism); therein providing another example of a mutilated gear.

The driver of the present invention according to certain embodiments can comprise a means of rotation such as a bearing, a positioning unit for positioning the driver such as a shaft, engagement units such as teeth capable of being in movable engagement with another one or more components of the dispensing mechanism, and an element capable of engaging with the medicament access body cover or lever.

According to certain aspects, which can be combined with any other aspect or embodiment or combination of aspects or embodiments described herein, the driver of the present invention can be suitably rotated. In some aspects, the driver rotates within a bearing, and the rotation occurs about a post, tube, or similar shaft. According to certain aspects, the bearing within which the driver rotates enables the driver gear to rotate about a stud of the inner housing assembly. Such a bearing and shaft can facilitate both the rotation and the positioning of the driver.

According to certain aspects, which can be combined with any other aspect or embodiment or combination of aspects or embodiments described herein, the driver gear of the present invention comprises i. primary driver wheel, ii. a spring loaded ratcheting disc, iii. a secondary driver gear and iv. torsion spring.

According to certain aspects, which can be combined with any other aspect or embodiment or combination of aspects or embodiments described herein, the driver gear of the present invention comprises i. primary driver wheel, ii. a spring loaded ratcheting disc, iii. a secondary driver gear and iv. torsion spring, wherein the primary driver wheel, a spring loaded ratcheting disc, a secondary driver gear and torsion spring are arranged coaxially.

According to certain aspects, which can be combined with any other aspect or embodiment or combination of aspects or embodiments described herein, the driver gear of the present invention comprises i. primary driver wheel, ii. a spring loaded ratcheting disc, iii. a secondary driver gear and iv. torsion spring, wherein the primary driver wheel is driven directly or indirectly by medicament access body cover.

According to certain aspects, which can be combined with any other aspect or embodiment or combination of aspects or embodiments described herein, the driver gear of the present invention comprises i. primary driver wheel, ii. a spring loaded ratcheting disc, iii. a secondary driver gear and iv. torsion spring, wherein the primary driver wheel is driven directly or indirectly by lever.

According to certain aspects, which can be combined with any other aspect or embodiment or combination of aspects or embodiments described herein, the driver gear of the present invention comprises i. primary driver wheel, ii. a spring loaded ratcheting disc, iii. a secondary driver gear and iv. torsion spring, wherein primary driver wheel may optionally include central ratchet, Gear tooth and locking arm, wherein the primary driver wheel is driven by the outer cover or lever via central ratchet and wherein Gear tooth drives the ratchet during each actuation.

According to certain aspects, which can be combined with any other aspect or embodiment or combination of aspects or embodiments described herein, the driver gear of the present invention comprises i. primary driver wheel, ii. a spring loaded ratcheting disc, iii. a secondary driver gear and iv. torsion spring, wherein a spring loaded ratcheting disc may optionally include ratchet wherein the ratchet is directly or indirectly engaged with secondary driver gear.

According to certain aspects, which can be combined with any other aspect or embodiment or aspects or embodiments described herein, the driver gear of the present invention comprises i. primary driver wheel, ii. a spring loaded ratcheting disc, iii. a secondary driver gear and iv. torsion spring, wherein secondary driver gear may include groove, driver gear tooth and inner gear teeth, wherein inner gear teeth include teeth arranged circumferentially on the body of the driver, which are uniformly distributed around the circumference of the gear.

According to certain aspects, which can be combined with any other aspect or embodiment or combination of aspects or embodiments described herein, the driver gear of the present invention comprises i. primary driver wheel, ii. a spring loaded ratcheting disc, iii. a secondary driver gear and iv. torsion spring, wherein the locking arm on the primary driver wheel locks within the grove of secondary wheel.

According to certain aspects, which can be combined with any other aspect or embodiment or combination of aspects or embodiments described herein, the driver gear of the present invention comprises i. primary driver wheel, ii. a spring loaded ratcheting disc, iii. a secondary driver gear and iv. torsion spring, wherein the driver gear is engaged with only one carrier engagement at a time.

According to certain aspects, which can be combined with any other aspect or embodiment or combination of aspects or embodiments described herein, the driver gear of the present invention comprises i. primary driver wheel, ii. a spring loaded ratcheting disc, iii. a secondary driver gear and iv. torsion spring, wherein, after completion of all doses in first carrier, driver gear tooth engages directly or indirectly with the second carrier thereby advancing second advancer for remaining doses.

According to certain aspects, which can be combined with any other aspect or embodiment or combination of aspects or embodiments described herein, driver gear tooth once engages directly or indirectly with the second carrier, driver gear assembly may able to rotate only one or more than one carriers during every actuation.

According to certain aspects, which can be combined with any other aspect or embodiment or combination of aspects or embodiments described herein, the first elongate form medicament carrier can be any elongate form medicament carrier that houses a plurality of discrete doses of medicament and wherein after completion of all the doses from a medicament carrier, it automatically switches to dispense medicament from a second elongate form medicament carrier and so on.

According to certain embodiments, the driver can be a gear having engagement units of a typical gear, such as teeth for engaging other components. In some aspects, teeth of a driver can be arranged in an annular fashion at or about the circumference of the driver. In one aspect, the driver can include teeth arranged circumferentially on the body of the driver, which are uniformly distributed around the circumference of the gear. In some aspects, a driver can comprise a number of such teeth, which typically is less than about 50 teeth, such as less than about 30 teeth, less than about 25 teeth or less, such as less than about 20 teeth, less than about 15 teeth, or less than about ten teeth (e.g., 5-50 teeth, 10-50 teeth, 10-40 teeth, or 10-30 teeth).

In certain alternative aspects, which can be combined with any other aspect or embodiment or combination of aspects or embodiments described herein, the driver can include engagement units such as teeth that are non-uniformly distributed about the driver. In certain aspects, such engagement units can be sets, or groups, of two or more teeth each set being arranged diametrically opposite to each other about the circumference of the driver. In another aspect, the driver can comprise a single tooth each arranged on diametrically opposite sides of the circumference of the driver. In some aspects, the driver is a mutilated gear, a mutilated gear being a movably engageable gear wherein the gear comprises areas of the circumference of the gear having engageable teeth while also comprising areas of the circumference of the gear having either no teeth or non-engageable teeth. Accordingly, in certain aspects of the present invention, the driver is capable of being positioned such that it is engageable with one or more other components of the dispenser; however, can also be positioned such that no engageable element of the driver is available and hence that area of the driver is non-engageable with another component of the dispenser. Such engagement and non-engagement of the driver can occur simultaneously such that the driver can be movably engaged with another one or more components of the dispenser via engageable units present on the driver in one area of the circumference of the driver while being unable to engage with another one or more components adjacent the gear due to the lack of present engagement units such as teeth on the driver gear in the area of circumference of the driver adjacent such a component.

According to certain aspects of the present invention, the driver is a mutilated driver gear such that not every movable engagement between the driver and a separate component of the dispenser must result in the movement of both the driver and the component with which it is engaged. In some aspects, it is an advancer that is intermittently in movable engagement with the mutilated gear. In some aspects, the design of the mutilated gear is such that it allows for idle motion of the driver prior to engagement of any other component. In certain embodiments, the design of the mutilated gear is such that it allows both for idle motion of driver prior to engagement of any other component and further allows for a sequential engagement of multiple other components. That is, according to certain embodiments, the driver is a mutilated gear which is capable of first experiencing an idle motion, then engaging with a first component, and upon the completion of engagement with the first component, is capable if then engaging with a second component, such that movement of the first component with which the driver engages is substantially complete prior to the start of movement of the second component.

The driver can be arranged to rotate about a first axis of rotation. In one aspect, the dispenser can have a unique driver for each medicament carrier present in the dispenser. In a common aspect, which can be combined with any other aspect or embodiment or combination of aspects or embodiments described herein, a single driver is common for each of the medicament carriers present within the dispenser; that is, movement of all present medicament carriers within the dispenser is driven by movement of the single driver. Such energy of motion from the driver can either be received directly or indirectly by each medicament carrier.

In certain aspects, the driver is engaged with the cover of the medicament access body such that the driver can be driven by a movement of the cover of the medicament access body. According to certain embodiments, which can be combined with any other aspect or embodiment or combination of aspects or embodiments described herein, the driver can comprise an element capable of engaging with the medicament access body cover. In some aspects, the engageable element can be a hook, clip, or other similar type fasteners. In some embodiments, the engageable element can be a slot, hole, indentation, protrusion, clip, snap, or other elements with which a correspondingly shaped element of a medicament access body cover element could engage. In some aspects, the driver can have an element similar to a ring, keyhole, or the like. In some aspects, the driver can comprise a flange which is designed to engage a complementarily designed flange key positioned on the mouthpiece cover. According to certain embodiments, which can be combined with any other aspect or embodiment or combination of aspects or embodiments described herein, the element of the driver which can engage with an element of the medicament access body cover to allow their cooperation is a flange.

In certain aspects, the driver is engaged with the lever such that the driver can be driven by a movement of the lever. According to certain embodiments, which can be combined with any other aspect or embodiment or combination of aspects or embodiments described herein, the driver can comprise an element capable of engaging with the lever. In some aspects, the engageable element can be a hook, clip, or other similar type fasteners. In some embodiments, the engageable element can be a slot, hole, indentation, protrusion, clip, snap, or other elements with which a correspondingly shaped element of a lever element could engage.

In certain aspects wherein the medicament access body cover is engaged with the driver and the driver is further engaged with at least one advancer, during the movement of the cover of the medicament access body the movement of the driver may be idle movement and thereby may not rotate the advancer. This aspect of the operation of some dispensers of the invention will be described further herein with reference to the exemplary figures provided herewith. In other words, in certain aspects, the driver may be intermittently in movable engagement with an advancer. In one aspect, which can be combined with any other aspect, embodiment, or combination of aspects or embodiments described herein, the idle movement of the driver can be in the range of about 5 degrees to about 50 degrees. In another aspect, the idle movement of the driver may be in the range of about 10 degrees to about 25 degrees (e.g., about 10-20 degrees, about 15-25 degrees, or about 12-20 degrees), such as about 10 degrees, about 12 degrees, about 14 degrees, about 16 degrees, about 18 degrees, about 20 degrees, about 22 degrees, or about 24 degrees, such as about 11 degrees, about 13 degrees, about 15 degrees, about 17 degrees, about 19 degrees, about 21 degrees, about 23 degrees, or about 25 degrees.

In some aspects, such idle movement of the driver allows a user of the dispenser to, for example, move the medicament access body cover to a limited extent allowing the user to expose the medicament access body (e.g., a mouthpiece) to wash it, without actuating the dispensing mechanism within the dispenser. In some aspects, the idle movement decreases the likelihood of the medicament dispenser being actuated by accident, such as by inadvertently being partially opened in the pocket or personal item such as a purse or backpack of a user. According to some aspects of the invention, there are three primary positions of the medicament access body cover: a first position (e.g., a rest or fully closed position), a second, and a third position, the third position being a fully opened position. In certain aspects, movement between the first and second position is idle motion; no actuation of the device occurs. In some aspects, movement from the second to the third position causes actuation. In some aspects, there is no exposure of the medicament access body until idle movement is complete. In certain embodiments, there is no uncovering of the medicament access body prior to the medicament access body cover reaching the second position upon which actuation of the device begins.

In some aspects, a mechanism is present to engage a medicament carrier to aid in its advancement. Such a carrier engagement can be provided for advancing the distinct medicament dose portions of the medicament carrier. According to one aspect of the disclosure, the invention relates to a carrier engagement comprising: a plurality of areas of the carrier engagement capable of engaging with the dose portion storage area of the elongated medicament carrier, such as the blister of a blister strip-form elongated medicament carrier, to aid in the movement of the medicament carrier, such areas of the carrier engagement commonly referred to herein as "cuvettes"; an engagement element capable of movingly engaging with another component of the dispenser to impart an energy of motion upon that component, such as, for example, a waste collector; and an engagement element capable of movingly engaging with a component of the dispensing mechanism capable of imparting an energy of motion to the carrier engagement, such as an advancer.

In some aspects, the carrier engagement can take any form capable of receiving an energy of motion to cause movement of the carrier engagement and, in turn, transfer such energy of motion to another one or more components of the dispenser. In some aspects, the carrier engagement can be a rotatable mechanism such as a hub, wheel, thimble- or cup-shaped element having an interior and an exterior, or the like. In some aspects, the carrier engagement is a thimble- or cup-shaped element having an interior and an exterior wherein, the element of the carrier engagement capable of engaging with the dose portion storage area of the elongated medicament carrier, e.g., such as a blister of a blister strip, is positioned on the exterior of the carrier engagement, and further wherein the element of the carrier engagement capable of receiving an energy of motion from another component of the dispensing mechanism, such as an advancer, is positioned on the interior of the carrier engagement.

According to certain embodiments, the carrier engagement can comprise a central hole in addition to the engagement elements described. Such a central hole can, in certain aspects, receive a part of the component conveying an energy of motion to the engagement carrier to appropriately position the two components relative to one another such that the energy of motion can be successfully and efficiently transferred between the two components.

According to certain aspects, which can be combined with any other aspect or embodiment, or combination of aspects or embodiments described herein, the element of the carrier engagement capable of engaging with a further component of the dispenser to impart an energy of motion upon that component (such a component being, for example, a waste collector), can be any element capable of forming a movable engagement, such as a magnet, a reversible clip mechanism, or a mechanism capable of forming an temporary mechanical connection to such another component, such as by means of the meshing or interfacing of protrusions or indentations between the two components, as can be the case wherein the carrier engagement component imparting energy is a gear having protrusions in the form of gear teeth. In such an embodiment, the element of the carrier engagement capable of engaging with a further component of the dispenser to impart an energy of motion upon that component is a gear having gear teeth.

In some aspects, the element of the carrier engagement capable of receiving an energy of motion from another component of the mechanism can be any element capable of being movingly engaged with the component imparting the energy, such as a gear mechanism, a magnet, a reversible clipping mechanism, or the like. In certain aspects, the element is a set of protrusions, e.g., a set of teeth, which can interface with one or more elements of the component imparting energy to the carrier engagement, such as a driven unit of an advancer, as will be described further herein. In some aspects, which can be combined with any other one or more aspects or embodiments, or combinations of aspects or embodiments described herein, the element of the carrier engagement capable of receiving an energy of motion from another component of the mechanism such as an advancer can be a set of ratchet teeth. In some aspects, the carrier engagement receives an energy of motion from an interim driver.

In some aspects, ratchet teeth of a carrier engagement are arranged such that they are capable of receiving energy from another component of the mechanism, such as an advancer or an interim driver, in such a manner that the carrier engagement is capable of rotating in a single direction. That is, in some aspects, an energy of motion is received by the carrier engagement is a rotational motion, and the rotational motion is in one direction, the carrier engagement is correspondingly rotated in that direction. Also or alternatively, if the energy of motion received by the carrier engagement is a rotational motion and the rotational motion is from a second direction, the ratchet teeth of the carrier engagement allow for the interfacing member of the component imparting the energy of movement to the carrier engagement, e.g., the advancer or interim driver, to slip past the ratchet teeth such that no energy of movement is imparted and the carrier engagement does not rotate.

According to certain embodiments, which can be combined with any other aspect or embodiment, or combination of aspects or embodiments, the element of the carrier engagement movably engaged with a component of the dispenser imparting energy of motion to the carrier engagement, e.g., ratchet teeth, can be positioned within the inner part, or interior, of the carrier engagement. In some aspects, the ratchet teeth can include a contacting surface. In some aspects, the ratchet teeth can comprise a non-contacting, or slipping surface, with which the corresponding interfacing element of the component of the dispenser imparting energy on the carrier engagement, e.g., an element on a driven unit of an advancer cannot sufficiently engage to impart an energy of motion.

According to certain embodiments, the dispensing mechanism can comprise a carrier engagement for each medicament carrier for individually advancing the distinct medicament dose portions thereof. In one aspect, the dispensing mechanism can comprise plural carrier engagements for advancing plural medicament carriers individually. In another aspect, the mechanism can comprise two carrier engagements for advancing two medicament carriers individually. In some aspects, the two carrier engagements can be positioned side by side (i.e., adjacent or close to one another) and/or the two carriers can be positioned within the same plane. According to certain embodiments, the two carrier engagements are distinct and can be decoupled from one another. In certain aspects, the advancement of the carrier engagement can occur when the cover of the medicament access body is moved. In certain aspects, the advancement of the carrier engagement can occur when the lever is moved. According to some aspects, the movement of the medicament access body cover or lever must be sufficiently moved to result in movement of a carrier engagement, such that limited movement of the medicament access body cover or lever does not result in movement of a carrier engagement. In some aspects, the advancement of each carrier engagement can happen in a substantially synchronous fashion. That is the advancement of each carrier engagement can happen at about the same time; as in, in some aspects, plural carrier engagements begin their rotation at substantially the same time. In some aspects, plural carrier engagements do not begin their rotation at substantially the same time. In one aspect, the two carrier engagements can be advanced non-synchronously. According to some aspects, the two carrier engagements can be advanced successively (i.e., one after the other). According to some aspects, the two carrier engagements do not begin their rotation at the same time. According to some aspects, the only one carrier engagements is movable at a time. According to some aspects, the two carrier engagements can be advanced substantially at the same time. According to some aspects, the only one carrier engagements is movable at a time till all the doses within the medicament carrier are indexed. According to some aspects, the second carrier engagement is substantially at stationary position while first carrier engagement is in the movement. According to some aspects, the second carrier engagement is indexed after all the doses within the first medicament carrier are indexed. In one aspect, a consecutive or sequential movement of a plurality of advancers, and also or alternatively the consecutive or sequential movement of a plurality of carrier engagements is an advantage of the present system over the prior art. In one aspect, the non-synchronous movement of a plurality of advancers, and also or alternatively the non-synchronous movement of a plurality of carrier engagements is an advantage of the present system over the prior art. According to certain aspects, a plurality of carrier engagements can be advanced non-synchronously, such that there is substantially no overlap or there is no detectable overlap in movement between any two or more carrier engagements in operation. In certain aspects, a plurality of carrier engagements move, e.g., rotate, successively; that is, one begins and completes its motion prior to any second carrier engagement beginning and completing its motion. In certain aspects, one carrier engagement can be in motion, advance a medicament carrier to the flow director, make a medicament dose available for administration, and complete its movement (e.g., complete its rotation), prior to a second carrier engagement beginning its movement, e.g., beginning its rotation and advancement of its associated medicament carrier. In certain embodiments, there is no overlap of motion, e.g., rotation, between the two carrier engagements. In some aspects, regardless of when a plurality of carrier engagements begin rotation and/or if the plural carrier engagements are decoupled from each other, the dispensing mechanism can make the medicament within a dose storage unit of each of a plurality of medicament carriers available for administration at substantially the same time. In certain alternative aspects, the medicament within a dose storage unit of each of a plurality of medicament carriers is made available for administration sequentially, that is, one becomes available (e.g., is opened) before another becomes available (e.g., is opened).

According to certain embodiments, which can be combined with any other aspect or embodiment, or combination of aspects or embodiments described herein, the carrier engagements can begin to rotate at different times and rotate at differing speeds. According to certain aspects, the carrier engagements advance each respective carrier at a different rate however provide a dose of medicament held within each respective medicament carrier, for example completely expose respective dose storage areas within each medicament carrier, for example completely open blister pockets of blister strip medicament carriers, at the same time. According to certain aspects, the carrier engagements advance each respective carrier at a different rate and provide a dose of medicament held within each respective medicament carrier, for example completely expose respective dose storage areas within each medicament carrier, for example completely open blister pockets of blister strip medicament carriers, at different times. In some aspects, the dose storage areas of a plurality of medicament carriers are exposed in sequence, such as, for example, the dose storage area of a first medicament carrier is exposed prior to the exposer of a dose storage area from a second medicament carrier. In certain aspects, regardless of the synchronicity of disparity of the timing of the exposure of doses from a plurality of medicament carriers, the medicaments can be available for providing to the user in a single administration, such as for example via a single inhalation. In some embodiments, the exposure of the medicament doses from a plurality of medicament carriers advanced into position by respective carrier engagements, e.g., the "opening" of the dose storage areas, can occur at a common "opening location," or a position within the dispenser wherein the medicament becomes available for administration.

According to some embodiments, a plurality of carrier engagements can rotate in a substantially synchronous fashion in at least some rotations. In an alternative embodiment, two or more carrier engagements, e.g., a plurality of carrier engagements, do not rotate in a substantially synchronous fashion in at least some rotations.

In some aspects, a plurality of carrier engagements are decoupled from one another.

In some aspects, the potential variation in the time of rotation initiation and rotation speeds of carrier engagements can support the accommodation of the use of a plurality of medicament carriers which are not the same as one another. In some aspects, one medicament carrier may comprise a medicament having a different administration or dosing schedule as another. In some aspects, it may be preferable not to have a dose from a particular carrier become available upon every actuation of the dispenser such that the user does not receive a dose from every medicament carrier upon every actuation of the dispenser. In some aspects, a variation in the time of rotation initiation and rotation speeds of carrier engagements can support the accommodation of medicament carriers comprising a difference in pitch, or spacing, between medicament doses.

According to certain embodiments, which can be combined with any other aspect or embodiment or combination of aspects or embodiments described herein, the carrier engagement comprises two or more elements capable of engaging with the dose portion storage area of the elongated medicament carrier, such as a blister of a blister strip-form elongated medicament carrier, to aid in the movement of the medicament carrier. Such elements can be provided for locating each dose storage area of the medicament carrier (e.g., blister pockets of a blister strip medicament carrier) sequentially during the operation of the dispenser. According to certain aspects, the dose portion engagement element of the carrier engagement is an indentation, a protrusion, a hook or scoop-shaped element, a fin, a ridge, or any similar or equivalently shaped element capable of engaging a dose storage area of a medicament carrier. In some aspects, the element is a series of carved out areas that circumferentially encircle the outer face of the carrier engagement. Such a "carved out" area, e.g., an area where material has been removed or not filled in by molding or the like to have created an indentation, is designed to have a complementary shape to the dose storage area, e.g., blister of a blister strip, to be suitable for engaging with the dose storage area to aid in its advancement and positioning. In some aspects, such areas of the carrier engagement are referred to herein as "cuvettes."

In some aspects, the cuvettes can not only participate in the advancement of the medicament carrier, but a cuvette can also aid in aligning the dose storage area of a medicament carrier, e.g., a blister pocket of a blister strip, with the flow director, to be described further elsewhere herein, when the cover of the medicament access body is rotated. The carrier engagement can include more than one equally spaced cuvette around its circumference. In one aspect, the carrier engagement can include more than two equally spaced cuvettes about its circumference, such as about two or more, about three or more, about four or more, about five or more, about six or more, about seven or more, about eight or more, about nine or more, about ten or more, or even about 12 or more, such as about 14 or more, about 16 or more, about 18 or more, or about 20 or more equally spaced cuvettes.

In some aspects, each carrier engagement comprises the same number of cuvettes about its circumference of each other carrier engagement within the dispenser. In some aspects, at least one carrier engagement can comprise a different number of cuvettes about its circumference than that of at least one other carrier engagement within the dispenser. In one aspect, a carrier engagement can include between about four to about 10 eight equally spaced cuvettes about its circumference. One embodiment, the dispenser comprises two carrier engagements, each having between about six and about ten cuvettes about its circumference, such as about eight cuvettes about its circumference. In some aspects, the two carrier engagements have the same number of cuvettes about their circumference.

According to certain embodiments, which can be combined with any other aspect or embodiment or combination of aspects or embodiments described herein, a carrier engagement can comprise more than one equally spaced ratchet tooth (teeth), a ratchet tooth being an element of an exemplary mechanism, e.g., a ratcheting mechanism, by which the carrier engagement receives an energy of motion from another component of the dispenser mechanism, such as from an advancer. According to certain aspects, such a mechanism can comprise at least one, such as at least about one, at least about two, at least about three, at least about 4, at least about 5, at least about 6, at least about 7, at least about 8, at least about 9, or at least about 10, such as at least about 12, at least about 14, at least about 16, at least about 18, or at least about 20 ratchet teeth (e.g., about 5-25 ratchet teeth, about 10-30 ratchet teeth, or about 7-21 ratchet teeth such as about 5-20 ratchet teeth or 5-15 ratchet teeth). In one aspect, the carrier engagement can be driven by, that is, receive an energy of motion from, resulting in movement of the carrier engagement, an advancer. A further description of an advancer is provided elsewhere herein. According to certain alternative embodiments, which can, in some aspects be present within the same device, a carrier engagement can be drive by, that is, receive an energy of motion from, resulting in movement of the carrier engagement, an interim mechanism such as an interim driver. In some embodiments, which can be combined with any other aspect or embodiment or combination of aspects or embodiments described herein, the carrier engagement can receive the energy of motion via a set non-ratchet teeth. In some aspects, an interim driver can comprise a ratcheting element and a non-ratcheting element, such that the ratcheting element movingly engages with an advancer and the non-ratcheting element engages with the carrier engagement.

In one aspect, a carrier engagement is driven directly by an advancer. In one aspect, the advancer is driven directly by the driver. In one aspect, the carrier engagement is indirectly engaged with an advancer via an interim mechanism, such as an interim driver, which, in some embodiments, receives its energy of motion from an advancer, which further in some aspects receives its energy of motion from a driver. In an alternative embodiment, the advancer is driven by an interim mechanism, such as an interim driver, which receives its energy of motion from the driver. In some aspects, when a plurality of carrier engagements are present, one or more carrier engagements can be driven by one or more advancers. In certain embodiments, one or more advancers can directly drive a carrier engagement or can indirectly drive a carrier engagement via an interim mechanism such as an interim driver. In some aspects, when a plurality of carrier engagements are present, one or more carrier engagements can be driven directly by one or more advancers; however one or more carrier engagements also or alternatively can be driven directly by an interim driver which receives its energy of motion from an advancer. Such an arrangement can be present within the same dispensing device. According to certain aspects, use of an interim driver provides for the ability for the dispensing mechanism to utilize a single driver to drive movement of a plurality of carrier engagements; however, each of the carrier engagements can be made to rotate in opposite directions. That is, a single driver can impart energy of motion to separate advancers; one such advancer can, in some aspects, directly engage with a carrier engagement, while a second such advancer can, in some aspects, engage with an interim driver which then engages a second carrier engagement. In one aspect, the carrier engagements being driven by separate mechanisms and having received their energy of motion from the same original source provide a means for utilizing a single original source of energy, e.g., a single driver, while permitting multiple carrier engagement mechanisms to receive their energy of motion in a manner that they each are made to move in a different way, for example they can be made to rotate in opposite directions.

According to certain aspects of the present invention, movement of the cover of the medicament access body can result in movement of the driver, movement of a plurality of advancers, and movement of a plurality of carrier engagements, and thereby advances a plurality of medicament carriers to align with the flow director for dispensing the medicament held within a dose storage area if present of each medicament carrier. The term "if present" is used to denote embodiments where a plurality of medicament carriers are held within the dispenser, but each has a different dosing schedule. In such scenarios, the pitch or spacing between the dose storage areas of one medicament carrier can be larger than that of another carrier. In such scenarios, it may be possible to advance one medicament carrier such that upon actuation, a dose storage area is opened or otherwise made available and positioned to align with the flow director for dispensation; while it may also be possible to advance another medicament carrier such that upon actuation, a blank area (an area of a medicament having no dose storage area; that is, a space between doses) is delivered/positioned to align with the flow director and hence no medicament from that carrier is administered. Such a configuration/operation can be advantageous when the dosing schedules between two medicaments vary.

An advancer can be provided for rotating a carrier engagement. According to certain embodiments, which can be combined with any other aspect or embodiment, or a combination of aspects or embodiments described herein, an advancer can be provided for each carrier engagement within the dispenser. In some aspects, therefore, each carrier engagement is separately driven, e.g., separately rotated by an individual advancer. In some aspects, an advancer can be driven by, e.g., can receive an energy of motion from the driver. In some aspects, an advancer can be driven by, e.g., can receive an energy of motion from, an interim driver, the interim driver receiving an energy of motion from the driver. In some aspects, use of an interim driver when two or more advancers are present, whether a) being driven by a driver and acting upon a carrier engagement via an advancer or b) being driven by an advancer which is driven by a driver and acting directly on a carrier engagement, allows for a plurality of carrier engagements to source their energy of motion from the same origination point (e.g., from a single driver), yet to rotate in opposite directions.

In one aspect of the present invention, the advancer is a single component. According to certain aspects, which can be combined with any aspect or embodiment, or combination of aspects or embodiments described herein, the single component advancer can comprise a ratcheting element to engage with one component of the dispenser and a set of non-ratcheting engagement element to engage with a second component of the system.

In some aspects, such a ratcheting element can be a set of protrusions or extensions that can be capable of interfacing with a corresponding element of another system component. In some aspects, the ratcheting element is a set of ratchet arms. In some aspects, the non-ratcheting engagement element can comprise a set of indentations or protrusions or other similar or equivalent engagement means capable of engaging with a separate corresponding element of another system component. In some aspects, the non-ratcheting element is a set of teeth.

According to an alternative embodiment, which can be combined with any aspect or embodiment, or combination of aspects or embodiments described herein, the advancer is comprised of two or more distinctly separate elements which operate together to form an assembly, an assembly being the combination of the elements making up the advancer. In some aspects, as will be described further, one such element can be an element that engages with a carrier engagement set of ratchet teeth; hence in some aspects, the advancer is sometimes referred to herein as a ratchet assembly.

According to certain aspects of the present invention, which can be combined with any aspect or embodiment, or combination of aspects or embodiments described herein, the advancer comprises a driving member, such a driving member being capable of being driven by, e.g., receiving a driving energy or energy of motion from, another component of a device, e.g., a driver or an interim driver. According to certain aspects, the advancer further comprises a driven member capable of being driven by, e.g., receiving an energy of motion from, the driving member of the advancer. In some aspects, the driving member of the advancer and the driven member of the advancer are movably engaged with one another.

In some aspects, the advancer comprises an element positioned between the driving member and driven member of the advancer capable of allowing axial displacement of the driven member during movable engagement (e.g., ratcheting motion which will be described further elsewhere herein), thereby preventing any axial (e.g., vertical) motion of the driving member. In some aspects, such an element can be any element capable of preventing such a motion, while retaining necessary tension/engagement in a similarly functional manner such as, for example, another type of tensioning element, e.g., an expandable/compressible member or component (e.g., a bellows, such as a metal bellows, or a compressible joint). In some aspects, the element is a spring, which can be any suitable type of spring, such as a compression spring. According to certain embodiments, the spring provides a means for the driven and driving member to maintain contact with one another however it further allows for the driving member and the driven member to be axially (e.g., vertically) displaceable relative to one another during ratcheting such that some movement of the driving member can result in movement of the driven member, however alternative movement of the driving member does not result in movement of the driven member. Such a relationship will be described further elsewhere herein.

According to certain embodiments, the driving member of the advancer can comprise an element for coaxially aligning the driving member with the driven member as well as to maintain the positioning of the driving member and the driven member in a relatively consistent position with one another. In some embodiments, which can be combined with any other aspect or embodiment, or combination of aspects or embodiments described herein, such an element can be any element capable of maintaining such a position between the two elements, such as a magnet, a clip, a lock, a pin, or the like. According to certain aspects, the element is a locking pin. According to certain aspects, the locking pin of the driving member is positioned within the driven member such that the driven member sits on top of, or about, the driving member, e.g., the driving member sits within the driven member.

According to certain aspects, the driving member can further comprise an element for interfacing with the driven member to impart a driving energy, such as an energy of motion, from the driving member to the driven member. In some aspects, such an element can also aid in the alignment between the driving member and the driven member to support the efficient and successful operation of the two components as they relate to one another. In some aspects, which can be combined with any other aspect or embodiment, or combination of aspects or embodiments described herein, such an element can be any element capable of imparting such an energy of motion, such as a protrusion, an indentation, a post, a pin, a structure or feature or a series of structures or features having a face, having a flat or curved shape or geometric shape, for interfacing with the driven member, or the like. In some aspects, the driving member comprises a series of curved faces which correspond with a series of oppositely curved faces of the driven member, the curved faces of the driving member being designed to nest within or about the curved faces of the driven member. The use of the term "pocket" or "driving pocket" can be used herein to describe such an inwardly curved surface or series of surfaces of the driving member. Because such a driving pocket or pocket can be used in part to align the driving member with the driven member, e.g., via the nesting within or about the corresponding feature of the driven member, in addition to providing a surface by which a driving force is passed from the driving member to the driven member, this element is can be referred to herein as an alignment pocket. Such elements aiding in alignment with, and driving of, the driven member of the advancer can be circumferentially arranged to be capable of transferring a rotational motion to the driven unit. While a series of indentations (or pockets) are provided here as an exemplary embodiment, it should be understood that multiple mechanisms would be capable of providing alignment and driving engagement interface between the driving and driven members of the advancer described herein, and this embodiment is not intended to be limiting. The interface between the driving member and the driven member of the advancer, and the design of such interface, is discussed further elsewhere herein.

In yet another aspect, the driving member of the advancer can further comprise an element capable of being movably engaged with a component of the dispenser capable of imparting a driving force, e.g., an energy of motion, to the driving member to result in movement of the driving member. Such an element can be any element capable of being engaged with a component providing a driving force, such as, for example, a conveyor, lever, switch, wheel or the like. In certain aspects, the element of the driving member of the advancer capable of receiving an energy of motion from another element can be in the form of a set of engagement units capable of engaging with corresponding engagement units of one or more other components. Such engagement units, in some aspects, can be protrusions or indentations of any shape or size. In some aspects, the engagement units are teeth, and the component of the driving member of the advancer capable of being in movable engagement with another component of the dispenser such that the other component of the dispenser can impart a driving energy to the driving member is a set of gear teeth.

According to certain embodiments, which can be combined with any other aspect or embodiment, or combination of aspects or embodiments described herein, the driving member of the advancer comprises a set of gear teeth capable of being in movable engagement with a driver, an interim driver, or both a driver and an interim driver. In some aspects, the driving member of the advancer is driven by, e.g., receives an energy of motion from, a driver, either directly from the driver or indirectly via an interim driver.

In some aspects, each advancer engages with a separate carrier engagement when a plurality of carrier engagements are present such that the movement of each advancer results in rotation of a separate carrier engagement. In some aspects, an advancer engages with a carrier engagement directly. In some aspects, an advancer engages with a carrier engagement indirectly via an interim driver. According to certain aspects, a plurality of advancers may be present within the same dispensing device. In such aspects, each of the plurality of advancers may have the same or similar functional design as any one or more other advancers present within the device, or, alternatively, any one advancer may have a different functional design as any one or more other advancers present within the device.

According to certain embodiments, which can be combined with any other aspect or embodiment, or combination of aspects or embodiments described herein, the driven member of the advancer can comprise an element responsible for aiding in the positioning of the driven member within a carrier engagement. According to certain embodiments, the element can further serve as a feature of the driven member about which the carrier engagement rotates. In some aspects, the driven member of the advancer is positioned within a carrier engagement, the driving member of the advancer further positioned within the driven member. According to some aspects, which can be combined with any other aspects, embodiments, or combinations of aspects or embodiments described herein, such an element capable of aiding in the positioning of the driven member and further serving as a feature of the driven member about which the carrier engagement can rotate can be any feature capable of providing such functionality, such as but not limited to a post, a tube, a boss, a ring or the like. In some aspects, the element is positioned centrally within the driving member. According to certain aspects, the element is a central boss.

According to certain embodiments, the driven member of the advancer and further comprise an element capable of engaging with a carrier engagement such that when the element is in motion, it is capable of imparting such motion as a driving motion to the carrier engagement. As described previously, according to a certain aspect, the element of the carrier engagement capable of receiving such a driving force, e.g., such an energy of motion, can be a set of ratchet teeth. In some aspects, the element of the driven member capable of engaging with the ratchet teeth of the carrier engagement is any element having a shape or configuration capable of engaging with the ratchet teeth of the carrier engagement in such a manner that when addressing the ratchet teeth of the carrier engagement with a motion of one direction, e.g., a rotational movement in one direction, the element engages with the ratchet teeth resulting in rotation of the carrier engagement to advance a medicament carrier; however the shape is such that when addressing the ratchet teeth of the carrier engagement with a motion of the opposite direction, e.g., a rotational movement in the opposite direction, the element does not engage with the ratchet teeth, instead slips or slides or otherwise passes over the ratchet teeth of the carrier engagement such that the carrier engagement does not rotate sufficiently to advance a medicament carrier. In some aspects, such an element can be a protrusion, an indentation, a post, a fin, a paddle, an extension, an arm, a lever, pin, a clip, sets of two or more of any such elements, or the like. Any such elements can, in some aspects, have a straight orientation or be curved or bent. According to certain embodiments, the element is a pin and is referred to herein as a drive pin. In some aspects, the drive pin has a straight orientation. In alternative embodiments, the drive pin is a set of bent arms; however both designs and other similar designs intended to be exemplary and not limiting to or of any similar or equivalent design which could be used to transfer energy of motion from the driven member of the advancer to a carrier engagement. The drive pin of the driven member of the advancer is an element which, is capable of engaging with an element of the carrier engagement capable of being driven by, or receiving an energy of motion from, the advancer, when the driven member and accordingly the drive pin is moving in a first direction; however is capable of not engaging with the element of the carrier engagement capable of being driven by the advancer when the drive pin is moving in a second direction.

According to certain embodiments of the present invention, which can be combined with any other aspect or embodiment, or combination of aspects or embodiments described herein, the driven member of the advancer can comprise an element capable interfacing with an element of the driving member of the advancer such that the element aids in the optimal alignment of the two components relative to one another and further is capable of receiving a driving energy from, e.g., an energy of motion from, the driving member of the advancer. According to certain embodiments, the element can be any element capable of receiving such an energy of motion, such as a protrusion, an indentation, a post, a pin, a structure or feature or a series of structures or features having a face, having a flat or curved shape or geometric shape, or the like for interfacing with the driving member. In some aspects, the driven member comprises a series of curved faces which correspond with a series of oppositely curved faces of the driving member, the curved faces of the driven member being designed to nest within or about the curved faces of the driving member. The use of the term driving pocket or pocket can be used herein to describe such an inwardly curved surface or series of surfaces of the driving member. Such a pocket-like or indented design of the driving element of the driving member of the advancer is simply one embodiment, and it should be well understood that multiple mechanisms for aligning and imparting motion between two elements of the advancer described herein can be envisioned, such as a geared mechanism, a ratcheted mechanism, a spoke and wheel mechanism, a clipped mechanism, and the like. In a particular embodiment where the driving element of the driving member of the advancer is designed as a series of indentations or pocket-like elements, the receiving element of the driving motion by the driven member from the driving member can be elements complementarily shaped to the indentations or pockets, such as correspondingly curved faces or "pocket inserts." Such curved faces can also be circumferentially arranged such that the curved faces of the driven member can be positioned in a complementary and aligned position with the curved faces of the driving member of the advancer such that the two faces are engaged with one another and wherein the rotation of the driving member of the advancer results in the rotation of the driven member of the advancer. Herein, the term "circumferential pins" may be used to describe the energy receiving element of the driven unit, "circumferential" because they can be positioned around the circumference of the driven unit and "pins" because they extend longitudinally about the circumference as is exemplified in provided figures. However, the term "circumferential pins" should be understood to mean any mechanism capable of being engaged with the corresponding interface of the driving element of the driving member of the advancer and receiving the driving force from the driving member of the advancer. Such a nesting relationship between the driving element of the driving member of the advancer and the driven element of the driven member of the advancer as exemplified herein and in provided figures has the advantage of providing both alignment support between the two components as well as energy transfer between the two components.

According to certain embodiments, such embodiments capable of being combined with any other aspect or embodiment, or combination of aspects or embodiments described herein, the energy receiving element of the driven member of the advancer receiving energy from the driving element of the driving member of the advancer, e.g., the circumferentially arranged pins, can be positioned within the inner part of the driving member. In some aspects, the advancer can be arranged within the dispenser to rotate about a second axis of rotation, that is, about an axis of rotation different from that of the driver. In some aspects, at least one advancer is arranged such that it is capable of being movably engaged with the driver. In certain embodiments, the carrier engagement can rotate about the central boss of the advancer, such that a carrier engagement and an advancer share a common axis of rotation. In some aspects, the drive pin can be in ratcheted engagement with ratchet teeth of the carrier engagement such that the rotational movement of the drive pin results in the rotation of the carrier engagement. In one aspect, which can be combined with any other aspect, embodiment, or combination of aspects or embodiments described herein, the dispensing mechanism comprises an advancer for each carrier engagement for rotating thereof. In some aspects, the advancer can directly drive the carrier engagement. In certain aspects, the advancer can indirectly drive the carrier engagement via an interim mechanism such as an interim driver. According to certain embodiments, the advancer can be movably engaged directly with the driver such that the movement of the driver results in the movement of the advancer. In some aspects, the movable engagement is constant; that is, the two elements are in constant contact with one another. In some aspects, the advancer can be movably engaged with an interim driver, which in turn is movably engaged with the driver.

In certain aspects, two carrier engagements are present within the dispenser device. In some aspects, at least one carrier engagement is directly movingly engaged with an advancer. In some aspects, both carrier engagements are directly movingly engaged with an advancer. In certain aspect, at least one carrier engagement is directly movingly engaged with an advancer and at least one other carrier engagement is not directly movingly engaged with an advancer. In some aspects, at least one carrier engagement is directly movingly engaged with an advancer comprised of a single component, the advancer having a mechanism for engaging both a driver, e.g., equally-spaced or non-equally spaced teeth for engaging teeth of a driver, and further comprising a mechanism for engaging the carrier engagement, such as for example ratchet arms capable of engaging corresponding ratchet teeth on the carrier engagement.

In certain other aspects, one or more carrier engagements are engaged with an advancer comprising multiple components, that is an assembly, such that at least one carrier engagement is engaged with the drive pin of the driven member of the advancer, the driven member of each advancer being engaged with the driving element of the advancer. In certain other aspects, two carrier engagements are present within the dispenser device with: a) the first carrier engagement being either i) engaged with the drive pin of the driven member of the advancer, the driven member of the advancer being engaged with the driving element of the advancer, and the driving element of the advancer being engaged with the driver; or ii) engaged with ratchet arms of the advancer, with a non-ratchet engagement element of the advancer being movably engaged with the driver; and b) a second carrier engagement engaged with an interim driving mechanism such as an interim driver, the interim driver being either i) engaged with the drive pin of the driven member of the advancer, the driven member of the advancer being engaged with the driving element of the advancer, the driving element of the advancer being engaged with a driver, or ii) engaged with the ratchet arms of the advancer, wherein the non-ratcheting element of the advancer is movingly engaged with the driver.

According to one aspect of the present invention, which can be combined with any other aspect or embodiment, or combination of aspects or embodiments described herein, the dispensing mechanism can include plural advancers, such advancers either being in the form of a single component or in the form of assemblies, e.g., ratchet assemblies, for driving plural carrier engagements within the dispenser individually. In one exemplary embodiment, the dispensing mechanism can include two advancers for driving two carrier engagements individually. In one aspect, an advancer and a carrier engagement can be positioned in a non-coaxial arrangement. In an alternative aspect, an advancer and a carrier engagement can be positioned in a coaxial manner or in a partially overlapping manner. In one aspect, an advancer and a carrier engagement can be coaxial in orientation, such that the advancer is an assembly of elements, a driving member being one such element and the driving member being positioned within, e.g., at least part of its body positioned inside of, a driven member of the advancer, the driven member and driving member of the advancer having a spring or other suitable compressible/expandable tensioning component positioned therebetween, and further wherein the three components of the advancer assembly (the driving member, the spring or similar means, and the driven member) are positioned within a carrier engagement, that is at least part of the assembly is positioned inside of the carrier engagement, and all components rotate about a common axis or that at least partially overlap with respect to such an orientation. In another aspect, the advancer and the carrier engagement can be positioned away from one another (i.e., non-adjacent to each other).

According to certain embodiments of the present invention, which can be combined with any other one or more aspects or embodiments, or combinations of any aspects or embodiments described herein, each advancer is distinct and is not coupled to any other advancer. In some aspects, a plurality of advancers, for example, two to four advancers or ratchet assemblies, can be positioned side by side (i.e., adjacent or close to) and/or in the same plane as one another. In some aspects, two advancers can be positioned away from one another (i.e., non-adjacent to one another).

In some aspects of the present invention, which can be combined with any other aspect or embodiment, or any combination of aspects or embodiments described herein, the advancement of each advancer can happen in a substantially simultaneous fashion, that is the advancement of each advancer occurs at about the same time. In another aspect, a plurality of advancers can be advanced successively (i.e., one after the other). In yet another aspect, the advancers may be advanced non-synchronously, that is whether or not the rotation of each advancer begins at about the same time, their rotation is asynchronous, e.g., they rotate at different rates, rotate at different speeds, and/or rotate at different periods of time, such periods of time being substantially non-overlapping or only overlapping in part such that there is at least one point in time when they are both in motion and at least one point in time when only one is in motion during a single actuation of a dispenser. In some aspects of the present invention, the advancers begin to rotate at different times and rotate at differing speeds. In some aspects, such an asynchronous movement is an advantage of the present invention over the prior art.

According to certain aspects, which can be combined with any other aspect or embodiment, or combination of aspects or embodiments described herein, idle movement of the advancer can exist. In such an embodiment, there is "play" in, or freedom of movement of, at least to a limited extent, an advancer such that movement of the advancer may not result in rotation of the carrier engagement. According to one embodiment, the idle movement of the advancer can be in the range of between about 5 degrees and about 50 degrees, such as between about 10 degrees to about 45 degrees, or for example between about 15 degrees and about 40 degrees, such as the idle movement of the advancer can be approximately 5 degrees, about 10 degrees, about 15 degrees, about 20 degrees, about 25 degrees, about 30 degrees, about 35 degrees, about 40 degrees, about 45 degrees, or for example approximately 50 degrees.

According to certain aspects, which can be combined with any other one or more aspects or embodiments or combinations of aspects or embodiments described herein, the device of the present invention incorporates operational functionality which helps to prevent the accidental opening of a dose storage area of a medicament carrier. In some aspects the functionality comprises configuration, selection, or operation of components such that an amount of idle movement of the driver gear, an amount of idle movement of the advancer, or an amount of idle movement of both the driver gear and the advancer can occur without actuating the device. Such an amount of idle movement of both the driver gear and the/an advancer can, in some aspects be described as degrees of movement, as described elsewhere herein. As stated elsewhere herein, in some aspects the idle movement of the driver gear can allow for partial, inadvertent opening of the medicament access body cover, or in some aspects may allow for cleaning of the medicament access body, in either or both cases without actuating the device. In some aspects the idle movement of the advancer or an advancer can prevent the advancement, e.g., the rotation, of the carrier engagement and hence the advancement of the medicament carrier associated with such a carrier engagement. In some aspects, accidental opening of a dose storage area and wasting of the medicament held therein is prevented by incorporating the idle movement of the/an advancer, the idle movement of the driver, or incorporation of both idle movement of the/an advancer and the idle movement of the driver.

According to certain aspects, a plurality of advancers within a dispensing device can have a different amount of idle movement. In some embodiments, which can be combined with any one or more aspects or embodiments described herein, a first of a plurality of advancers can experience an amount of idle motion, subsequently engage with a first carrier engagement, causing the rotation of the carrier engagement, followed by the carrier engagement completing its rotation prior to a second advancer completing a different amount of idle motion, subsequently engaging with a second carrier engagement upon the completion of the rotation of the first carrier engagement, causing the rotation of the second carrier engagement. In certain aspects, both advancers may be acted upon by the same dispenser component, e.g., a single driver gear.

As will be described further elsewhere herein, in other embodiments, idle movement can be present within the driver gear itself. In such embodiments, the driver gear can be a mutilated gear such that it first engages with a first advancer, such an advancer completing its work upon a movingly engaged component, followed by the driver gear engaging with a second advancer, such an advancer then completing its work upon a movingly engaged component, the motion of the two advancers being sequential to one another such that there is substantially no overlap in movement of the two advancers.

In some aspects, due to the idle motion of the driver gear, idle motion of one or more advancers, or idle motion of both the driver gear and one or more advancers, there is at least one period of time wherein no two or more carrier engagements are in motion at substantially the same time. In some aspects, during a device actuation, there is at least one period of time wherein there is no motion of both carrier engagements (e.g., no two or more carrier engagements are in motion at the same time) and there is at least one period of time wherein two or more carrier engagements are both in motion (e.g. two or more carrier engagements are in motion at the same time). In certain aspects, two or more carrier engagements are both in motion at the same time during at least one period during a device actuation, and further the carrier engagement causes the release of drug from a plurality of medicament carriers at substantially the same time. In certain aspects, two or more carrier engagements are both in motion at the same time during at least one period during a device actuation yet do not cause the release of drug from a plurality of medicament carriers at substantially the same time, instead such a release occurs substantially sequentially, one dose being made available for dispensation before another.

In some embodiments, which can be combined with any other one or more aspects or embodiments or combinations of aspects or embodiments described herein, the waste of each emptied medicament carrier present within the dispenser is collected separately from the waste of each other medicament carrier present within the dispenser. In certain aspects, each medicament carrier, e.g., a blister strip, when emptied produces two waste streams: a) a lid sheet and b) and empty base sheet. In some aspects, each type of waste stream is collected using a separate type of waste collector, each empty base sheet is collected using a first waste collector, and each lid sheet is collected using a second waste collector.

According to certain aspects of the present invention, a used medicament carrier being a medicament carrier having dispensed a dose of medicament held therein, can comprise two or more waste streams; e.g., a first waste stream and a second waste stream. According to some embodiments, one or more waste collectors can be present within the medicament dispenser of the present invention for collecting waste generated by the removal of doses of medicament from a medicament carrier yielding empty medicament carrier waste.

The mechanism of waste collection within the dispenser will be described as it applies to an embodiment wherein one or more medicament carriers comprise two layers reversibly sealed to one another, such that the two layers require separation to access a dose of medicament held within a storage compartment within one of the sheets, and wherein once the dose is administered, two waste streams are generated - one being a first layer or first sheet of the medicament carrier and the second being a second layer or second sheet of the medicament carrier.

In some aspects, as has been described, the medicament carrier can be an elongated sheet comprising a base sheet, such as a base sheet comprising distinct dose storage areas, e.g., blisters, and a lid sheet providing a sealed means by which the individual doses are held within the dose storage areas (e.g., blisters) of the base sheet, as in the medicament carrier can be a blister strip medicament carrier.

According to certain aspects, wherein a medicament carrier or medicament carriers within the dispenser is or are blister strips, the first waste stream can be the base sheet of the blister strip, and a second waste stream can be the lid sheet of the blister strip. According to certain aspects of the present invention, the dispenser comprises at least one waste collector designed to collect the lid sheet of a strip-style medicament carrier. Such a waste collector is referred to herein as a second waste collector, a distinction being made between such a collector and a first waste collector, the first waste collector being a waste collector designed to collect the base sheet of a used blister strip medicament carrier. The first waste collector for collecting the base sheet of a used blister strip medicament carrier will be described elsewhere herein, as it is distinctly different from that of the second waste collector described here.

According to certain aspects of the present invention, the second waste collector for collecting the second waste stream, e.g., the lid sheet of a used blister strip medicament carrier, can aid in peeling apart the base sheet and lid sheet of each such medicament carrier to open or otherwise access the blister pocket to make the dose stored therein available for administration. Further, the second waste collector is capable of providing tension to and maintaining tension on the waste stream being collected, e.g., the lid sheet. Yet further, the second waste collector can comprise a waste capture component, e.g., a specific component for collecting and storing the waste stream, e.g., a lid sheet. Such a waste capture component can also be referred to herein as a lid sheet collector. Accordingly, because the second waste collector comprises multiple elements having functionally different responsibilities, and because in some aspects, as will be described further herein, the component of the second waste collector can in some embodiments collect and store a waste stream by winding the waste stream about the waste collector, the second waste collector is commonly referred to herein as a "winding assembly."

According to one independent embodiment of the present invention, the invention described herein provides a collection system for capturing and storing an elongate media, which system comprises (a) a tensioning component for applying an initial, engaging tension on an end of the media (e.g., a portion of the media that has been subjected to processing), (b) a component or means for maintaining tension on the media, and (c) a collection component for collecting the media. In some aspects, the component capable of creating tension on the elongate media and the component capable of maintaining tension on the elongate media are positioned within the component capable of collecting the elongate media. In some aspects, all three components share a common axis of rotation.

According to some aspects of the present invention, the collection system comprises a tensioning component for applying an initial, engaging tension on an end of the media. In some aspects, such a tensioning mechanism comprises a mechanical attachment component. In some aspects, such a mechanical attachment component can be capable of engaging and securing an end of the media. In certain embodiments, such a mechanical attachment mechanism can be a slot, a clip, a clamp, a protrusion to engage the media or to engage a component of the media such as a hole, loop, or indentation in the media or alternatively a hole or indentation to engage a protrusion in the media. In some aspects, the tensioning component for applying an initial, engaging tension on an end of the media is a post. In some aspects, the post is capable of capturing an end of the media via a loop in the media which can be positioned about the post so as to secure the end of the media to the collection system.

According to some aspects of the present invention, the collection system comprises a component or means for maintaining tension on the media. In some aspects, such a component can be a guide, a clamp, a surface over which the media is stretched or against which the media is positioned during capture and storage of an elongate media. In some aspects, the component is a curved wall or a curved panel. In some aspects, the curved wall has a surface which increases the ability of the curved wall to engage with the media. In some aspects, such a surface is a sticky surface, such as a surface having an adhesive additive. In some aspects, such a surface is a rubber surface. In some aspects, the surface is a serrated surface.

According to some aspects of the present invention, the collection system comprises a collection component for collecting the media. In some aspects, such a collection component can be a surface about which an elongate media is wound or wrapped as the collection component moves, e.g., rotates. In some aspects, such a surface can be a flat surface. In alternative aspects, such a surface can be a curved surface. In some aspects, the collection component can comprise a media access point, an opening through which the elongate media can pass from one side of the collection component to the other. In certain embodiments, the collection component is a curved wall surface wherein the tensioning component for applying an initial, engaging tension on the media, and the component or means for maintaining tension on the media reside within the area defined at least in part by the curved wall; that is, if the curved wall were extended so as to create a circle, the tensioning component and the tension maintenance component reside within such a defined circle, as opposed to residing outside of the curved wall of the collection component. In some aspects, the media access point is a slit or slot or hole in the curved surface. In some aspects, the collection component rotates. In some aspects, an elongate media can be attached to the tensioning component for applying an initial, engaging tension on the end of the media, from which point the elongate media passes over the component or means for maintaining tension on the media, through the media access point of the collection component, to the outer surface of the collection component around which the elongate media is collected as the collection component rotates about both the tensioning component and the component or means for maintaining tension on the media. In some aspects, as the collection component rotates, the component capable of maintaining tension on the elongate media maintains tension on the media such that a sufficiently efficient, e.g. a relatively tight winding of the media is accomplished.

According to one aspect of the disclosure, which can be combined with any other aspect or embodiment, or combination of aspects or embodiments described herein, the invention described herein relates to a waste capture mechanism for use within a medicament dispenser to capture one or more waste streams generated once a medicament carrier held within the medicament dispenser has been opened and the medicament held therein dispensed. In some aspects, this waste capture mechanism is a winding assembly. According to one aspect of the disclosure, which can be combined with any other aspect or embodiment, or combination of aspects or embodiments described herein, the invention described herein relates to a winding assembly comprising: a waste capture component and a tensioning mechanism. In some aspects, the winding assembly can be incorporated into the devices described herein. In some aspects, the winding assembly can be incorporated into other devices, such as any type of device in which it is desirable to collect an elongate media, such as a tape, a string, a rope, a wire, a ribbon, or the like. In some aspects, such a device can be a medicament dispenser. In some aspects, the waste collector, e.g., the winding assembly, can collect at least one waste stream from at least one opened elongated medicament carrier held within such a dispenser, the winding assembly comprising a component capable of creating tension on the waste stream, a component capable of maintaining tension on the waste stream, and a component capable of collecting the waste stream.

According to one independent embodiment of the present invention, the invention described herein is a waste collector for use within a medicament dispenser, the collector capable of colleting at least one waste stream from at least one opened elongated medicament carrier held within the dispenser, the dispenser comprising a component capable of creating tension on the waste stream; a component capable of maintaining tension on the waste stream; and a component capable of collecting the waste stream. In some aspects, the component capable of creating tension on the waste stream and the component capable of maintaining tension on the waste stream are positioned within the component capable of collecting the waste stream. In some aspects, all three components share a common axis of rotation.

According to certain embodiments, the waste capture component comprises: (a) a waste collection surface for collecting a waste stream; (b) at least one waste access point within the waste capture component, the at least one waste access point allowing the waste stream to pass from inside of the waste capture component (e.g., from a tensioning mechanism positioned within the waste capture component; (c) a locking engagement for preventing the vertical motion of the tensioning mechanism, (d) inner movably engageable engagement elements for engaging with an engagement element of the tensioning mechanism, such an engagement between the two components being such that the tensioning mechanism is rotatable in a single direction relative to the waste capture component; and (e) a movably engageable driving element with engaging elements for engaging with a carrier engagement such that movement of the waste capture component is driven by the movement of carrier engagement.

According to certain aspects, the waste capture component can comprise any size, shape, or configuration providing for the effective and efficient interaction with the tensioning mechanism such that the waste stream being collected, e.g., the lid sheet, is maintained in a taught configuration and by which the lid sheet can be efficiently collected. According to certain aspects, the waste capture component has a circular, annular, cylindrical, tubular body shape. In some aspects, the waste capture component is configured such that it has an interior and an exterior, the interior being of sufficient size and shape to be capable of maintaining the positioning of the tensioning mechanism therein.

According to some aspects of the present invention, which can be combined with any other aspect or embodiment, or combinations of aspects or embodiments described herein, the waste capture component comprises a waste collection surface. Such a waste collection surface can be any component suitable for collecting a waste collection stream such as a lid sheet from an elongated medicament carrier by winding, gathering, compressing, folding, or otherwise collecting into a relatively compact area, the lid sheet. In some aspects, the waste collection surface is the outer or outside surface of the waste capture component. In a common aspect, the waste material, e.g., lid sheet, is collected by winding about, e.g., around, the waste collection surface. In some aspects, such winding can result in a spool-like collection of separated lid sheet. According to certain embodiments, the collection surface can be a cylindrical surface having a relatively flat surface, such as an annular surface such that similar to the surface of an empty thread spool. In some aspects, the waste collection surface can have a circumference about which the lid sheet is wound. In some aspects, the waste collection surface provided for winding up the spent lid sheet is referred to herein as a winding surface.

According to some aspects, which can be combined with any other aspect or embodiment or combination of aspects or embodiments described herein, the waste capture component, comprises a fixed-diameter winding surface on which said waste stream, e.g., the lid sheet or according to alternative embodiments the base sheet of an elongated medicament carrier is wound. That is, according to certain aspects, the diameter of the waste capture component is unchangeable and remains constant throughout the operation of the device.

In some aspects, which can be combined with any other aspect or embodiment, or combinations of aspects or embodiments described herein, the waste capture component can comprise one or more waste access points. In some aspects, the waste access point is an opening within the waste capture component, e.g., an opening in the winding surface. Such a waste access point can take any shape or configuration which allows for the waste stream being collected by the waste collector, such as the lid sheet of a blister strip medicament carrier to pass from the interior of the waste capture component to the exterior of the waste capture component, e.g., to the winding surface. In certain aspects a waste access point can comprise, but not be limited to a slit (which also may be referred to as a slot, describing a narrow opening within the waste capture component through which a waste stream may pass without significant extra space within the passage as the waste stream passes through); a passage; an opening or cut out having any size, shape, or configuration; or any similar or equivalent means allowing passage of the waste stream, e.g., the lid sheet, from the interior of the waste capture component (in which the tensioning mechanism can reside, typically comprising an element to which an end of the lid sheet may be engaged), to the exterior of the waste capture component, e.g., the winding surface. According to common aspects of the invention described herein, the waste access point of the waste capture component of the second waste collector is a slit and is commonly described herein as an entry slit.

According to certain aspects, the waste capture component comprises a plurality of waste access points. According to certain embodiments, which can be combined with any other aspect or embodiment or combination of aspects or embodiments, the waste capture component comprises a single waste access point. According to certain embodiments, two waste access points are present within the waste capture component.

In some embodiments, which can be combined with any other aspect or embodiment, or combinations of aspects or embodiments described herein, the waste capture component comprises a locking engagement. Such a locking engagement can be any engagement that maintains the waste capture component in relation to a tensioning mechanism positioned therein in such a manner to prevent the vertical motion of the tensioning mechanism upon rotation of the waste capture component. In some aspects, the locking engagement can be but may not be limited to a tongue-and-groove mechanism, a lip engagement, a clipping mechanism, or a ridged mechanism which maintains positioning of the tensioning mechanism relative to the waste capture component however does not prevent the independent rotation of the tensioning mechanism and the independent rotation of the waste capture component. In certain aspects, the locking engagement is a locking ridge, the ridge being, e.g., a (typically narrow) lip, or ridge, extending at least partially around the interior of the waste capture component such that it can be received by a corresponding receiving element of the tensioning mechanism or also or alternatively it can rest on a partial surface of an element of the tensioning mechanism such that the tensioning mechanism is held in place, preventing axial displacement of the tensioning mechanism without interfering with freedom of rotation of the tensioning mechanism. In some aspects, the lip or ridge extending about the circumference of the interior of the waste capture component is a continuous element, extending completely around the interior of the waste capture component to form a ring. In other aspects, the lip or ridge is noncontinuous, forming an incomplete ring or a series of discrete ridges, such as about two, about three, about four, about five, about six, about seven, or about eight or more ridges (e.g., about 2-10, about 2-8, about 2-6, about 3-9, or about 3-7 ridges). The term locking ridge can be used herein to refer to such an element of the waste capture component of the second waste collector.

According to certain aspects of the present invention, the waste capture component comprises a plurality of movably engageable components. In certain embodiments, a first of a plurality of movably engageable components can engage with the tensioning mechanism of the waste capture component while a second of a plurality of movably engageable components can engage with a separate component of the dispenser, such as for example a carrier engagement. In some aspects, the presence of such a plurality of engagement components negates the necessity of a spring as a tensioning mechanism. In some aspects of the present invention, no spring is present within the waste capture component.

According to certain aspects of the present invention, which can be combined with any other aspect or embodiment, or combinations of aspects or embodiments described herein, the waste capture component comprises inwardly positioned (inner) movably engageable engaging elements. In some aspects, such movably engageable engagement elements reside on the interior of the waste capture component. In some embodiments, the engagement elements can take any shape or form which enables the waste capture component to movably engage with the tensioning mechanism, or more specifically an engagement of the tensioning mechanism, such that the tensioning mechanism can only rotate in a single direction relative to the waste capture component. In some aspects, the inner movably engageable engaging elements are protrusions, indentations, levers, arms, teeth, or any other such similar or equivalent elements capable of being movably engaged with a complementary element of the tensioning mechanism. According to certain aspects of the present invention, the inner movably engageable engagement element of the waste capture component of the second waste collector is a set of inner teeth, such as the teeth of a common gear. Such a set of teeth can be referred to herein as the inner teeth of the waste capture component.

According to certain aspects of the present invention, the waste capture component, having a shape defining an inner or inside area and an outer or outside face, can have a component defining a bottom of the inner or inside area and/or the bottom of the waste capture component. The locking ridge, as described elsewhere herein, is positioned above such a bottom or base of the waste capture component. The inner teeth of the waste capture component are positioned within the interior of the waste capture component, circling the inner wall of the waste capture component and sitting above or on top of the bottom or base. In some aspects, the bottom or base of the waste capture component has an opening at its center. According to certain aspects, such an opening can aid in the proper positioning of the waste capture component, such that is allows for the waste capture component to be located within the inner housing assembly via a stud of the inner housing assembly, and to be positioned within the inner housing assembly and the base cover.

In certain embodiments, which can be combined with any other aspect or embodiment, or combinations of aspects or embodiments, described herein, the waste capture component can comprise a movably engageable element comprising engagement elements which are positioned on the outer perimeter of the waste capture component. In some aspects, there are two engagement elements present on the waste capture component: one on the interior of the waste capture component and on the exterior of the waste capture component. In some aspects, the movably engageable element on the exterior of the waste capture component can comprise any element capable of being driven by another component of the dispensing mechanism, such as a carrier engagement. In some aspects, the movably engageable element can be a conveyor, a lever, a switch, an engageable wheel, or the like. In certain aspects, the movably engageable element can be in the form of a wheel comprising engagement elements capable of engaging with corresponding engagement units of one or more other components such as a carrier engagement. Such engagement elements, in some aspects, can be protrusions or indentations of any shape or size capable of movably engaging with corresponding protrusions or indentations of the engaged component, such as elements of the carrier engagement. In some aspects, the engagement units are teeth, and the driver is a gear. Such engagement elements on the waste capture component typically are gear teeth capable of movably engaging with the gear and gear teeth of another component within a larger device in which the waste capture component resides. In some aspects, the other component is a carrier engagement of a/the medicament dispenser.

According to certain embodiments, the gear teeth of the waste capture component can be movingly engaged with the gear teeth of a carrier engagement, the gear teeth of the waste capture component being driven by the gear teeth of the carrier engagement. In certain aspects, the carrier engagement is driven by an advancer. In certain other aspects, the carrier engagement is driven by an interim driver. In further aspects, an advancer can be driven by a driver, either directly or indirectly via an interim driver.

According to certain aspects of the present invention, a second waste collector is movably engaged with a carrier engagement, and each second waste collector comprises multiple components to create an assembly, the assembly comprising a component capable of creating tension on the lid sheet and a lid sheet collector. In some aspects, the component capable of creating tension on the lid sheet is positioned within the lid sheet collector, and wherein the two components share a common axis of rotation, and further wherein sufficient movement of the carrier engagement results in the movement of the second waste collector.

In certain embodiments, which can be combined with any other aspect or embodiment, or combination of aspects or embodiments described herein, the tensioning mechanism of the second waste collector can comprise any mechanism, component, device, or element capable of creating and maintaining tension in the waste stream to be collected by the second waste collector. In certain aspects, creating and maintaining tension in the waste stream contributes to the functional operation of the waste collector.

In some aspects, the tensioning mechanism can be a single component such as but not limited to a winding wheel, or any other suitable movable element capable of applying a force on the waste stream such that the waste stream is extended or forced to take an indirect route to a waste collection surface of a waste capture element and create tension, (an elastic element, a compression element, or the like). In some aspects, the tension mechanism in the form of a single element can be an expandable and/or compressible wheel, having flexible members that expand to create tension on the waste stream wrapped or wound about them.

In alternative aspects, which can be combined with any other aspect or embodiment, or a combination of aspects or embodiments described herein, the tensioning mechanism can comprise multiple elements to form an assembly of elements. According to such embodiments, the tensioning mechanism may be referred to as a tensioning assembly.

According to certain embodiments, which can be combined with any other aspect or embodiment or combination of aspects or embodiments described herein, the tensioning mechanism can comprise a central connection element, such as a base, serving as a single component to which multiple elements of the tensioning mechanism are attached. In certain aspects, the tensioning mechanism can comprise an axle defining the rotational axis about which the waste capture component is rotatable. In some embodiments, the tensioning mechanism can comprise a connection element for connecting to the material being collected, such as, for example, to a waste stream, e.g., the lid sheet of a medicament carrier, such an element serving as a connection point to the tensioning mechanism to the waste stream. This collected material, e.g., this waste stream, is commonly embodied herein as a lid sheet of a medicament carrier but as stated elsewhere herein, such a collection mechanism is capable of being utilized within non-medicament dispenser devices and is capable of collecting other elongate media streams therein, such that it is capable of connecting to a waste stream other than a lid sheet. In some aspects, the tensioning mechanism can comprise a lid gripping element, an element capable of guiding, gripping, or guiding and gripping the waste stream as it moves within second waste collector, and more specifically as it moves within the tensioning mechanism positioned inside of the waste capture component of the waste collector, such a waste stream commonly embodied herein as a lid sheet of medicament carrier, but an element which can be capable of guiding and or gripping a waste stream other than a lid sheet. In some aspects, which can be combined with any other aspect or embodiment or combination of aspects or embodiments, the tensioning mechanism can comprise and an engagement element capable of engaging with the interior, or inner movably engageable engaging elements, e.g., inner teeth, of the waste capture component.

In some aspects of the present invention, which can be combined with any aspect or embodiment or combination of aspects or embodiments described herein, the tensioning mechanism of the second waste collector comprises a central connection element. In certain embodiments, the central connection element operates to secure multiple functional elements of the tensioning mechanism together to permanently establish their position to one another. The central connection element can take any form or shape capable of securing a plurality of functional elements of the tensioning mechanism together, such as it can take the shape of a plate or disk, having a circular or geometric shape. In some aspects, the central connection element is in the shape of a circular disk. In certain aspects, the central connection element can have one or more elements of the tensioning mechanism attached to a single side of the central connection element. In alternative aspects, the central connection element can have one or more elements of the tensioning mechanism attached to multiple sides, e.g., both of two sides, a top and a bottom, of the central connection element. In some aspects, the central connection element has elements attached to its upper or top side, such elements comprising one of, a combination of, or each of an axle, a lid connection element, and a lid gripping element. In some aspects, no lid gripping element is present. In some aspects, the central connection element has one or more elements attached to its lower or bottom side, such an element comprising an engagement for engaging with a waste capture component. In some embodiments, such positioning of topside elements and such positioning of bottom side elements are both present. In some aspects, the central connection element is a base. The term "base" in such contexts is a term commonly used herein to describe such a component of a tensioning mechanism.

According to certain embodiments, which can be combined with any other aspect or embodiment or combination of aspects or embodiments described herein, the axle of the tensioning mechanism of the second waste collector defines a rotational axis about which the waste capture component of the second waste collector is rotatable. In some aspects, the axle is centrally located on the base of the tensioning mechanism. In some aspects, the axle is an integral component of the central connection element and extends upward from the central connection element. In some aspects, the axle is formed separately and attached to the central connection element. In some embodiments, the axle is static. In certain aspects, the axle provides an axis about which the waste capture can rotate and further helps to position the waste capture element relative to the tensioning mechanism and vice versa. In some aspects the axle aids in the proper positioning of the waste capture component such that the waste stream is effectively wound about the waste capture component without interfering in the suitable operation of the second waste collector. According to certain aspects, the static nature of the axle enables the tensioning of the lid sheet during assembly.

According to certain aspects of the present invention, the tensioning mechanism further comprises an element designed to connect to and securely maintain an end of a waste stream. In some aspects, the waste stream is the lid sheet of a peelable elongated strip medicament carrier. In some aspects, the connection element is a clip, hook, squeezing device, slit, slot, post, or other similar or equivalent element capable of forming a secure connection to an end of a waste sheet. According to certain aspects, the securing mechanism for maintaining a connection with the end of the lid sheet waste stream is a post capable of receiving and maintaining a looped end of a lid sheet. A sealed looped end of a lid sheet can be placed over a post and maintained when a pulling pressure is applied to the lid sheet (e.g., a tensioning force). This element of the tensioning mechanism can be referred to herein as a lid connection element.

According to certain aspects, which can be combined with any other aspect or embodiment or combination of aspects or embodiments described herein, a tensioning mechanism comprises an element attached to the base tensioning mechanism capable of aiding in the guiding of waste stream, e.g., a lid sheet, from the lid connection element (e.g., an element capable of attaching to a waste stream) through the tensioning mechanism and out through the entry slit of the waste capture component to the surface of waste capture component. Such an element can be any element capable of providing additional support to the waste stream sheet such that it is properly guided from the lid connection element to the winding surface of the waste capture component. In some aspects, this element can be a post, a fin, a sheet or panel, or the like having a surface which is amenable to gripping to a limited extent the waste stream, e.g., the lid sheet, as it passes over. In some aspects, such a limited gripping provides an additional element of tensioning of the waste stream and further provides for proper positioning of the lid sheet in that it provides a means of reducing or preventing slipping of the lid sheet out of its intended path during collection, e.g., during winding. In some aspects, this additional tensioning element is a curved panel comprising a surface that provides a limited grip on the lid sheet as it passes over. In some aspects, the surface can be coated with a gripping component, such as comprising a rubberized surface. In some aspects, the surface could be coated with a sticky element such as a gum, glue, rubberized cement, or the like. In some aspects, the surface can be designed to comprise a rough or uneven surface to support gripping, such as having a sand-comprising coating, a series of protrusions, e.g., spikes or bumps, or a series of serrations. In some aspects, this element of the tensioning mechanism of the second waste collector can be a curved panel having a serrated surface. Such an element can be referred to as a lid gripping element or a blister guide. According to certain aspects, this element is capable of maintaining tension on the waste stream. In some aspects, no lid gripping element is present.

According to certain aspects of the present invention, the tensioning mechanism can further comprise an element capable of engaging with the interior, or inner movably engageable engaging elements, e.g., inner teeth of the waste capture component. In some aspects, such an element can be any element having a design suitable for interfacing with the inner movably engageable engaging elements (e.g., inner teeth) of the waste capture component. In some aspects, this element is a set of posts, pins, fins, arms, or the like. In some aspects, such elements are designed to have a fixed shape. In alternative aspects, such components are designed to have some level of flexibility. In some aspects, such components may have a varying degree of flexibility throughout their length or most of their length. In some aspects, such components can operate to only be capable of rotating the tensioning mechanism in a single direction relative to the waste capture component. According to some embodiments, the tensioning mechanism is bi-directional. According to certain aspects of the present invention, the components are flexible ratchet arms, having a level of flexibility within the non-central ends of each arm. In operation, the flexible ratchet arms of the tensioning mechanism are capable of movably engaging with the inner teeth of the waste capture component. Early in each operation of the dispenser device, the flexible ratchet arms can allow for the independent rotation of the ratchet mechanism and the waste capture components of the second waste collector. Typically, upon reaching an effective amount of tension, the arms lockably engage with the inner teeth of the waste capture component such that subsequently, the two elements rotate in unison. The operation of such a system in a dispenser device is further exemplified in the set of exemplary figures provided herein.

In one embodiment, the ratchet arms of the tensioning assembly can be provided for the ratcheted engagement with the inner teeth of the waste capture component. Such ratcheted engagement provides that the tensioning mechanism may only be rotated relative to the waste capture component in one rotational direction (e.g., only clockwise or only anti-clockwise) with rotation in the other rotational sense being prevented by the ratchet interaction between inner teeth of the tensioning mechanism and the ratchet arm of the base. In some aspects, the tensioning mechanism is bi-directional.

The tensioning mechanism can be located within the waste capture component. Further, the tensioning mechanism can be arranged concentrically with the waste capture component. In one aspect, the waste capture component is mounted on the axle such that it is rotatable in either a clockwise or anti-clockwise (counterclockwise) relative to the base. The lid connection element provides an anchor to hold the lid sheet at the sealed loop of the medicament carrier. The lid gripping element, if present, can include a serrated surface, as previously described, to guide and grip the lid sheet as it moves within the waste capture component during the tensioning process. The ratchet arms can be positioned on the underside surface of the base. The ratchet arms can be maintained in ratcheted engagement with the inner teeth of the waste capture component such that the base can only be rotated in one direction relative to the waste capture component. In operation, the uni-directional movement of the tensioning mechanism can provide the desired amount of tension on the lid sheet. The second waste collector, also referred to as described above as the winding assembly, can be driven by the carrier engagement during blister advancing via gear teeth. In use, the winding assembly can aid in the peeling apart the distinct medicament dose portions of each of the medicament carriers and further in the collection of a resulting waste stream.

The dispensing mechanism can include two winding assemblies for peeling apart the distinct medicament dose portions of each of the medicament carriers individually. In use, the dose storage area of the medicament strips, e.g., the blister pocket of a blister strip medicament carrier, is opened near or at a location aligned and accessible or otherwise closely associated with the grids of the flow director. As will be further described elsewhere herein, the tensioning assembly can aid in opening each dose storage area within the second waste collector as it applies tension to the lid sheet.

The waste capture component, as previously described, can, in one aspect, be located over, about, or otherwise on the outside of the tensioning mechanism and rotatable about the same rotational axis as the tensioning mechanism. In some aspects, as previously described, the waste capture component can include a waste collection surface for receipt of a waste stream, e.g., a waste element of an opened medicament carrier. As has been previously described, such a waste sheet can be the lid sheet of the medicament carrier. However, in alternative embodiments, the waste sheet collected could be the base sheet of the medicament carrier. As also previously described, the waste capture component is an independent embodiment of the present invention and is a mechanism capable of being utilized within many types of devices, a medicament dispenser being only one embodiment. In such aspects, the mechanism is better described as a device for the collection of an elongate media, such an elongate media being many possible types of media, a waste stream being only one example, and a lid sheet of a blister strip medicament carrier being only one example of such a waste stream.

In one embodiment, the waste capture component is mounted about the axle such that it can be rotatable in either a clockwise or anti-clockwise sense relative to the base unless otherwise locked by the ratcheting mechanism of the tensioning mechanism.

According to certain aspects of the present invention, an alternative type of waste collector is provided for collecting a different waste stream within the same dispenser. As described herein, this waste collector is referred to as a first waste collector. According to one embodiment, this first waste collector is capable of collecting a waste stream generated after a medicament carrier has a dose of medicament held therein removed. In some aspects, such a waste stream is the base sheet of an elongated medicament carrier, such as the base sheet of a blister strip. According to alternative embodiments, the waste stream could be the lid sheet. In some aspects, dispensers of the present invention can comprise one or more of a first waste collector and one or more of a second waste collector.

In one aspect, the dispensing mechanism comprises a single or common first waste collector. In such an embodiment, the same waste stream from each medicament carrier, e.g., the base sheet or the lid sheet, are both collected by the single first waste collector. In an alternative aspect, the dispensing mechanism can comprise two or more first waste collectors. Such first waste collectors are often referred to herein as one or more "wrapping wheels", e.g., one wrapping wheel for each medicament carrier present within the dispenser, for wrapping of two waste streams, e.g., the peeled base sheets from multiple medicament carriers individually.

According to some embodiments, which can be combined with any other aspects or embodiments or combinations of aspects or embodiments described herein, the first waste collector can collect a waste stream by any suitable means such as gathering, folding, winding/wrapping, or the like. In some aspects, the first waste collector collects a waste stream, such as the base sheet by winding or wrapping.

In some aspects of the present invention, the first waste collector wraps the base sheet about a central point and may alternatively be referred to as a wrapping wheel. In some aspects, the wrapping wheel has multiple elements that aid in its efficient and successful function. In certain aspects, the wrapping wheel (first waste collector) comprises a wrapping component, a waste access slit and gear teeth. Such a multi-component first waste collector element can also be referred to herein as the wrapping assembly.

According to some embodiments, which can be combined with any other aspects or embodiments or combinations of aspects or embodiments described herein, the wrapping wheel comprises a post, tube, pole, pin, or other similar or equivalent component about which the waste stream is wrapped. According to certain aspects, such an element is an elongated element having a length sufficient to provide a surface area about which a waste stream, such as the base sheet of a blister strip, can be collected by wrapping. In some aspects, such an element can be described as a pin, and the element about which the waste stream is wrapped is commonly referred to herein as a driving pin or a pin of the first waste collector or pin of the wrapping wheel or wrapping assembly.

According to some embodiments, which can be combined with any other aspects or embodiments or combinations of aspects or embodiments described herein, the pin of the wrapping wheel, or the pin of the wrapping assembly, or the pin of the first waste collector comprises an element for securing the end of a waste stream such that it can be maintained securely as the remainder of the waste stream is wrapped (such as the empty base sheet of a blister strip medicament carrier). This component provides access to the pin as previously described to secure the waste stream to the pin, about which the waste stream is wrapped. Accordingly, such a securing element can be referred to as a waste entry point to the first waste collector. The waste entry point to the first waste collector can be any means of securing the waste stream to the element about which the waste stream will be wrapped, wound, or coiled. Such a securing mechanism could be, for example, but not limited to, a clip, a hook, a squeezing mechanism, a pinching mechanism, or the like. In certain embodiments, the securing mechanism is a slit in the first waste collector driving pin. According to certain embodiments, the base sheet end of the medicament carrier is located with the slit and secured therein, the remainder of the emptied base sheet then wrapping around the wrapping wheel, e.g., more specifically around the pin, as it rotates during operation of the dispenser.

According to some embodiments, which can be combined with any other aspects or embodiments or combinations of aspects or embodiments described herein, the wrapping wheel comprises a movably engageable component for movably engaging with another component of the dispenser. In some aspects, such another component can be an interim component such as another movably engageable component. In some aspects, such another movably engageable component can be any component suitable for transmitting or receiving a driving force or a force of motion. In some aspects, such a component is a gear and can be an interim gear, such as is described herein as an interim gear, a secondary interim gear, or a tertiary interim gear. Such gears can be circularly shaped mechanisms having engagement elements about their outer circumference such that they operate, as has been previously described, and commonly embodied as gear teeth. In certain aspects, a gear having gear teeth is arranged about the bottom of the driving pin of the first waste collector (e.g., the wrapping wheel). Accordingly, upon receiving a driving force from a suitably arranged corresponding movably engaged component, the wrapping wheel can be rotated to wrap the connected waste stream about the driving pin. A first waste collector can be driven by the gear teeth of the carrier engagement via the interim gear. The use of interim gears is described further as part of the description of figures incorporated herein.

In some aspects, the dispenser comprises a component for receiving and directing airflow within the dispenser. This component is herein described as the flow director. In some aspects, a flow director is a single unit, functionally engaged with the medicament access body such that when a user places a body orifice on the opening of the medicament access body and inhales, an airflow is created which is received by the flow director. In certain embodiments, which can be combined with any other aspect or embodiment or combinations of aspects or embodiments, the flow director is capable of receiving a flow of air, in certain aspects splitting the flow of air into separate airflows, directing each airflow to allow the airflow to each entrain a medicament to be dispensed, and, in some aspects, maintaining each distinct airflow comprising entrained medicament separate from each other airflow until the airflow exits the dispenser.

According to one aspect of the present invention, the flow director can be provided for guiding airflow towards one or more available medicament doses, e.g., medicament doses within opened blister pocket(s) of a blister strip medicament carrier, for releasing the powder contained therein; and subsequently guiding the liberated powder to a medicament access body, e.g., mouthpiece, for inhalation by a patient.

According to one aspect of present invention, the disclosure herein relates to a flow director comprising: an inlet compartment for receiving air from outside of the medicament dispenser, an outlet compartment, and a pair of airflow guides for each medicament carrier present in the dispenser such that the pair of airflow guides is paired with a set of one inlet and one outlet compartments. In certain aspects, an inlet compartment exists for each medicament carrier present in the dispenser. In some aspects, an outlet compartment exists for each medicament carrier present in the dispenser. In some aspects an inlet compartments exists for each medicament carrier present in the dispenser, however only a single outlet compartment is present.

According to one aspect of the present invention, which can be combined with any other aspect or embodiment or combination of aspects or embodiments described herein, the flow director comprises a compartment for receiving external air entering the dispenser due to the negative pressure created within the dispenser by the inhalation of a patient via the medicament access body of the dispenser. In such embodiments, external air entering the dispenser due to the negative pressure created within the dispenser by the inhalation of a patient via the medicament access body of the dispenser directly enters one or more inlet compartments as will be described herein.

According to certain aspects of the present invention, the dispenser comprises a single inlet compartment. In some aspects, the inlet compartment, receiving air from inlet vents in the housing described elsewhere herein, defines the sole point of entry for external air into the dispenser. According to one aspect of the present invention, a separate inlet compartment can be present for each of a plurality of medicament carriers present within the dispenser. According to certain embodiments, the airflow entering the dispenser via the inlet vents upon the inhalation of the user causing a negative pressure within the devices is divided into separate airflows, directed to separate inlet compartments, one inlet compartment for each medicament carrier present within the dispenser. In such embodiments, a plurality of inlet compartments can sometimes be referred to as inlet channels. In certain aspects, there is an immediate separation of air as it passes from the exterior of the dispenser, through the inlet vents, into the interior of the dispenser, the air flow being separated immediately into separate inlet compartments. The function of the one or more inlet compartments is to direct airflow(s) toward a suitably positioned dose storage area of a medicament carrier, when present, in order to entrain the medicament held therein.

In some aspects, any one or more inlet compartments can be of any shape, size, or configuration to be suitable for receiving airflow from outside of the dispenser and directing such an airflow toward a path having the purpose of entraining a dose of medicament and carrying the entrained medicament to the user. In some aspects, the compartment has an ovular, square, circular, rectangular, trapezoidal, elliptical or another such geometric shape. In some aspects, the inlet compartment can comprise elements that guide airflow along a desired path or direction, such as fins, baffles, spirals, and the like. In some aspects, no such additional path director is present within the inlet compartment.

According to one aspect of the present invention, which can be combined with any other aspect or embodiment or combination of aspects or embodiments described herein, the flow director comprises an outlet compartment. In some aspects, such an outlet compartment can be a single outlet compartment such that there is a single airflow exiting the dispenser. In such an embodiment, the single airflow can entrain medicament from one or more dose storage areas from one or more medicament carriers. In certain other aspects, such an outlet compartment can be divided into a plurality of outlet compartments, sometimes referred to as outlet channels. As used herein, the term "outlet compartment" is used to describe the element of the flow director directing airflow out of the dispenser via a medicament access body, or, more specifically, via a medicament access body opening. The term "outlet compartment" can be used to describe a single passageway exiting the dispenser or a plurality of outlet channels, which, together, provide an exit for a plurality of airflows to exit the dispenser. In some aspects, where a plurality of outlet channels are present, the number of outlet channels present within the flow director matches the number of medicament carriers present within the dispenser. According to certain embodiments, the flow director comprises at least two, at least three, or at least four outlet channels. In some aspects, the flow director comprises a single outlet channel (e.g., a single outlet compartment having no plurality of channels). In some aspects, the flow director comprises about two outlet channels. In some aspects, the flow director comprises an outlet channel for each medicament carrier present within the dispenser. According to certain embodiments, the outlet compartment, which may be divided into a plurality of outlet channels, is provided for delivery of the medicament to the patient, for example, for delivering an airflow comprising entrained medicament, to the user via the medicament access body, or more specifically, via an opening in the medicament access body.

In some aspects of the present invention, an outlet compartment or outlet channel can be of any size, shape, or configuration to provide an exit for one or more airflows comprising an entrained medicament to exit the dispenser and to reach the user. In some aspects, an outlet compartment or outlet channel has an ovular, square, circular, rectangular, trapezoidal, elliptical or another such geometric shape. In some aspects, the outlet compartment or outlet channel can comprise elements that guide an airflow along a desired path or direction, such as fins, baffles, spirals, and the like. In some aspects, no such additional path director is present within the outlet compartment or outlet channel.

According to one aspect of the present invention, which can be combined with any other aspect or embodiment or combination of aspects or embodiments described herein, the flow director comprises one or more airflow guides to allow for an airflow to exit an inlet compartment or to enter an outlet compartment or an outlet channel. In common embodiments, a pair of airflow guides are incorporated in the dispenser, one airflow guide providing an exit from an inlet compartment and one other airflow guide providing an entrance to an outlet channel. In some aspects, a pair of airflow guides are associated with each set of one inlet compartment and one outlet compartment or channel. Alternatively, in some aspects, an airflow guide exists for every inlet compartment but only a single airflow guide exists when only a single outlet compartment is present. In many aspects, an airflow guide is positioned between the area directing the inlet airflow prior to reaching the dose storage area of a medicament carrier, and a second is positioned in the area directing the outlet airflow after having passed by the dose storage area of a medicament carrier, e.g., after having entrained the medicament dose in the airflow. According to certain embodiments, the inlet airflow paths and the outlet airflow paths are in communication only via the pair of airflow guides. In some aspects, the pair of airflow guides is separated by the space within which a dose storage area of a medicament carrier is present. The pair of airflow guides can be provided for communicating with an opened pocket of the medicament carrier to enable entrainment of powder by air drawn through the inlet vent openings via the inlet compartment as will be described.

According to certain embodiments, the airflow guides are suitably positioned openings within a wall or separation of the flow director, which aid in directing airflow from one area of the flow director to another. More specifically, the airflow guides can be positioned such that they are oriented to the two far ends, or to two far sides, (e.g., the opposite ends or opposite sides), of a medicament storage area of a medicament carrier when the dose storage area of the medicament carrier has been delivered to a position within the dispenser wherein the dose is available for dispensation (administration). In some aspects, the medicament storage area can have any suitable shape, such as a capsule shape, circular shape, rectangular shape, square shape, ovular shape, or any shape suitable for allowing an airflow to entrain the medicament held therein. According to certain aspects, one airflow guide is positioned within a wall of an inlet compartment, while a second airflow guide is positioned within a wall of an outlet compartment or outlet channel, such that the two airflow guides represent a set of guides, and an airflow exiting the inlet compartment via the first airflow guide of the set of airflow guides passes through, by, over, into, or around a dose storage area of a medicament carrier appropriately aligned thereto sufficiently so as to entrain the medicament held therein, and through the second airflow guide positioned within a wall of an outlet compartment or outlet channel.

As previously stated, the dose storage areas of medicament carriers, e.g., blisters pockets of blister strip medicament carriers, can have any suitable shape, including those with a square, circular, ovular or rectangular profile. The particular form, including shape and cross-sectional area, of the blister pocket can affect the airflow properties, and particularly airflow resistance and pressure drop experienced at the open pocket when a patient inhales through the flow director herein, and accordingly the positioning of the airflow guides can be considered during design so as to create a suitable airflow for entraining the medicament held within the dose storage area of a particular medicament carrier. According to certain alternative aspects, the positioning of the airflow guides is such that their positioning is suitable for a wide variety of shapes and sizes of medicament dose storage areas of a variety of medicament carriers.

In some aspects, the airflow guides can comprise any shape. In certain aspects, the airflow guides are circular, square, rectangular, triangular, trapezoidal, or any other such geometric or another shape sufficient to allow the passage of air therethrough. It can be appreciated that the size of an airflow guide can impact the amount of air, and also or alternatively the velocity of air which can pass therethrough. It can also be appreciated that the shape and or design characteristics of the airflow guide can impact the airflow pattern generated as the air passes through an airflow guide. It may be preferential and advantageous to select a size, shape, and design of the airflow guide according to the desired airflow characteristics to facilitate efficient and effective removal of medicament from a medicament storage area. For example, the flow director can, in some aspects, which can be combined with any other aspect, embodiment, or combination of aspects or embodiments described herein, also promote the de-agglomeration and/or the de-aggregation of a medicament, such as a released powder, that is transported therethrough having been entrained by an airflow. An airflow guide may, within its larger boundaries, further be divided to comprise one or more simple openings (i.e., apertures) or alternatively, in aspects certain features of an airflow guide may be provided thereto to modify the airflow pattern, such as inclusion of a 'cross-piece' (e.g., cruciform-shaped) divider within the airflow guide. According to alternative embodiments, such a subdivision of the larger opening comprises a cross-hatching design (e.g., a screen-like design), a target design, a pie-like design, or any such similar or equivalent design which can suitable modify the airflow patter or suitably impact the medicament entrained within an airflow, such as for example to promote the de-agglomeration and/or the de-aggregation thereof.

As previously described, a pair of airflow guides for each of the sets of e.g., inlet compartments and outlet compartments and/or channels can be provided for allowing an airflow receipt of, and an airflow directing of, the dose from the dose storage area of a medicament carrier, e.g., blister pocket of each blister strip medicament carrier. The exact orientation of the airflow guides will be determined to an extent by the shape of the dose storage area within a medicament carrier, e.g., a blister pocket, and the desired function of entrainment of medicament powder particles in the airflow directed into the pocket as previously briefly described. According to one specific embodiment, which can be combined with any other aspect or embodiment or combination of aspects or embodiments described herein, the open blister pocket of a blister strip medicament carrier has a generally elongate oval profile and the airflow guides are positioned above opposite ends of the elongate oval open pocket profile.

According to certain aspects of the present invention, the flow director can be located between a medicament access body and a location, e.g., an opening location, at which an opened dose storage area of a medicament carrier, e.g., blister pocket of a blister strip medicament carrier, is presented. Such a location can be referred to as an opening location or an opening station; as such a station or location within the device is the location in which the dose storage areas, e.g., blister pockets, are accessed.

In one aspect, the flow director comprises an opening in communication with a medicament access body. In some aspects, the opening comprises a component facilitating the attachment of the medicament access body, such as a mouthpiece, to the flow director. Such an attachment can be, for example, by a clip, snap-fit, screw threads, or similar or equivalent attachment means. In some embodiments, the opening comprises a rim that can be snap-fitted with the medicament access body, e.g., mouthpiece, opening.

In certain embodiments, which can be combined with any other aspect or embodiment or combination of aspects or embodiments described herein, a medicament access body opening, e.g., a mouthpiece opening, is located adjacent to the flow director opening. Thus, during the use of such a dispenser, the patient inhales through the medicament access body opening, which creates negative pressure in the flow director, which causes air to be drawn from outside of the dispenser device through the inlet vent openings and into the inlet chamber, or, in alternative embodiments, directly into one or more inlet compartment(s), of the flow director. The airflow is then directed from the inlet compartment to the outlet compartment via, airflow guides, during which the airflow passes over, by, around, within, or otherwise through the open dose storage area of an appropriately positioned medicament carrier, e.g., a blister pocket of an appropriately positioned blister pack, to entrain the medicament powder contents thereof.

In certain aspects of the present invention, which can be combined with any other aspect or embodiment or combination of aspects or embodiments described herein, the inlet compartment and outlet compartments can lie adjacent to each other such that the airflow can be directed from the inlet compartment(s) to the outlet compartment(s) via the open dose storage area of a medicament carrier, e.g., blister pocket, to entrain the medicament powder contents thereof. The inlet compartment and outlet compartment (e.g., the inlet flow path or paths and outlet flow path or flow paths) can, in some aspects, lie side-by-side (i.e., adjacent or close to) each other such that when said opened or otherwise available dose storage area, e.g., an open blister pocket of a blister strip medicament carrier, is positioned adjacent thereto, the airflow can be directed from the inlet compartment to the outlet compartment, such as one of a plurality of outlet channels, via the open blister pocket to entrain the medicament powder contents thereof. Transport of the so-entrained medicament particles is thereby enabled in the airflow from the inlet compartment to the outlet compartment.

In some aspects, the flow director can provide a path for the inlet air to evacuate the drug from the dose storage area of a medicament carrier, e.g., a blister pocket of a blister strip medicament carrier, during inhalation. In such an aspect, the drug leaves the blister pocket and enters the flow director through the airflow guide that helps the drug powder to de-agglomerate or de-aggregate before inhalation. The medicament in the blister pocket enters the airflow guide located on either side of the flow director. The outer compartment can locate the medicament access body. In an aspect, the inlet compartment and outlet compartment may each contain one or more airflow guides (e.g., cruciform-shaped). According to one aspect, the airflow drawn into an inlet compartment of the flow director flows primarily, essentially solely, or solely through the inlet vent openings. During use, the patient inhales through the medicament access body, which creates negative pressure in the flow director, which causes air to be drawn from outside the dispenser through the inlet vent openings and into one or more inlet compartment(s). The airflow is then directed from the inlet compartment to the outlet compartment (or in alternative embodiments into one of a plurality of outlet channels) via an open blister pocket to entrain the medicament powder content thereof. In some aspects, a plurality of outlet channels, when a plurality of medicament carriers are present, allows for each medicament to be maintained in a separate airflow until the airflow exits the dispenser and reaches the user. In some aspects, a plurality of outlet channels each exit the dispenser via the same opening within the medicament access body. In some aspects, after entraining separate medicaments, each of a plurality of airflows are joined and exit the dispenser via a single outlet compartment, e.g., a single outlet channel.

According to certain embodiments, the flow director can also contain a surface for guiding the medicament carrier, such a surface being on the external area of the flow director, an area not responsible for the passage of an airflow comprising and entrained medicament. Such a surface could comprise a slot, an edge, a slit, a curve, a compression, a ridge or other type of protrusion, or other similar or equivalent element suitable to impact the direction of or maintain the appropriate direction or positioning of a medicament carrier.

According to certain embodiments, e.g., the inlet vent openings provide the primary or the sole entry point for airflow into the medicament dispenser device, and particularly to the open dose storage area, e.g., blister pocket of a blister strip medicament carrier, during inhaled use of the dispenser device by a patient. Thus, suitably no other air inlet or other air entry point is provided to the housing and the housing itself provides a relatively airtight barrier to the entry of outside air therein by any other means.

According to certain embodiments, the dispensing mechanism can comprise any number of inlet vent openings, having any shape or design, to facilitate the drawing in of air external to the device to the inlet compartment of the flow director. It can be appreciated that the number of air inlet vents can impact the volume and velocity of air drawn into the dispenser. Further, it can be appreciated that the shape, design, and orientation of the air inlets can impact the volume and velocity of the air drawn through them. In certain aspects, the dispensing mechanism can comprise a plurality of inlet air vents, such as approximately two to about 30 air inlet vents, such as about 3 to about 25 air vents, about 4 to about 20 air inlet vents, such as between about five and about 15 air inlet vents, such as approximately two, approximately six, approximately 10, approximately 14, approximately 18, approximately 22, approximately 26, or approximately 30 air vents. In some aspects, the shape of any one air vent can be the same as any other one or more air vents. In some aspects, the shape of any one air vent can differ from that of any other one or more air vents. In some aspects, the air vents can have a circular, square, rectangular, oval, squircular, or another geometric shape. In some aspects, the air vents can have an elongated shape of a slit or slot. In some aspects, such an elongated shape such as a slit or slot can be straight or curved. In some aspects, slit or slot-shaped vents are arranged such that are oriented one on top of the other to create a stack of air vents located in relatively close proximity to one another and the medicament access body.

In one embodiment of the disclosure, a single flow director is present in the dispenser for use with two medicament carriers. In some aspects, the flow director can define more than one inlet compartment and outlet compartment, such as multiple inlet compartments and multiple outlet channels, as would typically be the case in an embodiment of the dispenser where the flow director is designed for use in the dispensing of medicament from more than one open dose storage area at a time, e.g., from open dose storage areas of multiple medicament carriers (e.g., an open blister pocket of multiple blister strip medicament carriers). In one aspect, the flow director described herein is suitable for use in a medicament dispenser device for the delivery of medicament powder from an open blister pocket of each of plural medicament carriers, the flow director comprising plural pairings of airflow guides, each said pairing associated with an open blister pocket of one of said plural medicament carriers. Thus, for example, in a medicament dispenser herein arranged to dispense powder from a pair of opened pockets, each pair associated with a single elongate form medicament carrier, and each one of the pair associated with a pair of inlet and outlet channels.

According to one aspect, the dispensing mechanism can include a flow director for each opened dose storage area of a medicament carrier, e.g., opened blister pocket of a blister strip medicament carrier. In such an embodiment, the dispensing mechanism can include two flow directors for dispensing the medicament from two medicament carriers individually. In one aspect, the flow directors can be distinct for each of the medicament carriers and can be positioned adjacent or close to one another, such as being positioned in a side-by-side orientation with respect to one another.

The flow director can either be designed as a single, integral component or as a sub-assembly or part of an adjacent component and is typically formed as a molded part. The flow directors and similarly any flow channel or compartment therein, e.g., an inlet compartment, outlet compartment, or outlet channel, can be provided as a unitary molded component. In an alternative aspect, multiple flow directors or individual compartments within a flow director can be separated by a partition wall. In another aspect, the flow directors can be molded separately and then joined together during the assembly process. The flow directors or individual compartments within a flow director can be joined through permanent bonding methods, including, but not limited to ultrasonic welding and ultraviolet cured adhesives and the like. According to some embodiments, the flow director can be permanently joined to the inner housing assembly on a notch during the assembly process.

In one aspect, the flow director can include a pair of inlet compartments and a pair of outlet compartments for each medicament carrier. Such a pair of outlet compartments can be referred to as outlet channels. In one aspect, the flow director can be common for each of the medicament carriers. In certain embodiments, the flow director can include a common inlet compartment and distinct outlet channels (which, together, may be referred to as an outlet compartment) for each of the medicament carriers. In certain embodiments, the flow director can comprise distinct inlet compartments and a common outlet compartment. In certain other embodiments, the flow director can include distinctly separate inlet compartments and distinctly separate outlet channels for each medicament carrier present in the dispenser. In one aspect, the distinct dose portion of each of the medicament carriers does not combine or mix with any other distinct dose portion of any another medicament carrier within the flow director. According to certain aspects of the invention, which can be combined with any other aspect or embodiment or combination of aspects or embodiments, separate airflows entrain medicament from each open dose storage unit, e.g., from each medicament carrier, and maintain such separate airflows comprising entrained medicament separately until each airflow exits the dispenser or also or alternatively reaches the user. According to alternative embodiments, separate airflows can entrain medicament from each open dose storage unit., e.g., from each of a plurality of medicament carriers, and, after having entrained such medicament, be joined with other airflows to create a single airflow which exits the dispenser or also or alternatively reaches the user as a mixed, single airflow.

In use, all airflow into the flow director is via the inlet vents. In use, airflow is then directed to one or more inlet compartment(s). In common embodiments, the inlet compartment(s) and outlet compartment or outlet channels are positioned such that when a medicament-containing, open blister pocket of each medicament carrier is positioned adjacent thereto, the airflow can be directed from the inlet compartment(s) to the outlet compartment or outlet channels via the open blister pocket to entrain said medicament and enable transport thereof in the airflow from the inlet compartment(s) to the outlet compartment(s).

In certain aspects, the flow director is integral to one or more other components of the medicament dispenser. In alternative aspects, the flow director is separable from the other components of the medicament dispenser. In one aspect, as has been previously described, the flow director is provided as a separable snap-fit component to the medicament dispenser, and the flow director and/or medicament dispenser device is provided with snap-fit features (e.g., located on the body of the dispenser device) to enable this mode of fitting. In common embodiments, the flow director is arranged for receipt by a medicament dispenser at a location that is intermediate between a medicament access body for the delivery of medicament in inhaled form by a patient; and an opening location (such an opening location can also be referred to herein as an opening station), at which an opened dose storage area of a medicament carrier, e.g., an opened blister pocket of a blister strip medicament carrier is presented to the flow director (i.e., at which its medicament contents may be accessed and entrained).

According to embodiments of the dispenser described herein, the dispenser is designed such that all airflow into the dispenser is via the inlet vent openings, and that all airflow passing through the inlet vent openings enters the flow director. In some aspects, the flow director then can serve to "separate" that total airflow into a first portion and a second portion, It may thus be appreciated that, in use, the total airflow entering the inlet vent openings is separated into a) a first portion, which can be directed to a first open dose storage area, e.g., open blister pocket, of a first medicament carrier to entrain the medicament powder as a first airflow, and b) a second portion which can be directed to a second open dose storage area, e.g., open blister pocket, of a second medicament carrier to entrain the medicament powder as a second airflow.

The flow director herein is suitable for use in a medicament dispenser device in which the patient breathes in, e.g., inhales, to create all airflow through the flow director. In some aspects, the airflow is separated within the dispenser into a plurality of separate airflows, such as for example a first and a second airflow. In an embodiment, the first airflow and the second airflow remain distinct within the flow director and do not mix while within the device. In another embodiment, the first portion of the airflow transports entrained medicament powder of a first medicament carrier and the second portion of the airflow transports entrained medicament powder of a second medicament carrier separately without mixing both medicament powders together in the flow director/dispenser. In certain embodiments, one portion of the airflow does not impact another airflow portion within the flow director or within the dispenser.

According to certain aspects, the flow director can, wholly or in part, comprise one or more elements that can aid in reducing the tendency of medicament to stick thereto as it passes within an airstream from the dose storage area to the exit of the flow director. For example, in some aspects, the flow director can be comprised of or be coated with one or more materials acting as a deterrent to the adhesion of medicament thereto. In some aspects, such a material could be a material that modifies the surface tension of the flow director's surface. According to certain aspects, helpful such materials can comprise but not be limited to high-density polyethylene (HDPE), a fluoropolymer, or a modified acetal material. In some aspects, no such effort is made to reduce the potential adhesion of medicament to the flow director.

According to certain embodiments, the dispenser described herein comprises a medicament access body to provide access by the user to the medicament held therein. In some aspects, a medicament access body cover is provided to protect the medicament access body, e.g., mouthpiece, from the ingress of dirt and contaminants during storage. According to certain embodiments, the medicament access body cover can also be engaged with a driver such that the medicament access body cover becomes part of the actuation mechanism of the device, e.g., the medicament access cover can be provided for actuating the dispensing mechanism.

According to certain embodiments, the medicament access body cover can comprise an element for engaging the medicament access body cover with the driver, a means for establishing a rotational access point, and an edge which can aid in the positioning of a medicament access body cover when in a fully opened position against the dispenser housing.

According to certain aspects, which can be combined with any other aspect or embodiment or combination of aspects or embodiments described herein, the medicament dispenser can comprise an element for engaging the medicament access body cover with the driver. Such an engagement mechanism can be any mechanism suitable for attaching or otherwise connecting the medicament access body cover to a driver such that the medicament access body cover becomes an element of the actuation mechanism of the dispenser. In some aspects, such a connection can be a permanent connection, such as, for example, when the two elements (medicament access body cover and driver) are mechanically fused or are designed as a single component. According to alternative aspects, the medicament access body cover can be designed as a separate and, in some aspects, a removable component that is attached via a reversible mechanism. In some aspects, such removal cannot be performed by the user. According to certain aspects, the driver comprises an element for interfacing with and engaging an element of the medicament body cover, as previously described. Accordingly, the element of the medicament body cover can be any element suitable for engaging with that element of the driver, e.g., an insert for a slit, hole or indentation, or a protrusion, or a clip, snap or another correspondingly shaped element. According to certain embodiments, the element on the driver designed to engage an element of the medicament access body cover is a flange. Hence, in some embodiments, the element on the medicament access body cover designed to engage the corresponding element on the driver is a mounting key, also referred to herein as a flange key.

In one aspect, the medicament access body cover is adapted to couple with the dispensing mechanism. In one aspect, the medicament access body cover can interact with the driver and actuate the dispensing mechanism. In certain aspects, the engagement of the flange on the driver gear with the mounting key of the medicament access body cover provides for a rotational relationship such that the mounting key engaged with the flange rotates the flange, and hence the driver, in the same direction as the direction of rotation of the mouthpiece cover.

According to certain aspects, which can be combined with any other aspect or embodiment or combination of aspects or embodiments described herein, the medicament dispenser can comprise an element for establishing a rotational access point. In some aspects, such a rotation access point is a pivot point, and a hole in the medicament access body cover, herein referred to as a pivot hole, is provided to allow for appropriately positioning the medicament access body about the pivot point. Such a pivot point could alternatively be established using a hinge mechanism, a flexible member, e.g., an elastic member, or the like. According to certain embodiments, as is further herein and as is described in the description of figures provided herein, a pin attached to a medicament housing base plate can be positioned within the pivot hole of the medicament access body cover to appropriately and securely position the medicament access body relative to the dispenser. In some aspects, the medicament access body cover rotates about an axis passing through the mounting key and the pivot hole, the pivot hole rotating about the pin of the base plate.

According to certain aspects of the present invention, which can be combined with any other aspect or embodiment or combination of aspects or embodiments described herein, the medicament access body cover can be arranged for rotational movement about an axis. The medicament access body cover can, in some embodiments, be configured such that it moves from a first position to a third position over a rotational path. The movement of the medicament access body cover can, in some aspects, be reversible. The cover can also or alternatively be configured such that it slides along the surface of the assembly, e.g., an inside surface of the medicament access body cover sliding over or along the outside surface of the medicament dispenser (e.g., the medicament dispenser housing).

In some aspects, the medicament access body cover is pivotally mounted to the housing. In some aspects, the medicament access body cover may be movably mounted to the medicament dispenser for sequential movement from a first position, in which the medicament access body, e.g., mouthpiece, is covered, to a second position, in which the mouthpiece is still covered, to a third position in which the mouthpiece is uncovered. According to one embodiment, the inlet vent openings are covered at the first and the second positions of the medicament access body cover. In another aspect, the inlet vent openings are uncovered at the third position of the medicament access body cover. Suitably, the medicament access body cover, e.g., a mouthpiece cover, is pivotally movable relative to the housing. Also or alternatively, the mouthpiece cover is rotationally movable relative to the housing. Suitably, in some aspects, the medicament access body cover, e.g., mouthpiece cover, is arranged to rotate and slide over the housing.

The movement of the medicament access body cover, e.g., a mouthpiece cover, from a first position to the second position, can, in some embodiments, result in no movement of any one or more carrier engagements. In one aspect, the range of motion of the medicament access body cover, e.g., a mouthpiece cover, from the first position to the second position is about 10 degrees to about 20 degrees. In yet another aspect, the motion of the medicament access body cover, e.g., a mouthpiece cover, from the first position to the second position is about 12 degrees. In yet another aspect, the motion of the medicament access body cover, e.g., a mouthpiece cover, from the first position to the second position is about 18.75 degrees. According to certain embodiments, the idle movement of the medicament access body cover, e.g., a mouthpiece cover, can be in the range of between about 5 degrees to about 25 degrees. In another aspect, the idle movement of the medicament access body cover, e.g., a mouthpiece cover, can be about 10 degrees, about 12 degrees, about 15 degrees, about 18 degrees, or about 20 degrees. In certain aspects, the idle movement of about 15 degrees of an advancer can correspond to about 12 degrees of idle movement of the medicament access body, e.g., a mouthpiece cover. In certain other aspects, idle movement of about 15 degrees of an advancer can correspond to about 20 degrees of the idle movement of the medicament access body, e.g., mouthpiece, cover. In certain other aspects, idle movement of about 20 degrees of an advancer can correspond to about 20 degrees of idle movement of the medicament access body cover, e.g., a mouthpiece cover.

According to certain aspects, which can be combined with any other aspect or embodiment or combination of aspects or embodiments described herein, the medicament dispenser can comprise an element to aid in the positioning of a medicament access body cover when in a fully opened position against the dispenser housing. In certain aspects, the medicament access body cover rests over the housing after having completed an actuation, e.g., upon the medicament access body cover having reached the third position. In some aspects, a ridge or stopping point is present within the exterior of the dispenser housing such that an edge of the medicament access body cover rests on such a ridge to aid in holding the medicament access body in place, not allowing it to rotate further past an actuated position, and, for example, preventing damage to the medicament access body in doing so. The ability of the edge of the medicament access cover to rest against a component on the exterior of the housing can provide physical support to create damage, provide a haptic signal that the actuation is complete (e.g., the medicament access body cover has reached a fully actuated position), and also or alternatively aid in holding the medicament access cover in an open position.

The medicament access body cover can comprise a circular form, a non-circular form, a spherical form, a semi-spherical form, an elongate form, an ovular form, an oblong form, a triangular form, a quadrilateral form, including, but not limited to, a square form, a rectangular form, and various trapezium forms, an hourglass form, or other shapes. The medicament access body cover can provide a flat surface or a resting surface. At least a portion of the medicament access body cover can be shaped for ease of grip by the user. The medicament access body cover may include dots or waves gripping to provide for an efficient gripping.

The medicament dispenser can include at least one locking mechanism, enabling the dispenser to remain locked in at least one position in which the dispenser is ready for inhalation and/or the cover is in the closed position. According to certain aspects, the locking mechanism can be any locking mechanism sufficient to maintain the medicament access body cover in a stable position, such as a clipping, snapping, ridged lip fitting, sliding lock fitting, or a similar or equivalent mechanism. In some aspects, the locking mechanism can be used for preventing unintended and accidental opening of the cover, providing the need for at least a limited amount of force to open or close the medicament access body cover. In some aspects, the locking mechanism can be used for preventing further rotation of the cover once it is completely opened (actuated). The locking mechanism can, in certain embodiments, be used for indicating that the medicament dispenser is ready for dispensing the dose. The locking mechanism can be used for providing audible and haptic or kinesthetic feedback to the user indicating complete opening, complete closing, or both complete opening or complete closing of the cover.

According to certain aspects of the invention, the medicament dispenser can comprise a component designed to hold and encase all components of the dispensing mechanism. In some aspects, such an element is designed to hold all components of the dispensing mechanism. According to certain embodiments, such an element is designed as a first part of a two-part housing of the medicament dispenser, such as may be described as a bottom or base cover and a top cover. In certain aspects, the element designed to hold all components of the dispensing mechanism is a base cover. In certain aspects, the top cover and bottom cover are reversibly engageable such that the top and bottom cover can be separated. In certain aspects, the top cover and bottom cover are maintained as a single piece by means of a connection mechanism placed entirely around the lip or rim of the base cover or alternatively placed in two or more locations about the lip or rim of the base cover and/or the top cover to hold the two units together. In some aspects, such a connection mechanism could be a clip, snap, slide fit, tongue-and-groove (the base cover comprising either the tongue or the groove of such a mechanism), or the like. One will appreciate that many such mechanisms could be feasible for holding the two components together to support optimal and successful stability of the components held therein and for the efficient and successful operation of the device.

According to certain embodiments, the base cover can comprise an element that allows the medicament access body cover to enter and rotate the driver, e.g., to enter and rotate the flange of the driver. In some aspects, such an element can be a cutout, indentation, or groove. In some aspects, such an element is centrally located and can be referred to as a central groove. The central groove can allow the medicament access body cover to enter and rotate the flange of the driver. The central groove along with other design elements of the base cover, are exemplified within the description of figures provided herein. According to certain aspects of the invention, the medicament dispenser can comprise a component designed to aid in encasing all components of the dispensing mechanism. In some aspects, such an element is designed to cover all components of the dispensing mechanism in coordination with a first element, as in, for example, the element being designed as a second part of a two-part housing of the medicament dispenser, such as may be described as a bottom or base cover and a top cover. In certain aspects, the element designed to hold all components of the dispensing mechanism is a top cover.

As previously stated, in certain aspects, the top cover and bottom cover are maintained as a single piece by means of a connection mechanism placed entirely around the lip or rim of the base cover or alternatively placed in two or more locations about the lip or rim of the base cover and/or the top cover to hold the two units together. In some aspects, such a connection mechanism could be a clip, snap, slide fit, tongue-and-groove (the top cover comprising either the tongue or the groove of such a mechanism), or the like. One will appreciate that many such mechanisms could be feasible for holding the two components together to support optimal and successful stability of the components held therein and for the efficient and successful operation of the device.

According to certain aspects, the top cover can comprise a central groove to allow the medicament access body cover to enter and rotate about the pin of the base plate, inlet vent openings, whose function has been previously described herein, and status identifier window for visualizing an event status.

According to certain embodiments, the medicament access body cover is not engaged with a driver and the medicament access body cover is not functional as a part of the actuation mechanism of the device.

In some aspects, a lever is provided on the housing. According to certain embodiments, the lever can also be engaged with a driver such that the lever becomes part of the actuation mechanism of the device, e.g., the lever can be provided for actuating the dispensing mechanism.

According to certain aspects, which can be combined with any other aspect or embodiment or combination of aspects or embodiments described herein, the medicament dispenser can comprise an element for engaging the lever with the driver. Such an engagement mechanism can be any mechanism suitable for attaching or otherwise connecting the lever to a driver such that the lever becomes an element of the actuation mechanism of the dispenser. In some aspects, such a connection can be a permanent connection, such as, for example, when the two elements (lever and driver) are mechanically fused or are designed as a single component. According to alternative aspects, the lever can be designed as a separate and, in some aspects, a removable component that is attached via a reversible mechanism. In some aspects, such removal cannot be performed by the user.

In one aspect, the lever is adapted to couple with the dispensing mechanism. In one aspect, the lever can interact with the driver and actuate the dispensing mechanism.

In some aspects, the lever is pivotally mounted to the housing. In some aspects, the lever is directly or indirectly engaged with the central gear. In some aspects, the flange on the central gear fits within the accepting grooves on the lever. In some aspects, the lever may be movably mounted to the medicament dispenser for sequential movement from a first position to a third position.

The movement of the lever, from a first position to the second position, can, in some embodiments, result in no movement of any one or more carrier engagements. In one aspect, the range of motion of lever from the first position to the second position is about 10 degrees to about 20 degrees. In yet another aspect, the motion of the lever, from the first position to the second position is about 12 degrees. In yet another aspect, the motion of the lever, from the first position to the second position is about 18.75 degrees. According to certain embodiments, the idle movement of the lever, can be in the range of between about 5 degrees to about 25 degrees. In another aspect, the idle movement of the lever, can be about 10 degrees, about 12 degrees, about 15 degrees, about 18 degrees, or about 20 degrees. In certain aspects, the idle movement of about 15 degrees of an advancer can correspond to about 12 degrees of idle movement of the lever. In certain other aspects, idle movement of about 15 degrees of an advancer can correspond to about 20 degrees of the idle movement of the lever. In certain other aspects, idle movement of about 20 degrees of an advancer can correspond to about 20 degrees of idle movement of the lever

The lever can comprise, a square form, a rectangular form, and various trapezium forms, an hourglass form, or other shapes including, but not limited to, circular form, a non-circular form, a spherical form, a semi-spherical form, an elongate form, an ovular form, an oblong form, a triangular form, a quadrilateral form. At least a portion of the lever can be shaped for ease of grip by the user. The lever may include dots or waves gripping to provide for an efficient gripping. lever can comprise, a slot to facilitate user to rotate the lever wherein the slot is elongated portion of the lever which can be of any shape and size.

In some aspects, a central groove is provided within the top cover to allow the medicament access body cover to enter and rotate about the pin of the base plate. This possible aspect of dispenser devices of the invention is further exemplified in the figures and their descriptions incorporated herein.

As previously described, and which will not be repeated in detail here, inlet vent openings can be provided, e.g., within the top cover, to provide an inlet for external air drawn into the dispenser by inhalation of the user during device use. In some aspects, the inlet vent openings can be located discreetly on the top cover to provide for an air inlet through the flow director. According to certain embodiments, the air can enter the inner compartment of the flow director through the inlet vent openings and can then pass through the outer compartment via the opened dose storage area of the medicament carrier, e.g., blister pocket of a blister strip medicament carrier, carrying the medicament powder. The air, along with medicament powder, can exit the dispenser through the medicament access body opening, e.g., mouthpiece opening. The flow of air contributes to the aerosolization of the contents of the blister pocket and thereby emptying of the contents of the blister pockets via the medicament access body opening.

According to certain aspects, which can be combined with any other aspect or embodiment or combination of aspects or embodiments described herein, the medicament dispensers of the present invention can comprise an event-related status indicator. Such an event-related status indicator is often referred to in the art as a dose counter, and may be referred to as a dose counter elsewhere herein; however it should be made clear that a status indicator incorporated within the medicament dispenser can be capable of providing an indicator status that may be a dose count (e.g., doses administered or doses remaining), however, can also provide information such as that described in US provisional patent applications numbers 62/910,551 and 62/913,982. Accordingly, the top cover can comprise a status identifier window to relay dose status information to the user. The term "dose indicator window" can also be used herein to describe this window. Such a window is an opening within the top cover of any size, shape, or configuration which allows the user to view the event indicator or one or more event indicators within the dose counter.

In some aspects, the dispenser comprises an event-related status indicator for providing information about the dose status of medicament or medicaments within the dispenser. Such an event status indicator can be referred to herein as a "dose counter," however, the indicator can provide other information aside from or in addition to a dose count, as is described in previously referred to US provisional patent applications numbers 62/910,551 and 62/913,982. Accordingly, according to certain embodiments, the medicament dispenser can include a dose counting or an indicating mechanism for counting or indicating the number of doses released from the dispenser in total. According to certain aspects, the event-related status indicator incorporated into the dispenser described herein can also or alternatively describe the number of doses dispensed from each medicament carrier individually. In some aspects, such an indication can be the number of doses having been dispensed or alternatively such an indication can be of the number of doses remaining, in total, or for any one or combination of more than one medicament carriers. In some aspects, the event-related status indicator can provide a count of the number of doses left to be taken or a count of the number of doses taken (e.g., having been dispensed). The status identifier of the event-related status indicator can, in some aspects, be visible via a status indicator window in the top cover of the dispenser.

According to some embodiments, an event-related status indicator of the medicament dispenser can, in certain aspects, provide an actuation counter for counting the number of actuations of the dispenser.

According to certain aspects of the present invention, an event-related status indicator incorporated within the dispensers of the present invention can be entirely or essentially entirely mechanical (e.g., non-electronic). According to alternative aspects, an event-related status indicator incorporated within the dispensers of the present invention can be non-mechanical (e.g., electronic, digital) in form. According to yet further aspects, an event-related status indicator incorporated within the dispensers of the present invention can comprise both mechanical (non-electronic) and non-mechanical (electronic and/or digital) components.

According to certain aspects of the present invention, the medicament dispenser can include a mechanism of actuation wherein the mechanism of actuation also provides for a mechanism for the increase in dose counter reading during the dispensation of the medicament.

In some embodiments, which can be combined with any other aspect or embodiment or combination of aspects or embodiments described herein, the medicament dispenser can include a mechanism of actuation wherein the movement of the mechanism of actuation of the dispenser, e.g., the sufficient movement of the medicament access body cover, the driver, and all necessary components to result in a complete actuation and delivery of one or more doses of medicament from one or more carriers to a position available for administration also provides a mechanism for updating the event-related status indicator. Such an actuation of the event-related event indicator can result in a mechanical (i.e., non-electronic), a non-mechanical (e.g., electronic, digital), or a combination of a mechanical and a non-mechanical event status display.

According to certain aspects of the present invention, the medicament dispenser can include a space, e.g., a compartment within the dispenser, for housing and storage of a power source (e.g., battery), an electronic circuit, and also or alternatively electronic measurement instruments. According to certain aspects, the power source (e.g., battery), electronic circuit and/or electronic measurement instruments can be located in a compartment of the medicament access body cover or within the housing having dedicated space to accommodate said power source (e.g., battery), electronic circuit and electronic measurement instruments.

The medicament dispenser of the present invention can, in some aspects, comprise an electronic data management system. In some embodiments, which can be combined with any other aspect or embodiment or combination of aspects or embodiments described herein, the electronic data management system can include data input, data output, and or a combination of data input and data output capability, and can in some aspects further comprise a memory for storage of data; a microprocessor for performing operations on said data; and a transmitter for transmitting a signal relating to the data or the outcome of an operation on the data.

In some aspects, the electronic data management system is integral with the body of the dispenser. Alternatively, in alternative aspects, the electronic data management system forms part of a base unit that is reversibly associable with the body.

The medicament dispenser described herein, according to any one of the aspects, embodiments, or combinations of aspects or embodiments disclosed herein, can be used to treat a patient in need having a condition treatable by a medicament capable of being administered via inhalation. According to certain aspects, the invention described herein is a method of treating a condition treatable by an inhalable medicament, the method comprising administering one or more medicaments via a medicament dispenser of any one of the aspects or embodiments described herein. In certain aspects, the method of administration comprises first loading a medicament dispenser with a plurality of elongate form medicament carriers comprising a plurality of discrete medicament doses in powder form. Upon medicament carrier loading, the method of administration comprise actuating the dispenser such that before any advancement of any medicament carrier upon actuation, tension is applied to at least one waste stream of multiple waste streams generated by the emptied medicament carrier; the application of tension being followed by the advancing of the plurality of medicament carriers within the dispenser independently from one another to a common opening location in the dispenser such that a medicament dose within a dose storage location, if present, of each medicament carrier becomes available for administration. Once a dose becomes available, e.g., a dose storage area has been opened and made available by appropriate positioning near the flow director, the method comprises the user inhaling the one or more medicament doses available for administration via a medicament access body. Upon inhalation, the airstream generated by the inhalation separately entrains the medicament of each available medicament dose is maintained separately from one another until the airstream exits the dispenser and reaches the user via the medicament access body.

According to certain aspects, the medicament access body is a mouthpiece, and therefore the method of administration comprises administering a medicament via oral inhalation. According to alternative aspects, the medicament access body is configured for being utilized with a human nose, and thus the method of administration comprises administering a medicament via nasal inhalation.

According to certain aspects of the present invention, the dispensers described herein can be used to treat any condition treatable by an inhalable medicament, such conditions being exemplified by but not limited to asthma, chronic obstructive pulmonary disease (COPD), bronchitis, lung infections, interstitial pulmonary fibrosis (IPF), pulmonary arterial hypertension (PAH), cystic fibrosis, Non-CF Bronchiectasis or lung transplant.

According to certain aspects, the invention described herein is a method of administering a plurality of medicaments by inhalation to an individual, the plurality of medicaments being maintained in separate airstreams prior to reaching the orifice of the individual into which the plurality of medicaments is being inhaled, the method comprising administering the plurality of medicaments to the individual via the dispenser according to any one of aspects, embodiments, or combinations of aspects or embodiments described herein.

Any feature, structure, or step disclosed herein can be replaced with or combined with any other feature, structure, or step disclosed herein, or omitted. Further, for purposes of summarizing the disclosure, certain aspects, advantages, and features of the inventions have been described herein. It is to be understood that not necessarily any or all such advantages are achieved in accordance with any particular embodiment of the inventions disclosed herein. No individual aspects of this disclosure are essential or indispensable.

To further aid in the description of the present invention, a set of drawings ("figures") has been provided and described. The drawings further explain and elaborate on, through exemplification, at least some of the embodiments described herein. In some cases, the drawings introduce alternative embodiments to those embodiments which have been previously described or are elsewhere herein described. Accordingly, the figures aim to provide a more thorough understanding of the breadth and spirit of the present invention without limiting the scope of the full disclosure provided here.

### BRIEF DESCRIPTION OF THE DRAWINGS

The following figures and related description thereof are incorporated to explain better and exemplify the invention described herein, without limiting its scope.

Some elements of the embodiments of the dispenser or its components are exemplified in a particular form and, accordingly, are sometimes described in such exemplary embodiments with a descriptive name in connection with the description of the embodiments shown in the figures that follow below. To aid the reader in understanding the various exemplary embodiments of the invention described in connection with figures, the following description of several such terms is provided here:
a. The use of the term "meshed" is commonly used within the description of figures to describe a movable engagement between two components, e.g., which is present when two gear-like mechanisms interface with each other.
b. The driver is exemplified as a driver gear (the driver may be described herein as a "driver gear" or "drive gear").
c. The carrier engagement is exemplified as a "blister cuvette," blister cuvette being a term to describe the carrier engagement being capable of engaging the blister of a blister strip-form elongated medicament carrier by means of pockets, or carved out areas of the carrier engagement capable of engaging with the dose storage area of a medicament carrier described as "cuvettes."
d. The advancer is exemplified as a "ratchet assembly," ratchet assembly being a term to describe an advancer comprising multiple elements as described herein, one such component being a ratcheting component.
e. The dose storage area of one or more medicament carriers is exemplified as a blister compartment, blister pocket, or blister cavity, each term being synonymous and representing the dose storage area of a blister strip medicament carrier wherein the dose storage area is an area of the base sheet of the carrier describable as a pocket, compartment, or cavity, wherein the lid sheet of the carrier is not sealed but is capable of holding the dose of medicament therein.
f. As described elsewhere herein but included here for clarity, the first waste collector is exemplified herein as a wrapping wheel or wrapping assembly.
g. The second waste collector is exemplified as a winding assembly, and further wherein the waste capture component of the winding assembly is exemplified as a winding wheel, comprising the waste collection surface exemplified as a winding surface, a waste access point exemplified as an entry slit, a locking engagement exemplified as a locking ridge, inner movably engageable engaging elements exemplified as inner teeth, and movably engageable driving element with engagement elements, such elements being gear teeth. The component of the second waste collector responsible for tensioning the waste stream being collected is exemplified as a tensioning mechanism; the tensioning mechanism is exemplified as comprising a lid connection element embodied as a blister post and the lid gripping element embodied as a blister guide, a central connection element in the form of a base, and an engagement unit of the tensioning mechanism in the form of ratchet arms.
h. The medicament access body is exemplified as a mouthpiece and the medicament access body cover is exemplified as a mouthpiece cover.
i. The element capable of providing a connection between the driver and the medicament access body (mouthpiece) cover is a flange on the driver, the mouthpiece cover exemplified as having a suitably configured flange key such that the two elements facilitate cooperation between the two components.
j. The flow director for directing airflow within the dispenser is exemplified as a "flow channel," and the airflow guides being a part of the flow director/flow channel are exemplified as grids.
k. While not described in detail, references are made to the event-related status indicator, exemplified as a dose counter ("dose counter" being a term which can in some cases, be limited, but which, however, is intended to describe the multiple functionalities of an event-related status indicator described previously herein). The status identifier window, wherein an event status is viewable, is referred to as a dose indicator window.

As noted above, this description of terms used in connection with the embodiments described below in connection with the figures is intended to aid the reader in understanding such exemplary aspects of the invention and should not be interpreted as limiting the scope of the invention in any way. Additional aspects of the invention described in connection with the figures will also be discussed below, but generally using terminology already introduced in this disclosure, such as the first waste capture component.
FIGS. 1A-1B show front and rear views of an embodiment of the medicament dispenser.
FIGS. 2A-2D show a demonstration of the "Open, Inhale, Close" mechanism of the medicament dispenser shown in FIGS. 1A-1B.
FIG. 3 shows a partially exploded view of the medicament dispenser shown in FIGS. 1A-1B.
FIG. 4 shows an exploded view of a first embodiment of the dispensing mechanism of the medicament dispenser shown in FIGS. 1A-1B.
FIG. 5 shows the front view of the first embodiment of the dispensing mechanism where the dispenser is shown absent its housing.
FIG. 6 shows a rear view of the first embodiment of the dispensing mechanism where the dispenser is shown absent its housing.
FIGS. 7A and 7B show a view of the gear mechanism of the first embodiment of the medicament dispenser shown in FIGS. 1A-1B.
FIGS. 8A to 8E show details of the dispensing mechanism when prepared for use in sequential steps corresponding to those of FIGS. 2A-2D.
FIG. 9 shows an exploded view of a second embodiment of the dispensing mechanism of the medicament dispenser shown in FIGS. 1A-1B.
FIGS. 10A-10B show a view of gear mechanisms where the dispenser is shown absent its housing.
FIG. 11 shows an interaction of the first ratchet assembly and first blister cuvette of the medicament dispenser shown in FIGS. 1A-1B.
FIG. 12 shows an interaction of the second ratchet assembly and second blister cuvette of the medicament dispenser shown in FIGS. 1A-1B.
FIG. 13 shows an exploded view of a third embodiment of the dispensing mechanism of the medicament dispenser shown in FIGS. 1A-1B.

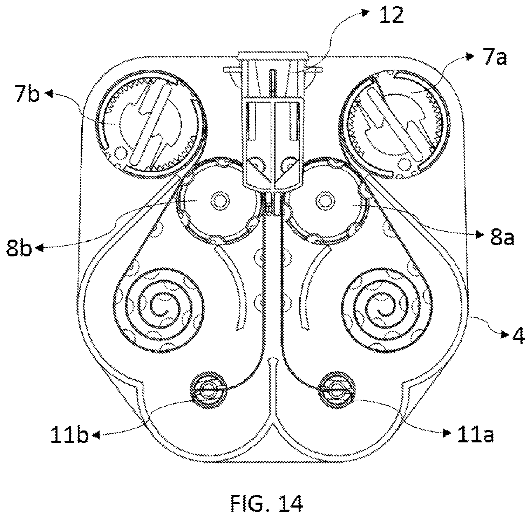

FIG. 14 shows a front view of the third embodiment of the dispensing mechanism where the dispenser is shown absent its housing.
FIG. 15 shows a rear view of the third embodiment of the dispensing mechanism where the dispenser is shown absent its housing.
FIG. 16 shows an interaction of the first ratchet assembly and first blister cuvette of the medicament dispenser shown in FIGS. 1A-1B.
FIG. 17 shows an interaction of the second ratchet assembly and second blister cuvette of the medicament dispenser shown in FIGS. 1A-1B.
FIGS. 18A to 18E show details of the third embodiment of the dispensing mechanism when prepared for use in sequential steps corresponding to those of FIGS. 2A-2D.
FIG. 19 shows an exploded view of a fourth embodiment of the dispensing mechanism of the medicament dispenser shown in FIGS. 1A-1B.
FIG. 20 shows a front view of the fourth embodiment of the dispensing mechanism where the dispenser is shown absent its housing.
FIG. 21 shows a rear view of a fourth embodiment of the dispensing mechanism where the dispenser is shown absent its housing.
FIGS. 22A to 22G show details of the fourth embodiment of the dispensing mechanism when prepared for use in sequential steps corresponding to those of FIGS. 2A-2D.
FIG. 23 shows a mouthpiece cover of the medicament dispenser shown in FIGS. 1A-1B.
FIG. 24 shows an embodiment of the outer view of the top cover that is compatible with the medicament dispenser described herein.
FIG. 25 shows an embodiment of the inner view of the top cover that is compatible with the medicament dispenser described herein
FIG. 26 shows an embodiment of the outer view of the base cover that is compatible with the medicament dispenser described herein.
FIG. 27 shows an embodiment of the inner view of the base cover that is compatible with the medicament dispenser described herein.
FIG. 28 shows a view of the inner housing assembly that is compatible with the medicament dispenser described herein.
FIG. 29 shows an underside view of an embodiment of the inner housing assembly shown in FIG. 28.
FIG. 30 shows an underside view of another embodiment of the inner housing assembly that is compatible with the medicament dispenser described herein.
FIG. 31 shows a view of the driver gear that is compatible with the medicament dispenser described herein.
FIG. 32 shows an underside view of the driver gear shown in FIG. 31.
FIG. 33 shows a view of the driver gear that is compatible with the medicament dispenser described herein.
FIG. 34 shows an underside view of the driver gear shown in FIG. 33.
FIG. 35 shows a view of the interim driver gear that is compatible with the medicament dispenser described herein.
FIG. 36 shows a view of the ratchet gear that is compatible with the medicament dispenser described herein.
FIG. 37 shows an exploded view of a first embodiment of the blister cuvette and the ratchet assembly that is compatible with the medicament dispenser described herein.
FIG. 38 shows a view of the blister cuvette shown in FIG. 37.
FIG. 39 shows an embodiment of the inside view of the blister cuvette shown in FIG. 38.
FIG. 40 shows another embodiment of the inside view of the blister cuvette shown in FIG. 38.
FIG. 41 shows a view of the driven member of the ratchet assembly shown in FIG 37.
FIG. 42 shows an underside view of the driven member of the ratchet assembly shown in FIG. 37 (and FIG. 41).
FIG. 43 shows a view of the driving member of the ratchet assembly shown in FIG. 37.
FIG. 44 shows a view of the ratchet assembly shown in FIG. 37.
FIG. 45 shows an exploded view of a second embodiment of the blister cuvette and ratchet assembly that is compatible with the medicament dispenser described herein.
FIG. 46 shows an inside view of the blister cuvette shown in FIG. 45.
FIG. 47 shows a view of the ratchet assembly shown in FIG. 45.
FIG. 48 shows an inside view of the ratchet assembly shown in FIG. 45 (and FIG. 47).
FIG. 49 shows an exploded view of a third embodiment of the blister cuvette and ratchet assembly that is compatible with the medicament dispenser described herein.
FIG. 50 shows an inside view of the blister cuvette shown in FIG. 49
FIG. 51 shows a view of the driven member of the ratchet assembly shown in FIG. 49.
FIG. 52 shows a view of the driving member of the ratchet assembly shown in FIG. 49.
FIG. 53 shows an exploded view of a fourth embodiment of the first blister cuvette and the first ratchet assembly that is compatible with the medicament dispenser described herein.
FIG. 54 shows an inside view of the first blister cuvette shown in FIG. 53.
FIG. 55 shows a view of the first ratchet assembly shown in FIG. 53.
FIG. 56 shows an exploded view of a fourth embodiment of the second blister cuvette and the second ratchet assembly that is compatible with the medicament dispenser described herein.
FIG. 57 shows an inside view of the second blister cuvette shown in FIG. 56.
FIG. 58 shows an embodiment of the second ratchet assembly shown in FIG. 56.
FIG. 59 shows an exploded view of a fifth embodiment of the first blister cuvette and first the ratchet assembly that is compatible with the medicament dispenser described herein.
FIG. 60 shows an inside view of the first blister cuvette shown in FIG. 59.
FIG. 61 shows an embodiment of the first ratchet assembly shown in FIG. 59.
FIG. 62 shows an exploded view of another embodiment of the first blister cuvette and the first ratchet assembly that is compatible with the medicament dispenser described herein.
FIG. 63 shows a view of the first ratchet assembly shown in FIG. 62.
FIGS. 64 and 65 show different views of the second ratchet assembly and interim driver gear.
FIG. 66 shows a first embodiment of the winding assembly that is compatible with the medicament dispenser described herein.
FIG. 67 shows an exploded view of the first embodiment of the winding assembly shown in FIG. 66.
FIG. 68 shows a view of the tensioning mechanism of the winding assembly shown in FIG. 66 (and FIG. 67).
FIG. 69 shows an underside view of the tensioning mechanism of the winding assembly shown in FIG. 66 (and FIGS. 67 and 68).
FIG. 70 shows a view of the winding wheel of the winding assembly shown in FIG. 66 (and FIG. 67).
FIGS. 71A-71C show a demonstration of the sheet tensioning mechanism.
FIG. 72 shows a second embodiment of the winding assembly that is compatible with the medicament dispenser described herein.
FIG. 73 shows an exploded view of the second embodiment of the winding assembly shown in FIG. 72.
FIG. 74 shows a view of the tensioning mechanism of the winding assembly shown in FIG. 72 (and FIG. 73).
FIG. 75 shows a view of the winding wheel of the winding assembly shown in FIG. 72 (and FIG. 73).
FIGS. 76A-76B show a demonstration of the sheet tensioning mechanism.
FIG. 77 shows a third embodiment of the winding assembly that is compatible with the medicament dispenser described herein.

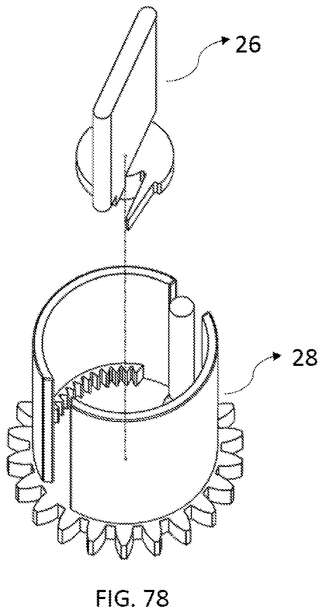

FIG.78 shows an exploded view of the third embodiment of the winding assembly shown in FIG. 77.

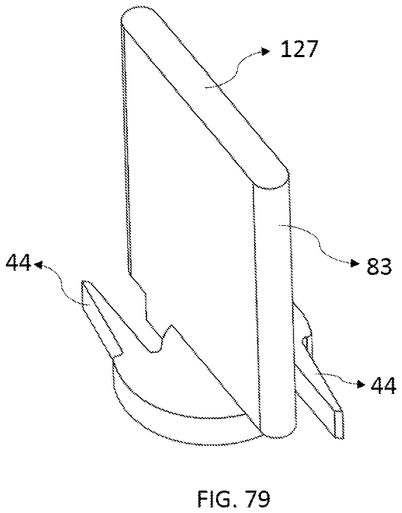

FIG. 79 shows a view of the tensioning mechanism of the winding assembly shown in FIG. 77 (and FIG. 78).
FIG. 80 shows a view of the winding wheel of the winding assembly shown in FIG. 77 (and FIG. 78).
FIGS. 81A-81B show a demonstration of the sheet tensioning mechanism.
FIG. 82 shows a fourth embodiment of the winding assembly that is compatible with the medicament dispenser described herein.

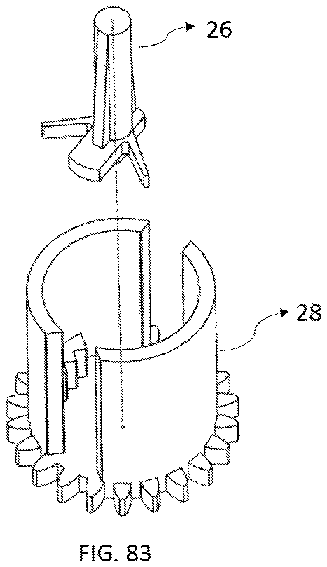

FIG. 83 shows an exploded view of the fourth embodiment of the winding assembly shown in FIG. 82.

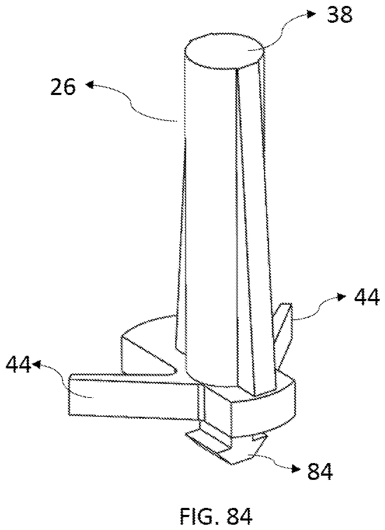

FIG. 84 shows a view of the tensioning mechanism of the winding assembly shown in FIG. 82 (and FIG. 83).
FIG. 85 shows a view of the winding wheel of the winding assembly shown in FIG. 82 (and FIG. 83).
FIGS. 86A-86B show a demonstration of the sheet tensioning mechanism.
FIG. 87 shows a fifth embodiment of the winding assembly that is compatible with the medicament dispenser described herein.

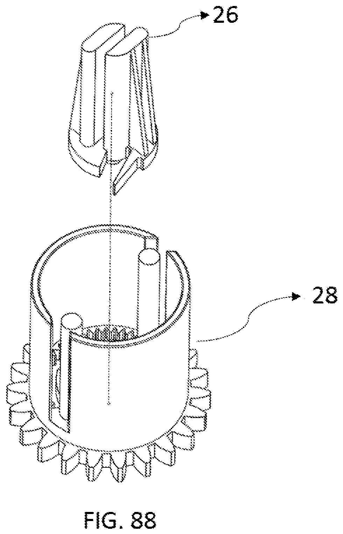

FIG. 88 shows an exploded view of the fifth embodiment of the winding assembly shown in FIG. 87.

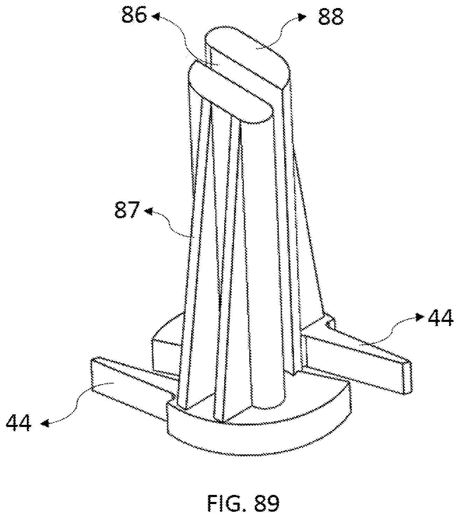

FIG. 89 shows a view of the tensioning mechanism of the winding assembly shown in FIG. 87 (and FIG. 88).
FIG. 90 shows a view of the winding wheel of the winding assembly shown in FIG. 87 (and FIG. 88).
FIGS. 91A-91B show a demonstration of the sheet tensioning mechanism.
FIG. 92 shows a view of an embodiment of the wrapping wheel that is compatible with the medicament dispenser described herein.
FIG. 93 shows a view of another embodiment of the wrapping wheel that is compatible with the medicament dispenser described herein.
FIG. 94 shows a view of the base plate that is compatible with the medicament dispenser described herein.
FIG. 95 shows a view of the medicament dispenser, illustrating the inward airflow and outward airflow.
FIG. 96 shows a view of the medicament dispenser, illustrating the inward airflow in the flow channel.
FIG. 97 shows a view of the flow channel illustrating the inward airflow and outward airflow.
FIG. 98A shows a cut-away view of the flow channel illustrating the inward airflow and outward airflow.
FIG. 98B shows a cut-away view of the flow channel illustrating the inward and outward airflow.
FIG. 99 shows a top view of the flow channel.
FIGS. 100A-100B show views of the medicament carrier that is compatible with the medicament dispenser described herein.

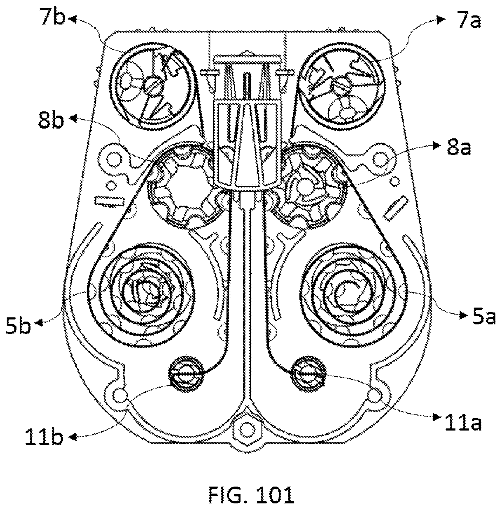

FIG. 101 shows the front view of the fifth embodiment of the dispensing mechanism where the dispenser is shown absent its housing.

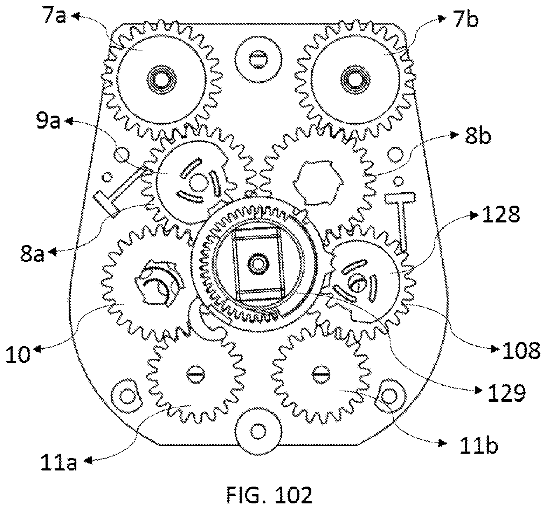

FIG. 102 shows a rear view of the fifth embodiment of the dispensing mechanism where the dispenser is shown absent its housing.

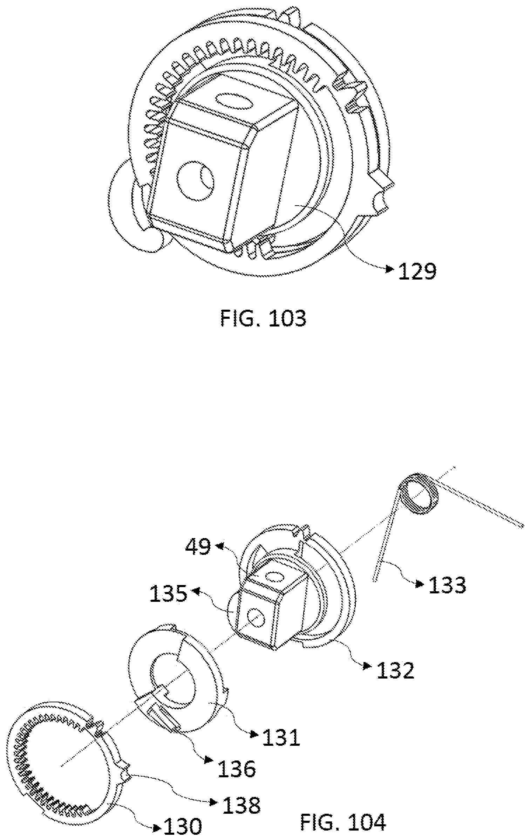

FIG. 103 shows driver gear or central gear assembly that is compatible with fifth embodiment of the medicament dispenser described herein.
FIG. 104 shows an exploded view of driver gear assembly that is compatible with fifth embodiment of the medicament dispenser described herein.

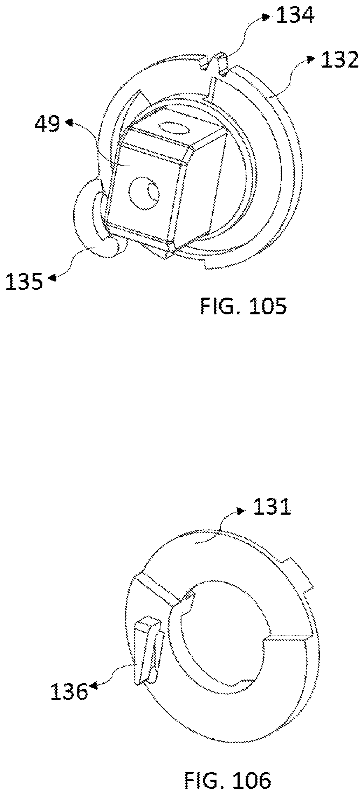

FIG. 105 shows view of primary driver gear that is compatible with fifth embodiment of the medicament dispenser described herein.
FIG. 106 shows view of spring loaded ratcheting disc that is compatible with fifth embodiment of the medicament dispenser described herein.

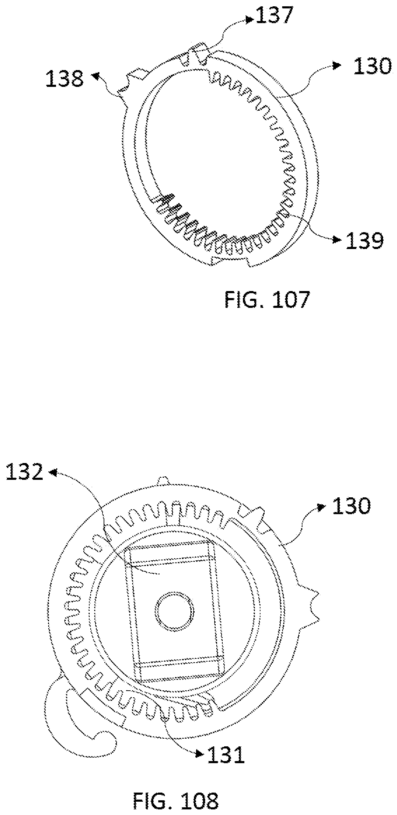

FIG. 107shows view of secondary gear that is compatible with fifth embodiment of the medicament dispenser described herein.
FIG. 108 shows position of the driver gear or central gear when it is at initial position.

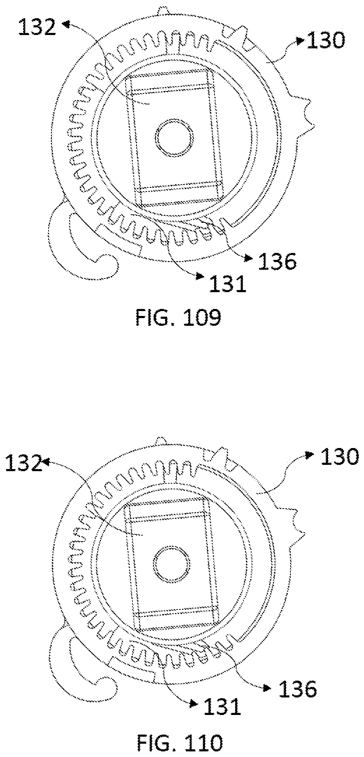

FIG. 109 shows position of the driver gear or central gear after first actuation.
FIG. 110 shows position of the driver gear or central gear after second actuation.

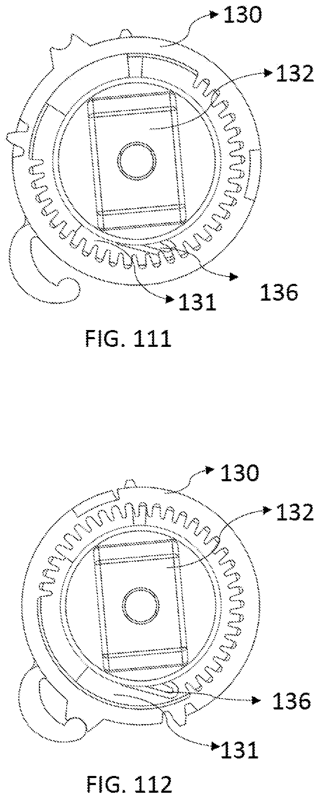

FIG. 111 shows position of the driver gear or central gear after fifteen actuation.
FIG. 112 shows position of the driver gear or central gear after thirty one actuation.

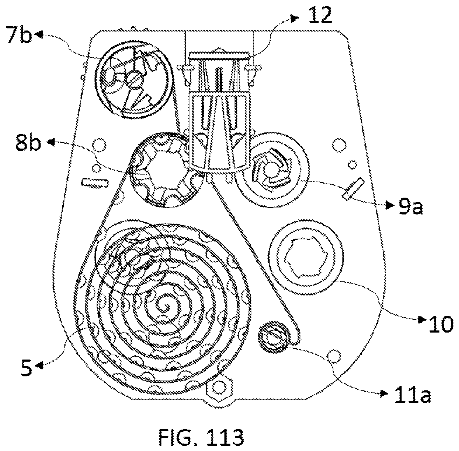

FIG. 113 shows the front view of the sixth embodiment of the dispensing mechanism where the dispenser is shown absent its housing.

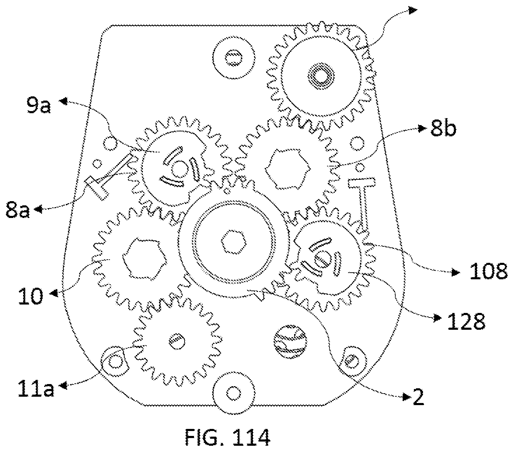

FIG. 114 shows a rear view of the sixth embodiment of the dispensing mechanism where the dispenser is shown absent its housing.
FIG. 115 show front closed view of an embodiment of the medicament dispenser.
FIG. 116 show front open view of an embodiment of the medicament dispenser.
FIG. 117 show rear open view of an embodiment of the medicament dispenser.
FIG. 118 show rear closed view of an embodiment of the medicament dispenser.
FIGS. 119-FIG. show a demonstration of the "Open, Click, Inhale, Close" mechanism of the medicament dispenser shown in FIG. 130- FIG. 133.
FIG. 123 shows outer cover that is compatible with the medicament dispenser described herein.
FIG. 124 shows lever compatible with the medicament dispenser described herein.
FIG. 125 shows front view of housing that is compatible with the medicament dispenser described herein.
FIG. 126 shows central gear that is compatible with the medicament dispenser described herein.

### DETAILED DESCRIPTION OF EXEMPLARY EMBODIMENTS

To better illustrate aspects of the invention, particular embodiments and their operation will be described in this section, often with reference to the devices and components shown in the Figures. It is intended that the scope of the present invention herein disclosed should not be limited by any particular embodiment described herein. While various embodiments of the present invention have been described above, it should be noted that they have been presented by way of example only, and not limitation. Numerous changes to the disclosed embodiments can be made in accordance with the disclosure herein without departing from the spirit or scope of the invention. Further, any single embodiment or aspect described within this disclosure can be combined with any one or more other herein described aspects or embodiments, the description of such combinations considered to be incorporated herein as if such a combination had been described in detail.

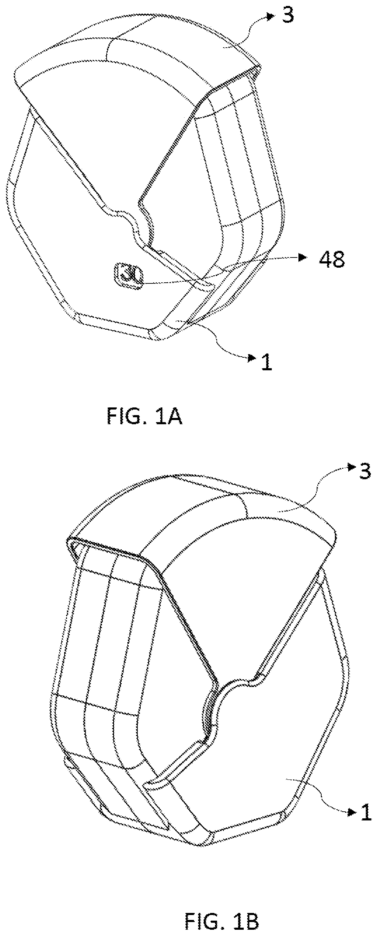

Figures 1A and 1B illustrate a medicament dispenser (DPI) according to one aspect of the disclosure. The medicament dispenser can include a housing (1) and a mouthpiece cover (3). Figure 1A also shows a dose indicator window (48) through which dose indicia '30' is visible. The window (48) provides a unified view of the dose indicator for user feedback on the number of remaining doses.

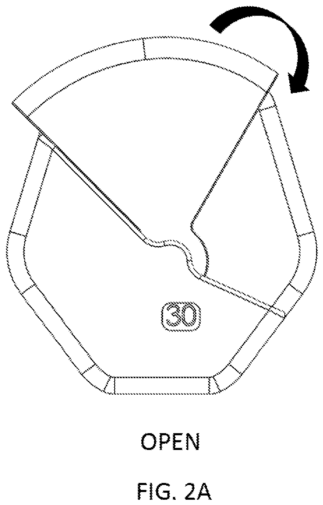

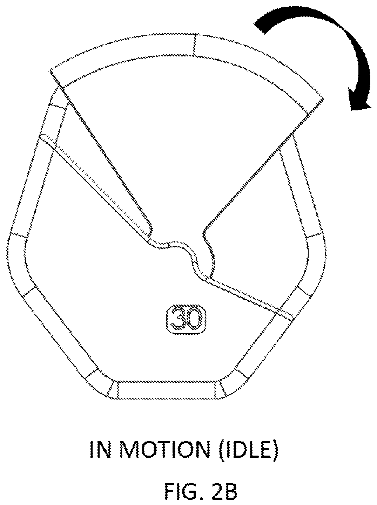

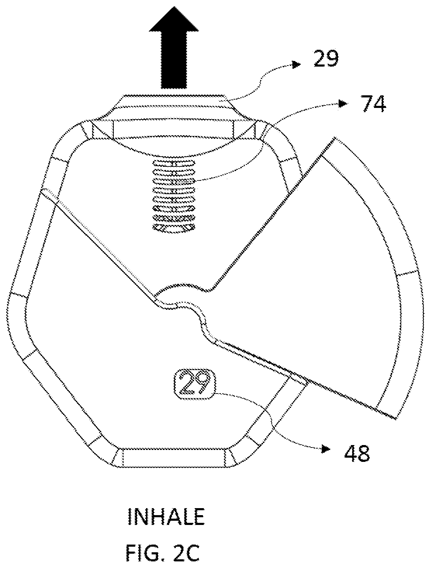

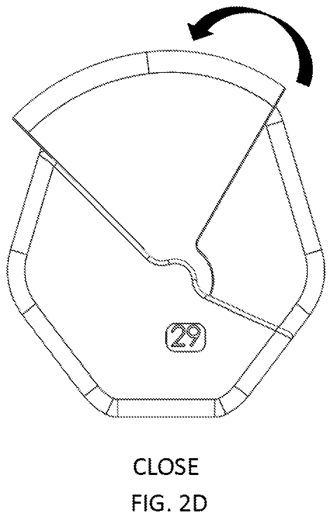

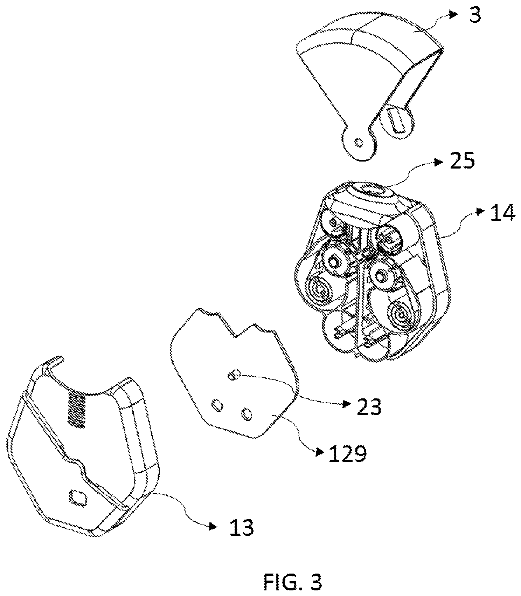

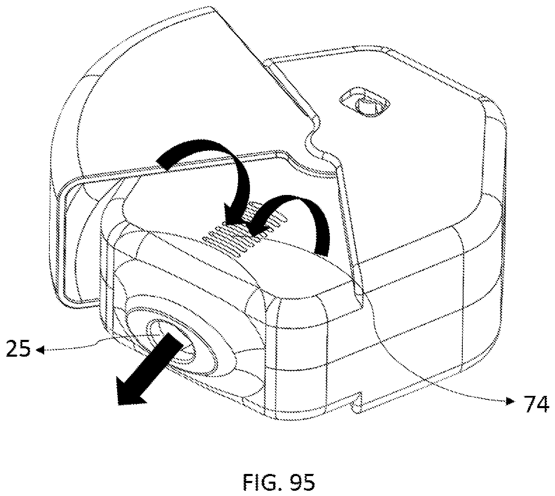

Figures 2A and 2D illustrate the demonstration of the "Open, Inhale, Close" mechanism of the medicament dispenser shown in Figures 1A and 1B. In Figure 2A, the mouthpiece cover is in a first/closed position in which mouthpiece (29) and inlet vent openings (74) are not visible. In Figure 2B, the mouthpiece cover (3) has been moved to a second position in which mouthpiece (29) and inlet vent openings (74) are not yet visible. The movement of the mouthpiece cover (3) from the first position to the second position does not actuate the dispensing mechanism (not shown) in the dispenser nor actuation of the dose counter (the mechanism is not shown). In Figure 2C, the mouthpiece cover (3) has been moved further to a third position in which mouthpiece (29) and inlet vent openings (74) are visible. As a result of the further movement from the second position to the third position, the dispensing mechanism (not visible) is actuated in the dispenser to make a medicament dose available for inhalation. The movement has also resulted in the actuation of the dose counter of the dispenser such as to decrease the dose count indicia shown in the dose indicator window (48) by one unit, here to a new reading of '29'. The patient can inhale the medicament through the mouthpiece opening (25) (see Figure 3 and 95). In Figure 2D, after inhalation, the mouthpiece cover (3) has been returned to the first/closed position (as in Figure 2A). As shown in Figures 2A to 2C, the inlet vent openings (74) of the top cover (13) may be covered by the mouthpiece cover (3) when its first and second positions (Figures 2A and 2B) and fully revealed when the mouthpiece cover (3) is in its third or open position (Figure 2C).

Figure 3 shows a partially exploded view of the medicament dispenser according to one aspect of the disclosure. As shown, the medicament dispenser can comprise a top cover (13), a base plate (129) which in some aspects can incorporate other elements aiding in the construction of or operation of the medicament dispenser such as but not limited to one or more pins (e.g., (23) of Figure 3), a base cover (14) which in this embodiment is shown as comprising a mouthpiece opening (25), and a mouthpiece cover (3), each of which is further described elsewhere herein.

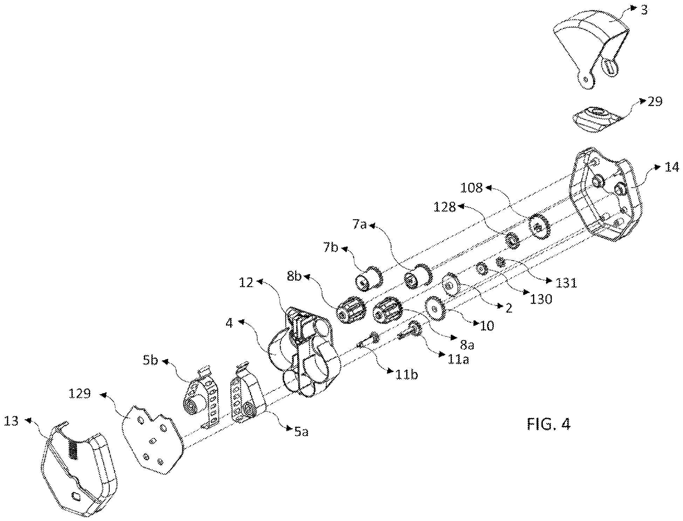

Figures 4 illustrate a fully exploded view of a first dispensing mechanism that is compatible with the medicament dispenser described herein. Figure 4 illustrates in more detail components of the medicament dispenser, which can reside, when fully assembled, between the base cover (14) and the base plate (129) shown previously in Figure 3. The components shown in the embodiment of the dispenser illustrated in Figure 4 are described elsewhere herein but include a top cover (13), a base plate (129), two medicament carriers shown as a first medicament carrier (5a) and a second medicament carrier (5b), an inner housing assembly (4), a flow channel (12), a first blister cuvette (8a) and a first wrapping wheel (11a) which associate with the first medicament carrier (5a), a second blister cuvette (8b) and a second wrapping wheel (11b) which associates with the second medicament carrier (5b), a first winding assembly (7a) which associates with the first medicament carrier (5a), a second winding assembly (7b) which associates with the second medicament carrier (5b), an interim gear (10), a driver gear (2), a secondary interim gear (130), a tertiary interim gear (131), a ratchet gear (128), an interim driver gear (108), a base cover (14), a mouthpiece (29), and a mouthpiece cover (3).

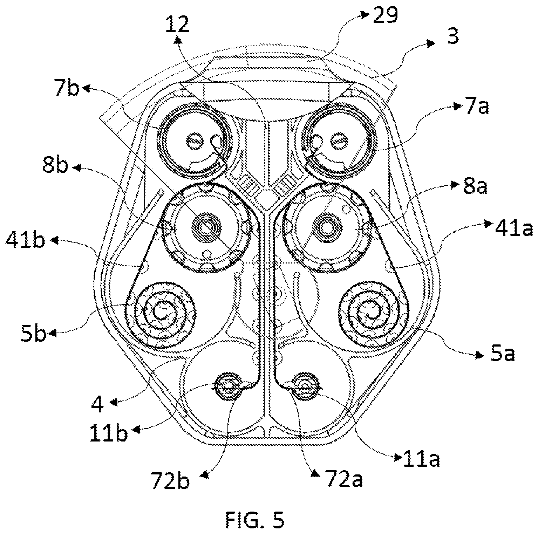

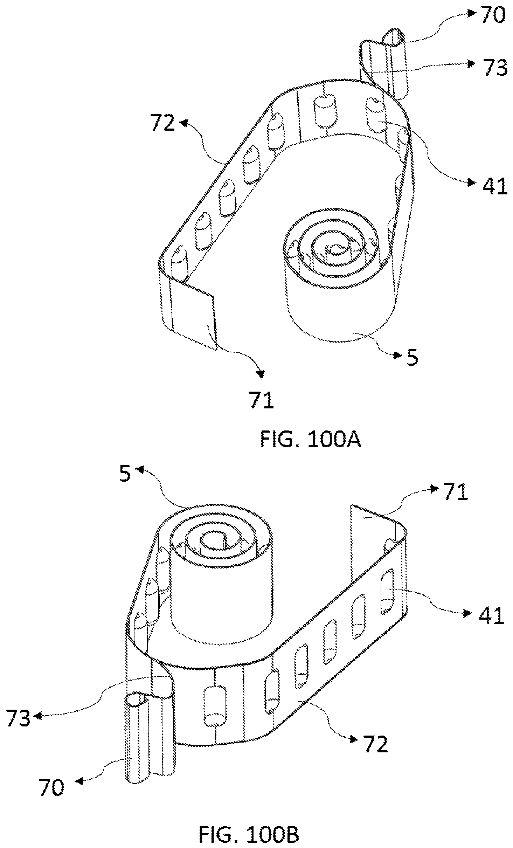

Figure 5 shows a front view of the dispensing mechanism and first and second medicament carriers (5a) and (5b). The first and second medicament carriers (5a) and (5b) are positioned within respective left and right compartments of the inner housing assembly (4). Each medicament carrier (5a) and (5b) engages with respective blister cuvettes (8a) and (8b). One carrier may contain one or more different medicament(s) than the other carrier. At an opening location (an opening location is a position within the medicament dispenser wherein the medicament carrier is opened or where an individual dose of medicament is otherwise held within the medicament carrier and positioned for access), the base sheet and the lid sheet parts (72) and (73) (best exemplified in Figures 100a and 100b) of each medicament carrier (5a) and (5b)) are peelably separable. The resulting empty base sheet (72a) and (72b) (shown in Figure 5) coils up in respective compartments. The wrapping wheels (11a) and (11b), for wrapping the base sheets of the two medicament carriers, each anchor the end of each respective base sheet (72a) and (72b) in the compartments. Progressive rotation of each respective wrapping wheel results in the base sheets (72a) and (72b) being wound up therearound into a tight coil. The rotation of each winding assembly (7a) and (7b) is coupled to that of the respective blister cuvettes (8a) and (8b) such the rotation of the blister cuvettes (8a) and (8b) rotates new blister packs of the medicament carriers (5a) and (5b) into the appropriate position within the opening location, the winding assemblies (7a) and (7b) correspondingly winding the lid sheets peeled away from each respective medicament carrier base sheet. The resulting separated lid sheets (73a) and (73b) are wound onto/into the respective winding assemblies (7a) and (7b) respectively.

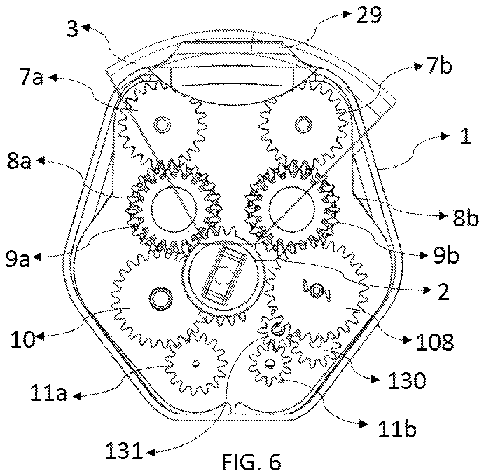

The mechanism of operation of the first dispensing mechanism and the medicament dispenser, according to one embodiment, is presented in Figures 6, 7A-7B and 8A-8E, each figure providing a rear view of the dispenser (as in from the opposing side of the dispenser than that shown in Figure 5).

In use, the mouthpiece cover (3) is movable along the housing (1). In Figure 6, the movable mouthpiece cover (3) is shown in its first position as previously described. As a means of introducing other figures to be later described, as in Figure 5, the mouthpiece cover (3) and housing (1) are transparent to provide a view into the medicament dispenser. A similar approach to illustration has been taken in many figures incorporated herein. From this perspective, many elements of the dispenser of this embodiment, and when used in describing further embodiments elsewhere herein many elements of the dispenser of that particular embodiment, are visible. From this perspective, the following minimum elements of the embodiment shown in Figure 6 are visible (more elements can be visible; however a select number are illustrated and labeled, as is the case similarly with other figures disclosed herein): a mouthpiece cover (3); a mouthpiece (29); a first winding assembly (7a) and a second winding assembly (7b); a first blister cuvette (8a) and a second blister cuvette (8b); a first ratchet assembly (9a) and a second ratchet assembly (9b); a driver gear (2); an interim gear (10); an interim driver gear (108); a secondary interim gear (130); a first wrapping wheel (11a) and a second wrapping wheel (11b); and a tertiary interim gear (131).

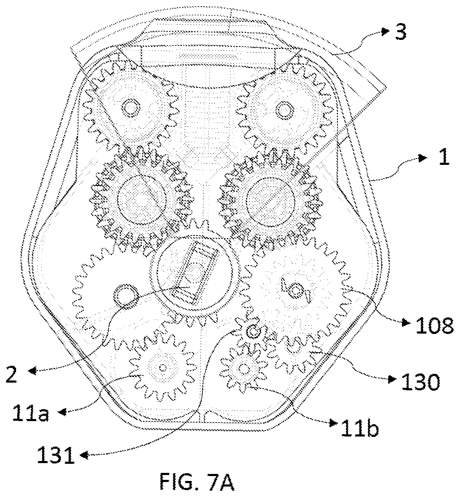

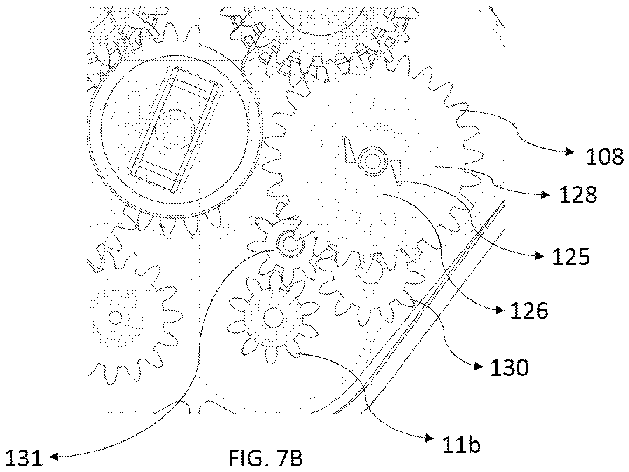

Figures 7A-7B are a similar illustration to Figure 6; however, Figures 7A-7B provide views of the gear mechanism of the dispenser with other non-labeled components in light shadowing as they relate to the positions of select components illustrated in Figure 6. Figure 7A illustrates the gear mechanism of the dispenser having the following components specifically labeled for orientation: mouthpiece cover (3); interim driver gear (108); secondary interim gear (130); driver gear (2); tertiary interim gear (131); and first and second wrapping wheels (11a) and (11b). Figure 7B illustrates a closeup view of one section of the gear mechanism of the medicament dispenser having the following components labeled: interim driver gear (108); ratchet gear (128); ratchet (125); inner gears (of ratchet gear) (126); secondary interim gear (130); second wrapping wheel (11b); and tertiary interim gear (131).

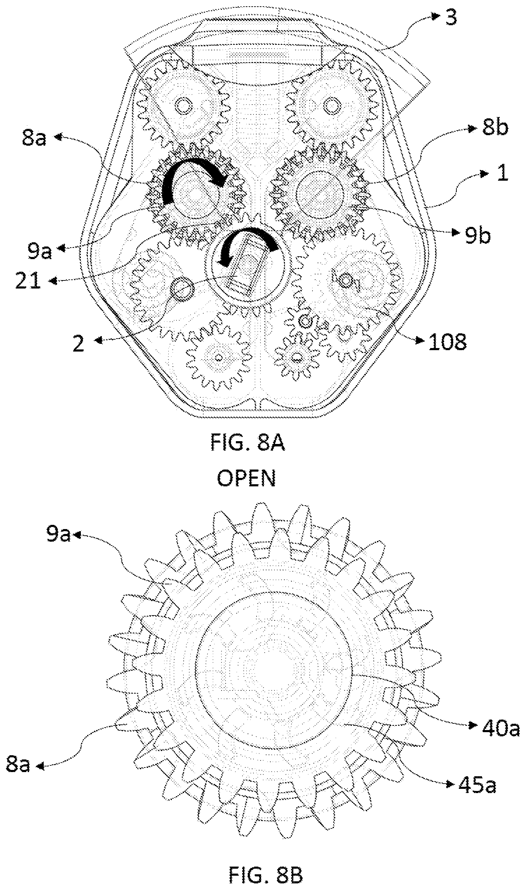

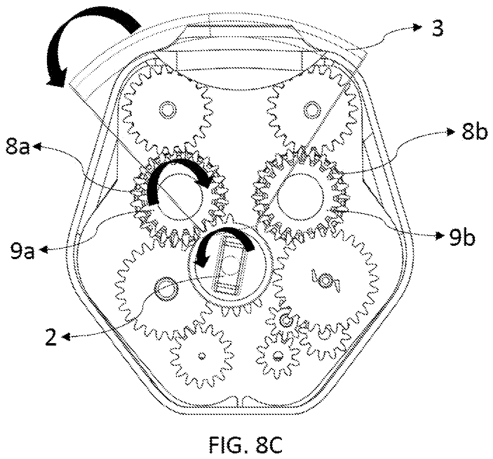

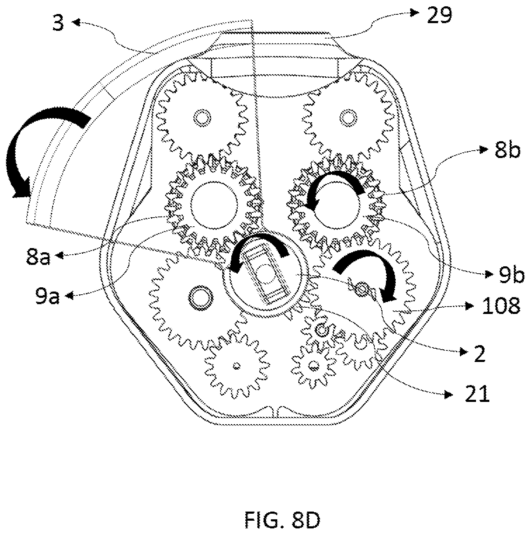

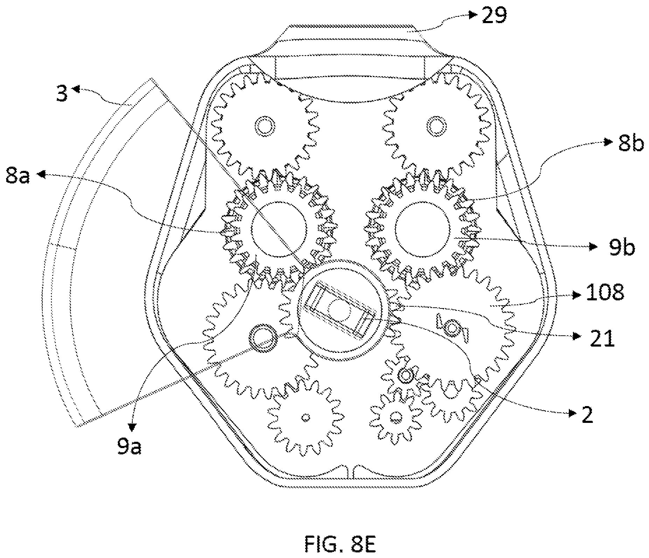

Figures 8A - 8E illustrate the dispensing mechanism when prepared for use, the figures corresponding to the mouthpiece positioning illustrated in Figures 2A - 2D. In Figure 8A, the mouthpiece cover (3) is in a first/closed position in which the mouthpiece cover (3) covers the mouthpiece (29) (not labeled). In Figure 8C, the mouthpiece cover has been moved about 12 degrees to a second position, in which the mouthpiece cover (3) still covers the mouthpiece (29) (not labeled). The movement of the mouthpiece cover from the first position to the second position does not result in the actuation of the blister cuvettes. In Figure 8D and 8E, the mouthpiece cover has been moved further to a third position, in which the mouthpiece cover (3) uncovers the mouthpiece (29). The further movement of the mouthpiece cover (3) from the second position to the third position results in movement of the blister cuvettes. The mouthpiece cover (3) rotates the driver gear (2) which in turn rotates the first ratchet assembly (9a) (see Figure 8A). The interim driver gear (108) is located between the driver gear (2) and the second ratchet assembly (9b) for allowing the rotation of ratchet assemblies (9a) and (9b) in the opposite direction. The first ratchet assembly (9a) is displaced at about 15 degrees from the ratchet tooth (45a) of the first blister cuvette (8a) (see Figure 8B). The first ratchet assembly (9a) does not rotate the first blister cuvette (8a) for the initial about 15 degrees rotation of the first ratchet assembly (9a). This idle movement of about 15 degrees of the first ratchet assembly (9a) corresponds to about 12 degrees of the idle movement of the mouthpiece cover (3) thus allowing for some "play" within the movement of the mouthpiece cover, such that if the mouthpiece cover is only slightly moved, or for example is bumped or wiggled such that it is rotated less than about 12 degrees, no rotation or actuation of the blister cuvette occurs. The first ratchet assembly (9a) rotates 1.25x faster than the driver gear (2). The first ratchet assembly (9a) rotates about 15 degrees and comes in contact with the ratchet tooth (45a) of the first blister cuvette (8a), and thereby the first blister cuvette (8a) rotates synchronously with the first ratchet assembly (9a). Upon rotation, the first blister cuvette (8a) advances a blister pocket, also referred to herein as a blister cavity (41a), the two terms being equivalent and both referring to the same element (not shown in Figures 8A - 8E), from the first medicament carrier (5a) (also not shown in Figures 8A - 8E). The driver gear (2) rotates the second ratchet assembly (9b) via interim driver gear (108). The driver gear (2) does not come in contact with the interim driver gear (108) while it is in contact with the first ratchet assembly (9a) (see Figures 8A and 8C - 8E). To provide context, additional components beyond those described in Figures 8A - 8E are described briefly here. These elements are described further elsewhere herein. The driver gear (2) does not rotate the second ratchet assembly (9b) until the blister cavity (41a) from the first medicament carrier (5a) is completely peeled open at the first pair of grids (62a) of the flow channel (12). Once the first medicament carrier (5a) is completely peeled open, the driver gear teeth (21) come into contact with the interim driver gear (108) and begins to rotate the second ratchet assembly (9b) and thereby the second blister cuvette (8b). The second medicament carrier (5b) continues to peel open until the mouthpiece cover (3) cannot be rotated any further. During this time, the second medicament carrier (5b) is completely peeled open at the second pair of grids (62b) of the flow channel (12). Both the medicament carriers (5a) and (5b) are peeled open successively (one after the other) during actuation. Advancement of the medicament carriers (5a) and (5b) cause the respective leading blister cavity (41a) and (41b) to be peeled open and successively brought into communication with the pair of grids (62a) and (62b) of the flow channel (12), which itself communicates with mouthpiece opening (25). It is at this time that the medicaments of the blister cavities are available for inhalation. The first blister cuvette gear teeth (34a) directly drive the first winding assembly (7a) and drive the first wrapping wheel (11a) via interim gear (10). The second blister cuvette gear teeth (34b) directly drive the second winding assembly (7b). The ratchet gear (128) located concentrically with the interim driver gear (108) drives the second wrapping wheel (11b) via secondary interim gear (130) and tertiary interim gear (131). The lid sheet of each of the medicament carriers (5a) and (5b) is successively peeled during each blister movement via a constant tension applied by the winding assemblies (7a) and (7b). The lid sheet of each of the medicament carriers (5a) and (5b) winds around the winding assemblies (7a) and (7b) during the operation of the dispenser. The base sheet of each of the medicament carriers (5a) and (5b) is advanced via the wrapping wheels (11a) and (11b). The first wrapping wheel (11a) is driven by the first blister cuvette (8a) via the interim gear (10). The second wrapping wheel (11b) is driven by the ratchet gear (128) via secondary interim gear (130) and tertiary interim gear (131). The ratchet gear (128) located under the interim driver gear (108) (e.g., is positioned at a lower plane than the interim driver gear (108) when viewed from a perspective of looking down on the interim driver gear (108) rotates the second wrapping wheel (11b) in one direction. The open end (71) of each of the base sheets is located within the respective wrapping wheel slits (69a) and (69b), and wraps around the wheel as it rotates during actuation. To access the medicament of the opened cavities of medicament carriers (5a) and (5b), the user places their mouth on the mouthpiece opening (25) and inhales. This state results in air (22) entering the device through the inlet vent openings (74) and passing through the opened blister cavities (41a) and (41b) at the pair of grids (62a) and (62b) and exiting the device through the mouthpiece opening (25). In doing so, the powder contained within the opened cavities (41a) and (41b) at the pair of grids (62a) and (62b) of the flow channel (12) is aerosolized and guided through the flow channel (12) and the mouthpiece opening (25) and delivered to the user as an orally inhaled medicament dose. Thus, in use, the user can simultaneously inhale one dose portion from each medicament carrier (5a) and (5b). In Figure 8D, the mouthpiece cover (3) after inhalation has been moved in the opposite direction (from the third position to the first position) to cover the mouthpiece (29). At this stage, the motion of the mouthpiece cover (3) rotates the ratchet assemblies (9a) and (9b) without moving the medicament carriers (5a) and (5b). The ratcheting mechanism of the ratchet assemblies (9a) and (9b) and blister cuvettes (8a) and (8b) prevents the rotation of the blister cuvettes (8a) and (8b) in the opposite direction.

Returning to Figures 8A to 8E, Figures 8A - 8E show the interaction and the movements of the components of the first dispensing mechanism. In the first/closed position of Figure 8A, the teeth (21) of the driver gear (2) are in a meshed relationship with the first ratchet assembly gear teeth (39a). The teeth (21) of the driver gear (2) do not mesh with the interim driver gear (108). In Figure 8B, the drive pin (40a) of the first ratchet assembly (9a) is not in engagement with the first blister cuvette ratchet tooth (45a). The about 12 degrees movement of the mouthpiece cover (3) from the first position to the second position results in about 15 degrees movement of the first ratchet assembly (9a) via driver gear (2), which further results in movement of the drive pin (40a) which then engages with the ratchet tooth of the first blister cuvette (45a) through the contacting surface (46a). When the mouthpiece cover (3) is moved to the second position, as illustrated in Figure 8C, the first blister cuvette (8a) is ready for movement but has not yet been moved, and hence the first medicament carrier (5a) has not been advanced. The further movement of the mouthpiece cover (3) from the second position to the third position, as shown in Figure 8D, results in simultaneous movement of the first ratchet assembly (9a) and the first blister cuvette (8a) through the engagement of the drive pin (40a) (refer to figure 8B) and the ratchet tooth (45a) to peel open the blister pocket (41a) of the first medicament carrier (5a) (not shown) at the first pair of grids (62a) (not shown) of the flow channel (12) (refer to Figure 5). Until this position, the teeth (21) of the driver gear (2) do not mesh with the interim driver gear (108). The further movement of the mouthpiece cover (3), illustrated in Figure 8E, causes the teeth (21) of the driver gear (2) to mesh with the interim driver gear (108) which meshes with the second ratchet assembly (9b). The drive pin (40b) (refer to Figure 8B) of the second ratchet assembly (9b) engages with the ratchet tooth (45b) of the second blister cuvette (8b) through the contacting surface (46b). The further movement of the mouthpiece cover (3) to the third position results in simultaneous movement of the second ratchet assembly (9b) and the second blister cuvette (8b) to peel open the blister pocket (41b) (not shown) of the second medicament carrier (5b) at the second grids (62b) (not shown) of the flow channel (12) (refer to Figure 5). At the third position, the opened blister pocket of each of medicament carriers (5a) and (5b) are available at the flow channel (12) (refer to Figure 5) for simultaneous inhalation by the patient through the mouthpiece opening (25) (within mouthpiece (29)). When the mouthpiece cover (3) is returned to its first/closed position, the reverse rotation of the ratchet assemblies (9a) and (9b) is not transmitted to the respective blister cuvettes (8a) and (8b). Thus, on each occasion, the mouthpiece cover (3) is fully opened and closed, the blister cuvettes (8a) and (8b) are incremented in one rotational direction only.

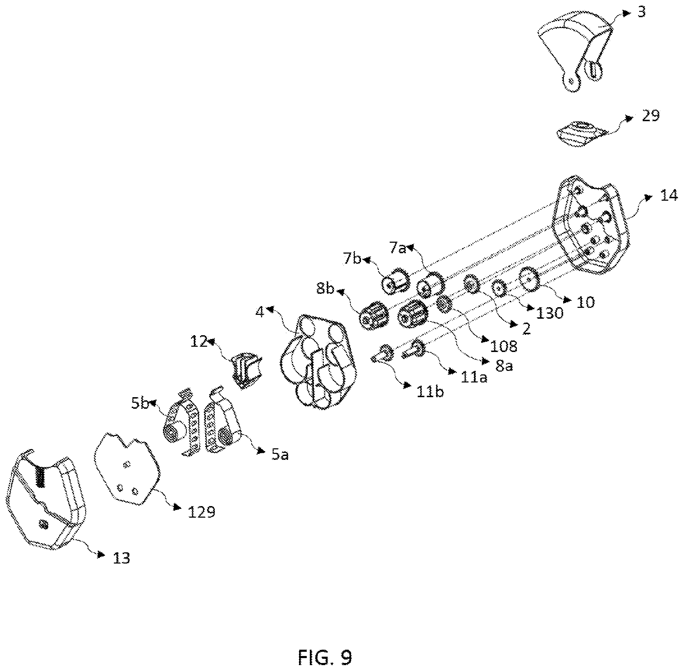

Figure 9 illustrates a fully exploded view of a second dispensing mechanism of the medicament dispenser described herein, e.g., a second embodiment of a dispensing mechanism of the medicament dispenser described herein. Figure 9 illustrates some components of the mechanism of this embodiment in relation to one another, the components comprising: a mouthpiece cover (3); a mouthpiece (29); a base cover (14); an interim gear (10); a secondary interim gear (130); a driver gear (2); an interim driver gear (108); a first winding assembly (7a) and a second winding assembly (7b); a first blister cuvette (8a) and a second blister cuvette (8b); a first wrapping wheel (11a) and a second wrapping wheel (11b); an inner housing assembly (4); a flow channel (12); a first medicament carrier (5a) and a second medicament carrier (5b); a base plate (129) and a top cover (13).

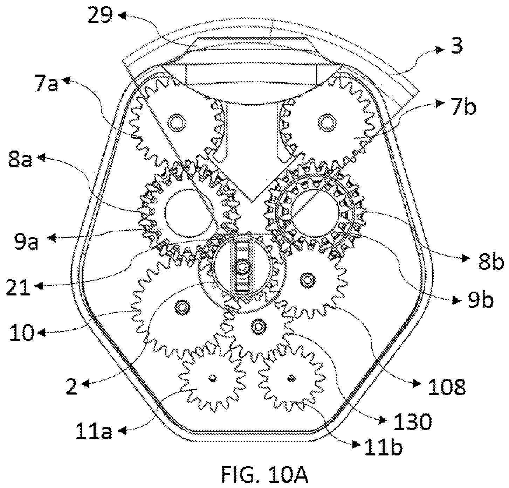

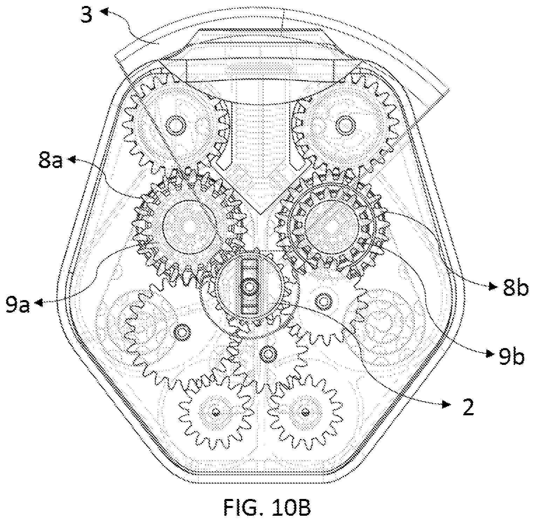

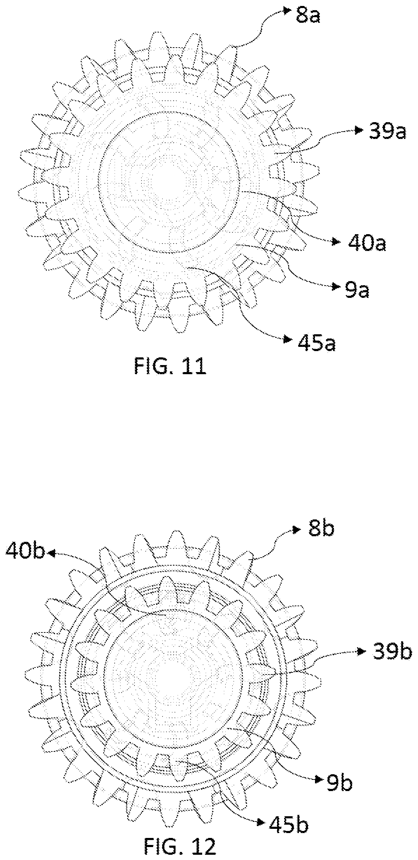

In use, as illustrated in Figures 10A and 10B, the mouthpiece cover (3) is movable along the housing. The mouthpiece cover (3), when sufficiently moved, rotates the driver gear (2) which in turn rotates the first ratchet assembly (9a) and second ratchet assembly (9b). The interim driver gear (108) is located between the driver gear (2) and the second ratchet assembly (9b) for allowing the rotation of ratchet assemblies (9a) and (9b) in opposite directions. The driver gear (2) simultaneously rotates ratchet assemblies (9a) and (9b). The first ratchet assembly (9a) is displaced at about 15 degrees from the first blister cuvette ratchet tooth (45a) (see Figure 11). The first ratchet assembly (9a) does not rotate the first blister cuvette (8a) for the initial about 15 degrees rotation of the first ratchet assembly (9a). The first ratchet assembly (9a) rotates 1.25x slower than the driver gear (2). The second ratchet assembly (9b) is displaced at about 30 degrees from the second blister cuvette ratchet tooth (45b) (see Figure 12). The second ratchet assembly (9b) does not rotate the second blister cuvette (8b) for the initial about 30 degrees rotation of the second ratchet assembly (9b). The idle movement of about 15 degrees of the first ratchet assembly (9a) corresponds to about 18.75 degrees of the idle movement of the mouthpiece cover (3). Once the ratchet assemblies (9a) and (9b) rotate about 15 degrees and about 30 degrees and come in contact with the blister cuvettes (8a) and (8b) respectively, the blister cuvettes (8a) and (8b) rotate synchronously with ratchet assemblies (9a) and (9b), thereby advancing the medicament carrier (5a) and (5b). The following further description, though not illustrated in Figures 10A - 10B, Figure 11, or Figure 12, is included here to aid in understanding and are further described elsewhere herein. The driver gear (2) rotates the second ratchet assembly (9b) via interim driver gear (108). Advancement of the medicament carriers (5a) and (5b) cause the respective leading blister cavity (41a) and (41b) to be peeled open and brought into communication with the pair of grids (62a) and (62b) of the flow channel (12), which itself communicates with mouthpiece opening (25), and the medicaments of the blister cavities (41a) and (41b) are made available for inhalation. The first blister cuvette gear teeth (34a) drive the first winding assembly (7a), and the first wrapping wheel (11a) via interim gear (10). The second blister cuvette gear teeth (34b) drives the second winding assembly (7b). The interim driver gear (108) drives the second wrapping gear (11b) via secondary interim gear (130). The lid sheet of each of the medicament carriers (5a) and (5b) is peeled during each blister movement via a constant tension applied by the winding assemblies (7a) and (7b). The lid sheet of each of the carriers (5a) and (5b) winds around the winding assemblies (7a) and (7b) during the operation of the dispenser. The base sheet of each of the medicament carriers (5a) and (5b) is advanced via the wrapping wheels (11a) and (11b). The open end (71) of both the base sheets is located with the respective wrapping wheel slits (69a) and (69b) and wraps around the wheel as it rotates during actuation. To access the medicament of the opened cavities of medicament carriers (5a) and (5b), the user places their mouth on the mouthpiece opening (25) and inhales. This action by the user results in air (22) entering the device through the inlet vent openings (74) and passing through the opened blister cavities (41a) and (41b) at the pair of grids (62a) and (62b). The air then exits the device through the mouthpiece opening (25). This exiting of air results in the powder contained within the opened cavities (41a) and (41b) at the pair of grids (62a) and (62b) of the flow channel (12) being aerosolized and the powder being guided through the flow channel and the mouthpiece opening (25), ultimately reaching the user as an inhaled medicament dose. Thus, in use, the user can simultaneously inhale one dose portion from each medicament carrier (5a) and (5b). The mouthpiece cover (3) after inhalation is moved in the opposite direction (from the third position to the first position) to cover the mouthpiece (29). At this stage, the motion of the mouthpiece cover (3) rotates the ratchet assemblies (9a) and (9b) without moving the medicament carriers (5a) and (5b). The ratcheting mechanism of the ratchet assemblies (9a) and (9b) and blister cuvettes (8a) and (8b) prevents the rotation of the blister cuvettes (8a) and (8b) in the opposite direction.

Figures 10A, 10B, 11, and 12 show the interaction and the movements of the components of the second dispensing mechanism. At a first/closed position, the teeth (21) of the driver gear (2) are in the meshed relationship with the first ratchet assembly gear teeth (39a) and the interim driver gear (108). The interim driver gear (108) meshes with the second ratchet assembly gear teeth (39b). The drive pins (40a) and (40b) of the respective first and second ratchet assemblies (9a) and (9b) are not in engagement with the respective ratchet tooth (45a) and (45b) of the first and second blister cuvettes (8a) and (8b) (see Figures 11 and 12). The sufficient movement of the mouthpiece cover (3) results in the simultaneous movement of the first and second ratchet assemblies (9a) and (9b) via driver gear (2). The about 15-degree movement of the first ratchet assembly (9a) results in movement of the drive pin (40a) of the first ratchet assembly (9a) and thereby engagement with the ratchet tooth (45a) through the contacting surface (46a). The further movement of the mouthpiece cover (3) results in simultaneous movement of the first ratchet assembly (9a) and the first blister cuvette (8a) to peel open the blister pocket (41a) of the first medicament carrier (5a) at the first pair of grids (62a) of the flow channel (12) (not shown). The about 30-degree movement of the second ratchet assembly (9b) via interim driver gear (108) results in movement of the drive pin (40b) of the second ratchet assembly (9b) and thereby engagement with the ratchet tooth (45b) through the contacting surface (46b). The further movement of the mouthpiece cover (3) results in simultaneous movement of the second ratchet assembly (9b) and the second blister cuvette (8b) to peel open the blister pocket (41b) of the second medicament carrier (5b) at the second pair of grids (62b) of the flow channel (12) (not shown). The first and second blister cuvettes (8a) and (8b) begin to rotate at different times and differing speeds and thereby advance the respective medicament carriers (5a) and (5b) at different rates but completely open the respective blister pockets (41a) and (41b) at the same time at the opening location. The opened blister pocket (41a) and (41b) of each medicament carriers (5a) and (5b) are available at the flow channel (12) for simultaneous inhalation by the patient through the opened mouthpiece opening (25). When the mouthpiece cover (3) is closed, the reverse rotation of the ratchet assemblies (9a) and (9b) is not transmitted to the respective blister cuvettes (8a) and (8b). Therefore, on each occasion that the mouthpiece cover (3) is fully opened and closed in the second embodiment of the medicament dispenser mechanism, blister cuvettes (8a) and (8b) are incremented in one rotational direction only.

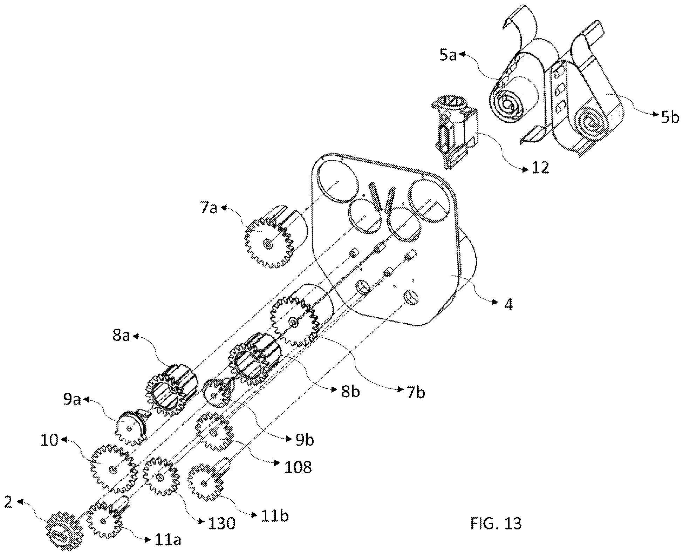

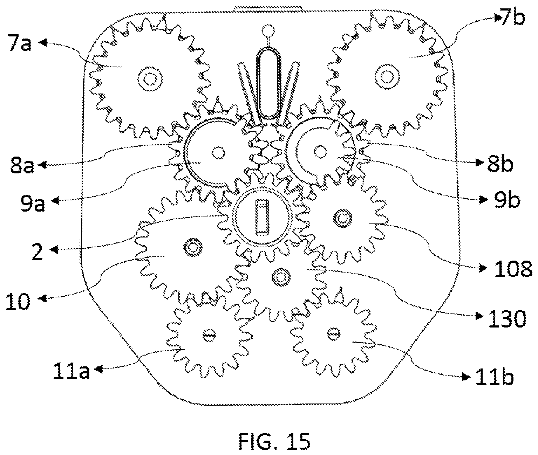

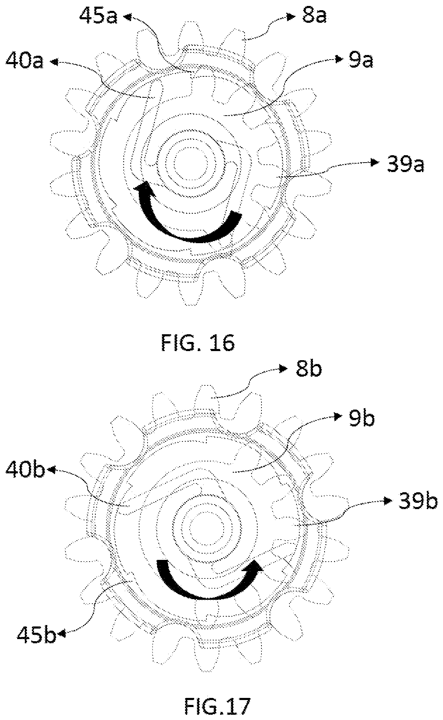

Figure 13 illustrates a fully exploded view of a third dispensing mechanism of the medicament dispenser described herein. In use, the mouthpiece cover (3) (not shown) is movable along the housing. The mouthpiece cover (3) rotates the driver gear (2) which in turn rotates the first ratchet assembly (9a) and the second ratchet assembly (9b) (see Figure 15). The interim driver gear (108) is located between the driver gear (2) and the second ratchet assembly (9b) for allowing the rotation of ratchet assemblies (9a) and (9b) in opposite directions. The driver gear (2) simultaneously rotates the ratchet assemblies (9a) and (9b). The first ratchet assembly (9a) is displaced at about 20 degrees from the first blister cuvette ratchet tooth (45a) (see Figure 16). The first ratchet assembly (9a) does not rotate the first blister cuvette (8a) for the initial about 20 degrees rotation of the first ratchet assembly (9a). The first ratchet assembly (9a) rotates slower than the driver gear (2). The second ratchet assembly (9b) is displaced at about 40 degrees from the ratchet tooth (45b) of the second blister cuvette (8b) (see Figure 17). The second ratchet assembly (9b) does not rotate the second blister cuvette (8b) for the initial about 40 degrees rotation of the second ratchet assembly (9b). The idle movement of about 20 degrees of the first ratchet assembly (9a) corresponds to about 18.75 degrees of the idle movement of the mouthpiece cover (3). Once the ratchet assemblies (9a) and (9b) rotate about 20 degrees and about 40 degrees and come in contact with the blister cuvettes (8a) and (8b) respectively, the blister cuvettes (8a) and (8b) rotate synchronously with the ratchets assemblies (9a) and (9b), thereby advancing the medicament carrier (5a) and (5b). The driver gear (2) rotates the second ratchet assembly (9b) via interim driver gear (108). As a result of the phase lag between the first and second ratchet assemblies (9a) and (9b) and the respective gear ratios, both the blister cuvettes (8a) and (8b) are out of phase during the entire duration of the rotation. Both the blister cuvettes (8a) and (8b) advance the respective carrier (5a) and (5b) at a different rate but completely open the respective blister pockets (41a) and (41b) at the same time once they reach the opening location. Advancement of the medicament carriers (5a) and (5b) cause the respective leading blister cavity (41a) and (41b) to be peeled open and brought into communication with the pair of grids (62a) and (62b) of the flow channel (12), which itself communicates with mouthpiece opening (25), and the medicaments of the blister cavities (41a) and (41b) are available for inhalation. The first blister cuvette gear teeth (34a) drives the first winding assembly (7a) and the first wrapping wheel (11a) via interim gear (10). The second blister cuvette gear teeth (34b) drive the second winding assembly (7b). The second blister cuvette gear teeth (34b) drives the second winding assembly (7b). The interim driver gear (108) drives the second wrapping wheel (11b) via secondary interim gear (130). The lid sheet of each of the medicament carriers (5a) and (5b) is peeled during each blister movement via a constant tension applied by the winding assemblies (7a) and (7b). The lid sheet of each of the carriers (5a) and (5b) winds around the winding assemblies (7a) and (7b) during the operation of the dispenser. The base sheet of each of the medicament carriers (5a) and (5b) is advanced via the wrapping wheels (11a) and (11b). The open end (71) of both the base sheets is located with the respective wrapping wheel slits (69a) and (69b) and wraps around the wheel as it rotates during actuation. To access the medicament of the opened cavities of medicament carriers (5a) and (5b), the user places the mouth on the mouthpiece opening (25) of the mouthpiece (29) and inhales. This action by the user results in air (22) entering the device through the inlet vent openings (74) and passing through the opened blister cavities (41a) and (41b) at the pair of grids (62a) and (62b) and exiting the device through the mouthpiece opening (25). This exit of air results in the powder contained within the opened cavities (41a) and (41b) at the pair of grids (62a) and (62b) of the flow channel (12) being aerosolized and guided through the flow channel and the mouthpiece opening (25) and hence to the user as an inhaled medicament dose. Thus, in use, the user can simultaneously inhale one dose portion from each medicament carrier (5a) and (5b). The mouthpiece cover (3) after inhalation has been moved in the opposite direction (from the third position to the first position) to cover the mouthpiece (29). At this stage, the motion of the mouthpiece cover (3) rotates the ratchet assemblies (9a) and (9b) without moving the medicament carriers (5a) and (5b). The ratcheting mechanism of the ratchet assemblies (9a) and (9b) and blister cuvettes (8a) and (8b) prevents the rotation of the blister cuvettes (8a) and (8b) in the opposite direction.

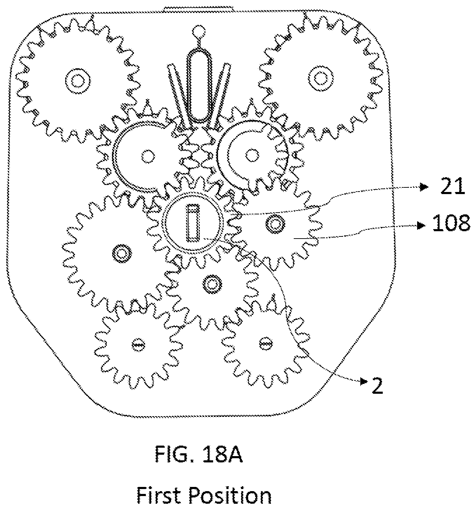

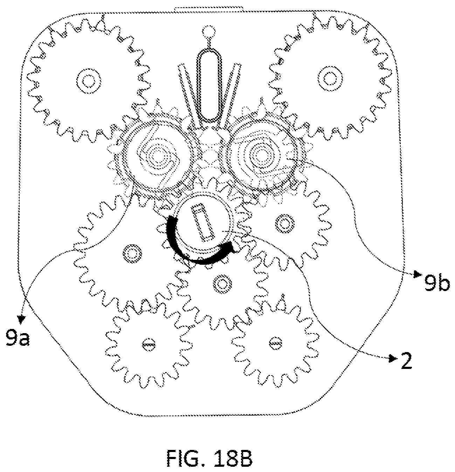

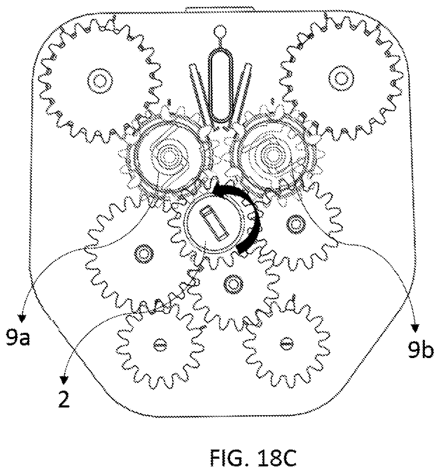

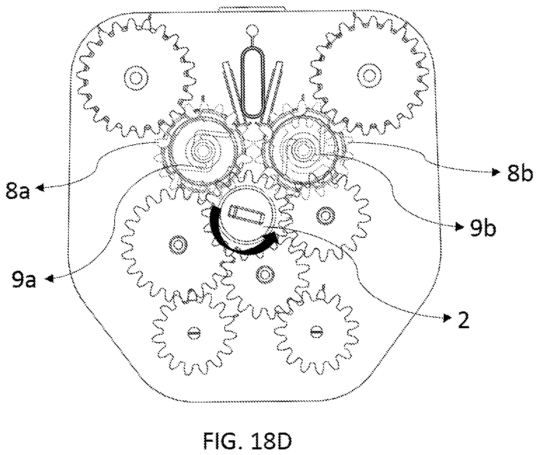

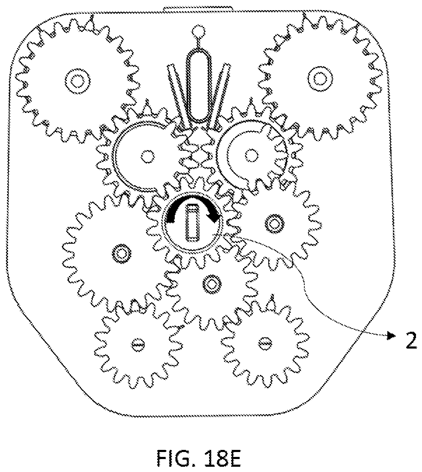

Figures 18A to 18E show the interaction and the movements of the components of the third dispensing mechanism. Referring to Figure 18A, at first/closed position, the teeth (21) of the driver gear (2) are in the meshed relationship with the first ratchet assembly (9a) gear teeth (39a) and the interim driver gear (108). The interim driver gear (108) meshes with the second ratchet assembly (9b) gear teeth (39b). The drive pin (40a) and (40b) of the respective first and second assemblies (9a) and (9b) are not in engagement with the respective ratchet tooth (45a) and (45b) of the first and second blister cuvettes (8a) and (8b) (see Figures 16 and 17). The movement of the mouthpiece cover (3) results in simultaneous movement of the first and second ratchet assemblies (9a) and (9b) via driver gear (2). Referring Figure 18B, the driver gear (2) rotates 18.75 degrees and hence rotates the first ratchet assembly (9a) by 20 degrees and second ratchet assembly (9b) by 25 degrees. The first ratchet assembly (9a) completes its idle motion and the second ratchet assembly (9b) needs to rotate another 15 degrees to complete its idle motion. No medicament carrier advancement occurs during this time. Referring to Figure 18C, the driver gear (2) rotates a total of 30 degrees. The first ratchet assembly (9a) rotates 12 degrees and hence begins to peel open the first medicament carrier (5a). The second ratchet assembly (9b) rotates an additional 15 degrees to complete its idle motion and begins to peel open the second medicament carrier (5b) thereafter. Referring Figure 18D, the driver gear (2) rotates a total of 75 degrees and hence rotates the first ratchet assembly (9a) by 80 degrees and second ratchet assembly (9b) by 100 degrees. Accounting for the idle motion of 20 degrees and 40 degrees in each ratchet assembly (9a) and (9b) respectively, the total advancement of each blister cuvette (8a) and (8b) is 60 degrees. The first and second blister cuvettes (8a) and (8b) begin to rotate at different times and differing speeds and thereby advance the respective carriers (5a) and (5b) at different rate but completely open the respective pockets (41a) and (41b) at the same time at the opening location (not shown). The opened blister pocket (41a) and (41b) of each of medicament carriers (5a) and (5a) is available at the flow channel (12) for simultaneous inhalation by the patient through the opened mouthpiece opening (25) (not shown). Referring to Figure 18E, the driver gear (2) returns back to the first/closed position as the mouthpiece cover (3) is closed after device use. When the mouthpiece cover (3) is closed, the reverse rotation of the ratchet assemblies (9a) and (9b) is not transmitted to the respective blister cuvettes (8a) and (8b). Therefore, on each occasion, the mouthpiece cover (3) is fully opened and closed, blister cuvettes (8a) and (8b) are incremented in one rotational direction only.

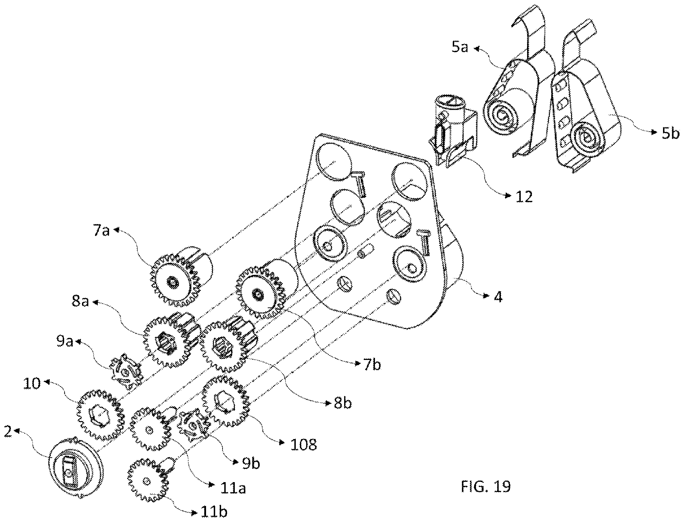

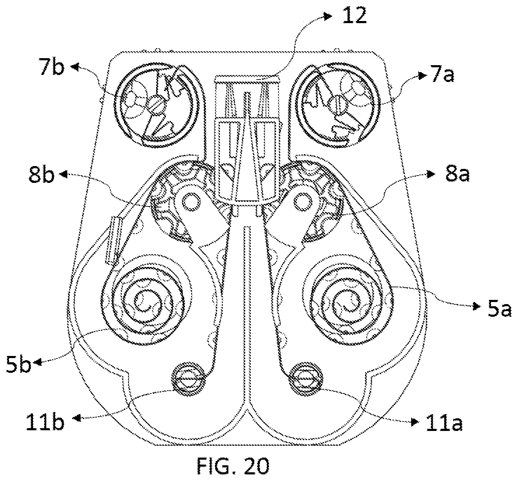

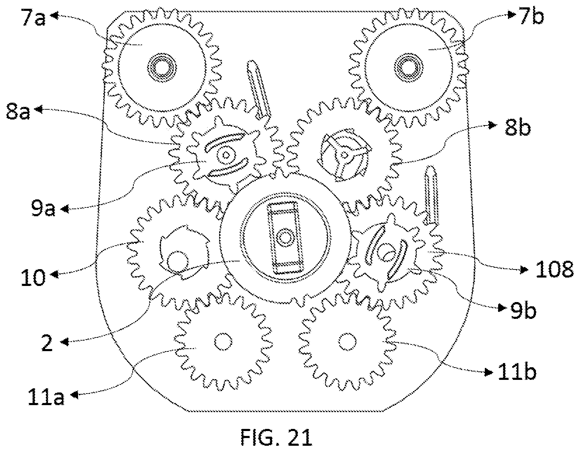

Figure 19 illustrates a fully exploded view of a fourth dispensing mechanism of the medicament dispenser described herein. The mechanism of operation of the medicament dispenser according to an aspect of the disclosure is presented in Figures 20, 21 to 22A-22G. In use, the mouthpiece cover is movable along the housing. The mouthpiece cover (3) rotates the driver gear (2), which in turn rotates the first ratchet assembly (9a). The driver gear (2) is displaced at about 12 degrees from the first ratchet assembly (9a). In other words, the driver gear rotates about 12 degrees before coming in contact with the first ratchet assembly gear teeth (39a) and thus provides an idle movement of about 12 degrees during the opening of the mouthpiece cover (3). The driver gear (2) does not rotate the first ratchet assembly (9a) for the initial about 12 degrees rotation of the driver gear (2). Once the driver gear (2) comes in contact with the first ratchet assembly (9a), the first ratchet assembly (9a) rotates the first blister cuvette (8a). The first blister cuvette (8a) rotates synchronously with the first ratchet assembly (9a) and advances a blister pocket (41a) from the first medicament carrier (5a). The driver gear (2) does not rotate the second blister cuvette (8b) until the blister pocket (41) from the first medicament carrier (5a) is completely peeled open at the first pair of grids (62a) of the flow channel (12). Further movement of the driver gear (2) rotates the second blister cuvette (8b) via the second ratchet assembly (9b) and interim driver gear (108). The driver gear (2) does not come in contact with gear teeth (39b) of the second ratchet assembly (9b) while it is in contact with the first ratchet assembly (9a). Once the first medicament carrier (5a) is completely peeled open at the first pair of grids (62a) of the flow channel (12), the driver gear (2) meshes with the second ratchet assembly gear teeth (39b) and starts rotating the second ratchet assembly (9b). The driver gear (2) rotates the second ratchet assembly (9b) which in turn synchronously rotates the second blister cuvette (8a) via the interim gear (108) and advancing a blister pocket (41b) from the second medicament carrier (5a) to peel open at the second pair of grids (62b) of the flow channel (12). Both the medicament carriers (5a) and (5b) are peeled open successively (one after the other) during the actuation of the device. Advancement of the medicament carriers (5a) and (5b) cause the respective leading blister cavity (41a) and (41b) to be peeled open and successively brought into communication with the pair of grids (62a) and (62b) of the flow channel (12), which itself communicates with mouthpiece opening (25) and the medicaments of the blister cavities are available for inhalation. The gear teeth (34a) of the first blister cuvette (8a) drive the first winding assembly (7a), and the first wrapping gear (11a) via interim gear (10). The gear teeth (34b) of the second blister cuvette (8b) drive the second winding assembly (7b). The interim driver gear (108) located concentrically with the second ratchet assembly (9b) drives the second wrapping wheel (11b) via gear teeth (68b). The lid sheet of each of the medicament carriers (5a) and (5b) is successively peeled during each blister movement via a constant tension applied by the winding assemblies (7a) and (7b). The lid sheet of each of the medicament carrier (5a) and (5b) winds around the winding assemblies (7a) and (7b) during the operation of the dispenser. The base sheet of each of the medicament carriers (5a) and (5b) is advanced via the wrapping wheels (11a) and (11b). The open end (71) of both of the base sheets is located with the respective wrapping wheel slits (69a) and (69b) and wraps around the wheel as it rotates during actuation. To access the medicament of the opened cavities of medicament carriers (5a) and (5b) the user places the mouth on the mouthpiece opening (25) and inhales. This action by the user results in air (22) enters the device through the inlet vent openings (74) and pass through the opened blister cavities (41a) and (41b) at the pair of grids (62a) and (62b) and exits the device through the mouthpiece opening (25). This flow of air results in the powder contained within the opened cavities (41a) and (41b) at the pair of grids (62a) and (62b) of the flow channel (12) being aerosolized and guided through the flow channel (12) and the mouthpiece opening (25) and hence to the user as an inhaled medicament dose. Thus, in use, the user can simultaneously inhale one dose portion from each medicament carrier (5a) and (5b). The mouthpiece cover after inhalation has been moved in the opposite direction (from the third position to the first position) to cover the mouthpiece (29). At this stage, the motion of the mouthpiece cover (3) rotates the ratchet assemblies (9a) and (9b) without moving the medicament carriers (5a) and (5b). The ratcheting mechanism of the first ratchet assembly (9a) and the first blister cuvettes (8a) prevents the rotation of the first blister cuvettes (8a) in the opposite direction. The ratcheting mechanism of the second ratchet assembly (9b) and the interim driver gear (108) prevents the rotation of the interim driver gear (108) and thereby the second blister cuvettes (8b) in the opposite direction. The second ratchet assembly (9b) does not rotate the interim driver gear (108) during the reverse motion or the closing of the mouthpiece cover (3).

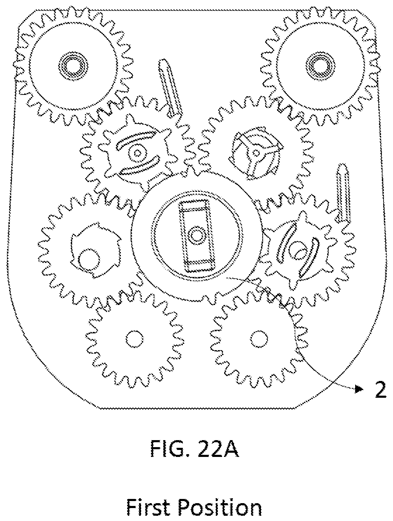

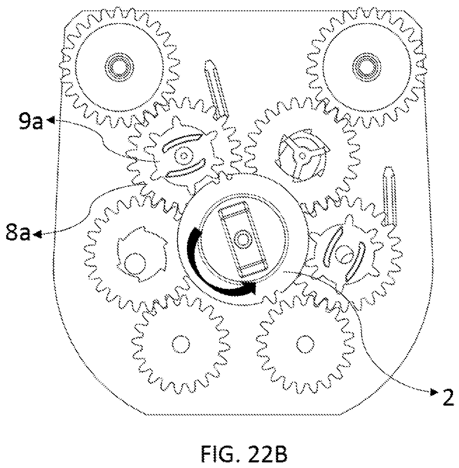

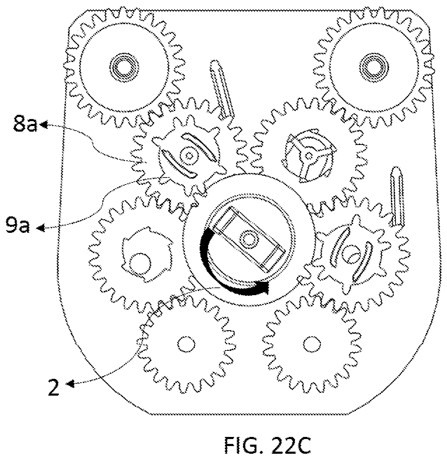

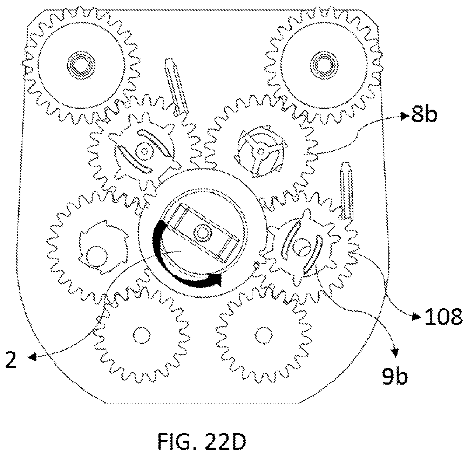

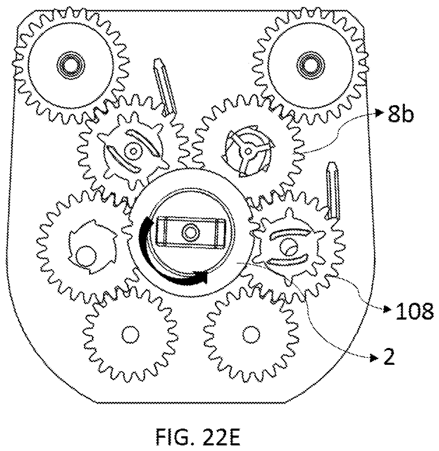

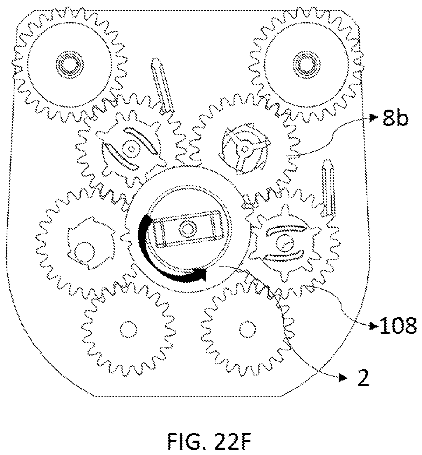

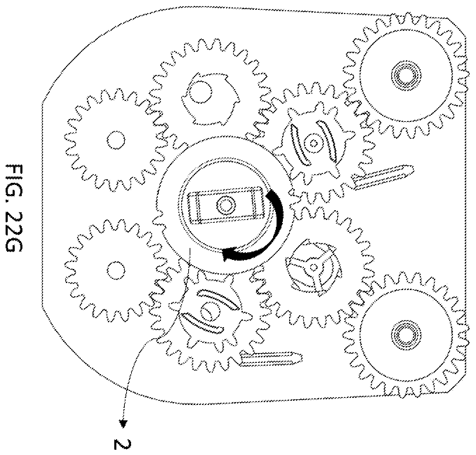

Figures 22A to 22G show the interaction and the movements of the components of the fourth dispensing mechanism. In Figures 22A at first/closed position, the driver gear (2) is not in a meshed relationship with the first and second ratchet assemblies (9a) and (9b). In Figures 22B, the driver gear (2) rotates about 12 degrees before contacting the first ratchet assembly (9a). About 12 degrees movement of the driver gear (2) results in the engagement of the driver gear (2) with the first ratchet assembly (9a). In Figures 22C, the driver gear (2) rotates the first ratchet assembly (9a) for 60 degrees to advance the first blister cuvette (8a) to peel open the blister pocket of the first medicament carrier (5a) at the first pair of grids (62a) of the flow channel (12). Until this position, the driver gear (2) does not mesh with the second ratchet assembly (9b). In Figures 22D, the driver gear (2) rotates the second ratchet assembly (9b) for 60 degrees to advance the second blister cuvette (8b) via the interim driver gear (108). In Figures 22E and 22F, the driver gear (2) completes the advancement of the second blister cuvette (8b) to peel open the blister pocket of the second medicament carrier (5b) at the second pair of grids (62b) of the flow channel (12). In the third position, the opened blister pocket of each of medicament carriers (5a) and (5a) is available at the flow channel (12) for simultaneous inhalation by the patient through the mouthpiece opening (25). In Figures 22G, the driver gear (2) returns to its original position as the mouthpiece cover (3) is closed after device use (i.e., first/closed position). When the driver gear (2)/mouthpiece cover (3) is returned to its original position, the reverse rotation of the ratchet assemblies (9a) and (9b) is not transmitted to the respective blister cuvettes (8a) and (8b). Thus, on each occasion, the mouthpiece cover is fully opened and closed, the blister cuvettes (8a) and (8b) are incremented in one rotary direction only.

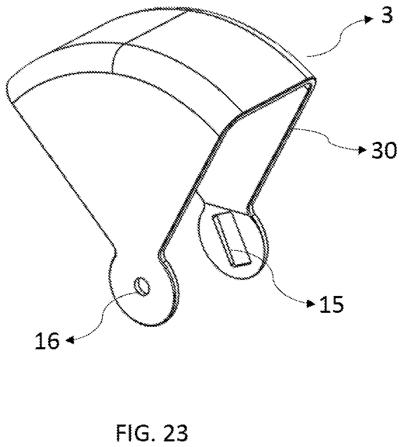

Figure 23 shows a mouthpiece cover (3) according to one aspect of the disclosure. The mouthpiece cover (3) can be provided for actuating the dispensing mechanism. The mouthpiece cover (3) can be mechanically mounted to the housing (1) via a pin (23) of the base plate (129) and the flange (49) of the driver gear (2). The mouthpiece cover (3) is provided with a mounting key for (15) for interaction with a flange (49) of a driver gear (2). In use, the mouthpiece cover (3) is rotationally movable about an axis defined by the rotational axis of the driver gear (2). The mouthpiece cover (3) can rotate about the axis passing through the mounting key (15) and the pivot hole (16). The pivot hole (16) rotates about the pin (23) of a base plate (129). The mounting key (15) engages with the flange (49) on the driver gear (2) and rotates it in the same direction as the direction of rotation of the mouthpiece cover (3). The edge (30) on the mouthpiece cover (3) rests over the housing (1) after complete actuation.

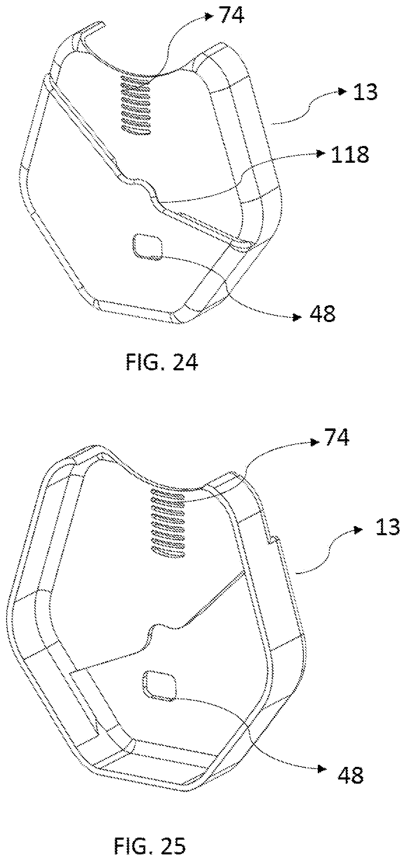

Figures 24 and 25 show outer and inner views of the top cover (13) according to one aspect of the disclosure. The top cover (13) covers and encloses the dispensing mechanism. Inlet vent openings (74) are located on the top cover (13) allows the air (22) to enter into the flow channel (12). In use, the inlet vent openings (74) allows the air (22) to pass from outside the dispenser into the flow channel (12) via the pair of inlet compartments (17a) and (17b) in response to inhalation by the patient at the mouthpiece opening (25). Notably, the inlet vent openings (74) provide the sole point of entry of inhalation air from outside the medicament dispenser into the flow channel (12). The central groove of the top cover (118) allows the mouthpiece cover (3) to enter and rotate about the pin (23) of the base plate (129).

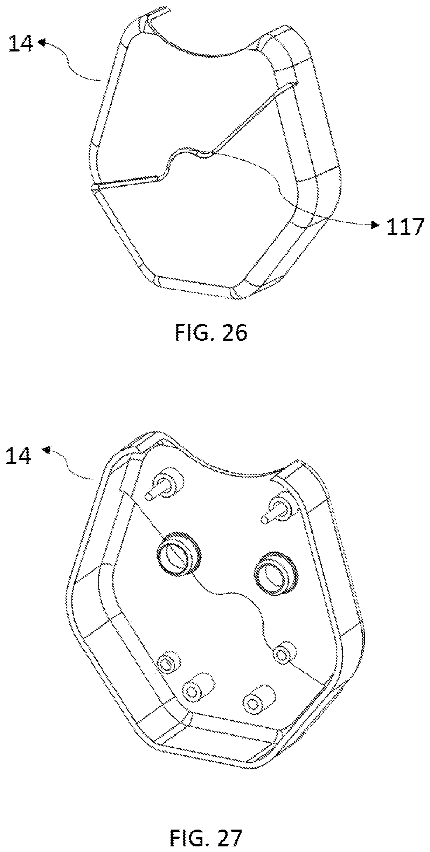

Figures 26 and 27 show outer and inner views of the base cover (14) according to one aspect of the disclosure. The base cover can hold and encase components of the dispensing mechanism. The central groove of the base cover (117) allows the mouthpiece cover (3) to enter and rotate the flange (49) of the driver gear (2).

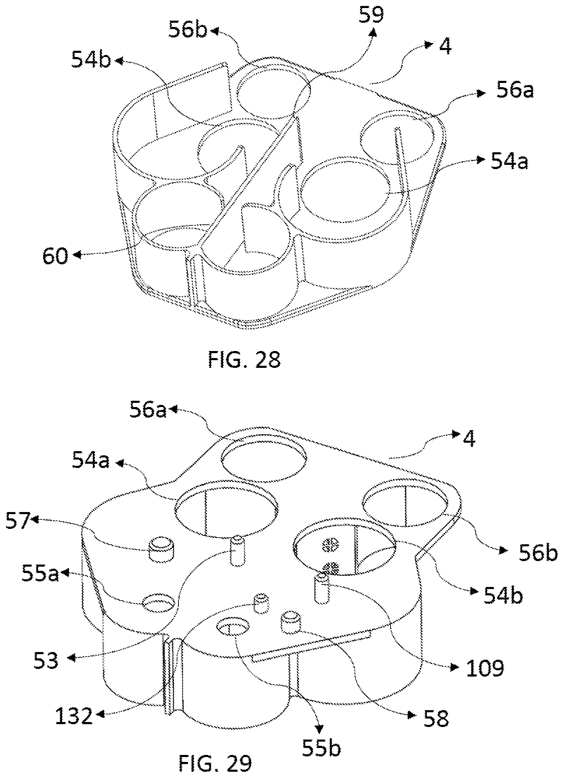

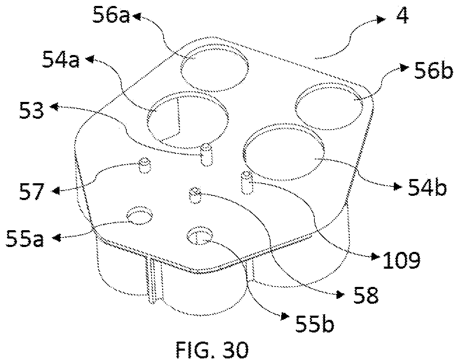

Figures 28 and 29 show an inner housing assembly (4) according to one aspect of the disclosure. The inner housing assembly may locate the medicament carriers (5a) and (5b) and components of the dispensing mechanism such as the blister cuvettes (8a) and (8b), the winding assemblies (7a) and (7b) and the wrapping wheels (11a) and (11b) pass through openings (54a) and (54b), (56a) and (56b) and (55a) and (55b) respectively. The wall (60) separates the medicament carriers (5a) and (5b). The underside part of the inner housing assembly (4) may locate the driver gear (2), an interim driver gear (108), an interim gear (10), a secondary interim gear (130) and a tertiary interim gear (131) on studs (53), (109), (57), (58) and (132) respectively. Figures 30 show an inner housing assembly according to another aspect of the disclosure. The inner housing assembly (4) locates the gears responsible for driving the dispensing mechanism. The underside part of the inner housing assembly (4) locates the driver gear (2), interim driver gear (108), interim gear (10) and secondary interim gear (130) on the studs (53), (109), (57) and (58) respectively.

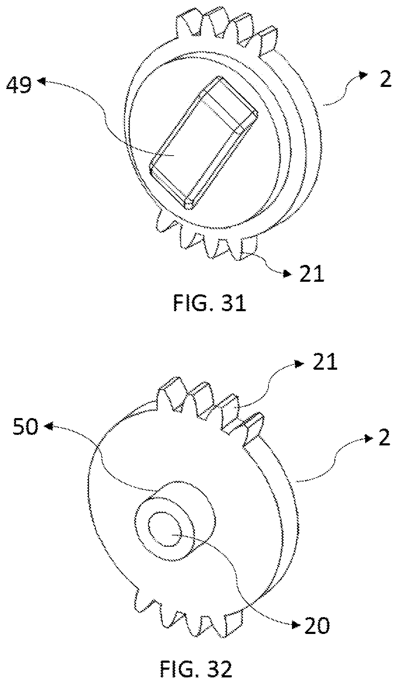

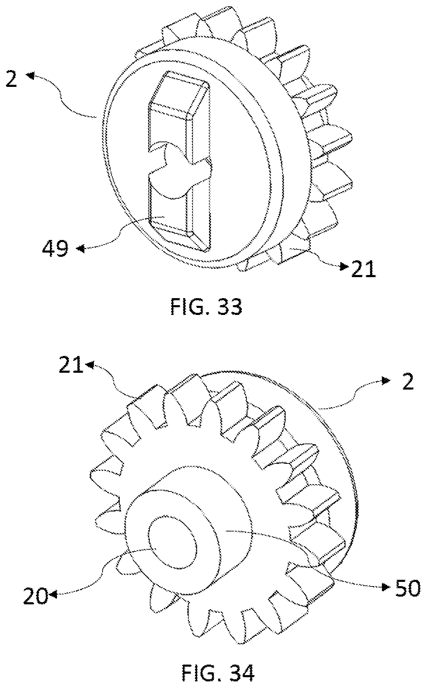

Figures 31 and 32 show an embodiment of a driver gear (2). The driver gear (2) is having inner and outer faces and a circumferential surface. The outer surface can include a driving flange (49), which fits in the mounting key (15) of the mouthpiece cover (3) for establishing a direct drive connection between the mouthpiece cover (3) and the driver gear (2). The inner surface can include a shaft (50) and a bearing (20). Bearing (20) enables the driver gear (2) to freely rotate about stud (53) on the inner housing (4). The circumferential surface can include a set of driver gear teeth (21) particularly on the opposite side. The teeth may not be arranged annularly. The driver gear (2) can be located on a stud (53) of the inner housing assembly (4) and rotates within the bearing (20) about a shaft (50). The driver gear (2) can be driven by the mouthpiece cover (3). The rotary movement of the mouthpiece cover (3) causes the rotary movement of the driver gear (2). The driver gear (2) rotates the ratchet assembly (9). The driver gear (2) may also rotate the ratchet assembly (9) via the interim driver gear (108). The teeth (21) of the driver gear (2) can be provided for driving the gear teeth (39) of the ratchet assemblies (9a) and (9b). Figures 33 and 34 show another embodiment of a driver gear (2). The driver gear teeth (21) arranged annularly on the circumferential surface. The teeth of the driver gear (21) are provided for drive interaction (meshing) with the ratchet assembly gear teeth (39) via the interim driver gear (108).

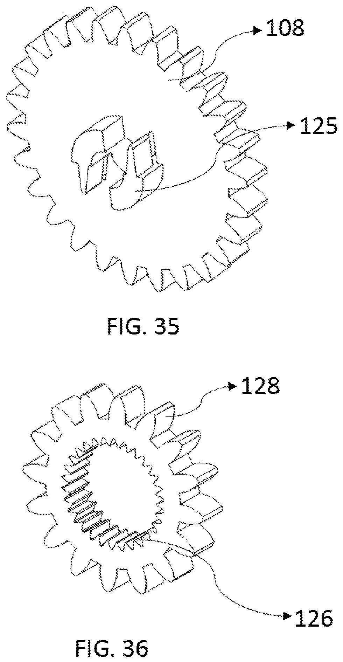

Figures 35 and 36 show other possible components of the dispensing mechanism. The ratchet gear (128) is located concentrically with the interim driver gear (108). The ratchets (125) on the interim driver gear (108) drive the ratchet gear (128) via the inner gears (126) of the ratchet gear (128). The interim driver gear (108) rotates in both directions when the mouthpiece cover (3) is opened and closed. Due to the ratcheting mechanism, the ratchets (125) allow the ratchet gear (128) to move in one direction only. The ratchet gear (128) drives the wrapping wheel (11b) via interim gears (130) and (131). The interim driver gear (108) may also mesh with the ratchet gear (128), which in turn meshes with the wrapping wheel gear teeth (68b) via interim gears (130) and (131).

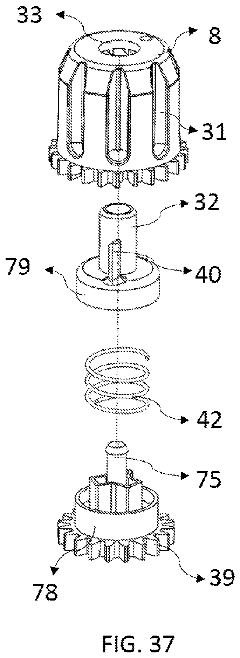

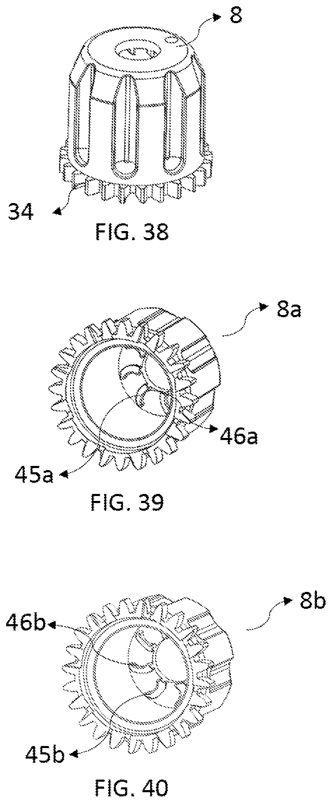

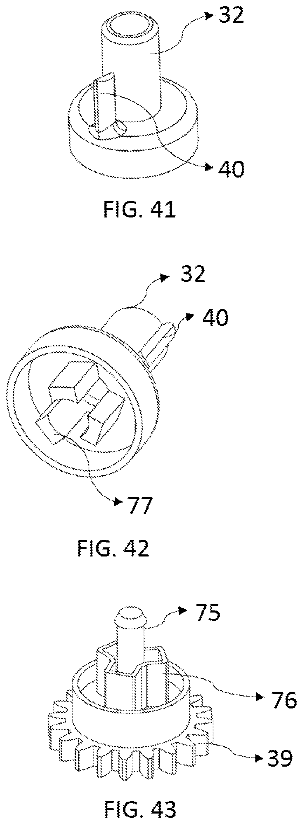

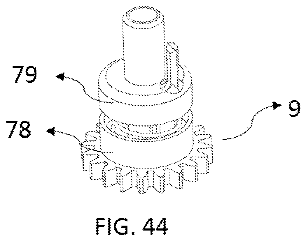

Figures 37 to 44 show a first embodiment of a blister cuvette (8) and a ratchet assembly (9). The blister cuvette (8) locates each blister pocket (41) during the operation of the dispenser. The blister cuvette (8) also helps to locate and align the blister pocket (41) along with the pair of grids (62) of the flow channel (12) when the dispensing mechanism is actuated. The gear teeth (34) of the blister cuvette (8) are provided for drive interaction with the winding wheel (28) of the winding assembly (7). The rotation of the blister cuvette (8) may result in the rotation of the winding wheel (28). The blister cuvette (8) rotates about a central boss (32) of the ratchet assembly (9). The first embodiment of the blister cuvette (8) may be used for both the medicament carriers (5a) and (5b) with only difference in the orientation of the ratchet teeth (45) and the contacting surface (46). Figures 39 and 40 show blister cuvettes (8a) and (8b) for advancing the respective medicament carriers (5a) and (5b). The ratchet teeth (45a) and (45b) may be provided to the inner part of the first and second blister cuvettes (8a) and (8b) respectively. Each ratchet tooth (45a) and (45b) may include the contacting surface (46a) and (46b) respectively. The gear teeth of the blister cuvettes (34a) and (34b) mesh with the respective winding assembly outer teeth (27a) and (27b). The gear teeth of the first blister cuvette (34a) also mesh with the interim gear (10), which in turn meshes with the first wrapping wheel gear teeth (68a). Figures 41 to 44 show the components of the ratchet assembly (9). The ratchet assembly (9) can be provided for rotating the blister cuvette (8). The blister cuvette (8) can be driven by the ratcheting mechanism of the ratchet assembly (9). The ratchet assembly (9) may be made of a driving member (78) and a driven member (79). The locking pin (75) can lock the driving member (78) and the driven member (79) together. The driving member (78) can drive the driven member (79) via the circumferential pins (77) and the locating pockets (76). The circumferential pins (77) extend axially along the driven member (79). The surfaces on the axial plane of the circumferential pins (77) are in contact with the respective axial surfaces on the locating pockets (76) of the driving member (78). The surfaces in contact in the axial plane enable torque transfer from the driving member (78) to the driven member (79). The spring (42) allows axial displacement of the driven member (79) during ratcheting motion thereby preventing any axial motion of the driving member (78). The central boss (32) of the driven member (79) passes through and locates the central hole (33) of the blister cuvette (8). The drive pin (40) can drive the blister cuvette (8) via the ratchet teeth (45) of the blister cuvette (8). The ratchet teeth (45) drives the blister cuvette (8) in one direction only through the contacting surface (46). The first embodiment of the ratchet assembly (9) may be used for both the medicament carriers (5a) and (5b). The gear teeth (39) of the first ratchet assembly (9a) may be in the meshed relationship with the driver gear (2), and the gear teeth (39) of the second ratchet assembly (9b) may be meshed with the interim gear (108).

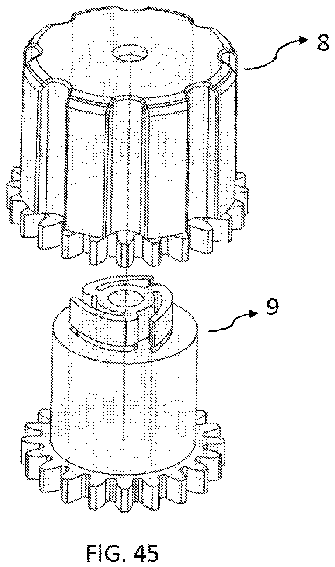

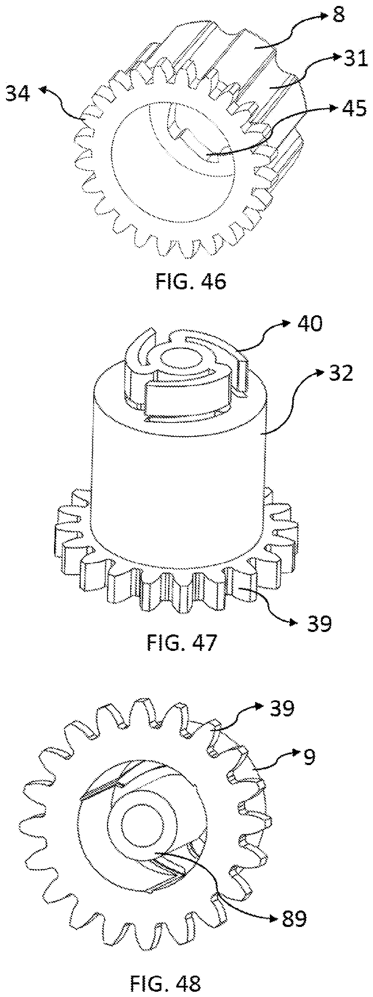

Figures 45 to 48 show a second embodiment of a blister cuvette (8) and a ratchet assembly (9). The blister cuvette (8) rotates about the central boss (32) of the ratchet assembly (9) while the ratchet assembly (9) rotates about the boss (89). The driver gear (2) rotates the ratchet assembly gear teeth (39). The gear teeth (34) of the blister cuvette (8) drive the winding assembly (7). The driving drive pin (40) in this embodiment is spiraled and concentric to the central boss (32). The ratchet teeth (45) are located at the distal end from the gear teeth (34) on the blister cuvette (8). The ratchet teeth (45) are placed in continuity and concentric to the center of the blister cuvette (8). The driving drive pin (40) drives the blister cuvette (8) via the ratchet teeth (45) on the blister cuvette (8). The ratchet teeth (45) drive the blister cuvette (8) in one direction. The second embodiment of the blister cuvette (8) may be used for both the medicament carriers (5a) and (5b) with only difference in the orientation of the ratchet teeth (45) and the driving drive pins (40). The gear teeth (34) of the blister cuvettes (8a) and (8b) mesh with the respective winding assembly outer teeth (27a) and (27b). The gear teeth of the first blister cuvette (34a) also mesh with the interim gear (10), which in turn meshes with the first wrapping wheel gear teeth (68a). The gear teeth (39) of the first ratchet assembly (9a) mesh with the driver gear (2), and the gear teeth (39) of the second ratchet assembly (9b) mesh with the interim driver gear (108).

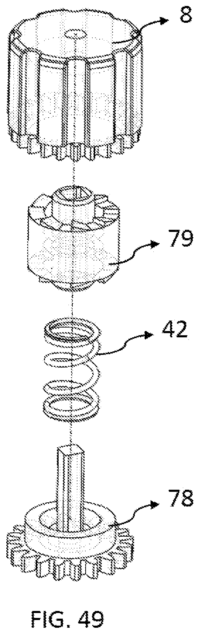

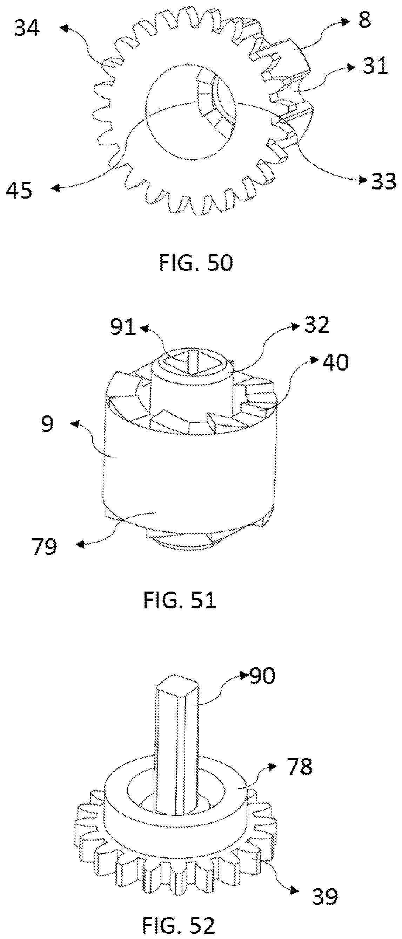

Figures 49 to 52 show a third embodiment of a blister cuvette (8) and a ratchet assembly (9). The blister cuvette (8) rotates about the central boss (32) of the ratchet assembly (9). The driver gear (2) rotates the ratchet assembly (9) via gear teeth (39) of the ratchet assembly (9). The ratchet assembly (9) is made of a driving member (78) and a driven member (79). The driving member (78) drives the driven member (79) via the rectangular shaft (90) of driving member (78) and receiving key (91) of the driven member (79). The spring (42) allows axial displacement of the driven member (79) during ratcheting motion thereby preventing any axial motion of the driving member (78). The drive pins (40) are concentric to the central boss (32). The ratchet teeth (45) are located at the distal end from the gear teeth (34) on the blister cuvette (8). The ratchet teeth (45) are placed discreetly and concentric to the center of the blister cuvette (8). The central boss (32) of the ratchet assembly (9) passes through and locates the central hole (33) on the blister cuvette (8). The gear teeth (34) of the blister cuvette (8) drive the winding assembly (7). The driving ratchets (40) drives the blister cuvette (8) via the ratchet teeth (45) of the blister cuvette (8b). The ratchet teeth (45) drive the blister cuvette (8) in one direction only. The third embodiment of the blister cuvette (8) may be used for both the medicament carriers (5a) and (5b) with only difference in the orientation of the ratchet teeth (45) and the driving ratchets (40).

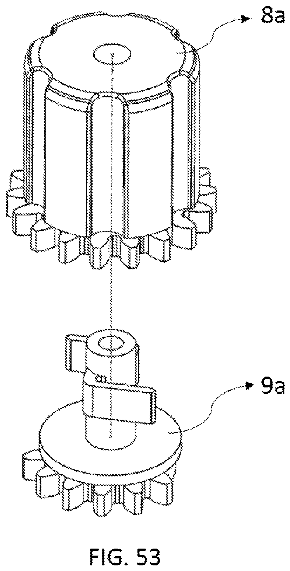

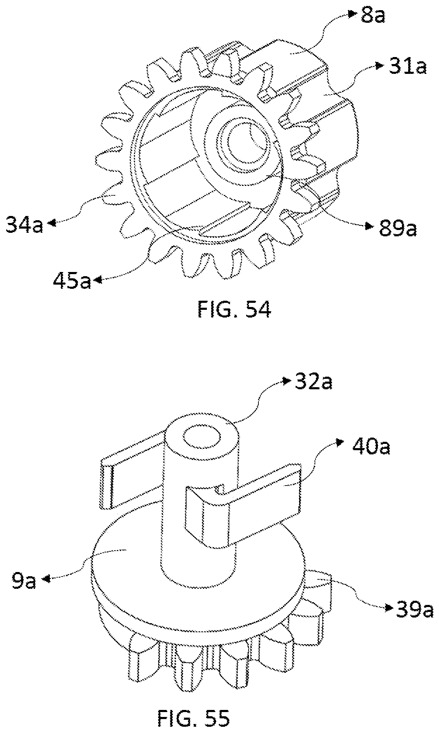

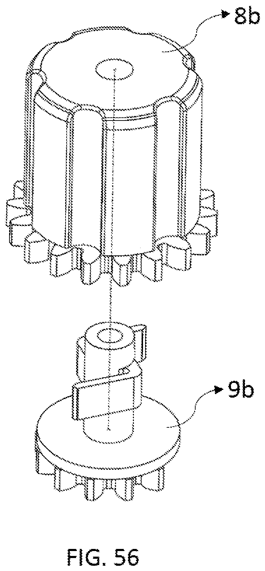

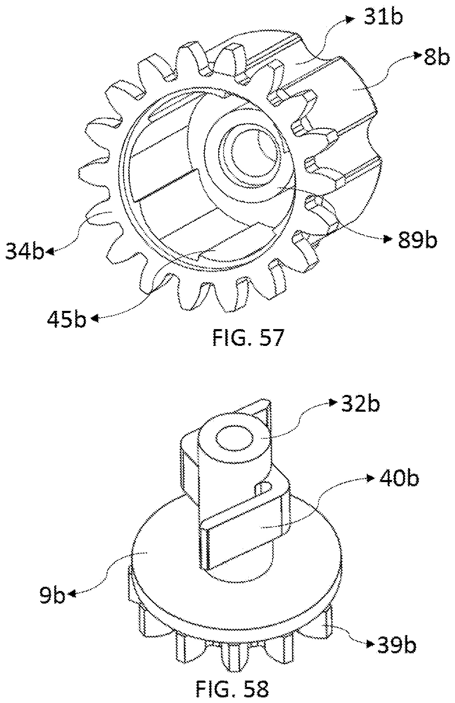

Figures 53 to 58 show a fourth embodiment of a blister cuvette (8) and a ratchet assembly (9). The first blister cuvette (8a) may be used for advancing the first medicament carrier (5a). The first blister cuvette (8a) contains six such equally spaced cuvettes (31a) around the circumference. The blister cuvette (8a) rotates about the central boss (32a) of the ratchet assembly (9a) while the first ratchet assembly (9a) rotates about the boss (89a). The driver gear (2) rotates the ratchet assembly (9a) via gear teeth (39a) of the ratchet assembly (9a). The drive pin (40a) drives the first blister cuvette (8a) via the ratchet teeth (45a) on the blister cuvette (8a). The ratchet teeth (45a) drive the first blister cuvette (8a) in one direction. The second blister cuvette (8b) may be used for advancing the second medicament carrier (5b). The second blister cuvette (8b) contains six such equally spaced cuvettes (31b) around the circumference. The second blister cuvette (8b) rotates about the central boss (32b) on the second ratchet assembly (9b) while the second ratchet assembly (9b) rotates about the boss (89b). The driver gear (2) rotates the second ratchet assembly (9b) via gear teeth (39b) of the ratchet assembly (9b) and interim driver gear (108). The drive pin (40b) drives the second blister cuvette (8b) via the ratchet teeth (45b) on the blister cuvette (8b). The ratchet teeth (45b) drive the second blister cuvette (8b) in one direction.

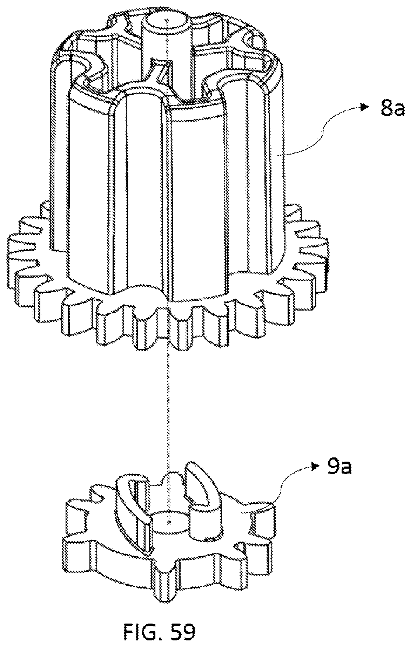

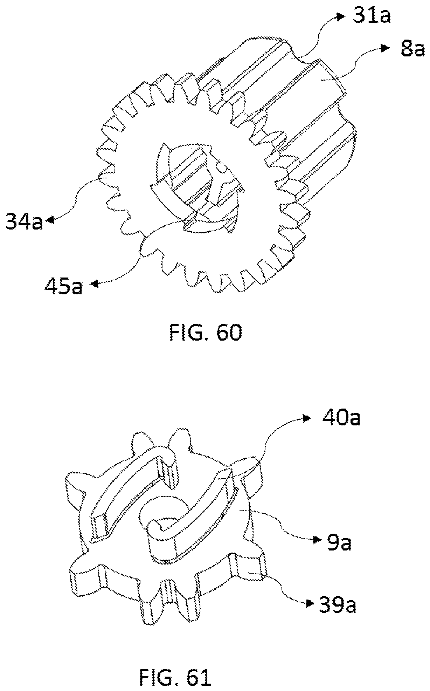

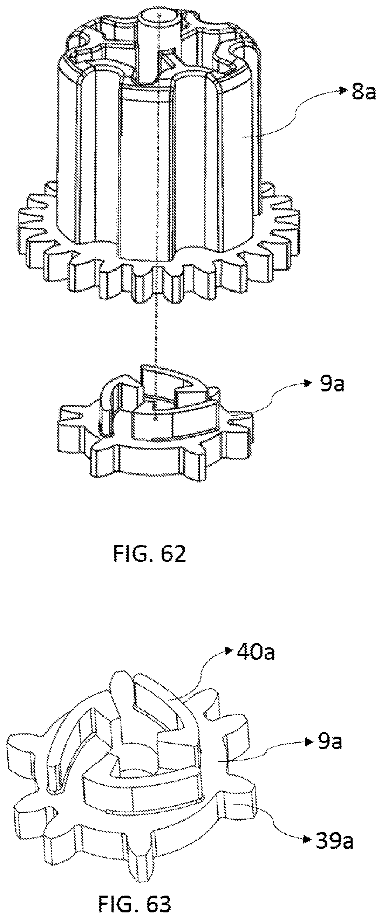

Figures 59 to 63 show a fifth embodiment of a blister cuvette (8) and a ratchet assembly (9). The blister cuvette (8) contains six such equally spaced cuvettes (31) around the circumference. The driver gear (2) rotates the ratchet assembly (9) via gears (39) of the ratchet assembly (9). The drive pin (40) drives the blister cuvette (8) via the ratchet teeth (45) on the blister cuvette (8a). The ratchet assembly (9) may have more than one drive pins to drive the blister cuvettes (8). The ratchet assembly (9) may have two to three drive pins (40) to drive the blister cuvette (8). The ratchet teeth (45) drive the blister cuvette (8) in one direction. The fifth embodiment of the blister cuvette (8) may be used for both the medicament carriers (5a) and (5b) with only difference in the orientation of the ratchet teeth (45) and the drive pin (40).

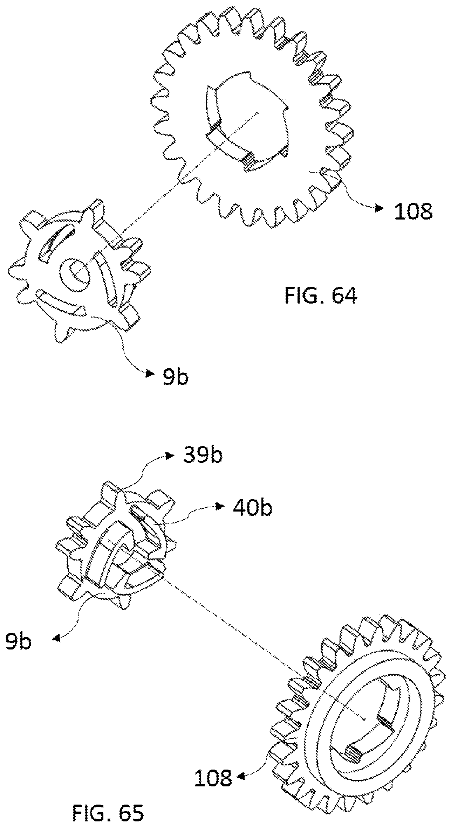

Figures 64 and 65 show different views of the second ratchet assembly (9b) and interim driver gear (108). The second ratchet assembly (9b) is located concentrically with the interim driver gear (108). The ratcheting mechanism of the second ratchet assembly (9b) and the interim driver gear (108) prevents the rotation of the interim driver gear (108) and thereby the second blister cuvettes (8b) in the opposite direction. The second ratchet assembly (9b) does not rotate the interim driver gear (108) during the reverse motion or the closing of the mouthpiece cover (3).

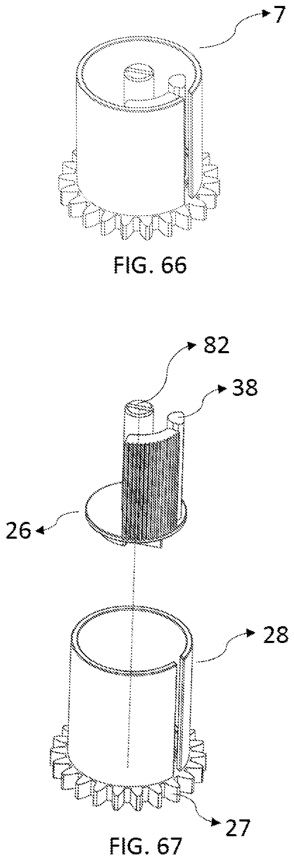

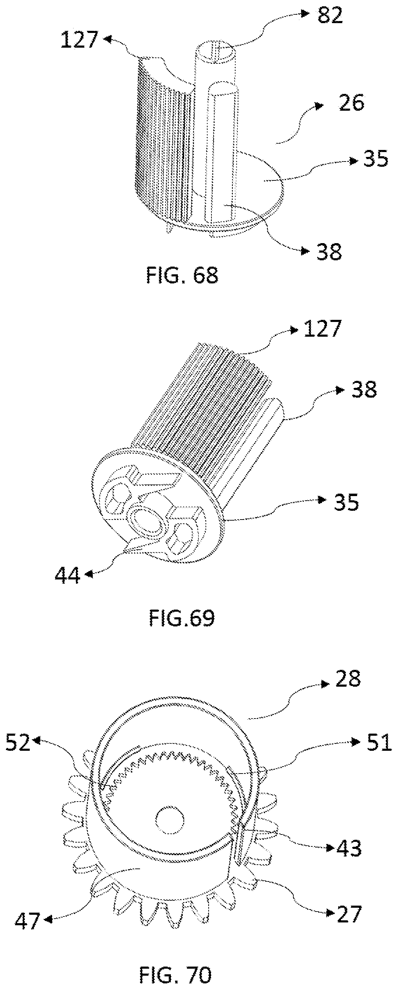

Figures 66 to 70 show a first embodiment of the winding assembly (7) that is compatible with the medicament dispenser described herein. The winding assembly (7), comprising a tensioning mechanism (26) and a winding wheel (28), each with their associated components as described herein can be capable of operating within the devices described herein and also or alternatively can operate an independent embodiment of the present invention. That is, the winding assembly (7), comprising a tensioning mechanism (26) and a winding wheel (28), each with their associated components, can operate as an elongate media collection mechanism within other devices, such as, for example, other non-medicament dispensers or other medicament dispensers. The tensioning mechanism (26) can include a circular form base (35) having two ratchet arms (44) in its underside surface and integral, upwardly extending axle (82) and lid sheet guide (127). The ratchet arms (44) are symmetrically located about the central plane passing through the central axis of rotation of the tensioning mechanism (26) facing away from each other and the central axis. The ratchet arms (44) flex at the distal end while ratcheting over the inner teeth (52) of the winding wheel. The ratchet arms (44) of the tensioning mechanism (26) can ride over the inner teeth (52) of the winding wheel (28). The uni-directional movement of the tensioning mechanism (26) provides the required tension on the lid sheet (73). The lid sheet guide (127) may have a serrated surface to guide and grip the lid sheet (73) as it moves within the winding wheel (28) during the tensioning process. The base of the tensioning mechanism (35) may also include an upwardly standing post (38) arranged for receipt of the looped end of the sheet. The post (38) provides an anchor to hold the lid sheet (73) at the sealed loop (70) of the medicament carrier (5). The ratchet arm (44) of the base (35) may be in ratcheted engagement with the inner teeth (52) of the winding wheel (28) such that the winding wheel (28) may only be rotated in one direction relative to the base (35). In Figure 66, the tensioning mechanism (26) is located in the winding wheel. The tensioning mechanism (26) is concentric with the winding wheel (28). The winding wheel (28) can include the winding surface (47), the slit (43) and the locking ridges (51). The vertical motion of the tensioning mechanism (26) is prevented by the locking ridges (51) of the winding wheel (28). The winding wheel (28) allows the lid sheet (73) to enter via the vertical slit (43). The lid sheet (73) tensioning takes place due to the unidirectional rotation of the tensioning mechanism (26). Once the lid sheet (73) tension is achieved, the lid sheet (73) winds over the winding surface (47) of the winding wheel (28) during medicament carrier advancement. The winding wheel (28) can also include inner teeth (52) provided to its inner part and outer teeth (27) provided to its outer part. The winding wheel (28) is driven by the blister cuvette (8) during blister advancement via outer teeth (27).

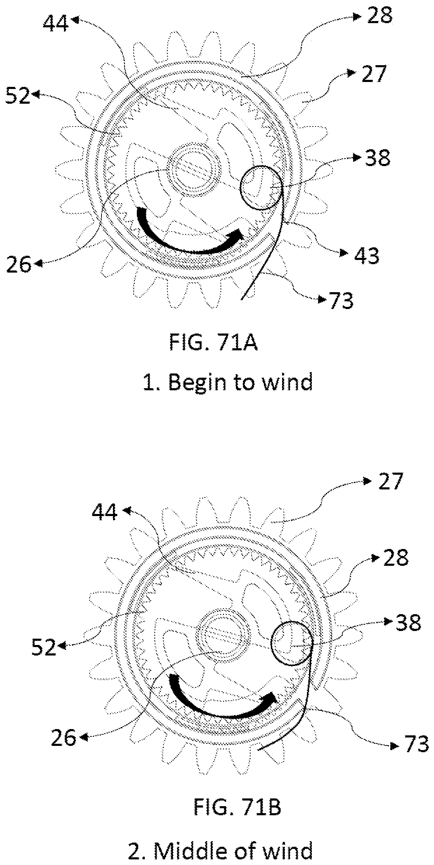

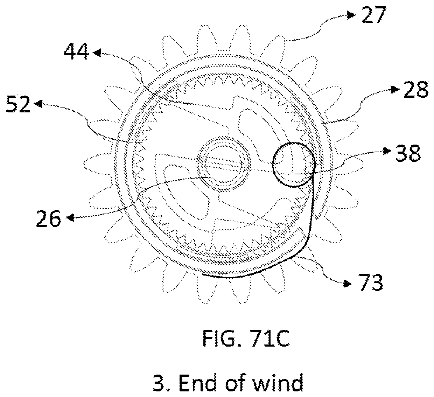

Figures 71A to 71C show the sheet tensioning mechanism. The tensioning mechanism (26) is rotated in a counter-clockwise direction (in alternative embodiments, anticlockwise rotation is envisaged) by a defined rotation relative to the winding wheel (28) clockwise to begin the tensioning of the lid sheet (73). During sheet tensioning, the winding wheel (28) remains stationary. It will be appreciated that because the ratchet arm (44) of the base of the tensioning mechanism (35) mesh with the inner teeth (52) of the winding wheel (28), such rotation results in tensioning of the lid sheet (73). The lid sheet (73) continues to move along with the tensioning mechanism (26) as it ratchets over the inner teeth (52) of the winding wheel (28). At the same time, the eccentric surface of the lid sheet guide (127) on the tensioning mechanism (26) grips and guides the lid sheet (73) thereby facilitating the sheet tensioning. The winding wheel (28) does not rotationally unwind in the opposite direction from its tensed state as a result of the ratcheted interaction between the ratchet arm (44) of the base (35) and the inner teeth (52) of the winding wheel (28). The sealed loop (70) on the end of the lid sheet (73) of a medicament carrier is looped over the post (38) of the tensioning mechanism (26). The winding assembly (7) is rotated thereby causing the lid sheet (73) to wrap around the winding wheel surface (47). As soon the preset tension is achieved on the lid sheet (73) during the rotation of the tensioning mechanism (26), it stays locked in place due to the ratchet arms (44). The winding wheel (28) and the tensioning mechanism (26) now rotate as a single assembly during the carrier advancement. The winding assembly (7) is now ready for use in a suitable medicament dispenser.

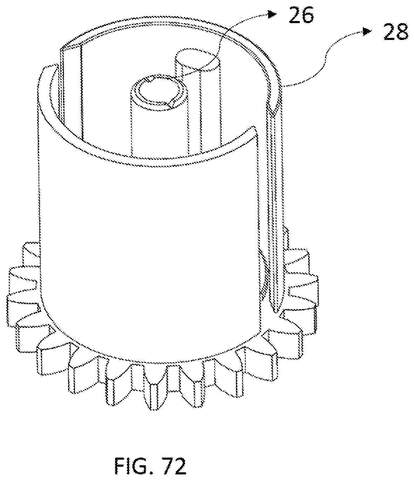

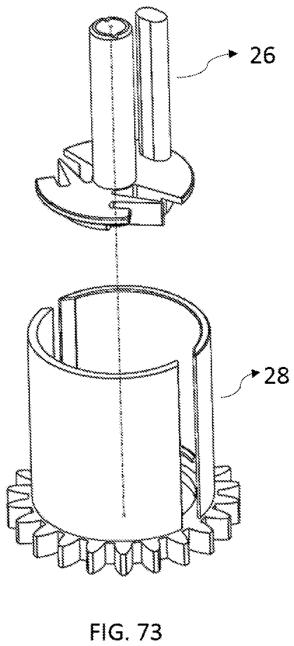

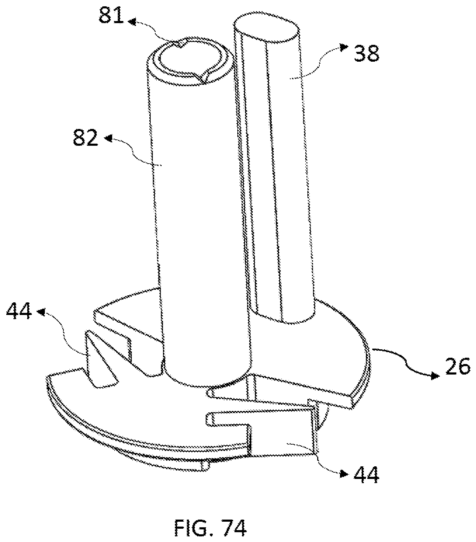

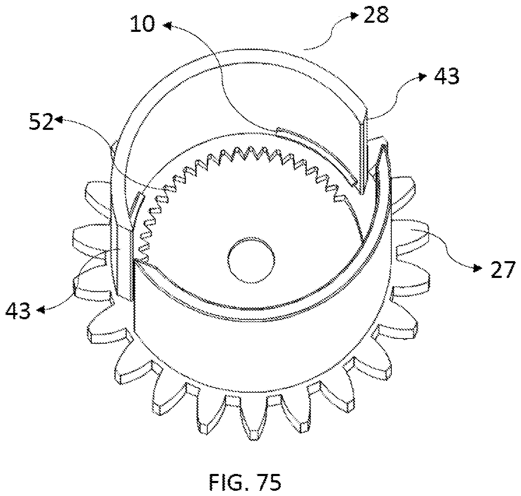

Figures 72 to 75 show a second embodiment of the winding assembly (7) that is compatible with the medicament dispenser described herein. The tensioning mechanism (26) can include a circular form base (35) having two ratchet arms (44) and integral, upwardly extending axle (82). The ratchet arms (44) of the tensioning mechanism (26) can ride over the inner teeth (52) of the winding wheel (28). The tensioning mechanism (26) is bi-directional and can be rotated both clockwise and counter-clockwise to facilitate the tensioning of the lid sheet (73). The base (35) may also include an upwardly standing post (38) arranged for receipt of the looped end of the sheet. The post (38) provides an anchor to hold the lid sheet (73) at the sealed loop (70) of the medicament carrier (5). The tensioning mechanism (26) rotates about the axle (82). The ratchet arms (44) are symmetrically located about the central plane passing through the central axis of rotation of the tensioning mechanism (26). The ratchet arms (44) flex at the distal end while ratcheting over the inner teeth (52). The ratchet arms (44) lock the ratchet position and hence the lid sheet (73) once tensioned. Groove (81) allows rotation of the tensioning mechanism (26) in either direction through manual or automated means. The bi-directional feature allows the use of the same tensioning mechanism (26) on either side of the advancing mechanism.

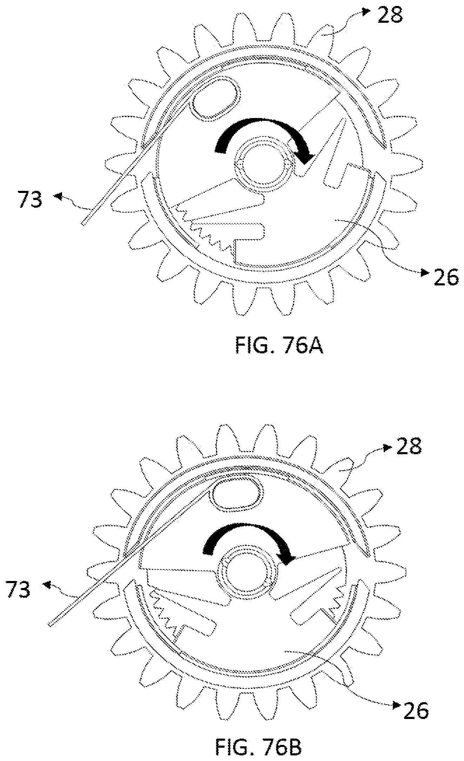

Figures 76A to 76B show the sheet tensioning mechanism. The winding wheel (28) is stationary during the initial tensioning of the lid sheet (73). The tensioning mechanism (26) rotates either clockwise or counterclockwise concentric to the winding wheel (28). Once the lid sheet (73) is adequately tensioned, the winding wheel (28) rotates to wind the lid sheet (73) as it peels during the actuation of the dispenser. The ratchet arms (44) ride over the inner teeth (52). Only one ratchet arm (44) out of the two ratchets is in contact with the inner teeth (52). This manner of operation of these components allows for the tensioning mechanism (26) to rotate clockwise or counterclockwise. The locking ridges (51) prevent the axial motion of the tensioning mechanism (26). Slits (43) located diametrically opposite to each other on the winding wheel (28) allow the lid sheet (73) to enter the winding wheel (28) and secure over the post (38) of the tensioning mechanism (26). Outer teeth (27) on the winding wheel (28) engage with the blister cuvette (8).

The lid sheet (73) enters the winding wheel (28) and is secured with a sealed loop (70) around the post (38). In this case, the tensioning mechanism (26) is rotated clockwise, thereby tensioning the lid sheet (73). Only one ratchet arm (44) is in contact with the inner teeth (52). Once tensioned, the tensioning mechanism (26) is locked in place and rotates in synchronous motion with the winding wheel (28) as the medicament carrier advances during dispenser actuation.

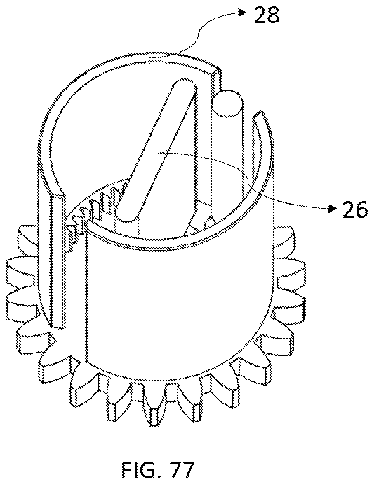

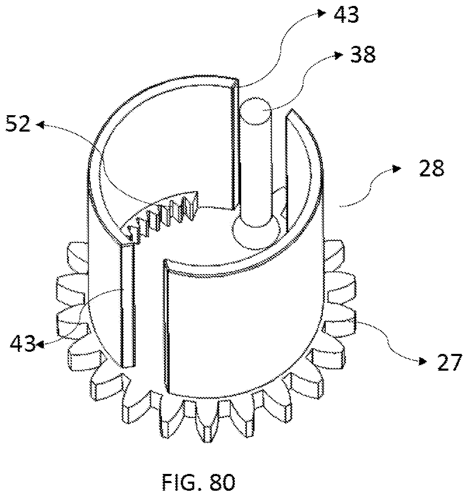

Figures 77 and 80 show a third embodiment of the winding assembly (7) that is compatible with the medicament dispenser described herein. The tensioning mechanism (26) can include a circular form base (35) having two ratchet arms (44) and integral, upwardly extending lid sheet guide (127). The ratchet arms (44) of the tensioning mechanism (26) can ride over the inner teeth (52) of the winding wheel (28). The tensioning mechanism (26) is rotated counter-clockwise to facilitate the tensioning of the lid sheet (73). The lid sheet (73) is attached to the post (38) of the winding wheel (28) via the sealed loop (70). The ratchet arms (44) are symmetrically located about the central plane passing through the central axis of rotation of the tensioning mechanism (26) facing away from each other and the central axis. The ratchet arms (44) flex at the distal end while ratcheting over the inner teeth (52). The ratchet arms (44) lock the ratchet position and hence the lid sheet (73) once tensioned. The lid sheet (73) forms around the filleted edge (83) of the lid sheet guide (127) before securing over the post (38) of the winding wheel (28). The lid sheet guide (127) is part of the tensioning mechanism (26) and tensions the lid sheet (73) as it rotates about the center during ratcheting.

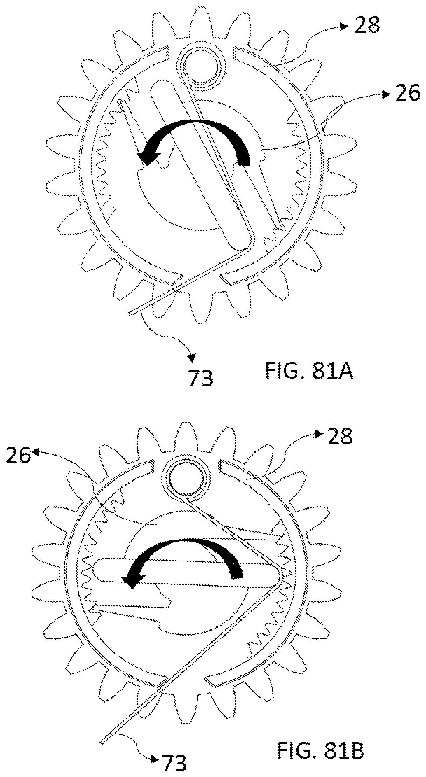

Figures 81A to 81B show the sheet tensioning mechanism. The winding wheel (28) is stationary during the initial tensioning of the lid sheet (73). The tensioning mechanism (26) rotates counterclockwise concentric to the winding wheel (28). Once the lid sheet (73) is adequately tensioned, the winding wheel (28) rotates to wind the lid sheet (73) as it peels during the actuation of the dispenser. The ratchet arms (44) ride over the inner teeth (52). The post (38) of the winding wheel (28) facilitates securing one end of the lid sheet (73). Slits (43) on the winding wheel (28) allow the lid sheet (73) to enter the winding wheel (28) and secure over the post (38). Outer teeth (27) of the winding wheel (28) engage with the blister cuvette (8).

The lid sheet (73) enters the winding wheel (28) and is secured with a sealed loop (70) around the post (38). The tensioning mechanism (26) is rotated counterclockwise thereby tensioning the lid sheet (73). The ratchet arms (44) along with the inner teeth (52) on the winding wheel (28) prevent the clockwise motion of the tensioning mechanism (26). Once tensioned, the tensioning mechanism (26) is locked in place and rotates in synchronous motion with the winding wheel (28) as the medicament carrier advances during dispenser actuation.

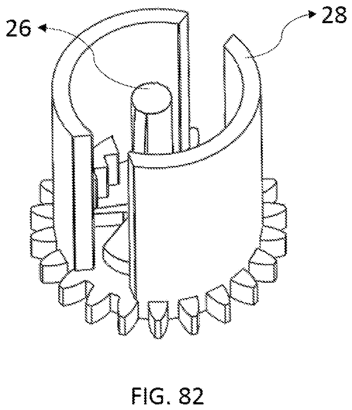

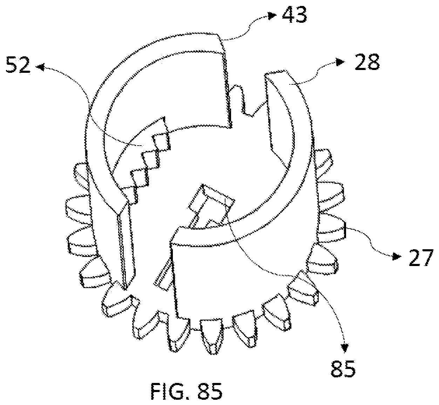

Figures 82 and 85 show a fourth embodiment of the winding assembly (7) that is compatible with the medicament dispenser described herein. The tensioning mechanism (26) can include a base (35) having two ratchet arms (44), integral, upwardly extending post (38) and a slider (84). The tensioning mechanism (26) moves along the long axis of the winding wheel (28) to facilitate the tensioning of the lid sheet (73). The lid sheet (73) is attached to the post (38) of the tensioning mechanism (26) via the sealed loop (70). The ratchet arms (44) are symmetrically located about the central plane passing through the central axis of rotation of the tensioning mechanism (26). The ratchet arms (44) flex at the distal end while ratcheting over the inner teeth (52) of the winding wheel (28). The ratchet arms (44) lock the ratchet position and hence the lid sheet (73) once tensioned. Slider (84) locks the tensioning mechanism (26) inside the groove (85) and thereby prevents the axial motion of the tensioning mechanism (26), Slider (84) facilitates the sliding motion of the tensioning mechanism (26) during tensioning of the lid sheet (73).

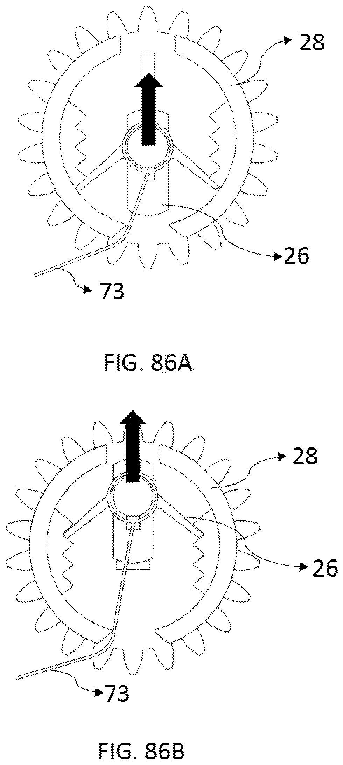

Figures 86A to 86B show the sheet tensioning mechanism. The winding wheel (28) is stationary during the initial tensioning of the lid sheet (73). The tensioning mechanism (26) moves along with the long axis of the winding wheel (28) to facilitate the tensioning of the lid sheet (73). Once the lid sheet (73) is adequately tensioned, the winding wheel (28) rotates to wind the lid sheet (73) as it peels during the actuation of the dispenser. The ratchet arms (44) ride over the inner teeth (52) while the slider (84) slides within the groove (85). Slit (43) on the winding wheel (28) allows the lid sheet (73) to enter the winding wheel (28) and secure over the post (38) of the tensioning mechanism (26). Outer teeth (27) on the winding wheel (28) engage with the blister cuvette (8).

The lid sheet (73) enters the winding wheel (28) and is secured with the sealed loop (70) around the post (38). The tensioning mechanism (26) is pushed along the long axis of the winding wheel (28), thereby tensioning the lid sheet (73). The ratchet arms (44), along with the inner teeth (52) of the winding wheel (28), prevent the reverse motion of the tensioning mechanism (26). Once tensioned, the tensioning mechanism (26) is locked in place and rotates in synchronous motion with the winding wheel (28) as the medicament carrier advances during dispenser actuation.

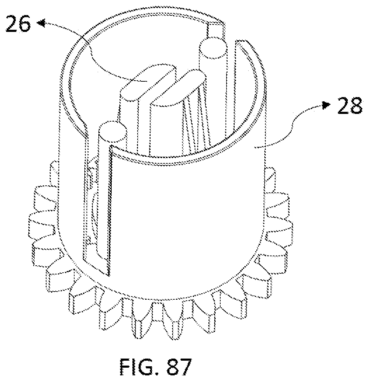

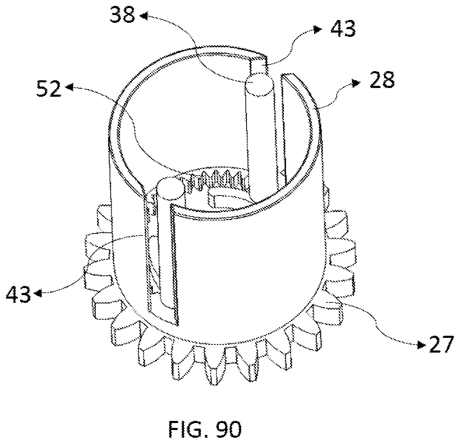

Figures 87 and 90 shows a fifth embodiment of the winding assembly (7) that is compatible with the medicament dispenser described herein. The tensioning mechanism (26) can include a base (35) having two ratchet arms (44) and integral, upwardly extending split axle (88) and slot (86). In this aspect, the split axel (88) provides for a means of guiding the lid sheet (73) as it is tensioned. The tensioning mechanism (26) can be rotated counter-clockwise to facilitate the tensioning of the lid sheet (73). The lid sheet (73) is attached to the post (38) of the winding wheel (28) via the sealed loop. The lid sheet (73) passes through the slot (86) on the tensioning mechanism (26) while it loops around the post (38) of the winding wheel (28). Ribs (87) are provided along the length of the split axle (88) to provide stability to the split axle (88). The lid sheet (73) passes through the slot (86) of the split axle (88) and secures over the post (38) of the winding wheel (28). The split axle (88) is split and divided along the central plane passing through the axis of rotation of the tensioning mechanism (26). The ratchet arms (44) lock the ratchet position and hence the lid sheet (73) once tensioned.

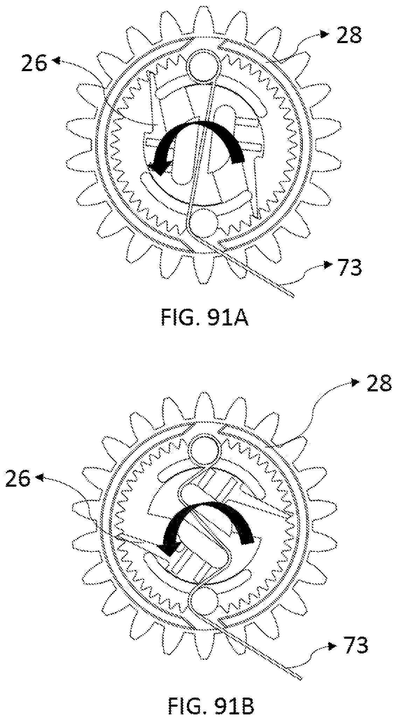

Figures 91A to 91B show the sheet tensioning mechanism. The winding wheel (28) is stationary during the initial tensioning of the lid sheet (73). The tensioning mechanism (26) rotates counterclockwise concentric to the winding wheel (28). Once the lid sheet (73) is adequately tensioned, the winding wheel (28) rotates to wind the lid sheet (73) as it peels during the actuation of the dispenser. The ratchet arms (44) ride over the inner teeth (52). The post (38) on the winding wheel (28) facilitates securing one end of the lid sheet (73). Slits (43) on the winding wheel (28) allow the lid sheet (73) to enter the winding wheel (28) and secure over the post (38). Outer teeth (27) on the winding wheel (28) engage with the blister cuvette (8).

The lid sheet (73) enters the winding wheel (28) and is secured with the sealed loop (70) around the post (38). The ratchet arm (44) is rotated counterclockwise thereby tensioning the lid sheet (73). The ratchet arms (44) along with the inner teeth (52) of the winding wheel (28), prevent the clockwise motion of the tensioning mechanism (26). Once tensioned, the tensioning mechanism (26) is locked in place and rotates in synchronous motion with the winding wheel (28) as the medicament carrier advances during dispenser actuation.

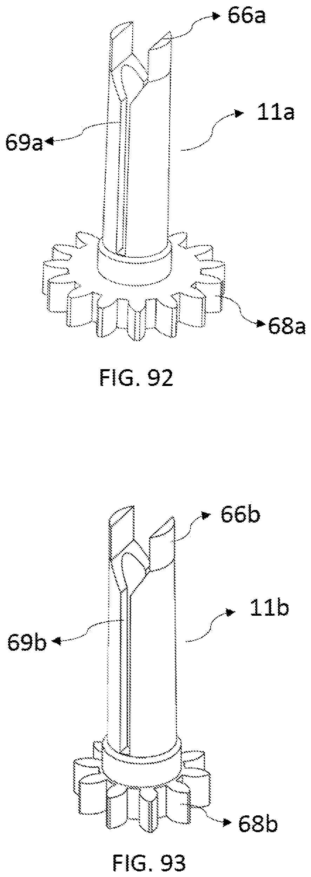

Figures 92 and 93 show the first and second wrapping wheels 11a and 11b according to one aspect of the disclosure. The first wrapping wheel (11a) is driven by the first blister cuvette (8a) via the interim gear (10). The second wrapping wheel (11b) is driven by the interim driver gear (108) and secondary interim gear (130). The open end (71) of each of the base sheets (72a) and (72b) is located with the respective slits (69a) and (69b) and wraps around each of the respective wrapping wheels (11a) and (11b) as they rotate during actuation.

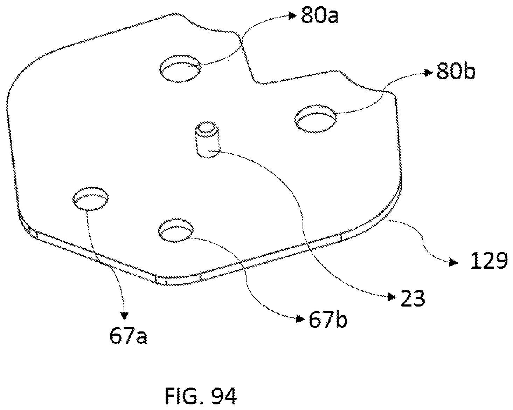

Figure 94 shows a base plate (129) according to one aspect of the disclosure. The base plate (129) may be mechanically or permanently bonded to the inner housing assembly (4). The pin (23) can locate the mouthpiece cover (3) via the pivot hole (16). The openings (67a) and (67b) are provided to allow passage of the pins (66a) and (66b) of the wrapping wheels (11a) and (11b). The openings (80a) and (80b) are provided to allow passage of the ratchet assemblies (9a) and (9b).

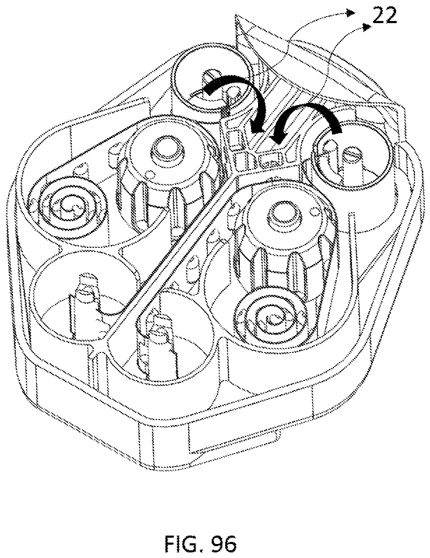

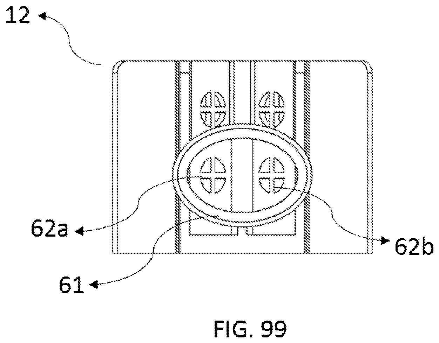

Figures 95 to 99 provide a more detailed description of the flow of the air (22) into the dispenser, the inlet vent openings (74) and the flow channel (12). As described above, and as shown in Figures 2A to 2D, the inlet vent openings (74) of the top cover (13) is covered by the mouthpiece cover (3) when in its first and second position (Figures 2A and 2B) and fully revealed when the mouthpiece cover (3) is in its third or open position (Figure 2C). In use, the inlet vent openings (74) allow air (22) to pass from outside the medicament dispenser into the inlet compartments (17a) and (17b) in response to inhalation by the patient through the mouthpiece opening (25), as indicated schematically by the arrow in Figures 95 and 96. Notably, the inlet vent openings (74) provide the sole intended point of entry of air from the outside into the medicament dispenser upon patient inhalation at the mouthpiece opening (25). More particularly, the inlet vent openings (74) provide the sole entry point for air outside the dispenser device to pass into the flow channel (12) upon patient inhalation on the mouthpiece opening (25). Air enters the dispenser through the inlet vent openings (74) once the user inhales the drug through the mouthpiece opening (25).

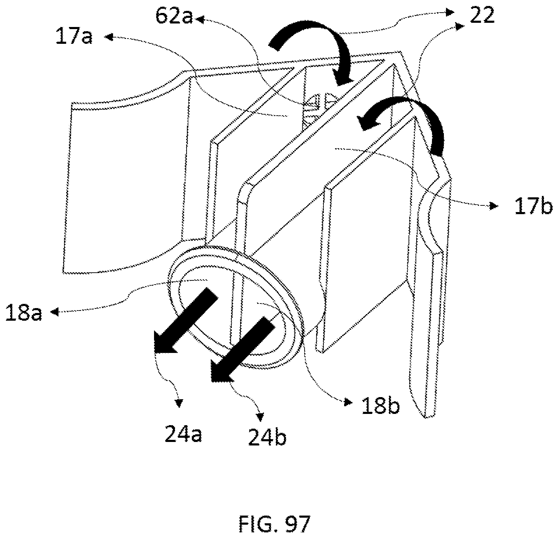

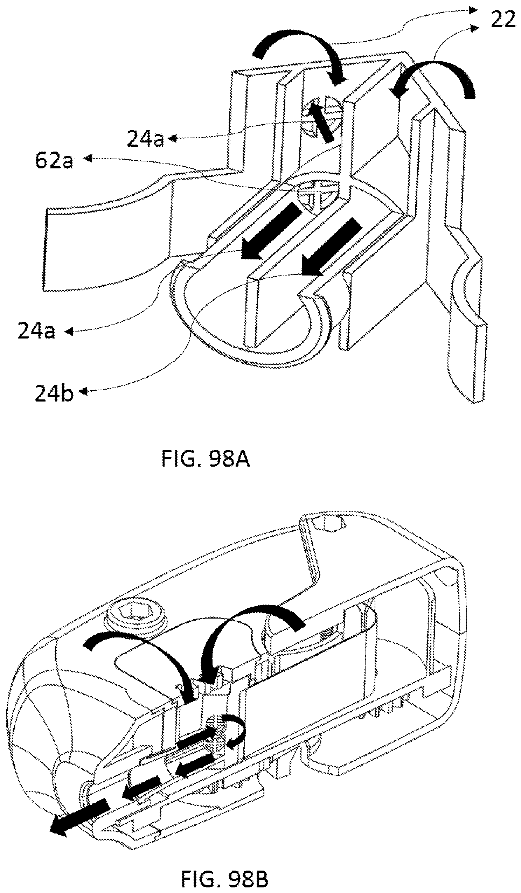

The flow channel (12) can be permanently attached to the inner housing assembly (4) on the notch (59) during the assembly process. The flow channel (12) provides a path for the inlet air to evacuate the powder from the blister pockets (41) during inhalation. The flow channel (12) also helps the powder to de-agglomerate before inhalation. Referring to Figures 97 and 98 (98A and 98B), the flow channel (12) has inlet compartments (17a) and (17b) and outlet compartments (18a) and (18b) and plural pairings of grids (62a) and (62b). The first pair of grids (62a) may be seen in Figure 98A and 98B. As may also be seen by reference to Figures 97 and 98 (98A and 98B), the inlet inner compartments (17a) and (17b) locate adjacent to the outlet compartments (18a) and (18b) and shares a common wall therewith.

Each inlet compartment (17a) and (17b) has an inlet and an exit in the form of a grid opening. In use, each inner compartment directs inward airflow (as exclusively received through the inlet vent openings on patient inhalation) from the inlet to the grid opening.

Each outlet compartment has an inlet in the form of a grid opening and exit in the form of a flow channel opening. The mouthpiece is provided to the flow channel opening (61) and snap-mounts thereto via a snap-mounting feature. In use, the inlet compartment directs inward airflow received through the inlet vent openings (74) to the outlet compartments via the plural pairing of grids (62a) and (62b).

The flow channel (12) is received by the inner housing assembly (4) such that inlet compartments (17a) and (17b) to the flow channel are located adjacent to the top cover (13), which defines the inlet vent openings (74).

In use, the inlet vent openings (74) directs inward airflow (as exclusively received through the inlet vent openings on patient inhalation) from the inlet compartments (17a) and (17b) to the outlet compartments (18a) and (18b) via respective plural pairings grids (62a) and (62b). The plural pairings grids (62a) and (62b) are defined by a pair of circular openings. Each circular opening is provided with a respective cruciform. The mouthpiece (29) is provided to the flow channel opening (61) and snap-mounts thereto via a snap-mounting feature. Each pair of grids is positioned adjacent to one another.

As detailed hereinabove, when the mouthpiece cover (3) is fully opened to its third position, the blister cuvettes (8a) and (8b) are advanced to cause each medicament carrier (5a) and (5b) to be advanced and a single pocket (41) of each carrier to be peeled open. The peeled open blister pocket (41) of each medicament carrier (5a) and (5b) lies adjacent to a respective one of the pairs of grids (62a) and (62b). Specifically, the open blister pocket (41a) of the first medicament carrier (5a) locates adjacent to the first pair of grids (62a) and the open blister pocket (41b) of the second medicament carrier (5b) likewise locates adjacent the other pair of grids (62b).

Figures 100A and 100B show different views of an embodiment of the medicament carrier. When the open pockets (41a) and (41b) are presented to the flow channel (12) at the opening location, the pockets (41a) and (41b) are oriented so that the sideways orientation thereof is aligned to the direction between the respective pairing of grids (62a) and (62b).

As shown in Figures 95 to 98A and 98B, when a patient inhales at the mouthpiece openings (25), an airstream (air) (22) flows from outside of the dispenser device into the flow channel (12) solely through the inlet vent openings (74) into the inlet compartments (17a) and (17b), which is in juxtaposed relation with the inlet vent openings (74).

As graphically represented in Figures 97 and 98A and 98B, the airstream is divided into first portion (24a) and second portion (24b) and flow into the opened blister pocket (41a) and (41b) of each medicament carrier (5a) and (5b) at the opening locations via the respective grid openings, thereby entraining the medicament powder contained in the pockets in the airstream, and thence out of the pocket (41a) and (41b) into the respective outlet compartments (18a) and (18b) via the grid openings. The respective airstream (24a) and (24b) with entrained medicament powder then flows out of the mouthpiece opening (25) into the patient's respiratory tract. The first and second airstream portions (24a) and (24b) are created concurrently in the flow channel (12) upon patient inhalation at the mouthpiece opening (25).

As detailed hereinabove, when the mouthpiece cover (3) is fully opened to its third position, the gear and dispensing mechanisms are actuated to cause each medicament carrier (5a) and (5b) to be advanced and a single pocket (41) of each carrier to be peeled open. The peeled open blister pocket of each medicament carrier (5a) and (5b) lies adjacent to a respective one of the pairs of grids (62a) and (62b). When a patient inhales at the mouthpiece, an airstream (air) (22) flows from outside of the dispenser into the flow channel solely through the inlet vent openings (74) into the inlet compartments (17a) and (17b). First portion of the air (22) flows into the opened blister pocket of the first medicament carrier (5a) via the opening of the first pair of grids (62a), thereby entraining the medicament powder contained in the pockets in the airstream, and then out of the pockets into the outlet compartments (18a) via another opening of grids (62a). Likewise, second portion of this airstream (24b) flow into the opened blister pocket of the second medicament carrier (5b) via the opening of the grids (62b), thereby entraining the medicament powder contained in the pockets in the airstream, and then out of the pockets into the outer compartments (18b) via another opening of grids (62b). The separate portions of the airstream with entrained medicament powder then flows out of the mouthpiece opening (25) into the patient's respiratory tract. The portions of the airstream are distinct in the dispenser.

Figure 95 shows the mouthpiece and its opening according to one aspect of the disclosure. The mouthpiece (29) allows the user to receive the contents of the medicament carrier (5) by placing the mouthpiece opening (25) inside the oral cavity. The mouthpiece (29) can be covered by the mouthpiece cover (3) when the dispenser is not in use.

Figures 100A and 100B show different views of the medicament carrier according to one aspect of the disclosure. The medicament carrier (5) can include a plurality of blister pockets (41), each of which contains a portion of a dose of medicament in the form of powder. The medicament carrier (5) can include a base sheet (72) in which blisters are formed to define the pockets (41) and a lid sheet (73) which is sealed to the base sheet such a manner that the base sheet (72) and the lid sheet (73) can be peeled apart. The sheets (72) and (73) are sealed to one another over their whole width except for the end portions (70) and (71). One end of the lid sheet has a sealed loop (70) that can be placed in the blister post (38) of the winding assembly (7). Open end (71) of the base sheet (72) of a medicament carrier (5) can be placed in the slit (69) of the wrapping wheel (11). In an embodiment, the medicament dispenser may contain two identical medicament carriers. The pockets (41) of each medicament carrier contain the same medicament powder. However, one medicament carrier may have different medicaments in the powder than the other medicament carrier.

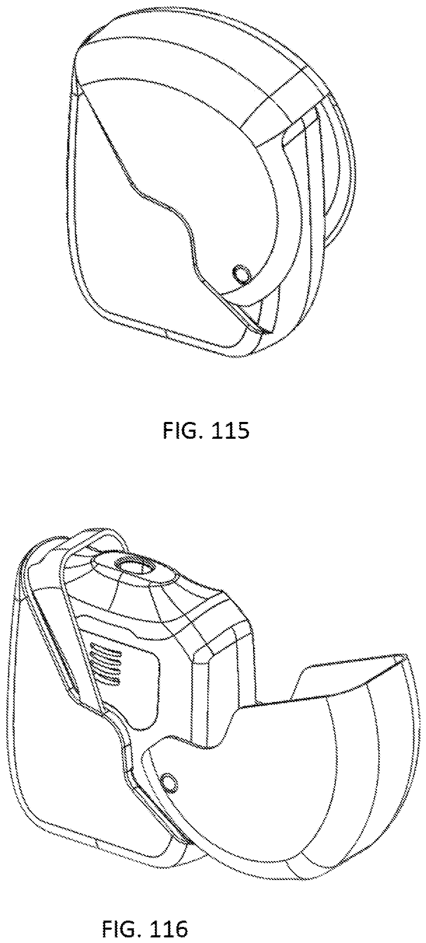

Figure 116 shows the front view of the fifth embodiment of the dispensing mechanism. Figure 116 shows modification in 30 dose dispensing device to dispense 60 doses of medicament wherein first 31 doses being indexed by first indexer and after completion of all the doses of first indexer, second indexer is moved to dispense remaining 30 doses of medicament. Figure 116 shows first and second medicament carriers (5a) and (5b). Each medicament carrier (5a) and (5b) engages with respective blister cuvettes (8a) and (8b). One carrier may contain one or more different medicament(s) than the other carrier. At an opening location (an opening location is a position within the medicament dispenser wherein the medicament carrier is opened or where an individual dose of medicament is otherwise held within the medicament carrier and positioned for access), the base sheet and the lid sheet parts (72) and (73) (best exemplified in Figures 100a and 100b) of each medicament carrier (5a) and (5b)) are peelably separable. The resulting empty base sheet (72a) and (72b) coils up in respective compartments. The wrapping wheels (11a) and (11b), for wrapping the base sheets of the two medicament carriers, each anchor the end of each respective base sheet (72a) and (72b) in the compartments. Progressive rotation of each respective wrapping wheel results in the base sheets (72a) and (72b) being wound up therearound into a tight coil. The rotation of each winding assembly (7a) and (7b) is coupled to that of the respective blister cuvettes (8a) and (8b) such the rotation of the blister cuvettes (8a) and (8b) rotates new blister packs of the medicament carriers (5a) and (5b) into the appropriate position within the opening location, the winding assemblies (7a) and (7b) correspondingly winding the lid sheets peeled away from each respective medicament carrier base sheet. The resulting separated lid sheets (73a) and (73b) are wound onto/into the respective winding assemblies (7a) and (7b) respectively.

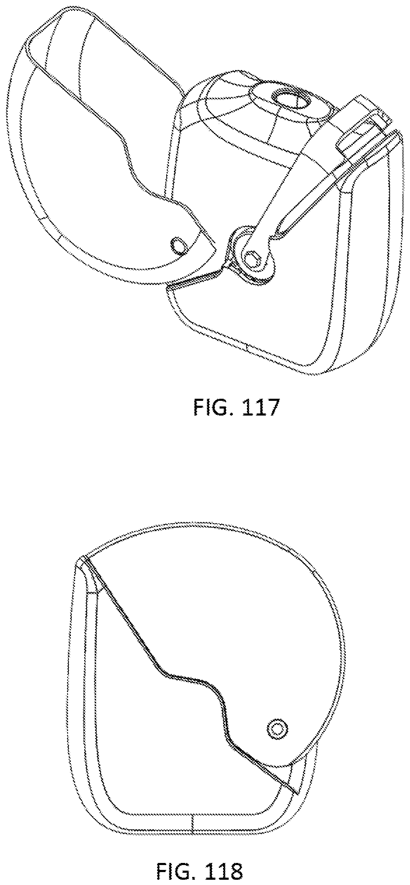

As illustrated in Figure 117, the mouthpiece cover (3) (not shown in Fig. 117) is movable along the housing. The mouthpiece cover (3), when sufficiently moved, rotates the driver gear assembly (129) which in turn rotates the first ratchet assembly (9a) only thereby indexing only one blister. During the first 31 actuations, the driver gear assembly (129) does not come in contact with and does not ratchet (128). Hence the blister on the indexer (8b) is not indexed for the first 31 actuations. After the first 31 actuations, the driver gear tooth (137) on the driver gear assembly (129) comes in contact with the ratchet (128) during every actuation. At this time, the driver gear assembly (129) rotates both the ratchets (9a) and (128) during every actuation. As a result, the driver gear assembly (129) allows the first 31 blisters to be indexed using indexer (8a) during each actuation followed by the indexing of remaining 30 blisters indexed using indexer (8b) during every actuation. This design results in converting the 30 dose blisters that are indexed during every actuation to a 61 dose blisters that are indexed sequentially by indexers 8a and 8b respectively.

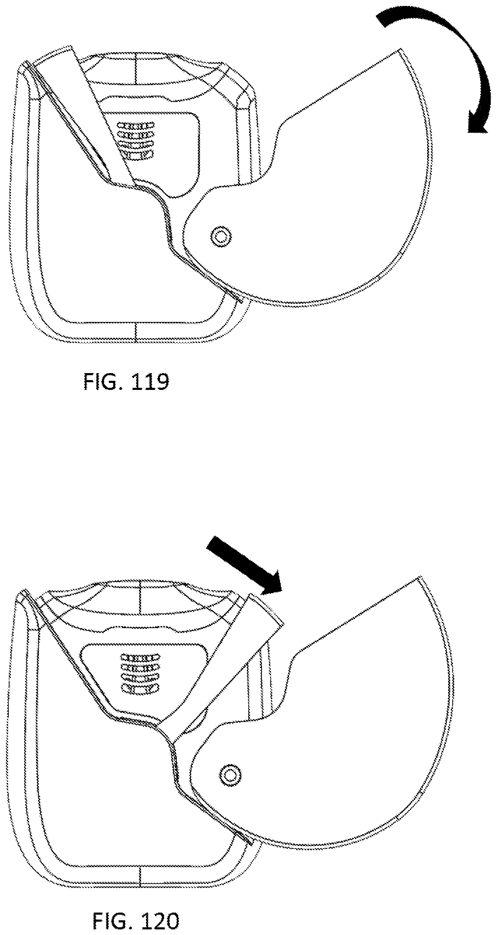

Figure 118 shows driver gear or central gear assembly that is compatible with fifth embodiment of the medicament dispenser described herein. As illustrated in Figure 119, the driver gear assembly (129) consists of a primary driver wheel (132), a spring loaded ratcheting disc (131), a secondary driver gear (130) and a torsion spring (133). The primary driver gear (132) is driven by the outer cover (3) via the central ratchet (49). The outer cover (3) rotates for a total angle of 90 deg. during each actuation. When the outer cover (3) and hence the primary driver wheel (132) rotate first 82 deg., it comes in contact with the spring loaded ratcheting disc (131). The primary driver wheel (132) hence drives the ratcheting disc (131) for the remaining 8 deg. before completing its actuation. The ratchet (136) on the ratcheting wheel (131) is in contact with the inner gears (139) on the secondary gear (130). The (136) ratchets and rotates the secondary wheel (130) by 8 deg. When the outer cover (3) is closed thereby rotating the primary driver wheel (132) in the opposite direction, the spring loaded ratchet disc (131) returns to its original position ratcheting over the inner gear teeth (139) of the secondary wheel (130) without rotating the secondary wheel (130) in the opposite direction. Each rotation of the primary wheel (132) results in an incremental rotation of the secondary wheel (130) by 8 deg. via the ratcheting disc (131). Once the secondary wheel (130) has ratcheted 31 times and reached its final position, the locking arm (135) on the primary driver wheel (132) locks within the groove (138) of the secondary wheel (130). After locking, the primary driver wheel (132) and the secondary wheel (130) rotate as a single unit. At this time, the ratcheting disc (131) is not able to ratchet and drive the secondary wheel (130) as it does not contact any inner gears (139) on the secondary wheel (130). The secondary wheel (130) is now able to ratchet (128) and drive the indexer (8b) during each actuation.

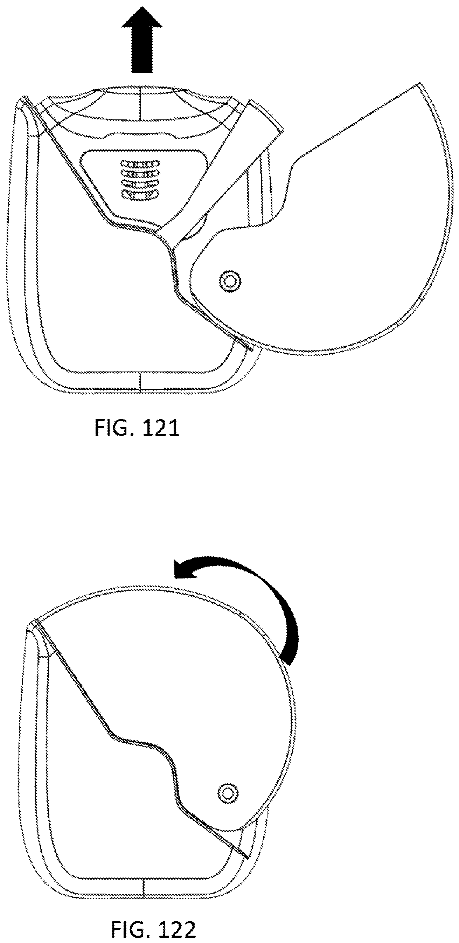

Figure 120,121 and 122 shows different components of central gear assembly. Figure 120 illustrates primary driver gear (132) having central ratchet (49), Gear tooth (134) and locking arm (135). Figure 121 shows a view of spring load ratcheting disc (131) having a ratchet (136). Figure 122 illustrates secondary gear (130) on which groove (138) and driver gear tooth (137) are situated at the outer circumference and inner gears (139) at inner circumference. The primary driver gear (132) is driven by the outer cover (3) via the central ratchet (49). Gear tooth (134) drives the ratchet (128) during each actuation. When the outer cover (3) and hence the primary driver wheel (132) rotate first 82 deg., it comes in contact with the spring loaded ratcheting disc (131). The ratchet (136) on the ratcheting wheel (131) is in contact with the inner gears (139) on the secondary gear (130). The ratchet (136) ratchets and rotates the secondary wheel (130) by 8 deg. When the outer cover (3) is closed thereby rotating the primary driver wheel (132) in the opposite direction, the spring loaded ratchet disc (131) returns to its original position ratcheting over the inner gear teeth (139) of the secondary wheel (130) without rotating the secondary wheel (130) in the opposite direction. Once the secondary wheel (130) has ratcheted 31 times and reached its final position, the locking arm (135) on the primary driver wheel (132) locks within the groove (138) of the secondary wheel (130). After locking, the primary driver wheel (132) and the secondary wheel (130) rotate as a single unit. The secondary wheel (130) is now able to ratchet (128) and drive the indexer (8b) during each actuation.

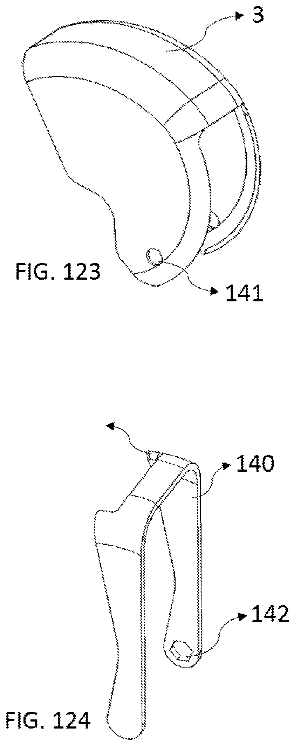

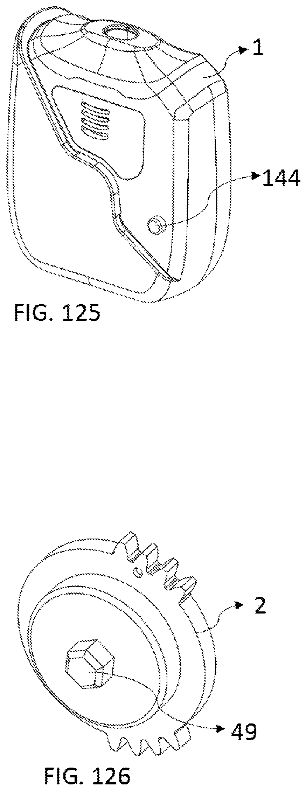

Figure 123 shows position of the driver gear or central gear when it is at initial position wherein the ratchet (136) on the spring loaded ratchet disc (131) is engaged with the first groove of inner gears (139) on the secondary gear (130). Figure 124 shows position of the driver gear or central gear after first actuation wherein the ratchet (136) on the spring loaded ratchet disc (131) is moved and engaged to with the second groove of inner gears (139) on the secondary gear (130). Figure 125 shows position of the driver gear or central gear after second actuation wherein the ratchet (136) on the spring loaded ratchet disc (131) is moved and engaged to with the subsequent third groove of inner gears (139) on the secondary gear (130). Figure 126 shows position of the driver gear or central gear after fifteen actuation and Figure 127 shows position of the driver gear or central gear after thirty one actuation wherein the ratchet (136) on the spring loaded ratchet disc (131) is moved to the last groove of inner gears (139) on the secondary gear (130) and the locking arm (135) on the primary driver wheel (132) locks within the groove (138) of the secondary wheel (130).

Figure 128 shows front view of the sixth embodiment of the dispensing mechanism. Figure 128 shows modification in 30 dose dispensing device to dispense 60 doses of API wherein dual 30 dose blister is modified to meet the requirements of blister advancement of single 60 dose blister. Fig 129 shows the driver gear (2) which is driven by the lever (140). The driver gear (2) rotates the blister advancing ratchets (9a and 128). Interim driver gear (108) is located between the driver gear (2) and the indexer (8b) to allow the rotation of indexers (8a & 8b) in opposite direction. The blister (5) contains the active drug in the blister pockets and are laminated at other end. The blister (5) aligns with the flow channel opening (12) during inhalation. The lidding foil (73) on the blisters (5) winds around the winder assembly (7b) during the operation of the inhaler. Similarly, the base foil (72) of the blister (5) is also advanced via the wrapping wheel (11a). The wrapping wheel (11a) rotates in the same direction of the respective blister cuvette or indexer (8a) via the interim gear (10). The blister (5) is separated from the blister advancing assembly via an inner housing (4). The inner housing (4) is fixed and supported inside the inhaler assembly.

Figure 130 show front closed view of an embodiment of the medicament dispenser. Figure 131 show front open view of an embodiment of the medicament dispenser wherein the medicament dispenser is actuated by the sufficient movement of lever (140). Figure 132 show rear open view of an embodiment of the medicament dispenser. Figure 133 show rear closed view of an embodiment of the medicament dispenser. Figure 134- figure 137 show a demonstration of the "Open, Click, Inhale, Close" mechanism of the medicament dispenser shown in figure 130- figure 133.

Figure 138 shows mouthpiece cover (3) which pivots around the boss (144) of the housing (1). Pivoting holes (141) accepts the boss (144) on the housing (1). Figure 140 shows the housing. Figure 139 shows lever (140) which directly drives the central gear (2) via flange (49). Flange (49) fits within the accepting grooves (142) on the lever (140). The lever (140) has slot feature (143) to facilitate user to rotate the lever (140). Figure 141 shows position of flange (49) on the central gear (2).

Conditional language used herein, such as, among others, "can," "might," "may," "e.g.," and the like, unless specifically stated otherwise, or otherwise understood within the context as used, is generally intended to convey that some embodiments include, while other embodiments do not include certain features, elements, and/or states. Thus, such conditional language is not generally intended to imply that features, elements, blocks, and/or states are in any way required for one or more embodiments or that one or more embodiments necessarily include logic for deciding, with or without author input or prompting, whether these features, elements and/or states are included or are to be performed in any particular embodiment.

The ranges disclosed herein also encompass any and all overlap, sub-ranges, and combinations thereof. Numbers preceded by a term such as "about" or "approximately" include the recited numbers and values that are ±10%.

Although certain embodiments and examples have been described herein, it will be understood by those skilled in the art that many aspects of the medicament dispensers shown and described in the present disclosure may be differently combined and/or modified to form still further embodiments or acceptable examples. All such modifications and variations are intended to be included herein within the scope of this disclosure. A wide variety of designs and approaches are possible. No feature, structure, or step disclosed herein is essential or indispensable.

### EXAMPLE EMBODIMENTS

The following example embodiments identify some possible permutations of combinations of features disclosed herein, although other permutations of combinations of features are also possible.

A medicament dispenser for dispensing medicament from plural elongate form medicament carriers, each carrier having multiple distinct dose portions, said dispenser comprising: a) a dispensing mechanism comprising: i) a driver gear, ii) a blister cuvette for each medicament carrier for individually advancing the distinct medicament dose portions thereof, iii) a ratchet assembly for each blister cuvette for rotating thereof, iv) a flow channel, v) a winding assembly, vi) a wrapping wheel; b) a mouthpiece; and c) a cover for the mouthpiece.

A medicament dispenser according to Embodiment 1, wherein the cover and the driver gear are arranged to rotate about a first axis of rotation.

A medicament dispenser according to Embodiment 1, wherein the lever and the driver gear are arranged to rotate about a first axis of rotation.

A medicament dispenser according to any one of Embodiments 1 and 2, wherein the cover is coupled to the driver gear such that movement of the cover results in rotation of the driver gear.

A medicament dispenser according to any one of Embodiments 1 and 2, wherein the lever is coupled to the driver gear such that movement of the lever results in rotation of the driver gear.

A medicament dispenser according to Embodiment 1, wherein the ratchet assembly is arranged to rotate about a second axis of rotation.

A medicament dispenser according to Embodiment 4, wherein one ratchet assembly is in meshed relationship with the driver gear.

A medicament dispenser according to Embodiment 4, wherein the other ratchet assembly is in meshed relationship with the driver gear via an interim driver gear.

A medicament dispenser according to Embodiment 5 to 6, wherein the ratchet assembly is constantly in meshed relationship with the driver gear.

A medicament dispenser according to any one of Embodiments 1 to 3, wherein the driver gear is a mutilated gear.

A medicament dispenser according to any one of Embodiments 1 to 3, wherein the driver gear may comprises primary driver wheel, a spring loaded ratcheting disc, a secondary driver gear and a torsion spring.

A medicament dispenser according to any one of Embodiments 5 and 7, wherein the ratchet assembly is intermittently in meshed relationship with the mutilated gear.

A medicament dispenser according to Embodiment 1, wherein the dispensing mechanism comprised plural distinct ratchet assemblies.

A medicament dispenser according to Embodiment 1, wherein the ratchet assembly and the blister cuvette are coaxial.

A medicament dispenser according to Embodiment 1, wherein the ratchet assembly and the blister cuvette are non-adjacent to one another.

The medicament dispenser according to Embodiment 1, wherein the ratchet assembly interacts with the blister cuvette such that movement of the ratchet assembly results in rotation of the blister cuvette.

A medicament dispenser according to Embodiment 1, wherein the dispensing mechanism comprised plural blister cuvettes.

A medicament dispenser according to Embodiment 13, wherein the plural blister cuvettes rotate in a successive fashion.

A medicament dispenser according to Embodiment 13, wherein the plural blister cuvettes are decoupled to each other.

The medicament dispenser according to any one of the preceding Embodiments, wherein movement of the cover rotates the driver gear, the ratchet assembly, and the blister cuvette, and thereby advances the medicament carrier to align with the flow channel for dispensing the medicament.

The medicament dispenser according to any one of the preceding Embodiments, wherein movement of the lever rotates the driver gear, the ratchet assembly, and the blister cuvette, and thereby advances the medicament carrier to align with the flow channel for dispensing the medicament.

The medicament dispenser according to any one of the preceding Embodiments, wherein the medicament comprises one or more active pharmaceutical ingredients and optionally, one or more pharmaceutically acceptable carriers.

The medicament dispenser according to Embodiment 16, wherein the active pharmaceutical ingredient is selected from group consisting of anticholinergics, anti-inflammatories, analgesics, anginal preparations, anti-allergic, antihistamines, antitussives, bronchodilators, anti-infectives, anti-fungals, leukotriene inhibitors, PDE IV inhibitors, diuretics, hormones, cromolyns, xanthines, therapeutic proteins and peptides, vaccines, diagnostics and gene therapies or combinations thereof.

The medicament dispenser according to any one of the preceding Embodiments, wherein the medicament is in the form selected from a group consisting of powder, tablet, capsule, pellet or puck, or mixtures thereof.

The medicament dispenser according to any one of the preceding Embodiments, wherein the dispenser is used for dispensing medicament for the treatment of asthma, chronic obstructive pulmonary disease (COPD), bronchitis, lung infections, interstitial pulmonary fibrosis (IPF), pulmonary arterial hypertension (PAH), cystic fibrosis, Non-CF Bronchiectasis or lung transplant.

The medicament dispenser according to any one of the preceding Embodiments, wherein the dispenser further comprises a dose counting mechanism for counting the number of doses released from the dispenser.

The medicament dispenser according to Embodiment 20, wherein the dose counter is in the form of mechanical or non-mechanical.

The medicament dispenser according to any one of the preceding Embodiments, wherein the dispenser further comprises a resting surface.

The medicament dispenser according to any one of the preceding Embodiments, wherein the dispenser further comprises a power source, an electronic circuit, and an electronic measurement instrument.

### ADDITIONAL EXEMPLARY EMBODIMENTS

The following further exemplary embodiments identify further possible permutations of combinations of features disclosed herein, although other permutations of combinations of features are also possible.
1. A medicament dispenser for dispensing medicament from a plurality of elongate form medicament carriers held therein, each carrier comprising multiple dose portions of one or more medicaments, said dispenser comprising:
   a. A dispensing mechanism for dispensing the medicament held within the dispenser comprising:
      i) a driver,
      ii) a carrier engagement for each medicament carrier for individually advancing the medicament dose portions thereof,
      iii) an advancer for each carrier engagement located coaxially with the carrier engagement for rotating thereof,
      iv) a flow director; and
   b. a medicament access body; and
   c. a cover for the medicament access body,
   wherein the medicament access body cover is engaged with the driver and wherein sufficient movement of the medicament access body cover results in movement of the driver, the driver being movingly engaged, either directly or indirectly, with the advancers such that sufficient movement of the driver results in movement of the advancers, each advancer being directly engaged with a carrier engagement such that sufficient movement of the advancers results in movement of the carrier engagements, and further wherein movement of each carrier engagement results in advancement of a medicament carrier to a position wherein a medicament dose portion of the medicament carrier, when present, is in sufficient alignment with the flow director such that separate airflows directed by the flow director entrain at least one medicament dose portion extracted from a present dose storage area, and further wherein the separate airflows containing the entrained medicament are maintained until the medicament exits the dispenser.
2. A medicament dispenser for dispensing medicament from a plurality of elongate form medicament carriers held therein, each carrier comprising multiple dose portions of one or more medicaments, said dispenser comprising:
   a. A dispensing mechanism for dispensing the medicament held within the dispenser comprising:
      i) a driver,
      ii) a carrier engagement for each medicament carrier for individually advancing the medicament dose portions thereof,
      iii) an advancer for each carrier engagement, wherein one or more advancers is located non-coaxially with the carrier engagement for rotating thereof,
      iv) a flow director; and
   b. a medicament access body; and
   c. a cover for the medicament access body,
   wherein the medicament access body cover is engaged with the driver and wherein sufficient movement of the medicament access body cover results in movement of the driver, the driver being movingly engaged, either directly or indirectly, with the advancers such that sufficient movement of the driver results in movement of the advancers, each advancer being directly engaged with a carrier engagement such that sufficient movement of the advancers results in movement of the carrier engagements, and further wherein movement of each carrier engagement results in advancement of a medicament carrier to a position wherein a medicament dose portion of the medicament carrier, when present, is in sufficient alignment with the flow director such that separate airflows directed by the flow director entrain at least one medicament dose portion extracted from a present dose storage area, and further wherein the separate airflows containing the entrained medicament are maintained until the medicament exits the dispenser.
3. A medicament dispenser for dispensing medicament from a plurality of elongate form medicament carriers held therein, each carrier comprising multiple dose portions of one or more medicaments, said dispenser comprising:
   a. A dispensing mechanism for dispensing the medicament held within the dispenser comprising:
      i) a driver,
      ii) a carrier engagement for each medicament carrier for individually advancing the medicament dose portions thereof,
      iii) an advancer for each carrier engagement, wherein one or more advancers is located coaxially with the carrier engagement for rotating thereof,
      iv) a flow director; and
   b. a medicament access body; and
   c. a cover for the medicament access body,
   wherein the medicament access body cover is engaged with the driver and wherein sufficient movement of the medicament access body cover results in movement of the driver, the driver being movingly engaged, either directly or indirectly, with the advancers such that sufficient movement of the driver results in movement of the advancers, at least one advancer being indirectly engaged with a carrier engagement via an interim driver such that sufficient movement of the advancers results in movement of the carrier engagements either directly via an advancer or indirectly via an interim driver, and further wherein movement of each carrier engagement results in advancement of a medicament carrier to a position wherein a medicament dose portion of the medicament carrier, when present, is in sufficient alignment with the flow director such that separate airflows directed by the flow director entrain at least one medicament dose portion extracted from a present dose storage area.
4. A medicament dispenser for dispensing medicament from a plurality of elongate form medicament carriers held therein, each carrier comprising multiple dose portions of one or more medicaments, said dispenser comprising:
   a. A dispensing mechanism for dispensing the medicament held within the dispenser comprising:
      i) a driver,
      ii) a carrier engagement for each medicament carrier for individually advancing the medicament dose portions thereof,
      iii) an advancer for each carrier engagement, wherein at least one advancer is located co-axially with a carrier engagement for rotating thereof and wherein at least one advancer is located non-coaxially with a carrier engagement for rotating thereof,
      iv) a flow director; and
   b. a medicament access body; and
   c. a cover for the medicament access body,
   wherein the medicament access body cover is engaged with the driver and wherein sufficient movement of the medicament access body cover results in movement of the driver, the driver being movingly engaged, either directly or indirectly, with the advancers such that sufficient movement of the driver results in movement of the advancers, at least one advancer being directly engaged with a carrier engagement and at least one advancer being indirectly engaged with a carrier engagement via an interim driver such that sufficient movement of the advancers results in movement of the carrier engagements either directly via an advancer or indirectly via an interim driver, and further wherein movement of each carrier engagement results in advancement of a medicament carrier to a position wherein a medicament dose portion of the medicament carrier, when present, is in sufficient alignment with the flow director such that an airflow is directed by the flow director to entrain at least one medicament dose portion extracted from a present dose storage area.
5. The dispenser of any one of aspects 1 - 4, wherein the driver is movingly engaged with at least a first advancer and at least one interim driver, the interim driver being movingly engaged with at least a second advancer, such that sufficient movement of the driver directly causes rotational movement of the first advancer and indirectly causes rotational movement of the second advancer via the interim driver such that the first advancer and the second advancer rotate in opposite directions.
6. The dispenser of any one of aspects 1 - 4, wherein the driver is movingly engaged with at least a first and a second advancer, and wherein at least one advancer is movingly engaged directly with a first carrier engagement and at least one advancer is movingly engaged indirectly with a second carrier engagement via an interim driver, such that sufficient movement of the driver causes direct rotational movement of the first and second advancers, yielding indirect movement of the first carrier engagement via the first advancer in one direction, and indirect movement of the second carrier engagement via the interim driver, driven by the second advancer, in the opposite direction.
7. The dispenser of any one of aspects 1 - 4, wherein the driver is movingly engaged with only one advancer such that sufficient movement of the driver causes direct rotational movement of advancer yielding indirect movement of the first carrier engagement via the first advancer.
8. The dispenser of any one of aspects 1 - 4, wherein the driver is movingly engaged with only one advancer such that sufficient movement of the driver causes direct rotational movement of first advancer yielding indirect movement of the first carrier engagement via the first advancer and wherein after completion of all the doses form the blisters of the first carrier, driver engages with the second advancer such that sufficient movement of the driver causes direct rotational movement of second advancer yielding indirect movement of the second carrier engagement.
9. The dispenser of any one of aspects 1 - 6, wherein each advancer is movingly engaged, either directly or indirectly, with a first waste collector, and further either directly or indirectly engaged with a second waste collector, such that sufficient movement of each advancer results in rotational movement of the first waste collector and the second waste collector, and wherein the first and second waste collectors collect different medicament carrier waste material after at least one dose of medicament held within the medicament carrier has been removed.
10. The dispenser of aspect 7, wherein each advancer is movingly engaged, either directly or indirectly, with a first waste collector, and further wherein each advancer is indirectly engaged with a second waste collector via a carrier engagement, such that sufficient movement of each advancer results in rotational movement of the first waste collector and the second waste collector, and wherein the first and second waste collectors collect different medicament carrier waste material after at least one dose of medicament held within the medicament carrier has been removed.
11. A medicament dispenser for dispensing medicament from a plurality of elongate form medicament carriers held therein, each carrier comprising multiple dose portions of one or more medicaments, said dispenser comprising:
   a. A dispensing mechanism for dispensing the medicament held within the dispenser comprising:
      i) a driver,
      ii) a carrier engagement for each medicament carrier for individually advancing the medicament dose portions thereof,
      iii) an advancer for each carrier engagement located either coaxially or non-coaxially with the carrier engagement for rotating thereof,
      iv) a flow director for directing airflow within the dispenser,
      v) at least one first waste collector, and
      vi) at least one second waste collector; and
   b. a medicament access body; and
   c. a cover for the medicament access body,
   wherein the medicament access body cover is engaged with the driver and wherein sufficient movement of the medicament access body cover results in movement of the driver, the driver being movingly engaged, either directly or indirectly, with the advancers such that sufficient movement of the driver results in movement of the advancers, at least one advancer being movingly engaged, either directly or indirectly, with a first waste collector and each advancer being either directly or indirectly engaged with a carrier engagement such that sufficient movement of the advancers results in movement of at least one first waste collector and in movement of the carrier engagements, movement of at least one carrier engagement resulting in movement of at least one second waste collector, the movement of each carrier engagement resulting in advancement of a medicament carrier to a position wherein a medicament dose portion of the medicament carrier, when present, is in sufficient alignment with the flow director such that separate airflows directed by the flow director entrain at least one medicament dose portion extracted from a present dose storage area..
12. The dispenser of aspect 9, wherein the separate airflows directed by the flow director entraining at least one medicament dose portion extracted from a present dose storage area are maintained as separate airflows until the medicament exits the dispenser.
13. The dispenser of any one of aspects 9 - 10, wherein the driver is movingly engaged with at least a first advancer and at least one interim driver, the interim driver being movingly engaged with at least a second advancer, such that sufficient movement of the driver directly causes rotational movement of the first advancer and indirectly causes rotational movement of the second advancer via the interim driver such that the first advancer and the second advancer rotate in opposite directions.
14. The dispenser of any one of aspects 9 - 10, wherein the driver is movingly engaged with at least a first advancer movingly engaged with a first carrier engagement, and a second advancer movingly engaged with an interim driver which is movingly engaged with a second carrier engagement, such that sufficient movement of the driver causes direct rotational movement of the first and second advancers and indirectly causes rotational movement of the carrier engagements, either via an advancer or an interim driver.
15. A medicament dispenser for dispensing medicament from a plurality of elongate form medicament carriers held therein, each carrier comprising multiple dose portions of one or more medicaments, said dispenser comprising:
   a. a dispensing mechanism for dispensing the medicament held within the dispenser comprising:
      i) a driver that movingly engages, either directly or indirectly, with a plurality of advancers,
      ii) an advancer for each carrier engagement for advancing the carrier engagement wherein at least one advancer is directly movingly engaged with the driver and wherein at least a second advancer is acted upon indirectly by movement of the driver via an interim component;
      iii) a carrier engagement for each medicament carrier for individually advancing the medicament dose portions thereof, wherein the carrier is movingly engaged with an advancer and further wherein the advancer is positioned coaxially with the carrier engagement for rotating thereof,
         wherein sufficient movement of the driver results in direct movement of at least a first advancer and indirect movement of at least a second advancer, movement of each advancer resulting in movement of each respective carrier engagement with which it is coaxially positioned, the movement of each carrier engagement resulting in the advancement of a medicament carrier;
      iv) a flow director for receiving and directing airflow, wherein, upon dispenser use, separate airflows entrain at least one medicament dose portion extracted from a dose storage area when present of at least one medicament carrier brought into position by the carrier engagement;
      v) at least one waste collector for collecting medicament carrier material after at least one dose of medicament contained therein has been removed; and
   b. a medicament access body for generating an airflow via user inhalation and for receipt of the medicament by the user as it exits the dispenser; and
   c. a cover for the medicament access body engaged with the driver such that sufficient movement of the medicament access body cover results in movement of the driver.
16. The dispenser of aspect 13, wherein the separate airflows entraining at least one medicament dose portion extracted from a dose storage area when present of at least one medicament carrier brought into position by the carrier engagement are maintained as separate airflows until the medicament exits the dispenser.
17. A medicament dispenser for dispensing medicament from a plurality of elongate form medicament carriers held therein, each carrier comprising multiple dose portions of one or more medicaments, said dispenser comprising:
   a. a dispensing mechanism for dispensing the medicament held within the dispenser comprising:
      i) a driver that movingly engages, either directly or indirectly, with a plurality of advancers,
      ii) an advancer for each carrier engagement for advancing the carrier engagement wherein at least one advancer is directly movingly engaged with the driver;
      iii) a carrier engagement for each medicament carrier for individually advancing the medicament dose portions thereof, wherein at least one carrier engagement is movingly engaged with an advancer and further wherein at least one advancer is positioned non-coaxially with the carrier engagement for rotating thereof,
         wherein sufficient movement of the driver results in movement of at least one advancer, movement of each advancer resulting in movement of each respective carrier engagement with which it is either coaxially or non-coaxially positioned, the movement of each carrier engagement resulting in the advancement of a medicament carrier;
      iv) a flow director for receiving and directing airflow,
         wherein, upon dispenser use, separate airflows entrain at least one medicament dose portion extracted from a dose storage area when present of at least one medicament carrier brought into position by the carrier engagement;
      v) at least one waste collector for collecting medicament carrier material after at least one dose of medicament contained therein has been removed; and
   b. a medicament access body for generating an airflow via user inhalation and for receipt of the medicament by the user as it exits the dispenser; and
   c. a cover for the medicament access body engaged with the driver such that sufficient movement of the medicament access body cover results in movement of the driver.
18. The dispenser of aspect 15, wherein the separate airflows entraining at least one medicament dose portion extracted from a dose storage area when present of at least one medicament carrier brought into position by the carrier engagement are maintained as separate airflows until the medicament exits the dispenser.
19. The dispenser of any one of aspects 13 - 16, wherein the indirect movement of the at least second advancer is via an interim driver movably engaged with at least the driver and the at least second advancer.
20. The dispenser of any one of aspects 13 - 16, wherein at least one carrier engagement is movingly engaged with an advancer indirectly via an interim driver.
21. The dispenser of aspect 17, wherein the interim driver is movably engaged with at least the driver, the at least second advancer, and is further movably engaged, either directly or indirectly, with at least one waste collector.
22. The dispenser of aspect 18, wherein the interim driver is movably engaged with at least one advancer, at least one carrier engagement, and is further movably engaged, either directly or indirectly, to at least one waste collector.
23. The dispenser of any one of aspects 13 - 20, wherein the advancer for each carrier engagement is capable of engaging with the carrier engagement and at least one other movable component.
24. The dispenser of aspect 21, wherein the at least one other movable component is a waste collector.
25. A medicament dispenser for dispensing one or more medicaments from plural elongate form medicament carriers, each carrier comprising multiple dose portions, said dispenser comprising:
   a. a dispensing mechanism comprising:
      i) a driver,
      ii) a carrier engagement for each medicament carrier for individually advancing the medicament dose portions thereof,
      iii) an advancer for each carrier engagement located coaxially with the carrier engagement for rotating thereof,
      iv) a flow director wherein separate airflows entrain medicament dose portions extracted from a dose storage area when present for administration, of a medicament carrier and further wherein the separate airflows containing the entrained medicament are maintained until the medicament exits the dispenser,
      v) a first waste collector; and
      vi) a second waste collector, and
   b. a medicament access body; and
   c. a cover for medicament access body.
26. A medicament dispenser for dispensing one or more medicaments from plural elongate form medicament carriers, each carrier comprising multiple dose portions, said dispenser comprising:
   a. a dispensing mechanism comprising:
      i) a driver,
      ii) a carrier engagement for each medicament carrier for individually advancing the medicament dose portions thereof,
      iii) an advancer for each carrier engagement, at least one of such advancer(s) located non-coaxially with the carrier engagement for rotating thereof,
      iv) a flow director wherein separate airflows entrain medicament dose portions extracted from a dose storage area when present for administration,
      v) a first waste collector; and
      vi) a second waste collector, and
   b. a medicament access body; and
   c. a cover for medicament access body.
27. The dispenser of any one of aspects 23 or 24, wherein the dispenser further comprises an interim driver, such that the driver advances each advancer either directly or indirectly, and each carrier engagement, driven either directly or indirectly by an advancer, is driven by the single driver to rotate in opposite directions.
28. A medicament dispenser for dispensing medicament from a plurality of elongate form medicament carriers held therein, each carrier comprising multiple dose portions of one or more medicaments, said dispenser comprising a dispensing mechanism for dispensing the medicament held within the dispenser, further an advancer for each medicament carrier held therein, each advancer:
   a. being engaged, either directly or indirectly with an actuator, for actuating the dispensation of medicament from the dispenser;
   b. being engaged directly or indirectly with a carrier engagement for each medicament carrier for individually advancing the medicament dose portions thereof; and
   c. being positioned coaxially or non-coaxially with a carrier engagement.
29. The dispenser of aspect 26, wherein each advancer is comprised of a driving member and a driven member driven by the driving member, the driving member in movable engagement with, either directly or indirectly, the driver, such that sufficient movement of the driver causes movement of the driving member of the advancer.
30. The dispenser of aspect 27, wherein the driven member is in movable engagement with a carrier engagement such that sufficient movement of the driven member causes movement of the carrier engagement resulting in the advancement of medicament carrier.
31. The dispenser of aspect 28, wherein advancement of the medicament carrier results in at least one medicament dose portion of a medicament carrier being delivered to a position where it can be extracted from a dose storage area of the medicament carrier and be made available to a user.
32. A medicament dispenser for dispensing medicament from a plurality of elongate form medicament carriers held therein, each carrier comprising multiple dose portions of one or more medicaments, said dispenser comprising a dispensing mechanism for dispensing the medicament held within the dispenser, further comprising at least one waste collector for collecting a portion of a medicament carrier having had a dose of medicament held therein dispensed, each waste collector:
   a. comprising a component capable of creating tension on the waste element being collected by the waste collector;
   b. comprising a component capable of maintaining tension on the waste element being collected by the waste collector;
   c. comprising a component capable of collecting the waste element being collected by the waste collector;
   d. wherein the component (a) and component (b) are positioned within the component (c) and wherein the component (a), component (b), and component (c) share a common axis of rotation.
33. The dispenser of aspect 30, wherein the at least one waste collector is different in function from at least one other waste collector present within the dispenser.
34. The dispenser of aspect 30, wherein the at least one waste collector collects at least one lid sheet of a medicament carrier.
35. The dispenser of any one of aspects 30 - 32, wherein the at least one waste collector is movingly engaged with a carrier engagement, the carrier engagement being movingly engaged with an advancer, either directly or indirectly, the advancer capable of receiving an actuation energy from an actuation component and transferring said actuation energy from the actuation component to the carrier engagement.
36. The dispenser of aspect 33, wherein the advancer receives the actuation energy from the actuation component directly.
37. The dispenser of aspect 33, wherein the advancer receives the actuation energy from the actuation component indirectly.
38. The dispenser of any one of aspects 33 - 35, wherein the carrier engagement is movingly engaged directly with an advancer.
39. The dispenser of any one of aspects 33 - 35, wherein the carrier engagement is movingly engaged indirectly with an advancer via an interim driver.
40. A medicament dispenser for dispensing medicament from a plurality of elongate form medicament carriers held therein, each carrier comprising multiple dose portions of one or more medicaments, said dispenser comprising a dispensing mechanism for dispensing the medicament held within the dispenser, further comprising a flow director, said flow director:
   a. receiving an airflow;
   b. separating the received airflow of (a) into two or more separate airflows each within a separate inlet compartment;
   c. directing each of the separated airflows of (b) such that each separate airflow entrains the medicament held within the dose storage areas, when present, of separate medicament carriers; and
   d. maintaining the separation of each airflow comprising entrained medicament from separate dose storage areas until each airflow exits the dispenser.
41. A medicament dispenser for dispensing medicament from a plurality of elongate form medicament carriers held therein, each carrier comprising multiple dose portions of one or more medicaments, said dispenser comprising a dispensing mechanism for dispensing the medicament held within the dispenser, further comprising a flow director, said flow director:
   a. receiving an airflow;
   b. optionally separating the received airflow into two or more separate airflows each within a separate inlet compartment;
   c. directing each of airflow of (a) such that each airflow entrains the medicament held within separate dose storage areas, when present, of separate medicament carriers; and
   d. directing each airflow comprising entrained medicament to the medicament access body where it exits the dispenser.
42. The dispenser of aspect 39, wherein the separation of each of a plurality of airflows comprising entrained medicament from separate dose storage areas is maintained by maintaining each airflow within separate outlet compartments.
43. The dispenser of any one of aspects 38 - 40, wherein the flow director further comprises a set of airflow guides for each airflow generated to guide each airflow from each inlet compartment to an outlet compartment, wherein the airflow received in an inlet compartment is directed out of the inlet compartment via one airflow guide of the airflow guide set, the airflow then passing to a dose storage area of a medicament carrier such that the airflow entrains the dose of medicament held therein, the airflow comprising entrained medicament then passing through the second airflow guide of the set of airflow guides, and into the outlet compartment where it is optionally maintained separately from other airflows within the flow director until each airflow exits the dispenser.
44. The dispenser of aspect 41, wherein at least one airflow guide of each airflow guide set is capable of breaking apart aggregated medicament, if present, entrained within the airflow passing through the airflow guide.
45. The dispenser of any one of aspects -38 - 42, wherein the airflow is generated by the inhalation of the user.
46. A medicament dispenser for dispensing medicament from a plurality of elongate form medicament carriers held therein, each carrier comprising multiple dose portions of one or more medicaments, said dispenser comprising a dispensing mechanism for dispensing the medicament held within the dispenser, further comprising an advancer according to any one of aspects 27 - 29, and a waste collector according to any one of aspects 31 - 37.
47. A medicament dispenser for dispensing medicament from a plurality of elongate form medicament carriers held therein, each carrier comprising multiple dose portions of one or more medicaments, said dispenser comprising a dispensing mechanism for dispensing the medicament held within the dispenser, further comprising an advancer according to any one of aspects 27 - 29 and a flow director according to any one of aspects 40 - 43.
48. A medicament dispenser for dispensing medicament from a plurality of elongate form medicament carriers held therein, each carrier comprising multiple dose portions of one or more medicaments, said dispenser comprising a dispensing mechanism for dispensing the medicament held within the dispenser, further comprising a waste collector according to any one of aspects - 31 - 37 and a flow director according to any one of aspects 40 - 43.
49. The dispenser of any one of aspects 1 - 46, wherein at least one of the plural elongate form medicament carriers is a blister strip comprising multiple individual dose portions of medicament stored within individual dose storage areas of the carriers.
50. The dispenser of aspect 47, wherein at least two of the plural elongate form medicament carriers are blister strips comprising separate, individual doses of medicament stored within individual dose storage areas of each blister strip.
51. The dispenser of any one of aspects 1 - 48, wherein the medicament is in the form selected from the group consisting of powder, tablet, capsule, pellet or puck, or mixtures thereof.
52. The dispenser according to aspect 49, wherein the medicament is in the form of a powder, and the dispenser is a dry powder inhaler (DPI).
53. The dispenser of any one of aspects 1 - 50, wherein the dose portions of medicament within any single medicament carrier are all the same.
54. The dispenser of any one of aspects 1 - 50, wherein at least one of the individual doses of medicament within any single medicament carrier is different from at least one other separated, individual dose of medicament within the same single medicament carrier.
55. The dispenser of any one of aspects 1 - 52, wherein at least one of the individual doses of medicament within any one medicament carrier is different from at least one of the individual doses of medicament within any second or more medicament carriers.
56. The dispenser of any one of aspects 1 - 53, wherein the spacing between each dose storage area of any single medicament carrier is essentially the same.
57. The dispenser of any one of aspects 1 - 53, wherein the spacing between at least two dose storage areas is different than the spacing between at least two other dose storage areas within a single medicament carrier.
58. The dispenser of any one of aspects 1 - 55, wherein the spacing between any at least two dose storage areas of a first medicament carrier is different from the spacing between any at least two dose storage areas of a second medicament carrier.
59. The dispenser of any one of aspects 1 - 56, wherein the medicament within each of the medicament storage areas of any single medicament carrier are the same.
60. The dispenser of any one of aspects 1 - 56, wherein the medicament within at least one of the medicament storage areas differs from the medicament held within at least one other medicament storage area within the same carrier.
61. The dispenser of any one of aspects 1 - 58, wherein the medicament within at least one of the medicament storage areas of any first carrier varies from the medicament held within at least one of the medicament storage areas of any second carrier.
62. The dispenser of any one of aspects 1 - 59, wherein the medicament comprises one or more active pharmaceutical ingredients and optionally, one or more pharmaceutically acceptable carriers.
63. The dispenser of any one of aspects 1 - 60, wherein the active pharmaceutical ingredient is selected from the group consisting of anti-cholinergics, anti-inflammatories, analgesics, anti-allergenics, antihistamines, antitussives, bronchodilators, anti-infectives, anti-fungals, leukotriene inhibitors, PDE IV inhibitors, anginal preparations, diuretics, or any combination thereof.
64. The dispenser of any one of aspects 1 - 61, wherein the active pharmaceutical ingredient is selected from the group consisting of hormones, cromolyns, xanthines, therapeutic proteins, therapeutic peptides, vaccines, polynucleotides, small molecule organic compounds, diagnostics, or any combinations thereof.
65. The dispenser of any one of aspects 1 - 62, wherein the dispenser is used for dispensing medicament for the treatment of asthma, chronic obstructive pulmonary disease (COPD), bronchitis, lung infections, interstitial pulmonary fibrosis (IPF), pulmonary arterial hypertension (PAH), cystic fibrosis, Non-CF Bronchiectasis, lung transplant, or any combination thereof.
66. The dispenser of any one of aspects 1 - 63, wherein at least one of the plurality of medicament carriers comprises medicament to be administered on a different dosing schedule than that of at least one other of the plurality of medicament carriers.
67. The dispenser of any one of aspects 1 - 64, wherein the medicament access body is a mouthpiece.
68. The dispenser of any one of aspects 1 - 65, wherein the cover for the medicament access body is a mouthpiece cover.
69. The dispenser of any one of aspects 1 - 66, wherein the cover for the medicament access body is mechanically engaged with the driver, the driver being the actuation mechanism of the dispenser.
70. The dispenser of aspect 67, wherein the cover of the medicament access body pivots about, and the driver rotates about, a shared axis.
71. The dispenser of any one of aspects 67 or 68, wherein the cover of the medicament access body is coupled to the driver such that sufficient movement of the cover can result in rotation of the driver.
72. The dispenser according to any one of aspects 1 - 68, wherein the driver is a mutilated driver gear such that not every movement of the driver must result in movement of the the component with which it is engaged.
73. The dispenser of aspect 70, wherein the cover of the medicament access body is a mouthpiece cover and the mouthpiece cover is capable of being rotated to an extent without resulting in rotation of an advancer such that insufficient movement of the mouthpiece cover does not result in rotation of an advancer.
74. The dispenser of any one of aspects -1 - 71, wherein the advancer is arranged to rotate about an axis of rotation different from that of the driver.
75. The dispenser of any one of aspects 1 - 72, wherein at least one advancer is arranged such that it is capable of being movably engaged with the driver.
76. The dispenser according to aspect 73, wherein at least the second advancer is arranged such that it is movably engaged with the driver via an interim driver.
77. The dispenser according to any one of aspects 73 or 74, wherein at least one advancer is constantly in movable engagement with the driver.
78. A medicament dispenser according to any one of aspects 1 - 75, wherein the driver is a mutilated driver gear such that not every movable engagement between the driver and a separate component of the dispenser must result in movement of both the driver and the component with which it is engaged and wherein the advancer is intermittently in a movable engagement with the mutilated driver gear.
79. The medicament dispenser according to any one of aspects 1 - 76, wherein each advancer movably engages with a separate carrier engagement such that movement of each advancer results in rotation of a separate carrier engagement.
80. The medicament dispenser according to any one of aspects 1 - 77, wherein the advancer comprises a plurality of parts contributing to the functionality of the advancer such that the advancer is an assembly of such parts.
81. The medicament dispenser according to aspect 78, wherein the advancer comprises a driving member receiving an actuation energy from the driver, and a driven member receiving energy from the driving component.
82. The medicament dispenser according to aspect 79, wherein the driving member of the advancer is movably engaged with the driven member of the advancer, and the driven member is movably engaged with the carrier engagement such that sufficient movement of the driving member results in movement of the driven member and sufficient movement of the driven member results in movement of the carrier engagement such that the carrier engagement advances a medicament carrier.
83. The dispenser of any one of aspects 1 - 80, wherein the plural carrier engagements begin their rotation at substantially the same time.
84. The dispenser of any one of aspects 1 - 80, wherein the plural carrier engagements do not begin their rotation at substantially the same time.
85. The dispenser of any one of aspects 1 - 82, wherein the plural carrier engagements rotate in a substantially synchronous fashion in at least some rotations.
86. The dispenser of any one of aspects 1 - 82, wherein the plural carrier engagements do not rotate in a substantially synchronous fashion in at least some rotations.
87. The dispenser of any one of aspects 1 - 82, wherein the plural carrier engagements experience substantially no overlap in rotation, such that one carrier engagement substantially completes its rotation prior to a second carrier engagement beginning its rotation.
88. The dispenser of any one of aspects 1 - 85, wherein the plural carrier engagements are decoupled from each other.
89. The dispenser of any one of aspects 81 - 84 or 86, wherein regardless of when the plural carrier engagements begin rotation and/or if the plural carrier engagements are decoupled from each other, the dispensing mechanism makes the medicament within a dose storage area of each medicament carrier available for administration at substantially the same time.
90. The dispenser of aspect 85, wherein the dispensing mechanism makes the medicament within a dose storage area of each medicament carrier available in sequence, one becoming available prior to another being made available.
91. The dispenser of any one of aspects 1 - 88, wherein movement of the cover of the medicament access body results in movement of the driver, movement of a plurality of advancers, and movement of a plurality of carrier engagements, and thereby advances a plurality of medicament carriers to align with the flow director for dispensing the medicament held within a dose storage area if present of each medicament carrier.
92. The dispenser of any one of aspects 1 - 89, wherein the waste of each emptied medicament carrier present within the dispenser is collected separately from the waste of each other medicament carrier present within the dispenser.
93. The dispenser of aspect 90, wherein each medicament carrier is a blister strip, and the emptied blister strip produces two waste streams: a) a lid sheet and b) an empty base sheet, and further wherein each type of waste stream is collected using a separate type of waste collector, each empty base sheet being collected using a first waste collector and each lid sheet being collected using a second waste collector.
94. The dispenser of aspect 91, wherein each second waste collector is movably engaged with a carrier engagement, and each second waste collector comprising multiple components to create an assembly, the assembly comprising:
   a. a component capable of creating tension on the lid sheet;
   b. a lid sheet collector;
   wherein component (a) is positioned within the component (b) and wherein component (a) and component (b) share a common axis of rotation, and further wherein sufficient movement of the carrier engagement results in the movement of the second waste collector.
95. The dispenser of aspect 92, wherein the component capable of creating tension on the lid sheet comprises a lid connection element to hold an end of a lid sheet and optionally a lid gripping element to support tensioning and positioning of the lid sheet, together forming a tensioning assembly.
96. The dispenser of any one of aspects 1 - 93, wherein the medicament carrier is a blister strip comprising a lid sheet and a base sheet, the lid sheet being removably sealed to the base sheet to maintain distinct doses of medicament within medicament storage spaces defined within the base sheet, and wherein an open end of a blister strip comprises a length of lid sheet which extends beyond the length of the base sheet such that the lid sheet is free of attachment to a base sheet and further wherein the lid sheet ends in a sealed loop.
97. The dispenser of aspect 94, wherein the sealed loop of the lid sheet is capable of engaging with the lid connection element to hold the lid sheet in a stable position such that upon rotation of the tensioning assembly, the lid sheet remains engaged with the lid connection element and the tensioning assembly pulls on the lid sheet to create tension on the lid sheet.
98. The dispenser of aspect 95, wherein the tensioning assembly is positioned within the lid sheet collector, the lid sheet collector and the tensioning assembly being movably engaged with one another, the lid sheet collector comprising an opening to allow passage of the lid sheet from the lid sheet connector and over the lid gripping element of the tensioning assembly, if present, and through the opening of the lid collector to the exterior surface of the lid sheet collector.
99. The dispenser of aspect 96, wherein the tensioning assembly first rotates independently of the lid sheet collector, establishing tension in the lid sheet sufficient to cause the engagement between the tensioning assembly and the lid sheet collector to become immovable such that the tensioning assembly and the lid sheet collector subsequently rotate in unison, the lid sheet being removed from the medicament carrier upon each actuation of the dispenser being wound around the lid sheet collector as the lid sheet collector and tensioning assembly rotate.
100. The dispenser of any one of aspects 1 - 97, wherein in use, the flow director splits a single airflow entering the device generated by inhalation of the patient into separate airflows for each medicament carrier within the dispenser, and wherein the airflows remain separate once separated until each deliver an entrained dose of medicament captured by each airflow in its path through the flow director to the user.
101. The dispenser of any one of aspects 1 - 97, wherein in use, the flow director splits a single airflow entering the device generated by inhalation of the patient into separate airflows for each medicament carrier within the dispenser, and wherein the airflows are rejoined after each entrains a dose of medicament such that a single airflow delivers the entrained medicament to the user.
102. The dispenser of aspect 98, wherein the flow director comprises separate inlet compartments for each separate airflow, and wherein the flow director comprises distinct sets of airflow guides for each airflow allowing the airflow to pass from each inlet compartment to a separate outlet channel for each airflow.
103. The dispenser of aspect 99, wherein the flow director comprises separate inlet compartments for each separate airflow, and wherein the flow director comprises airflow guides for each airflow allowing the airflow to pass from each inlet compartment to a single outlet compartment.
104. The dispenser of aspect 100, wherein the airflow guides are designed to allow airflow to pass from the inlet compartment, out of the inlet compartment via the first airflow guide in the set, into the outlet channel via the second airflow guide in the set, the airflow entraining medicament available from a dose storage area of a medicament carrier if present after exiting the inlet compartment via the first airflow guide and entering the outlet channel via the second airflow guide.
105. The dispenser of aspect 102, wherein the airflow guides comprise a design which is capable of disrupting the airflow pattern when passing through the airflow guide to promote de-aggregation of the medicament entrained within the airflow.
106. The dispenser of any one of aspects 1 - 103, wherein the dispenser further comprises an event-related status indicator for providing information about the dose status of medicament or medicaments within the dispenser.
107. The dispenser of aspect 104, wherein the event-related status indicator is in the form of a mechanical device.
108. The dispenser of aspect 104, wherein the event-related status indicator is a non-mechanical (electronic) device.
109. The dispenser of any one of aspects -104 - 106, wherein a status of medicament doses held within the dispenser is visible external to the device via a status identifier window within the housing of the dispenser.
110. The dispenser according to any one of aspects 1 - 107, wherein the advancer and the carrier engagement are non-adjacent to one another.
111. The dispenser according to any one of aspects 1 - 108, wherein the dispenser further comprises a resting surface capable of maintaining the dispenser in a stable position when placed upon a relatively flat surface.
112. The dispenser according to any one of aspects 1 - 109, wherein the dispenser can be operated using a single hand of the user.
113. The dispenser according to any one of aspects 1 - 110, wherein the dispenser further comprises a power source, an electronic circuit, and an electronic measurement instrument.
114. A medicament dispenser capable of independently advancing a plurality of medicament carriers at varying rates and at varying times comprising i) a driver for actuating the dispensing mechanism, ii) a carrier engagement for each medicament carrier for individually advancing the distinct medicament dose portions thereof, iii) an advancer for each carrier engagement located coaxially or non-coaxially with the carrier engagement for rotating thereof, iv) a flow director wherein separate airflows entrain medicament extracted from separate medicament carriers and, optionally, separate airflows containing the entrained medicament are maintained until the medicament reaches the user, v) at least one first waste collector for collecting a first waste stream, vi) at least one second waste collector for collecting at least one second waste stream; b) a medicament access body for user access to the medicaments held within the dispenser; and c) a cover for the medicament access body to protect the medicament access body and which is engaged with the driver such that movement of the medicament access body cover results in movement of the driver, and sufficient movement of the medicament access body cover results in movement of at least one advancer.
115. The method of treating a condition treatable by a medicament administered via inhalation, the method comprising administering one or more medicaments via a medicament dispenser, the administration comprising:
   a. loading a dispenser with a plurality of elongate form medicament carriers comprising a plurality of discrete medicament doses in powder form;
   b. actuating the dispenser such that prior to any advancement of any medicament carrier upon actuation, tension is applied to at least one waste stream of multiple waste streams generated by the emptied medicament carrier;
   c. advancing the plurality of medicament carriers within the dispenser independently from one another to a common opening location in the dispenser such that a medicament dose within a dose storage location, if present, of each medicament carrier becomes available for administration;
   d. the user inhaling the one or more medicament doses available for administration via a medicament access body, the airstream generated by the inhalation separately entraining the medicament of each available medicament dose and optionally being maintained separately from one another until the airstream exits the dispenser and reaches the user via the medicament access body.
116. The method of aspect 113, wherein the medicament access body is a mouthpiece.
117. The method of any one of aspects 113 - 114, wherein the condition is selected from the group comprising asthma, chronic obstructive pulmonary disease (COPD), bronchitis, lung infections, interstitial pulmonary fibrosis (IPF), pulmonary arterial hypertension (PAH), cystic fibrosis, Non-CF Bronchiectasis or lung transplant.
118. A method of administering a plurality of medicaments by inhalation to an individual, the plurality of medicaments being maintained in separate airstreams prior to reaching the orifice of the individual into which the plurality of medicaments is being inhaled, the method comprising administering the plurality of medicaments to the individual via the dispenser according to any one of aspects 1 - 112.
119. A collection system for capturing and storing an elongate media comprising:
   a. a component capable of applying an initial, engaging tension on an end of the media; and
   b. a component capable of collecting the media, the component capable of collecting the media comprising a plurality of movably engageable engagement mechanisms such that one engagement mechanism engages with component (a) and one component engages with a separate component of a larger device or environment in which the collection system is housed,
   wherein the component (a) is positioned within the component (b) and wherein the component (a) and component (b) share a common axis of rotation.
120. The collection system of aspect 117, wherein the elongate media is a media that has been subjected to processing.
121. The collection system of aspect 118, wherein the elongate media is a waste stream of a spent medicament carrier within a medicament dispenser.
122. The collection system of aspect 119, wherein the waste stream is one or more lid sheets of one or more blister sheet medicament carrier(s).
123. The collection system of aspect 117, wherein the component capable of collecting the media is movingly engaged with a component of a larger system of which the collection system is one component, such that actuation of the larger system causes actuation of the collection system.
124. The collection system of any one of aspects 117 - 121, wherein the component capable of applying an initial, engaging tension on the end of the media is a tensioning mechanism comprising an element for attaching to a media and a static element about which the media component can rotate.
125. The collection system of aspect 122, wherein the element for attaching to the media is a post capable of receiving one end of the media.
126. The collection system of any one of aspects 122 - 123, wherein the static element is an axle.
127. The collection system of any one of aspects 117 - 124, wherein the system further comprises a component capable of maintaining tension on the media, applying additional tension to the media, aiding in guiding the media toward the collection component, or any or all thereof.
128. The collection system of aspect 125, wherein the component capable of maintaining tension on the media, applying additional tension to the media, aiding in guiding the media toward the collection component, or any or all thereof is an element of the tensioning mechanism capable of guiding the media from the media attachment component of the tensioning mechanism away from the tensioning mechanism and to a component capable of collecting the media.
129. The collection system of aspect 126, wherein the component capable of maintaining tension on the media is a curved panel having a gripping surface.
130. The collection system of aspect 127, wherein the gripping surface is a serrated surface.
131. The collection system of any one of aspects 117 - 128, wherein the component capable of collecting the media is a collection component comprising a media collection surface for collecting an elongate media, the collection component having at least one media access point which allows the media to pass from an internally positioned tensioning mechanism to the media collection surface.
132. The collection system of aspect 129, wherein the collection component has at least two media access points.
133. The collection system of any one of aspects 117 - 130, wherein the collection component further comprises a locking engagement for preventing the vertical motion of the tensioning mechanism.
134. The collection system of any one of aspects 117 - 131, wherein a first movably engageable engagement mechanism is an inner movably engageable engagement element for engaging with an engagement element of the tensioning mechanism, such an engagement between the two components being such that the tensioning mechanism is rotatable in a single direction relative to the collection component in order to maintain tension on the media.
135. The collection system of any one of aspects 117 - 132, wherein a second movably engageable engagement mechanism is an outer movably engageable engagement element for engaging with one or more other component(s) of a larger system within which the collection system resides.
136. The collection system of aspect 133, wherein the movably engageable driving element for engaging with one or more components of a larger system within which the collection system resides is the driving component of the collection system, such that movement of the component of the larger system with which the collection system is movably engaged causes movement of the collection system.
137. The collection system of aspect 133, wherein the component of a larger system is a carrier engagement of a medicament dispenser.
138. A medicament dispenser for dispensing medicament from a plurality of elongate form medicament carriers held therein, each carrier comprising multiple dose portions of one or more medicaments, said dispenser comprising:
   a. A dispensing mechanism for dispensing the medicament held within the dispenser comprising:
      i) a driver,
      ii) a carrier engagement for each medicament carrier for individually advancing the medicament dose portions thereof,
      iii) an advancer for each carrier engagement,
      iv) a flow director; and
   b. a medicament access body;
   c. a cover for the medicament access body, and
   d. lever
   wherein the lever is engaged with the driver and wherein sufficient movement of the lever results in movement of the driver, the driver being movingly engaged, either directly or indirectly, with the advancers such that sufficient movement of the driver results in movement of the advancers, each advancer being directly engaged with a carrier engagement such that sufficient movement of the advancers results in movement of the carrier engagements, and further wherein movement of each carrier engagement results in advancement of a medicament carrier to a position wherein a medicament dose portion of the medicament carrier, when present, is in sufficient alignment with the flow director such that separate airflows directed by the flow director entrain at least one medicament dose portion extracted from a present dose storage area, and further wherein the separate airflows containing the entrained medicament are maintained until the medicament exits the dispenser.
139. The dispenser according to aspect 138 wherein an advancer for each carrier engagement is located non-coaxially with the carrier engagement for rotating thereof.
140. The according to aspect 138 wherein an advancer for each carrier engagement is located coaxially with the carrier engagement for rotating thereof.

## Claims

1. A medicament dispenser for dispensing medicament from one or more elongate form medicament carriers, each carrier comprising multiple dose portions, said dispenser comprising:
a. a dispensing mechanism comprising:
(i) a driver,
(ii) a carrier engagement for each medicament carrier for individually advancing the medicament dose portions thereof,
(iii) an advancer for each carrier engagement; and
b. a medicament access body; and
c. a medicament access body cover,
wherein the medicament access body cover is movably mounted for sequential movement from a first position, in which the medicament access body is covered, to a second position, in which the medicament access body is covered, to a third position in which the medicament access body is uncovered,
wherein the movement of the medicament access body cover from the first position to the second position results in no actuation of the medicament dispenser and movement of the medicament access body cover from the second position to the third position results in actuation of the medicament dispenser.

2. The dispenser according to claim 1, wherein movement of the medicament access body cover from the first position to the second position is in a range of about 5 degrees to about 25 degrees.

3. The dispenser according to claim 1 or claim 2, wherein movement of the medicament access body cover from the first position to the second position is about 12 degrees.
